(19)

Europäisches
Patentamt
European
Patent Office
Office européen
des brevets

(11) **EP 1 867 734 A1**

(12) **EUROPEAN PATENT APPLICATION**

(43) Date of publication:
**19.12.2007 Bulletin 2007/51**

(51) Int Cl.:
*C12Q 1/68* [(2006.01)]    *G01N 33/68* [(2006.01)]

(21) Application number: **07107064.3**

(22) Date of filing: **23.12.2003**

(84) Designated Contracting States:
**AT BE BG CH CY CZ DE DK EE ES FI FR GB GR HU IE IT LI LU MC NL PT RO SE SI SK TR**

(30) Priority: **24.12.2002 US 436301 P**
**27.12.2002 US 330696**
**20.02.2003 US 371149**
**24.06.2003 US 603891**
**26.09.2003 US 673077**
**14.11.2003 US 714078**

(62) Document number(s) of the earlier application(s) in accordance with Art. 76 EPC:
**03810896.5 / 1 587 955**

(71) Applicant: **Biosite Incorporated**
**San Diego, CA 92121 (US)**

(72) Inventors:
• **Maisel, Alan**
**Del Mar, CA 92014 (US)**

• **Hochstrasser, Denis François**
**1245 Geneva (CH)**
• **Sanchez, Jean-Charles**
**1208 Geneva (CH)**
• **Lescuyer, Pierre**
**74100 Annemasse (FR)**
• **Allard, Laure**
**F-74240, Gaillard (FR)**

(74) Representative: **Viering, Jentschura & Partner**
**Grillparzerstrasse 14**
**81675 München (DE)**

Remarks:
This application was filed on 26.04.2007 as a divisional application to the application mentioned under INID code 62.

(54) **Markers for differential diagnosis and methods of use thereof**

(57)    The invention relates to method of diagnosis of a brain damage-related disorder or the possibility thereof in a subject suspected of suffering therefrom, which comprises detecting one or more polypeptides or variants thereof selected from the group consisting of serum amyloid A, neuromodulin, calcyphosphine, RNA binding regulatory subunit, ubiquitin fusion degradation protein 1 homolog, nucleoside diphosphate kinase A, and cathepsin D in a sample of body fluid taken from the subject. The invention also refers to the use of one or more polypeptides or variants or mutants thereof, selected from the group consisting of serum amyloid A, neuromodulin, calcyphosphine, RNA binding regulatory subunit, ubiquitin fusion degradation protein 1 homolog, nucleoside diphosphate kinase A, and cathepsin D for diagnostic, prognostic, and therapeutic applications relating to brain damage-related disorders and to devices and kits for use in the diagnosis of brain damage-related disorders.

EP 1 867 734 A1

**Description**

**FIELD OF THE INVENTION**

The present invention relates to the identification and use of diagnostic markers for differential diagnosis of diseases and conditions. In a various aspects, the invention relates to methods and compositions able to determine the presence or absence of one, and preferably a plurality, of diseases and/or conditions that exhibit one or more similar or identical symptoms.

**BACKGROUND OF THE INVENTION**

The following discussion of the background of the invention is merely provided to aid the reader in understanding the invention and is not admitted to describe or constitute prior art to the present invention.

The clinical presentation of certain diseases and conditions can often be strikingly similar, even though the underlying diseases, and the appropriate treatments to be given to one suffering from the various diseases, can be completely distinct. For example, subjects may present in an urgent care facility exhibiting a deceptively simple constellation of apparent symptoms (e.g., fever, shortness of breath, dizziness, headache) that may be characteristic of a variety of unrelated conditions. Differential diagnosis methods involve the comparison of symptoms and/or diagnostic test results known to be associated with one or more diseases that exhibit a similar clinical presentation to the symptoms and/or diagnostic results exhibited by the subject, in order to identify the underlying disease or condition present in the subject.

Taking shortness of breath (referred to clinically as "dyspnea") as an example, patients often present in a clinical setting with this symptom as the initial clinical presentation. This symptom considered in isolation may be indicative of conditions as diverse as asthma, chronic obstructive pulmonary disease ("COPD"), tracheal stenosis, obstructive endobroncheal tumor, pulmonary fibrosis, pneumoconiosis, lymphangitic carcinomatosis, kyphoscoliosis, pleural effusion, amyotrophic lateral sclerosis, congestive heart failure, coronary artery disease, myocardial infarction, cardiomyopathy, valvular dysfunction, left ventricle hypertrophy, pericarditis, arrhythmia, pulmonary embolism, metabolic acidosis, chronic bronchitis, pneumonia, anxiety, sepsis, acute coronary syndrome, aneurismic dissection, *etc. See, e.g.,* Kelley's Textbook of Internal Medicine, 4th Ed., Lippincott Williams & Wilkins, Philadelphia, PA, 2000, pp. 2349-2354, "Approach to the Patient With Dyspnea"; Mulrow et al., J. Gen. Int. Med. 8: 383-92 (1993).

Differential diagnosis in the case of dyspnea involves identifying the particular condition causing shortness of breath in a given subject from amongst numerous possible causes. These methods often require that the clinician integrate information obtained from a battery of tests, leading to a clinical diagnosis that most closely represents the range of symptoms and/or diagnostic test results obtained for the subject. The tests required may include radiography, electrocardiogram, exercise treadmill testing, blood chemistry analysis, echocardiography, bronchoprovocation testing, spirometry, pulse oximetry, esophageal pH monitoring, laryngoscopy, computed tomography, histology, cytology, magnetic resonance imaging, *etc. See, e.g.,* Morgan and Hodge, Am. Fam. Physician 57: 711-16 (1998). Because of the variety of tests that may need to be performed, obtaining sufficient information to arrive at a diagnosis can take hours or even days.

Differential diagnosis of chest pain requires the clinician to consider many possible causes, including differentiating between respiratory pain and pain associated with angina, or myocardial infarction and pleuritic and chest wall pain.

Differential diagnosis of diastolic and systolic dysfunction in patients suffering from heart failure is important since the therapies for each dysfunction are different. Further differentiation of atrial fibrillation from heart failure is critical for appropriate therapy.

In the area of infection, differential diagnosis of viral versus bacterial is critical to the clinician delivering the appropriate therapy.

The acuteness or severity of the symptoms often dictates how rapidly a diagnosis must be established and treatment initiated. Immediate diagnosis and care of a patient experiencing a variety of acute conditions associated with dyspnea and chest pain can be critical. *See, e.g.,* Harris, Aust. Fam. Physician 31: 802-06 (2002) (asthma); Goldhaber, Eur. Respir. J Suppl. 35: 22s-27s (2002) (pulmonary embolism); Lundergan et al., Am. Heart J. 44: 456-62 (2002) (myocardial infarction). However, even in cases where the apparent symptoms appear relatively stable, rapid diagnosis, and the rapid initiation of treatment, can provide both relief from immediate discomfort and advantageous improvement in prognosis.

Each reference cited in the preceding section is hereby incorporated by reference in its entirety, including all tables, figures, and claims.

**SUMMARY OF THE INVENTION**

The present invention relates to the identification and use of diagnostic markers for differential diagnosis of diseases and conditions and prediction of clinical outcomes. The methods and compositions described herein can meet the need in the art for rapid, sensitive and specific diagnostic and prognostic assays to be used in the diagnosis and differentiation of various diseases that are related in terms of one or more clinical characteristics.

In various aspects, the invention relates to materials and procedures for identifying the underlying cause of one or more symptoms that, when considered in isolation, may be related to a plurality of possible underlying diseases or conditions; to using such markers in diagnosing and treating a patient and/or to monitor the course of a treatment regimen; to using such markers to identify subjects at risk for one or more adverse outcomes an underlying disease or condition; and for screening compounds and pharmaceutical compositions that might provide a benefit in treating or preventing such diseases or conditions.

In traditional methods to evaluate marker levels in the diagnosis or prognosis of disease, a "threshold" for a marker of interest is typically established, and the concentration of that marker in a sample is compared to that threshold amount; an amount greater than the pre-established threshold is indicative of one state (e.g., disease), and an amount less than the pre-established threshold is indicative of another state (e,g., normal). For example, the American Heart Association has stated that a cardiac troponin I concentration greater that the 99th percentile concentration in the normal population should be used to rule in myocardial infarction. In the methods described herein, such threshold concentrations may be established for one or more markers, and these thresholds used for determining the diagnosis/prognosis of a subject in a similar fashion. As the number of markers in a panel increase, however, applying individual thresholds to each marker can become unwieldy. Thus, in certain preferred embodiments in which a plurality of markers are evaluated, particular thresholds for one or more markers in the marker panel are not relied upon to determine a particular diagnosis and/or prognosis. Rather, the present invention may utilize an evaluation of the plurality of markers as a unitary whole. In a simple example, the ratio of two or more markers, rather than an absolute amount of the markers, may be used to determine a diagnosis/prognosis. Even more preferably, however, a particular "fingerprint" pattern of changes in such a panel of markers may, in effect, act as a specific diagnostic or prognostic indicator. Methods for determining a "panel response value" that integrates a plurality of marker concentrations into a single result are described hereinafter. In these methods, each marker concentration measured in a sample contributes to this panel response value, which may be compared to a threshold panel response as if it were simply the concentration of a single marker. This is an example of a diagnostic method wherein the amount of one or more the markers is not compared to a predetermined threshold level.

In a first aspect, the invention discloses methods for determining the presence or absence of a disease or condition (a "diagnosis") in a subject that is exhibiting a perceptible change in one or more physical characteristics (that is, one or more "symptoms") that are indicative of a plurality of possible etiologies underlying the observed symptom(s). These methods comprise analyzing a test sample obtained from the subject for the presence or amount of one or more markers for one or more of the possible etiologies of the observed symptom(s). The presence or amount of such marker (s) in a sample obtained from the subject can be used to rule in or rule out one or more of the possible etiologies, thereby either providing a diagnosis (rule-in) and/or excluding one or more diagnoses (rule-out).

In certain embodiments, these markers can be used to rule in or rule out one or more possible etiologies of shortness of breath, or "dyspnea." While the present invention is described hereinafter generally in terms of the differential diagnosis of diseases and conditions related to dyspnea, the skilled artisan will understand that the concepts of symptom-based differential diagnosis described herein are generally applicable to any physical characteristics that are indicative of a plurality of possible etiologies such as fever, chest pain (or "angina"), abdominal pain, neurologic dysfunction, disturbances in metabolic state, such as aberrant water, electrolyte, mineral, or acid-base metabolism, hypertension, dizziness, headache, *etc*.

In preferred embodiments, the present invention relates to methods in which a test sample is analyzed for the presence or amount of a plurality of markers related to a plurality of possible etiologies, so that the method is adapted to rule in or out a plurality of possible underlying causes based upon the analysis of a single sample.

In the case of dyspnea, the plurality of markers are preferably selected to rule in or out one or more, and preferably a plurality, of the following diagnoses: asthma, atrial fibrillation, chronic obstructive pulmonary disease ("COPD"), tracheal stenosis, obstructive endobroncheal tumor, pulmonary fibrosis, pneumoconiosis, lymphangitic carcinomatosis, kyphoscoliosis, pleural effusion, amyotrophic lateral sclerosis, congestive heart failure, coronary artery disease, myocardial infarction, acute coronary syndrome, cardiomyopathy, valvular dysfunction, left ventricle hypertrophy, pericarditis, arrhythmia, pulmonary embolism, metabolic acidosis, chronic bronchitis, pneumonia, anxiety, sepsis, or aneurismic dissection. In a particularly preferred embodiment, the methods relate to defining the cause of dyspnea to rule in or rule out myocardial ischemia and cardiac necrosis, heart failure and pulmonary embolism. In yet another particularly preferred embodiment, the methods relate to defining the cause of dyspnea to rule in or rule out myocardial ischemia and cardiac necrosis, heart failure, pulmonary embolism and atrial fibrillation. The plurality of markers may also be used for prediction of risk that a subject may suffer from a future clinical outcome such as death or one or more nonfatal complications such as might require rehospitalization. The skilled artisan will understand that the same plurality of markers may provide both diagnostic and prognostic information. The markers used for diagnosis may be the same as those used for prognosis, or may differ in that one or more markers used for one of these purposes may not be used for the other purpose.

In the case of abdominal pain, the plurality of markers are preferably selected to rule in or out a plurality of the following: aortic aneurysm, mesenteric embolism, pancreatitis, appendicitis, myocardial infarction, one or more infectious diseases described above, influenza, esophageal carcinoma, gastric adenocarcinoma, colorectal adenocarcinoma, pancreatic tumors including ductal adenocarcinoma, cystadenocarcinoma, and insulinoma.

In the case of disturbances of metabolic state, the plurality of markers are preferably selected to rule in or out a plurality of the following: diabetes mellitus, diabetic ketoacidosis, alcoholic ketoacidosis, respiratory acidosis, respiratory alkalosis, nonketogenic hyperglycemia, hypoglycemia, renal failure, interstitial renal disease, COPD, pneumonia, pulmonary and edema, asthma.

As described in detail herein, a plurality of markers are used as part of panels as described hereinafter to associate diagnosis and/or prognosis to the subject. Such panels may comprise 2, 3, 4, 5, 6, 7, 8, 9, 10, 15, 20, or more or individual markers. Preferred panels for the diagnosis of a cause of dyspnea comprise a plurality of markers independently selected from the group consisting of specific markers of cardiac injury, specific markers of neural tissue injury, non-specific markers of tissue injury, markers related to blood pressure regulation, markers related to inflammation, markers related to coagulation and hemostasis, markers related to pulmonary injury, and markers related to apoptosis. Exemplary markers in each of these groups are described hereinafter. Preferably, such a panel comprises markers from two, three, four, five, or more different members of this group. Thus, particularly preferred panels for the diagnosis of a cause of dyspnea comprise one or more specific markers of cardiac injury and one or more markers related to blood pressure regulation; one or more specific markers of cardiac injury and one or more markers related to coagulation and hemostasis; one or more markers related to blood pressure regulation and one or more markers related to coagulation and hemostasis; or one or more specific markers of cardiac injury, one or more markers related to blood pressure regulation, and one or more markers related to coagulation and hemostasis, where each of these particularly preferred panels may optionally comprise one or more non-specific markers of tissue injury, markers related to inflammation, markers related to pulmonary injury, and/or markers related to apoptosis. One or more markers may lack diagnostic or prognostic value when considered alone, but when used as part of a panel, such markers may be of great value in determining a particular diagnosis and/or prognosis.

Preferred specific markers of cardiac injury for use in the methods described herein comprise, for example, annexin V, β-enolase, cardiac troponin I (free and/or complexed), cardiac troponin T (free and/or complexed), creatine kinase-MB, glycogen phosphorylase-BB, heart-type fatty acid binding protein, phosphoglyceric acid mutase-MB, and S-100ao. Preferred non-specific markers of tissue injury for use in the methods described herein comprise, for example, aspartate aminotransferase, myoglobin, actin, myosin, and lactate dehydrogenase.

Preferred marker(s) related to blood pressure regulation for use in the methods described herein comprise, for example, one or more marker(s) selected from the group consisting of atrial natriuretic peptide ("ANP"), pro-ANP, B-type natriuretic peptide ("BNP"), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, and urodilatin, or markers related thereto.

Preferred marker(s) markers related to inflammation for use in the methods described herein comprise, for example, one or more marker(s) selected from the group consisting of acute phase reactants, cell adhesion molecules such as vascular cell adhesion molecule ("VCAM"), intercellular adhesion molecule-1 ("ICAM-1"), intercellular adhesion molecule-2 ("ICAM-2"), and intercellular adhesion molecule-3 ("ICAM-3"), myeloperoxidase (MPO), C-reactive protein, interleukins such as IL-1β, IL-6, and IL-8, interleukin-1 receptor agonist, monocyte chemoattractant protein-1, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), and vascular endothelial growth factor ("VEGF"), or markers related thereto. The term "acute phase reactants" as used herein refers to proteins whose concentrations are elevated in response to stressful or inflammatory states that occur during various insults that include infection, injury, surgery, trauma, tissue necrosis, and the like. Acute phase reactant expression and serum concentration elevations are not specific for the type of insult, but rather as a part of the homeostatic response to the insult.

In addition to those acute phase reactants listed above as "markers related to inflammation," one or more markers related to inflammation may also be selected from the group of acute phase reactants consisting of hepcidin, HSP-60, HSP-65, HSP-70, asymmetric dimethylarginine (an endogenous inhibitor of nitric oxide synthase), matrix metalloproteins 11,3, and 9, defensin HBD 1, defensin HBD 2, serum amyloid A, oxidized LDL, insulin like growth factor, transforming growth factor β, e-selectin, glutathione-S-transferase, hypoxia-inducible factor-1α, inducible nitric oxide synthase ("I-NOS"), intracellular adhesion molecule, lactate dehydrogenase, monocyte chemoattractant peptide-1 ("MCP-1"), n-acetyl aspartate, prostaglandin E2, receptor activator of nuclear factor ("RANK") ligand, TNF receptor superfamily member 1A, lipopolysaccharide binding protein ("LBP"), and cystatin C, or markers related thereto.

Preferred marker(s) related to coagulation and hemostasis for use in the methods described herein comprise, for example, one or more marker(s) selected from the group consisting of plasmin, fibrinogen, thrombus precursor protein, D-dimer, β-thromboglobulin, platelet factor 4, fibrinopeptide A, platelet-derived growth factor, prothrombin fragment 1+2, plasmin-α2-antiplasmin complex, thrombin-antithrombin III complex, P-selectin, thrombin, and von Willebrand factor, tissue factor, or markers related thereto.

Preferred markers related to pulmonary injury for use in the methods described herein comprise, for example, one or more marker(s) selected from the group consisting of neutrophil elastase, 7s collagen fragment, pulmonary surfactant protein(s), dipalmitoylphosphatidyl choline, KL-6, and ubiquitin-conjugated lung proteins, or markers related thereto.

Preferred marker(s) related to apoptosis for use in the methods described herein comprise, for example, one or more marker(s) selected from the group consisting of spectrin, cathepsin D, caspase 3, cytochrome c, s-acetyl glutathione, and ubiquitin fusion degradation protein 1 homolog.

As described in detail hereinafter, the methods and compositions of the present invention can be selected to subdivide congestive heart failure by distinguishing between systolic heart failure and diastolic heart failure by analyzing a test sample obtained from the subject for the presence or amount of one or more markers, the presence or amount of which can be used to rule in or out systolic heart failure and/or diastolic heart failure, or that can be used to distinguish between these two causes of congestive heart failure. Similarly, the methods and compositions herein may distinguish between atrial fibrillation and heart failure by analyzing a test sample obtained from the subject for the presence or amount of one or more markers, the presence or amount of which can be used to rule in or out heart failure or atrial fibrillation. Likewise, the methods can be used to distinguish between systolic and diastolic dysfunction and atrial fibrillation and/or to distinguish between systolic and diastolic dysfunction, atrial fibrillation, myocardial ischemia and cardiac necrosis.

For example, the differential diagnosis of various diseases underlying dyspnea may require discrimination between heart failure and atrial fibrillation. A preferred marker panel for performing such discrimination preferably includes a plurality of markers related to blood pressure regulation, preferably BNP or BNP related peptides, and ANP or ANP related peptides. Additional markers may be added to such a panel to distinguish between systolic and diastolic dysfunction and atrial fibrillation. A preferred marker panel for performing such discrimination preferably includes a plurality of markers related to blood pressure regulation. Most preferably, such a panel comprises BNP, calcitonin gene related peptide, calcitonin, urotensin 1, and ANP, or related peptides. Likewise, markers may be defined to distinguish between systolic and diastolic dysfunction, atrial fibrillation, myocardial ischemia and cardiac necrosis. Preferred marker panels in this case comprise a plurality of markers related to blood pressure regulation, one or more markers of cardiac necrosis, and optionally one or more non-specific markers of tissue damage. Most preferably, such a panel comprises BNP, calcitonin gene related peptide, calcitonin, urotensin 1, ANP, and cardiac troponin I or T (free and/or complexed), or related peptides, and optionally myoglobin, creatine kinase-MB and/or S100ao, or related peptides.

Moreover, one or more markers of coagulation and hemostasis, most preferably D-dimer and/or thrombus precursor protein or related peptides, may be added to assist such panels in ruling in or out pulmonary embolism. Similarly, one or more markers of vascular tissue injury, preferably smooth muscle myosin, and most preferably smooth muscle myosin heavy chain or related peptides, may be added to such panels assist such panels in ruling in or out aortic dissection. Finally, one or more markers related to inflammation, preferably IL-1ra, myeloperoxidase, MMP-9, and/or C-reactive protein may also provide additional information to such panels for the further discrimination of disease.

Particularly preferred markers for distinguishing causes of dyspnea include two or more markers selected from the group consisting of specific markers of cardiac injury, non-specific markers of tissue injury, markers related to inflammation, markers related to blood pressure regulation, and markers related to coagulation and hemostasis. Most preferred are panels comprising 2, 3, 4, 5, 6, 7, 8, or more such markers, which are most preferably selected from the group consisting of cardiac-specific troponin I (free and/or complexed), cardiac-specific troponin T (free and/or complexed), creatine kinase-MB, S100ao, A-type natriuretic peptide, B-type natriuretic peptide, calcitonin gene related peptide, calcitonin, urotensin 1, myoglobin, smooth muscle myosin light chain, thrombus precursor protein, D-dimer, smooth muscle myosin heavy chain, IL-1ra, myeloperoxidase, caspase-3, cytochrome C, C-reactive protein, monocyte chemoattractant peptide-1, and MMP-9, or markers related thereto. One or more markers may be replaced, added, or subtracted from this list of particularly preferred markers while still providing clinically useful results.

These markers may be combined in various combinations. For example, preferred panels may comprise 2, 3, 4, 5, or more of the following markers: B-type natriuretic peptide or a marker related to B-type natriuretic peptide, creatine kinase-MB, total cardiac troponin I, total cardiac troponin T, C-reactive protein, D-dimer, and myoglobin.

Particularly preferred panels comprise creatine kinase-MB, total cardiac troponin I, myoglobin, and B-type natriuretic peptide or a marker related to B-type natriuretic peptide; total cardiac troponin I, C-reactive protein, and B-type natriuretic peptide or a marker related to B-type natriuretic peptide; creatine kinase-MB, total cardiac troponin I, myoglobin, C-reactive protein, and B-type natriuretic peptide or a marker related to B-type natriuretic peptide; myoglobin, C-reactive protein, and B-type natriuretic peptide or a marker related to B-type natriuretic peptide; creatine kinase-MB, total cardiac troponin I, and myoglobin; or creatine kinase-MB, total cardiac troponin 1, C-reactive protein and myoglobin. These particularly preferred panels may further comprise D-dimer and/or myeloperoxidase; and D-dimer and/or myeloperoxidase may be used to replace one or two of the markers in these particularly preferred panels.

Such panels may diagnose one or more, and preferably distinguish between a plurality of, cardiovascular disorders selected from the group consisting of myocardial infarction, congestive heart failure, acute coronary syndrome, ST elevated ACS, non-ST elevated ACS, unstable angina, and/or pulmonary embolism; and/or predict risk that a subject may suffer from a future clinical outcome such as death, nonfatal myocardial infarction, recurrent ischemia requiring urgent revascularization, and/or recurrent ischemia requiring rehospitalization; and/or predict a risk of a future outcome in such diseases. Marker(s) able to differentiate congestive heart failure from diseases or conditions that present similar symptoms, but that are not congestive heart failure ("CHF mimics"), are referred to herein as "CHF differential diagnostic markers;" marker(s) able to differentiate myocardial infarction from diseases or conditions that present similar symptoms, but that are not myocardial infarction ("MI mimics"), are referred to herein as "MI differential diagnostic markers."

In similar fashion, a panel may comprise a plurality of markers selected to diagnose and/or distinguish amongst a plurality of cerebrovascular disorders. In preferred embodiments, the invention discloses methods for determining a diagnosis or prognosis related to stroke, or for differentiating between types of strokes and/or TIA. These methods comprise analyzing a test sample obtained from a subject for the presence or amount of one or more markers for cerebral injury. These methods can comprise identifying one or more markers, the presence or amount of which is associated with the diagnosis, prognosis, or differentiation of stroke and/or TIA. Once such marker(s) are identified, the level of such marker(s) in a sample obtained from a subject of interest can be measured. In certain embodiments, these markers can be compared to a level that is associated with the diagnosis, prognosis, or differentiation of stroke and/or TIA. By correlating the subject's marker level(s) to the diagnostic marker level(s), the presence or absence of stroke, the probability of future adverse outcomes, etc., in a patient may be rapidly and accurately determined.

The invention also discloses methods for determining the presence or absence of a disease or condition in a subject that is exhibiting a perceptible change in one or more symptoms that are indicative of a plurality of possible etiologies underlying the observed symptom(s), one of which is stroke. These methods comprise analyzing a test sample obtained from the subject for the presence or amount of one or more markers selected to rule in or out stroke, or one or more types of stroke, as a possible etiology of the observed symptom(s). Etiologies other than stroke that are within the differential diagnosis of the symptom(s) observed are referred to herein as "stroke mimics", and marker(s) able to differentiate one or more types of stroke from stroke mimics are referred to herein as "stroke differential diagnostic markers". The presence or amount of such marker(s) in a sample obtained from the subject can be used to rule in or rule out one or more of the following: stroke, thrombotic stroke, embolic stroke, lacunar stroke, hypoperfusion, subclinical cerebral ischemia, intracerebral hemorrhage, and subarachnoid hemorrhage, thereby either providing a diagnosis (rule-in) and/or excluding a diagnosis (rule-out).

In these aspects related to cerebrovascular disease, preferred marker panels comprise a plurality of markers independently selected from the group consisting of specific markers of neural tissue injury, markers related to blood pressure regulation, markers related to coagulation and hemostasis, markers related to inflammation, and markers related to apoptosis. Preferably, such a panel comprises markerd from two, three, four, or five different members of this group.

Exemplary markers related to blood pressure regulation, to inflammation, and to coagulation and hemostasis are described above. One or more markers related to neural tissue injury include those selected from the group consisting of precerebellin 1, cerebillin 1, cerebellin 3, chimerin 1, chimerin 2, calbrain, calbindin D, brain tubulin, brain fatty acid binding protein ("'B-FABP"), brain derived neurotrophic factor ("BDNF"), carbonic anhydrase XI, CACNA1A calcium channel gene, nerve growth factor β, atrophin 1, apolipoprotein E4-1, protein 4.1B, 14-3-3 protein, ciliary neurotrophic factor, creatine kinase-BB, C-tau, glial fibrillary acidic protein ("GFAP"), neural cell adhesion molecule ("NCAM"), neuron specific enolase, S-100β, prostaglandin D synthase, neurokinin A, neurotensin, and secretagogin. Additional exemplary markers related to neural tissue injury are described hereinafter.

Preferred marker panels selected to diagnose and/or distinguish amongst a plurality of cerebrovascular disorders comprise a plurality of markers selected from the group consisting of adenylate kinase, brain-derived neurotrophic factor, calbindin-D, creatine kinase-BB, glial fibrillary acidic protein, lactate dehydrogenase, myelin basic protein, neural cell adhesion molecule (NCAM), c-tau, neuropeptide Y, neuron-specific enolase, neurotrophin-3, proteolipid protein, S-100β, thrombomodulin, protein kinase Cγ, atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, urodilatin, acute phase reactants, MMP-9, cell adhesion molecules, C-reactive protein, interleukins, interleukin- receptor agonist, monocyte chemotactic protein-1, caspase-3, lipocalin-type prostaglandin D synthase, mast cell tryptase, eosinophil cationic protein, KL-6, haptoglobin, tumor necrosis factor α, tumor necrosis factor β, Fas ligand, soluble Fas (Apo-1), TRAIL, TWEAK, fibronectin, macrophage migration inhibitory factor (MIF), vascular endothelial growth factor (VEGF), caspase-3, cathepsin D, α-spectrin, plasmin, fibrinogen, D-dimer, β-thromboglobulin, platelet factor 4, fibrinopeptide A, platelet-derived growth factor, prothrombin fragment 1+2, plasmin-α2-antiplasmin complex, thrombin-antithrombin III complex, P-selectin, thrombin, von Willebrand factor, tissue factor, and thrombus precursor protein, or markers related thereto.

Most preferred marker panels comprise at least one marker related to neural tissue injury and at least one marker of inflammation, and preferably comprise 2, 3, 4, 5, 6, 7, 8, or more markers selected from the group consisting of IL-1ra, C-reactive protein, von Willebrand factor (vWF), creatine kinase-BB, creatine kinase-MB, c-Tau, D-dimer, thrombus precursor protein, vascular endothelial growth factor (VEGF), matrix metalloprotease-9 (MMP-9), neural cell adhesion molecule (NCAM), BNP, S100β, cytochrome c, and caspase-3.

Preferred markers of the invention can differentiate between ischemic stroke, hemorrhagic stroke, and TIA. Such markers are referred to herein as "stroke differentiating markers". Particularly preferred are markers that differentiate between thrombotic, embolic, lacunar, hypoperfusion, intracerebral hemorrhage, and subarachnoid hemorrhage types of strokes. For purposes of the following discussion, the methods described as applicable to the diagnosis and prognosis of stroke generally may be considered applicable to the diagnosis and prognosis of TIAs.

Still other preferred markers of the invention can identify those subjects suffering from stroke at risk for a subsequent adverse outcome. For example, a subset of subjects presenting with intracerebral hemorrhage or subarachnoid hemorrhage types of strokes may be susceptible to later vascular injury caused by cerebral vasospasm. In another example, a clinically normal subject may be screened in order to identify a risk of an adverse outcome. Preferred markers include caspase-3, NCAM, MCP-1, S100b, MMP-9, vWF, BNP, CRP, NT-3, VEGF, CKBB, MCP-1 Calbindin; thrombin-antithrombin III complex, IL-6, IL-8, myelin basic protein, tissue factor, GFAP, and CNP. Each of these terms is defined hereinafter. Particularly preferred markers are those predictive of a subsequent cerebral vasospasm in patients presenting with subarachnoid hemorrhage, such as von Willebrand factor, vascular endothelial growth factor, matrix metalloprotein-9, or combinations of these markers. Other particularly preferred markers are those that distinguish ischemic stroke from hemorrhagic stroke.

Still other particularly preferred markers are those predictive of a subsequent cerebral vasospasm in patients presenting with subarachnoid hemorrhage, such as one or more markers related to blood pressure regulation, markers related to inflammation, markers related to apoptosis, and/or specific markers of neural tissue injury. Again, such panels may comprise 2, 3, 4, 5, 6, 7, 8, 9, 10, 15, 20, or more or individual markers. Preferred marker(s) for use individually or in panels may be selected from the group consisting of IL-1ra, C-reactive protein, von Willebrand factor (vWF), vascular endothelial growth factor (VEGF), matrix metalloprotease-9 (MMP-9), neural cell adhesion molecule (NCAM), BNP, and caspase-3, or markers related thereto.

Obtaining information on the true time of onset can be critical, as early treatments have been reported to be critical for proper treatment. Obtaining this time-of-onset information can be difficult, and is often based upon interviews with companions of the stroke victim. Thus, in various embodiments, markers and marker panels are selected to distinguish the approximate time since stroke onset. For purposes of the present invention, the term "acute stroke" refers to a stroke that has occurred within the prior 12 hours, more preferably within the prior 6 hours, and most preferably within the prior 3 hours; while the term "non-acute stroke" refers to a stroke that has occurred more than 12 hours ago, preferably between 12 and 48 hours ago, and most preferably between 12 and 24 hours ago. Preferred markers for differentiating between acute and non-acute strokes, referred to herein as stroke "time of onset markers" are described hereinafter.

Preferred panels comprise markers for the following purposes: diagnosis of stroke; diagnosis of stroke and indication if an acute stroke has occurred; diagnosis of stroke and indication if an non-acute stroke has occurred; diagnosis of stroke, indication if an acute stroke has occurred, and indication if an non-acute stroke has occurred; diagnosis of stroke and indication if an ischemic stroke has occurred; diagnosis of stroke and indication if a hemorrhagic stroke has occurred; diagnosis of stroke, indication if an ischemic stroke has occurred, and indication if a hemorrhagic stroke has occurred; diagnosis of stroke and prognosis of a subsequent adverse outcome; diagnosis of stroke and prognosis of a subsequent cerebral vasospasm; and diagnosis of stroke, indication if a hemorrhagic stroke has occurred, and prognosis of a subsequent cerebral vasospasm.

As noted above, panels may also comprise differential diagnosis of stroke; differential diagnosis of stroke and indication if an acute stroke has occurred; differential diagnosis of stroke and indication if an non-acute stroke has occurred; differential diagnosis of stroke, indication if an acute stroke has occurred, and indication if an non-acute stroke has occurred; differential diagnosis of stroke and indication if an ischemic stroke has occurred; differential diagnosis of stroke and indication if a hemorrhagic stroke has occurred; differential diagnosis of stroke, indication if an ischemic stroke has occurred, and indication if a hemorrhagic stroke has occurred; differential diagnosis of stroke and prognosis of a subsequent adverse outcome; differential diagnosis of stroke and prognosis of a subsequent cerebral vasospasm; differential diagnosis of stroke, indication if a hemorrhagic stroke has occurred, and prognosis of a subsequent cerebral vasospasm.

The presence or amount of the markers in such panels may be correlated to the presence or absence of a plurality of cerebrovascular disorders. Additional markers are described hereinafter. As described hereinafter, the markers described herein may be indicative of a plurality of diseases, depending on the status of other markers in a panel. For example, certain markers are generally elevated in inflammation resulting from a variety of causes. Thus, alone, a single marker may not be diagnostic *per se,* but as part of a panel, the marker can provide important diagnostic and/or prognostic information.

In a related aspect, the presence or amount of markers that are selected to diagnose and/or distinguish amongst a plurality of cerebrovascular disorders may also be used prognostically, in order to identify patients at risk for a future onset of a cerebrovascular disorder. Such uses may find particular interest in monitoring patients known to be at increased risk for such onset. For example, patients undergoing carotid endarterectomy are known to be at risk for cerebral ischemia. Outcomes of such ischemia include intraoperative and perioperative stroke, neurologic deficit, and death. Cerebral ischemia is also a risk of procedures such as hypothermic circulatory arrest, aortic valve replacement, mitral valve replacement, coronary artery surgery, endograft repair of aortic aneurism, coronary artery bypass graft surgery, laryngeal mask insertion, and repair of congenital heart defects. Thus, the present invention also relates to methods and compositions for monitoring the status of patients undergoing such procedures to identify at-risk patients.

In yet another aspect, the present invention describes thrombus precursor protein ("TpP™"), and monocyte chemoattractant protein-1 (MCP-1) as representing independent markers for use in risk stratification and diagnosis of patients suffering from vascular diseases. In the case of ACS for example, TpP™ and/or MCP-1 may permit a determination of risk that a subject may suffer from a future clinical outcome such as death, nonfatal myocardial infarction, recurrent ischemia requiring urgent revascularization, and/or recurrent ischemia requiring rehospitalization. The time horizon over which risk stratification may be applied (that is, the period for which prognostic risk may be predicted) may be from 1 day to 5 years, more preferably from 1 week to 2 years, and most preferably from 1 month to 1 year. While described hereinafter with regard to acute coronary syndrom ("ACS") patients, TpP™ and MCP-1 may also be used in various aspects according to the methods described herein to provide diagnostic and prognostic information in a variety of vascular diseases in which coagulation and hemostasis and/or inflammation are implicated.

Preferred diseases to which the various aspects described herein may be applied include one or more diseases selected from the group consisting of sepsis, acute coronary syndrome, atherosclerosis, ischemic stroke, intracerebral hemorrhage, subarachnoid hemorrhage, transient ischemic attack, systolic dysfunction, diastolic dysfunction, aneurysm, aortic dissection, myocardial ischemia, angina pectoris, myocardial infarction, congestive heart failure, dilated congestive cardiomyopathy, hypertrophic cardiomyopathy, restrictive cardiomyopathy, cor pulmonale, arrhythmia, valvular heart disease, endocarditis, pulmonary embolism, venous thrombosis, and peripheral vascular disease.

In a preferred embodiment of this aspect, the invention features methods of predicting a risk of one or more clinical outcomes for a subject suffering from a vascular disease by analyzing a test sample obtained from the subject for the presence or amount of TpP™ and/or MCP-1, and using the presence or amount of TpP™ and/or MCP-1 measured in the sample to associate a risk of one or more clinical outcomes to the subject.

As described hereinafter, TpP™ and/or MCP-1 may be associated with a given risk of one or more clinical outcomes without considering any other markers. Thus, in certain embodiments, such an association may be made simply by providing one or more predetermined threshold concentrations, below which a subject has a first risk level, and above which a subject has a second risk level. A subject may be assigned a a relative prognostic risk based upon a population of vascular disease patients for whom TpP™ and/or MCP-1 concentrations have been measured, and subsequent clinical outcomes followed over a period of days, months, or years. The population TpP™ and/or MCP-1 (as relevant) concentrations may be divided into tertiles, quartiles, quintiles, *etc.,* and an associated risk level determined for each subpopulation by methods known in the art. Patients may then be assigned to one of these prognostic risk subpopulations according to a measured TpP™ and/or MCP-1 concentration.

In other embodiments, TpP™ and/or MCP-1 are used as part of panels as described herein to associate a risk of one or more clinical outcomes to the subject. Such panels may comprise 2, 3, 4, 5, 6, 7, 8, 9, 10, 15, 20, or more or individual markers, at least one of which is TpP™ or MCP-1. Preferred panels comprise a plurality of markers independently selected from the group consisting of TpP™, MCP-1, and one or more additional markers independently selected from the group consisting of specific markers of cardiac injury, specific markers of neural tissue injury, markers related to blood pressure regulation, markers related to inflammation, markers related to coagulation and hemostasis, and markers related to apoptosis. Exemplary markers in each of these groups are described hereina. One or more markers considered with TpP™ and/or MCP-1 may lack diagnostic or prognostic value when considered alone, but when used as part of a panel, such markers may be of great value in determining a particular diagnosis and/or prognosis.

Suitable additional markers for inclusion in such panels are described in detail hereinafter. Particularly preferred markers for use in such panels in addition to TpP™ include BNP, cardiac troponin I (free and/or complexed), cardiac troponin T (free and/or complexed), CRP, creatine kinase-MB, MMP-9, caspase-3, myoglobin, myeloperoxidase, sCD40L, or markers related thereto. One or more markers may be replaced, added, or subtracted from this list of particularly preferred markers while still providing clinically useful results.

In another aspect of the present invention, methods of diagnosing a vascular disease are described. Such methods comprise analyzing a test sample obtained from the subject for the presence or amount of TpP™ and/or MCP- and one or more additional markers, and using the presence or amount of TpP™ and/or MCP-1 and the additional marker(s) to determine the presence or absence of the vascular disease in the subject. In this aspect then, TpP™ and/or MCP-1 is used as part of a diagnostic panel. As above, such panels may comprise 2, 3, 4, 5, 6, 7, 8, 9, 10, 15, 20, or more or individual markers, at least one of which is TpP™ or MCP-1. Preferred panels comprise a TpP™ and/or MCP-1 and one or more additional markers independently selected from the group consisting of specific markers of cardiac injury, specific markers of neural tissue injury, markers related to blood pressure regulation, markers related to inflammation, markers related to coagulation and hemostasis, and markers related to apoptosis.

In a related aspect of the present invention, methods of diagnosing atherosclerosis are described. Such methods comprise analyzing a test sample obtained from the subject for the presence or amount of MCP- (and optionally one or more additional markers), and using the presence or amount of MCP-1 (and the additional marker(s) if measured) to determine the presence or absence of atherosclerosis in the subject. When MCP-1 is used as part of a diagnostic panel in this aspect, such panels may comprise 2, 3, 4, 5, 6, 7, 8, 9, 10, 15, 20, or more or individual markers, at least one of which is MCP-1. Preferred panels comprise MCP-1 and one or more additional markers independently selected from the group consisting of specific markers of cardiac injury, specific markers of neural tissue injury, markers related to blood pressure regulation, markers related to inflammation, markers related to coagulation and hemostasis, and markers related to apoptosis.

The marker panels of the present invention may be analyzed in a number of fashions well known to those of skill in the art. For example, each member of a panel may be compared to a "normal" value, or a value indicating a particular disease or outcome. A particular diagnosis/prognosis may depend upon the comparison of each marker to such a value; alternatively, if only a subset of markers are outside of a normal range, this subset may be indicative of a particular diagnosis/prognosis. For example, certain markers in a panel may be used to diagnose (or to rule out) a myocardial infarction, while other members of the panel may diagnose (or rule out) congestive heart failure, while still other members of the panel may diagnose (or rule out) aortic dissection. Markers may also be commonly used for multiple purposes by, for example, applying a different threshold or a different weighting factor to the marker for the different purpose(s). For example, a marker at one concentration or weighting may be used, alone or as part of a larger panel, to to diagnose (or to rule out) a myocardial infarction, and the same marker at a different concentration or weighting may be used, alone or as part of a larger panel, to diagnose (or rule out) congestive heart failure, *etc.*

In certain embodiments, one or more diagnostic or prognostic indicators are correlated to a condition or disease by merely the presence or absence of the indicator(s). For example, an assay can be designed so that a positive signal for a marker only occurs above a particular threshold concentration of interest, and below which concentration the assay provides no signal above background. In other embodiments, threshold concentration(s) of diagnostic or prognostic indicator(s) can be established, and the level of the indicator(s) in a patient sample can simply be compared to the threshold level(s).

The sensitivity and specificity of a diagnostic and/or prognostic test depends on more than just the analytical "quality" of the test--they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. *See, e.g.,* Hanley et al., Radiology 143: 29-36 (1982). Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

As described herinafter, a "panel response value" is preferably determined by plotting ROC curves for the sensitivity of a particular panel of markers versus 1-(specificity) for the panel at various cutoffs. In these methods, a profile of marker measurements from a subject are integrated to provide a single value that is considered to be the panel "result." Thus, the plurality of markers in a panel are considered together to provide a global probability (expressed either as a numeric score or as a percentage risk) of a diagnosis or prognosis. In such embodiments, an increase in a certain subset of markers may be sufficient to indicate a particular diagnosis/prognosis in one patient, while an increase in a different subset of markers may be sufficient to indicate the same or a different diagnosis/prognosis in another patient. Weighting factors may also be applied to one or more markers in a panel, for example, when a marker is of particularly high utility in identifying a particular diagnosis/prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

The skilled artisan will understand that associating a diagnostic or prognostic indicator, with a diagnosis or with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.*, Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

In yet other embodiments, multiple determinations of one or more diagnostic or prognostic markers described herein can be made, and a temporal change in the marker can be used to determine a diagnosis or prognosis. For example, a marker concentration in a subject sample may be determined at an initial time, and again at a second time from a second subject sample. In such embodiments, an increase in the marker from the initial time to the second time may be indicative of a particular diagnosis, or a particular prognosis. Likewise, a decrease in the marker from the initial time to the second time may be indicative of a particular diagnosis, or a particular prognosis. This "temporal change" parameter can be included as a marker in the marker panels of the present invention. In a related embodiment, multiple determinations of one or more diagnostic or prognostic markers can be made, and a temporal change in the marker can be used to monitor the efficacy of appropriate therapies. In such an embodiment, one might expect to see a decrease or an increase in the marker(s) over time during the course of effective therapy.

The skilled artisan will understand that, while in certain embodiments, comparative measurements are made of the same diagnostic marker at multiple time points, one could also measure a given marker at one time point, and a second marker at a second time point, and a comparison of these markers may provide diagnostic information. Similarly, the skilled artisan will understand that serial measurements and changes in markers or the combined result over time may also be of diagnostic and/or prognostic value.

In other embodiments, a threshold degree of change in the level of a prognostic or diagnostic indicator can be established, and the degree of change in the level of the indicator in a patient sample can simply be compared to the threshold degree of change in the level. A preferred threshold change in the level for markers of the invention is about 5%, about 10%, about 15%, about 20%, about 25%, about 30%, about 50%, about 75%, about 100%, and about 150%. The term "about" in this context refers to +/- 10%. In yet other embodiments, a "nomogram" can be established, by which a level of a prognostic or diagnostic indicator can be directly related to an associated disposition towards a given outcome. The skilled artisan is acquainted with the use of such nomograms to relate two numeric values with the understanding that the uncertainty in this measurement is the same as the uncertainty in the marker concentration because individual sample measurements are referenced, not population averages.

In yet another aspect, the invention relates to methods for determining a treatment regimen for use in a subject. The methods preferably comprise determining a diagnosis or prognosis as described herein, and selecting one or more treatment regimens appropriate to the diagnosis. In preferred embodiments, a treatment regimen is selected to improve the subject's prognosis by reducing the disposition for an adverse outcome associated with the diagnosis. Such methods may also be used to screen pharmacological compounds for agents capable of improving the patient's prognosis as above.

In a further aspect, the invention relates to kits and devices for determining the diagnosis or prognosis of a patient. Kits preferably comprise devices and reagents for performing the assays described herein, and instructions for performing the assays. Such devices preferably contain a plurality of discrete, independently addressable locations, or "diagnostic zones," each of which is related to a particular marker of interest. Following reaction of a sample with the devices, a signal is generated from the diagnostic zone(s), which may then be correlated to the presence or amount of the markers of interest. Optionally, the kits may contain one or more means for converting marker level(s) to a diagnosis or prognosis. Such kits preferably contain sufficient reagents to perform one or more such determinations, and/or Food and Drug Administration (FDA)- or other governmentally-approved labeling.

## BRIEF DESCRIPTION OF THE FIGURES

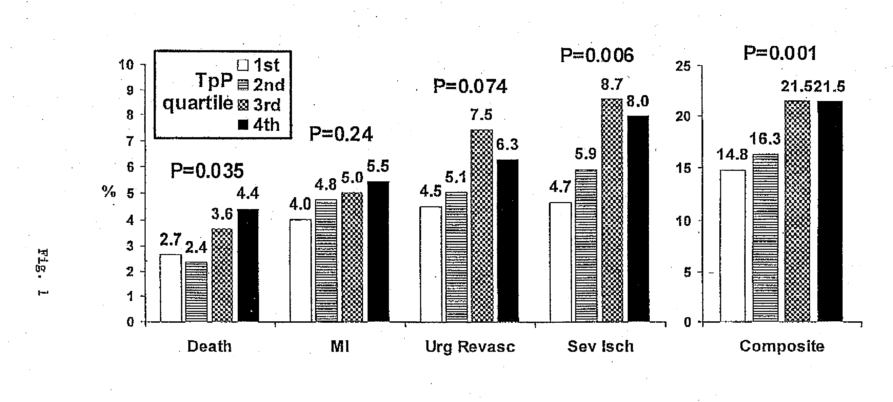

Fig. 1 shows the relationship of TpP™ concentration to clinical outcome through 12 months following enrollment of ACS subjects in the OPUS-TIMI 16 study.

Fig. 2 shows the relationship of MCP-1 concentration to atherosclerosis in subjects not exhibiting clinically apparent atherosclerosis as measured by determining CAC.

## DETAILED DESCRIPTION OF THE INVENTION

The present invention relates to methods and compositions for symptom-based differential diagnosis of diseases in subjects.

Patients presenting for medical treatment often exhibit one or a few primary observable changes in bodily characteristics or functions that are indicative of disease. Often, these "symptoms" are nonspecific, in that a number of potential diseases can present the same observable symptom or symptoms. A typical list of nonspecific symptoms might include one or more of the following: shortness of breath (or dyspnea), chest pain, fever, dizziness, and headache. These symptoms can be quite common, and the number of diseases that must be considered by the clinician can be astoundingly broad.

Taking shortness of breath (referred to clinically as "dyspnea") as an example, this symptom considered in isolation may be indicative of conditions as diverse as asthma, chronic obstructive pulmonary disease ("COPD"), tracheal stenosis, pulmonary injury, obstructive endobroncheal tumor, pulmonary fibrosis, pneumoconiosis, lymphangitic carcinomatosis, kyphoscoliosis, pleural effusion, amyotrophic lateral sclerosis, congestive heart failure, coronary artery disease, myocardial infarction, atrial fibrillation, cardiomyopathy, valvular dysfunction, left ventricle hypertrophy, pericarditis, arrhythmia, pulmonary embolism, metabolic acidosis, chronic bronchitis, pneumonia, anxiety, sepsis, aneurismic dissection, *etc. See, e.g.,* Kelley's Textbook of Internal Medicine, 4th Ed., Lippincott Williams & Wilkins, Philadelphia, PA, 2000, pp. 2349-2354, "Approach to the Patient With Dyspnea"; Mulrow et al., J. Gen. Int. Med. 8: 383-92 (1993).

Similarly, chest pain, when considered in isolation, may be indicative of stable angina, unstable angina, myocardial ischemia, atrial fibrillation, myocardial infarction, musculoskeletal injury, cholecystitis, gastroesophageal reflux, pulmonary embolism, pericarditis, aortic dissection, pneumonia, anxiety, *etc.* Moreover, the classification of chest pain as stable or unstable angina (or even mild myocardial infarction) in cases other than definitive myocardial infarction is completely subjective. The diagnosis, and in this case the distinction, is made not by angiography, which may quantify the degree of arterial occlusion, but rather by a physician's interpretation of clinical symptoms.

Differential diagnosis refers to methods for diagnosing the particular disease(s) and/or condition(s) underlying the symptoms in a particular subject, based on a comparison of the characteristic features observable from the subject to the characteristic features of those potential diseases. Depending on the breadth of diseases and conditions that must be considered in the differential diagnosis, the types and number of tests that must be ordered by a clinician can be quite large. In the case of dyspnea for example, the clinician may order tests from a group that includes radiography, electrocardiogram, exercise treadmill testing, blood chemistry analysis, echocardiography, bronchoprovocation testing, spirometry, pulse oximetry, esophageal pH monitoring, laryngoscopy, computed tomography, histology, cytology, magnetic resonance imaging, *etc. See, e.g.,* Morgan and Hodge, Am. Fam. Physician 57: 711-16 (1998). The clinician must then integrate information obtained from a battery of tests, leading to a clinical diagnosis that most closely represents the range of symptoms and/or diagnostic test results obtained for the subject.

The present invention describes methods and compositions that can assist in the differential diagnosis of one or more nonspecific symptoms by providing diagnostic markers that are designed to rule in or out one, and preferably a plurality, of possible etiologies for the observed symptoms. The concept of symptom-based differential diagnosis described herein can provide panels of diagnostic markers designed to be considered in concert to distinguish between possible diseases that underlie a nonspecific symptom observed in a patient.

The term "fever" as used herein refers to a body temperature greater than 100 °C orally or 100.8 °C rectally. In the case of fever, a plurality of markers are preferably selected to rule in or out a plurality of the following: sepsis; arteritis; sarcoidosis; and one or more infectious diseases, including infection by Staphyloccus species, Nisseria species, Pneumococcal species, Listeria species, Anthrax, Nocardia species, Salmonella species, Shigella species, Haemophilus species, Brucella species, Vibrio species including V. cholerae, Franciscella tularensis, Yersinia pestis, Pseudomonas species, Clostridia species including C. tetani, C. perfringens, C. ramosum, C. botulinum, and C. septicum, Actinomyces species, Treponema pallidum, Borrelia species including B. burgdorferi, Leptospira species, Mycobacterium species including M. tuberculosis, M. bovis, M. leprae, and M. africanum, Histoplasma species, Escherichia coli, Coccidioides species, Blastomyces species, Paracoccidioides species, Sporothrix species, Cryptococcus species, Candida species, and Aspergillus species; Rickettsial diseases including Rocky Mountain spotted fever, Q fever, typhus, trench fever, and cat-scratch fever; parasitic diseases including Malaria, Babesiosis, African sleeping sickness, Trypanosomiasis, Leishmaniasis, Toxoplasmosis, and Amebiasis; viral infection by influenza virus, parainfluenza virus, mumps virus, adenovirus, respiratory syncytial virus, rhinovirus, poliovirus, coxackievirus, echovirus, rubeola virus, rubella virus, parvovirus, hepatitis A, B, C, D, or E, cytomegalovirus, Epstein-Barr virus, Herpes simplex virus, Varicella-zoster virus, Alphavirus, Flaviviruses including yellow fever virus, dengue fever virus, Japanese encephalitis virus, and St. Louis encephalitis virus, West Nile virus, Colorado tick fever virus, Rabies virus, Arenavirus, Marburg agent, and Ebola virus.

The term "neurologic dysfunction" as used herein refers to a loss of one or more normal physiological or mental functions having a neurogenic etiology. The skilled artisan will understand that neurologic dysfunction is a common symptom in various systemic disorders (e.g., alcoholism, vascular disease, stroke, autoimmunity, metabolic disorders, aging, etc.). Specific neurologic dysfunctions include, but are not limited to, pain, headache, aphasia, apraxia, agnosia, amnesia, stupor, coma, delirium, dementia, seizure, migraine insomnia, hypersomnia, sleep apnea, tremor, dyskinesia, paralysis, etc.

The term "hypertension" as used herein refers to a systolic blood pressure of greater than or equal to 140 mm Hg and/or a diastolic blood pressure of greater than or equal to 90 mm Hg. Hypertension can include isolated systolic hypertension (i.e., no elevation in diastolic blood pressure). In the case of hypertension, the plurality of markers are preferably selected to rule in or out a plurality of the following: left ventricular failure, atherosclerosis, renal disease including chronic glomerulonephritis, and polycystic renal disease, coartation of the aorta, renal arterial stenosis, and hyperparathyroidism.

The term "condition within the differential diagnosis of a symptom" as used herein refers to a pathologic state that is known to be causative of a particular perceptible change in one or more physical characteristics exhibited by a subject suffering from the pathologic state, as compared to a normal subject. The concept of differential diagnosis is well established to those of skill in the art. *See, e.g.,* Beck, Tutorials in Differential Diagnosis, Churchill Livingstone, 2002; Zackon, Pulmonary Differential Diagnosis, Elsevier, 2000; Jamison, Differential Diagnosis for Primary Practice, Churchill Livingstone, 1999; Bouchier et al., French's Index of Differential Diagnosis, Oxford University Press, 1997.

The term "marker" as used herein refers to proteins, polypeptides, glycoproteins, proteoglycans, lipids, lipoproteins, glycolipids, phospholipids, nucleic acids, carbohydrates, etc., small molecules, or other characteristics of one or more subjects to be used as targets for screening test samples obtained from subjects. "Proteins or polypeptides" used as markers in the present invention are contemplated to include any fragments thereof, in particular, immunologically detectable fragments. "Marker" as used herein may also include derived markers as defined below, and may also include such characteristics as patient's history, age, sex and race, for example.

The term "derived marker" as used herein refers to a value that is a function of one or more measured markers. For example, derived markers may be related to the change over a time interval in one or more measured marker values, may be related to a ratio of measured marker values, may be a marker value at a different measurement time, or may be a complex function such as a panel response function.

The term "related marker" as used herein refers to one or more fragments of a particular marker or its biosynthetic parent that may be detected as a surrogate for the marker itself or as independent markers. For example, human BNP is derived by proteolysis of a 108 amino acid precursor molecule, referred to hereinafter as $BNP_{1-108}$. Mature BNP, or "the BNP natriuretic peptide," or "BNP-32" is a 32 amino acid molecule representing amino acids 77-108 of this precursor, which may be referred to as $BNP_{77-108}$. The remaining residues 1-76 are referred to hereinafter as $BNP_{1-76}$. Additionally, related markers may be the result of covalent modification of the parent marker, for example by oxidation of methionine residues, ubiquitination, cysteinylation, nitrosylation, glycosylation, *etc.*

Further considering BNP as an example, the sequence of the 108 amino acid BNP precursor pro-BNP ($BNP_{1-108}$) is as follows, with mature BNP ($BNP_{77-108}$) underlined:

```
HPLGSPGSAS  DLETSGLQEQ  RNHLQGKLSE  LQVEQTSLEP  LQESPRPTGV  50

WKSREVATEG  IRGHRKMVLY  TLRAPRSPKM  VQGSGCFGRK  MDRISSSSGL  100

GCKVLRRH                                                   108
```

(SEQ ID NO: 1).

$BNP_{1-108}$ is synthesized as a larger precursor pre-pro-BNP having the following sequence (with the "pre" sequence shown in bold):

```
MDPQTAPSRA  LLLLLFLHLA  FLGGRSHPLG  SPGSASDLET  SGLQEQRNHL  50

QGKLSELQVE  QTSLEPLQES  PRPTGVWKSR  EVATEGIRGH  RKMVLYTLRA  100

PRSPKMVQGS  GCFGRKMDRI  SSSSGLGCKV  LRRH                  134
```

(SEQ ID NO: 2).

While mature BNP itself may be used as a marker in the present invention, the prepro-BNP, $BNP_{1-108}$ and $BNP_{1-76}$ molecules represent BNP-related markers that may be measured either as surrogates for mature BNP or as markers in and of themselves. In addition, one or more fragments of these molecules, including BNP-related polypeptides selected from the group consisting of $BNP_{77-106}$, $BNP_{79-106}$, $BNP_{76-107}$, $BNP_{69-108}$, $BNP_{79-108}$, $BNP_{80-108}$, $BNP_{81-108}$, $BNP_{83-108}$, $BNP_{39-86}$, $BNP_{53-85}$, $BNP_{66-98}$, $BNP_{30-103}$, $BNP_{11-107}$, $BNP_{9-106}$, and $BNP_{3-108}$ may also be present in circulation. In addition, natriuretic peptide fragments, including BNP fragments, may comprise one or more oxidizable methionines, the oxidation of which to methionine sulfoxide or methionine sulfone produces additional BNP-related markers. *See, e.g.,* U.S. Patent No. 10/419,059, filed April 17, 2003, which is hereby incorporated by reference in its entirety including all tables, figures and claims.

Because production of marker fragments is an ongoing process that may be a function of, *inter alia,* the elapsed time between onset of an event triggering marker release into the tissues and the time the sample is obtained or analyzed; the elapsed time between sample acquisition and the time the sample is analyzed; the type of tissue sample at issue; the storage conditions; the quantity of proteolytic enzymes present; *etc.,* it may be necessary to consider this degradation when both designing an assay fou one or more markers, and when performing such an assay, in order to provide an accurate prognostic or diagnostic result. In addition, individual antibodies that distinguish amongst a plurality of marker fragments may be individually employed to separately detect the presence or amount of different fragments. The results of this individual detection may provide a more accurate prognostic or diagnostic result than detecting the plurality of fragments in a single assay. For example, different weighting factors may be applied to the various fragment measurements to provide a more accurate estimate of the amount of natriuretic peptide originally present in the sample.

In a similar fashion, many of the markers described herein are synthesized as larger precursor molecules, which are then processed to provide mature marker; and/or are present in circulation in the form of fragments of the marker. Thus, "related markers" to each of the markers described herein may be identified and used in an analogous fashion to that described above for BNP.

Removal of polypeptide markers from the circulation often involves degradation pathways. Moreover, inhibitors of such degradation pathways may hold promise in treatment of certain diseases. *See, e.g.,* Trindade and Rouleau, Heart Fail. Monit. 2: 2-7, 2001. However, the measurement of the polypeptide markers has focused generally upon measurement of the intact form without consideration of the degradation state of the molecules. Assays may be designed with an understanding of the degradation pathways of the polypeptide markers and the products formed during this degradation, in order to accurately measure the biologically active forms of a particular polypeptide marker in a sample.

The unintended measurement of both the biologically active polypeptide marker(s) of interest and inactive fragments derived from the markers may result in an overestimation of the concentration of biologically active form(s) in a sample.

The failure to consider the degradation fragments that may be present in a clinical sample may have serious consequences for the accuracy of any diagnostic or prognostic method. Consider for example a simple case, where a sandwich immunoassay is provided for BNP, and a significant amount (e.g., 50%) of the biologically active BNP that had been present has now been degraded into an inactive form. An immunoassay formulated with antibodies that bind a region common to the biologically active BNP and the inactive fragment(s) will overestimate the amount of biologically active BNP present in the sample by 2-fold, potentially resulting in a "false positive" result. Overestimation of the biologically active form(s) present in a sample may also have serious consequences for patient management. Considering the BNP example again, the BNP concentration may be used to determine if therapy is effective (e.g., by monitoring BNP to see if an elevated level is returning to normal upon treatment). The same "false positive" BNP result discussed above may lead the physician to continue, increase, or modify treatment because of the false impression that current therapy is ineffective.

Likewise, it may be necessary to consider the complex state of one or more markers described herein. For example, troponin exists in muscle mainly as a "ternary complex" comprising three troponin polypeptides (T, I and C). But troponin I and troponin T circulate in the blood in forms other than the I/T/C ternery complex. Rather, each of (i) free cardiac-specific troponin I, (ii) binary complexes (*e.g.*, troponin I/C complex), and (iii) ternary complexes all circulate in the blood. Furthermore, the "complex state" of troponin I and T may change over time in a patient, *e.g.*, due to binding of free troponin polypeptides to other circulating troponin polypeptides. Immunoassays that fail to consider the "complex state" of a protein marker may not detect all of the marker present.

Preferably, the methods described hereinafter utilize one or more markers that are derived from the subject. The term "subject-derived marker" as used herein refers to protein, polypeptide, phospholipid, nucleic acid, prion, glycoprotein, proteoglycan, glycolipid, lipid, lipoprotein, carbohydrate, or small molecule markers that are expressed or produced by one or more cells of the subject. The presence, absence, amount, or change in amount of one or more markers may indicate that a particular disease is present, or may indicate that a particular disease is absent. Additional markers may be used that are derived not from the subject, such as molecules expressed by pathogenic or infectious organisms that are correlated with a particular disease, race, time since onset, sex, etc. Such markers are preferably protein, polypeptide, phospholipid, nucleic acid, prion, or small molecule markers that identify the infectious diseases described above.

The term "test sample" as used herein refers to a sample of bodily fluid obtained for the purpose of diagnosis, prognosis, or evaluation of a subject of interest, such as a patient. In certain embodiments, such a sample may be obtained for the purpose of determining the outcome of an ongoing condition or the effect of a treatment regimen on a condition. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

As used herein, a "plurality" refers to at least two. Preferably, a plurality refers to at least 3, more preferably at least 5, even more preferably at least 10, even more preferably at least 15, and most preferably at least 20. In particularly preferred embodiments, a plurality is a large number, i.e., at least 100.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are "patients," i.e., living humans that are receiving medical care. This includes persons with no defined illness who are being investigated for signs of pathology.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, i.e., a marker, the presence, absence, or amount of which is indicative of the presence, severity, or absence of the condition.

Similarly, a "prognosis" is often determined by examining one or more "prognostic indicators." These are markers, the presence or amount of which in a patient (or a sample obtained from the patient) signal a probability that a given course or outcome will occur. For example, when one or more prognostic indicators reach a sufficiently high level in samples obtained from such patients, the level may signal that the patient is at an increased probability for experiencing a future stroke in comparison to a similar patient exhibiting a lower marker level. A level or a change in level of a prognostic indicator, which in turn is associated with an increased probability of morbidity or death, is referred to as being "associated with an increased predisposition to an adverse outcome" in a patient. Preferred prognostic markers can predict the onset of delayed neurologic deficits in a patient after stroke, or the chance of future stroke.

The term "correlating," as used herein in reference to the use of diagnostic and prognostic markers, refers to comparing the presence or amount of the marker(s) in a patient to its presence or amount in persons known to suffer from, or known to be at risk of, a given condition; or in persons known to be free of a given condition. As discussed above, a marker level in a patient sample can be compared to a level known to be associated with a specific diagnosis. The sample's marker level is said to have been correlated with a diagnosis; that is, the skilled artisan can use the marker level to determine whether the patient suffers from a specific type diagnosis, and respond accordingly. Alternatively, the sample's marker level can be compared to a marker level known to be associated with a good outcome (*e.g.*, the absence of disease, *etc.*). In preferred embodiments, a profile of marker levels are correlated to a global probability or a particular outcome.

The phrase "determining the diagnosis" as used herein refers to methods by which the skilled artisan can determine the presence or absence of a particular disease in a patient. The term "diagnosis" does not refer to the ability to determine the presence or absence of a particular disease with 100% accuracy, or even that a given course or outcome is more likely to occur than not. Instead, the skilled artisan will understand that the term "diagnosis" refers to an increased probability that a certain disease is present in the subject. In preferred embodiments, a diagnosis indicates about a 5% increased chance that a disease is present, about a 10% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 2%.

Similarly, the phrase "determining the prognosis" as used herein refers to methods by which the skilled artisan can determine the likelihood of one or more future clinical outcomes for a patient. The skilled artisan will understand that the term "prognosis" refers to an increased probability that a certain clinical outcome will occur at a future date in the subject. In preferred embodiments, a prognosis indicates about a 5% increased chance of a certain clinical outcome compared to a "control" population, about a 10% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 2%.

The term "discrete" as used herein refers to areas of a surface that are non-contiguous. That is, two areas are discrete from one another if a border that is not part of either area completely surrounds each of the two areas.

The term "independently addressable" as used herein refers to discrete areas of a surface from which a specific signal may be obtained.

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341: 544-546), which consists of a VH domain; and (vi) an isolated complementarity determining region (CDR). Single chain antibodies are also included by reference in the term "antibody."

The term "specific marker of myocardial injury" as used herein refers to molecules that are typically associated with cardiac tissue, and which can be correlated with a cardiac injury, but are not correlated with other types of injury. Such specific markers of cardiac injury include annexin V, B-type natriuretic peptide, β-enolase, cardiac troponin I (free and/or complexed), cardiac troponin T (free and/or complexed), creatine kinase-MB, glycogen phosphorylase-BB, heart-type fatty acid binding protein, phosphoglyceric acid mutase-MB, and S-100ao.

The term "specific marker of neural tissue injury" as used herein refers to molecules that are typically associated with neural tissue, and which can be correlated with a neural injury, but are not correlated with other types of injury. Exemplary specific markers of neural tissue injury are described in detail hereinafter.

<u>Differential Diagnosis of Dyspnea (Shortness of Breath)</u>

The present invention is described hereinafter generally in terms of the differential diagnosis of diseases and conditions related to dyspnea. The skilled artisan will understand, however, that the concepts of symptom-based differential diagnosis described herein are generally applicable to any physical characteristics that are indicative of a plurality of possible etiologies such as fever, neurologic dysfunction, chest pain ("angina"), dizziness, headache, *etc.*

A first step in the identification of suitable markers for symptom-bases differential diagnosis requires a consideration of the possible diagnoses that may be causative of the non-specific symptom observed. In the case of dyspnea, the potential causes are myriad. In a preferred embodiment, the following discussion considers three potential diagnoses: congestive heart failure, pulmonary embolism, and myocardial infarction; and three potential markers for inclusion in a differential diagnosis panel for these potential diagnoses: BNP, D-dimer, and cardiac troponin, respectively. In another preferred embodiment, markers for three potential diagnoses, congestive heart failure, pulmonary embolism, and myocardial infarction include three potential markers in a differential diagnosis panel, BNP related peptides, D-dimer, and cardiac troponin, respectively. In a preferred embodiment, three potential diagnoses in the case of dyspnea include congestive heart failure, pulmonary embolism, and myocardial infarction. In a second preferred embodiment, four potential diagnoses in the case of dyspnea include congestive heart failure, pulmonary embolism, and myocardial infarction, and atrial fibrillation. Potential markers for inclusion in a differential diagnosis panel include one or more of the following: BNP, BNP related peptides, D-dimer, cardiac troponin, ANP, and ANP related peptides.

*BNP*

B-type natriuretic peptide (BNP), also called brain-type natriuretic peptide is a 32 amino acid, 4 kDa peptide that is involved in the natriuresis system to regulate blood pressure and fluid balance. Bonow, R.O., Circulation 93: 1946-1950 (1996). The precursor to BNP is synthesized as a 108-amino acid molecule, referred to as "pre pro BNP," that is proteolytically processed into a 76-amino acid N-terminal peptide (amino acids 1-76), referred to as "NT pro BNP" and the 32-amino acid mature hormone, referred to as BNP or BNP 32 (amino acids 77-108). It has been suggested that each of these species - NT pro-BNP, BNP-32, and the pre pro BNP - can circulate in human plasma. Tateyama et al., Biochem. Biophys. Res. Commun. 185: 760-7 (1992); Hunt et al., Biochem. Biophys. Res. Commun. 214: 1175-83 (1995). The 2 forms, pre pro BNP and NT pro BNP, and peptides which are derived from BNP, pre pro BNP and NT pro BNP and which are present in the blood as a result of proteolyses of BNP, NT pro BNP and pre pro BNP, are collectively described as markers related to or associated with BNP.

BNP and BNP-related peptides are predominantly found in the secretory granules of the cardiac ventricles, and are released from the heart in response to both ventricular volume expansion and pressure overload. Wilkins, M. et al., Lancet 349: 1307-10 (1997). Elevations of BNP are associated with raised atrial and pulmonary wedge pressures, reduced ventricular systolic and diastolic function, left ventricular hypertrophy, and myocardial infarction. Sagnella, G.A., Clinical Science 95: 519-29 (1998). Furthermore, there are numerous reports of elevated BNP concentration associated with congestive heart failure and renal failure. Thus, BNP levels in a patient may be indicative of several possible underlying causes of dyspnea.

*D-dimer*

D-*dimer* is a crosslinked fibrin degradation product with an approximate molecular mass of 200 kDa. The normal plasma concentration of D-dimer is < 150 ng/ml (750 pM). The plasma concentration of D-dimer is elevated in patients with acute myocardial infarction and unstable angina, but not stable angina. Hoffmeister, H.M. et al., Circulation 91: 252.0-27 (1995); Bayes-Genis, A. et al., Thromb. Haemost. 81: 865-68 (1999); Gurfinkel, E. et al., Br. Heart J. 71: 151-55 (1994); Kruskal, J.B. et al., N. Engl. J. Med. 317: 1361-65 (1987); Tanaka, M. and Suzuki, A., Thromb. Res. 76: 289-98 (1994).

The plasma concentration of D-dimer also will be elevated during any condition associated with coagulation and fibrinolysis activation, including stroke, surgery, atherosclerosis, trauma, and thrombotic thrombocytopenic purpura. D-dimer is released into the bloodstream immediately following proteolytic clot dissolution by plasmin. The plasma concentration of D-dimer can exceed 2 µg/ml in patients with unstable angina. Gurfinkel, E. et al., Br. Heart J. 71: 151-55 (1994). Plasma D-dimer is a specific marker of fibrinolysis and indicates the presence of a prothrombotic state associated with acute myocardial infarction and unstable angina. The plasma concentration of D-dimer is also nearly always elevated in patients with acute pulmonary embolism; thus, normal levels of D-dimer may allow the exclusion of pulmonary embolism. Egermayer et al., Thorax 53: 830-34 (1998).

*Cardiac Troponin*

Troponin I (TnI) is a 25 kDa inhibitory element of the troponin complex, found in muscle tissue. TnI binds to actin in the absence of $Ca^{2+}$, inhibiting the ATPase activity of actomyosin. A TnI isoform that is found in cardiac tissue (cTnI) is 40% divergent from skeletal muscle TnI, allowing both isoforms to be immunologically distinguished. The normal plasma concentration of cTnI is < 0.1 ng/ml (4 pM). cTnI is released into the bloodstream following cardiac cell death; thus, the plasma cTnI concentration is elevated in patients with acute myocardial infarction. Investigations into changes in the plasma cTnI concentration in patients with unstable angina have yielded mixed results, but cTnI is not elevated in the plasma of individuals with stable angina. Benamer, H. et al., Am. J. Cardiol. 82: 845-50 (1998); Bertinchant, J.P. et al., Clin. Biochem. 29: 587-94 (1996); Tanasijevic, M.J. et al., Clin. Cardiol. 22: 13-16 (1999); Musso, P. et al., J. Ital. Cardiol. 26: 1013-23 (1996); Holvoet, P. et al., JAMA 281: 1718-21 (1999); Holvoet, P. et al., Circulation 98: 1487-94 (1998).

The plasma concentration of cTnI in patients with acute myocardial infarction is significantly elevated 4-6 hours after onset, peaks between 12-16 hours, and can remain elevated for one week. The release kinetics af cTnI associated with unstable angina may be similar. The measurement of specific forms of cardiac troponin, including free cardiac troponin I and complexes of cardiac troponin I with troponin C and/or T may provide the user with the ability to identify various stages of ACS. Free and complexed cardiac-troponin T may be used in a manner analogous to that described for cardiac troponin I. Cardiac troponin T complex may be useful either alone or when expressed as a ratio with total cardiac troponin I to provide information related to the presence of progressing myocardial damage. Ongoing ischemia may result in the release of the cardiac troponin TIC complex, indicating that higher ratios of cardiac troponin TIC:total cardiac troponin I may be indicative of continual damage caused by unresolved ischemia. *See,* U.S. Patent Nos. 6,147,688, 6,156,521, 5,947,124, and 5,795,725, which are hereby incorporated by reference in their entirety, including all tables, figures, and claims. One skilled in the art recognizes that in measuring cardiac troponin, one can measure the different isoforms of troponin I and troponin T.

One skilled in the art recognizes that in measuring cardiac troponin, one can measure the different forms of troponin I and troponin T. Thus, one may preferably measure free cardiac troponin 1, free cardiac troponin T, cardiac troponin I in a complex comprising one or both of troponin T and troponin C, cardiac troponin T in a complex comprising one or both of troponin I and troponin C, total cardiac troponin I (meaning free and complexed cardiac troponin I), and/or total cardiac troponin T, The term "at least one cardiac troponin form" as used herein refers to any one of these foregoing forms.

*ANP*

A-type natriuretic peptide (ANP) (also referred to as atrial natriuretic peptide or cardiodilatin Forssmann et al Histochem Cell Biol 110: 335-357 (1998)) is a 28 amino acid peptide that is synthesized, stored, and released atrial myocytes in response to atrial distension, angiotensin II stimulation, endothelin, and sympathetic stimulation (beta-adrenoceptor mediated). ANP is synthesized as a precursor molecule (pro-ANP) that is converted to an active form, ANP, by proteolytic cleavage and also forming N-terminal ANP (1-98). N-terminal ANP and ANP have been reported to increase in patients exhibiting atrial fibrillation and heart failure (Rossi et al. Journal of the American College of Cardiology 35: 1256-62 (2000). In addition to atrial natriuretic peptide (ANP99-126) itself, linear peptide fragments from its N-terminal prohormone segment have also been reported to have biological activity. As the skilled artisan will recognize, however, because of its relationship to ANP, the concentration of N-terminal ANP molecule can also provide diagnostic or prognostic information in patients. The phrase "marker related to ANP or ANP related peptide" refers to any polypeptide that originates from the pro-ANP molecule (1-126), other than the 28-amino acid ANP molecule itself. Proteolytic degradation of ANP and of peptides related to ANP have also been described in the literature and these proteolytic fragments are also encompassed it the term "ANP related peptides."

Elevated levels of ANP are found during hypervolemia, atrial fibrillation and congestive heart failure. ANP is involved in the long-term regulation of sodium and water balance, blood volume and arterial pressure. This hormone decreases aldosterone release by the adrenal cortex, increases glomerular filtration rate (GFR), produces natriuresis and diuresis (potassium sparing), and decreases renin release thereby decreasing angiotensin II. These actions contribute to reductions in blood volume and therefore central venous pressure (CVP), cardiac output, and arterial blood pressure. Several isoforms of ANP have been identified, and their relationship to stroke incidence studied. See, *e.g.,* Rubatu et al., Circulation 100: 1722-6, 1999; Estrada et al., Am. J. Hypertens. 7:1085-9, 1994.

Chronic elevations of ANP appear to decrease arterial blood pressure primarily by decreasing systemic vascular resistance. The mechanism of systemic vasodilation may involve ANP receptor-mediated elevations in vascular smooth muscle cGMP as well as by attenuating sympathetic vascular tone. This latter mechanism may involve ANP acting upon sites within the central nervous system as well as through inhibition of norepinephrine release by sympathetic nerve terminals. ANP may be viewed as a counter-regulatory system for the renin-angiotensin system. A new class of drugs that are neutral endopeptidase (NEP) inhibitors have demonstrated efficacy in heart failure. These drugs inhibit neutral endopeptidase, the enzyme responsible for the degradation of ANP, and thereby elevate plasma levels of ANP. NEP inhibition is particularly effective in heart failure when the drug has a combination of both NEP and ACE inhibitor properties.

Based on the foregoing discussion, the skilled artisan will recognize that, for example, increased BNP is indicative of congestive heart failure, but may also be indicative of other cardiac-related conditions such as myocardial infarction. Thus, the inclusion of a marker related to myocardial injury such as cardiac troponin I and/or cardiac troponin T can permit further discrimination of the disease underlying the observed dyspnea and the increased BNP level. In this case, an increased level of cardiac troponin may be used to rule in myocardial infarction.

Similarly, BNP may also be indicative of pulmonary embolism. The inclusion of a marker related to coagulation and hemostasis such as D-dimer can permit further discrimination of the disease underlying the observed dyspnea and the increased BNP level. In this case, a normal level of D-dimer may be used to rule out pulmonary embolism.

A detailed analysis of this exemplary marker panel is provided in the examples hereinafter. The skilled artisan will readily acknowledge that other markers may be substituted in or added to such marker panels to further discriminate the causes of dyspnea in accordance with the methods for identification and use of diagnostic markers described herein. Additional suitable markers are described in the following sections.

As discussed in detail herein, the foregoing principles of marker panel design may be applied broadly to symptom-based differential diagnosis. For example, in the case of abdominal pain, the plurality of markers are preferably selected to rule in or out a plurality of the following: aortic dissection, mesenteric embolism, pancreatitis, appendicitis, angina, myocardial infarction, one or more infectious diseases described above, influenza, esophageal carcinoma, gastric adenocarcinoma, colorectal adenocarcinoma, pancreatic tumors including ductal adenocarcinoma, cystadenocarcinoma, and insulinoma. In a preferred embodiment, the potential diagnoses for abdominal pain include aortic aneurysm, mesenteric embolism, pancreatitis, appendicitis, angina and myocardial infarction.

The foregoing principles may also be applied to subdivide differential diagnosis to a given level of detail required by the clinical artisan. For example, the differential diagnosis of various symptoms may require discrimination between heart failure and atrial fibrillation. An exemplary marker panel for performing such discrimination preferably includes BNP or BNP related peptides, and ANP or ANP related peptides, respectively. Additional markers may be defined to distinguish between systolic and diastolic dysfunction and atrial fibrillation. Preferred markers in this case include BNP, calcitonin gene related peptide, calcitonin and urotensin 1 for differentiation of systolic and diastolic dysfunction and ANP or ANP related peptides for the detection of atrial fibrillation. Likewise, markers may be defined to distinguish between systolic and diastolic dysfunction, atrial fibrillation, myocardial ischemia and cardiac necrosis. Preferred markers in this case include BNP, calcitonin gene related peptide, calcitonin and urotensin 1 for differentiation of systolic and diastolic dysfunction and ANP or ANP related peptides for the detection of atrial fibrillation and BNP and cardiac troponins for the detection of myocardial ischemia and necrosis.

In the case of chest pain, the present invention can provide markers able to distinguish between aortic dissection, myocardial ischemia, and cardiac necrosis; markers able to distinguish between aortic dissection, myocardial ischemia, and myocardial infarction; markers able to distinguish between aortic dissection, myocardial ischemia, cardiac necrosis and heart failure; markers able to distinguish between aortic dissection, myocardial ischemia, cardiac necrosis and myocardial infarction; markers able to distinguish between aortic dissection, myocardial ischemia, cardiac necrosis and atrial fibrillation; and/or markers able to distinguish between aortic dissection, myocardial ischemia and cardiac necrosis, myocardial infarction and atrial fibrillation. In accordance with the foregoing, a particularly preferred marker for aortic dissection is smooth muscle myosin, and most preferably smooth muscle myosin heavy chain, and a particularly preferred marker for atrial fibrillation is ANP or an ANP-related marker.

Preferred marker sets are those comprising smooth muscle myosin (or smooth muscle myosin heavy and/or light chains) and ANP or an ANP-related marker to distinguish aortic dissection and atrial fibrillation; smooth muscle myosin (or smooth muscle myosin heavy and/or light chains), ANP or an ANP-related marker, and BNP or a BNP-related marker to distinguish aortic dissection, atrial fibrillation and myocardial ischemia; smooth muscle myosin (or smooth muscle myosin heavy and/or light chains), BNP or a BNP-related marker, and a cardiac troponin form to distinguish aortic dissection, myocardial ischemia, and myocardial infarction; and smooth muscle myosin (or smooth muscle myosin heavy and/or light chains), BNP or a BNP-related marker, creatine kinase MB, myoglobin, and a cardiac troponin form to distinguish aortic dissection, myocardial ischemia, cardiac necrosis, and myocardial infarction.

Congestive heart failure is a heterogenous condition arising from two primary pathologies: left ventricular diastolic dysfunction and systolic dysfunction, which occur either alone or in combination. Gaasch, JAMA 271: 1276-80 (1994). As many as 40 percent of patients with clinical heart failure have diastolic dysfunction with normal systolic function. Soufer et al., Am. J. Cardiol. 55: 1032-6 (1984). Patient care decisions and prognosis hinge upon determination of the presence of one or both of these pathologies. Shamsham and Mitchell, Am. Fam. Physician 2000; 61:1319-28 (2000). Exemplary marker panels related to differentiating systolic and diastolic function comprise one or more markers selected from the group consisting of BNP, BNP related peptides, aldosterone, ANP, ANP related peptides, urodilatin, angiotensin 1, angiotensin 2, angiotensin 3, bradykinin, calcitonin, calcitonin gene related peptide, endothelin-2, endothelin-3, renin, urotensin 1, urotensin 2, antithrombin III, D-dimer, MMP-3, MMP-9, MMP-11, carboxy terminal propeptide of type I collagen (PICP), collagen carboxy terminal telopeptide (ICTP), fibrinogen, fibronectin, and vasopressin. Markers related to both systolic and diastolic dysfunction include BNP, ANP and ANP related markers. A preferred list of markers for differentiating systolic and diastolic heart failure include one or more markers selected from the group consisting of BNP, BNP related peptides, calcitonin gene related peptide, urotensin 2, endothelin 2, calcitonin and angiotensin 2. A particularly preferred list of markers for differentiating systolic and diastolic dysfunction include one or more markers selected from the group consisting of BNP, angiotensin 2, urotensin 2, and calcitonin gene related peptide.

Exemplary marker panels related to differentiating aortic dissection, myocardial ischemia, and myocardial infarction comprise one or more markers selected from the group consisting of smooth muscle myosin and/or smooth muscle myosin heavy chain, BNP and/or BNP related peptides, one or more troponin forms, and myoglobin.

Exemplary marker panels related to differentiating atrial fibrillation, myocardial infarction, and/or congestive heart failure comprise markers selected from the group consisting of ANP, ANP related peptides, one or more troponin forms, myoglobin, BNP, and BNP related peptides.

In the case of disturbanes of metabolic state, the plurality of markers are preferably selected to rule in or out a plurality of the following: diabetes mellitus, diabetic ketoacidosis, alcoholic ketoacidosis, respiratory acidosis, respiratory alkalosis, nonketogenic hyperglycemia, hypoglycemia, renal failure, interstitial renal disease, COPD, pneumonia, pulmonary edema and asthma.

Differential Diagnosis of Neurologic Dysfunction

In the case of neurologic dysfunction, the plurality of markers are preferably selected to rule in or out a plurality of the following: stroke, brain tumor, cerebral hypoxia, hypoglycemia, migraine, atrial fibrillation, myocardial infarction, cardiac ischemia, peripheral vascular disease and seizure. Preferred markers in this case include specific markers of cerebral injury such as adenylate kinase, brain-derived neurotrophic factor, calbindin-D, creatine kinase-BB, glial fibrillary acidic protein, lactate dehydrogenase, myelin basic protein, neural cell adhesion molecule, neuron-specific enolase, neurotrophin-3, proteolipid protein, S-100β, thrombomodulin, protein kinase C gamma; and/or one or more non-specific markers of cerebral injury such as β-thromboglobulin, D-dimer, fibrinopeptide A, plasmin-α-2-antiplasmin complex, platelet factor 4, prothrombin fragment 1+2, thrombin-antithrombin III complex, tissue factor, von Willebrand factor, adrenomedullin, cardiac troponin I (for myocardial ischemia and necrosis), head activator, hemoglobin $\alpha_2$ chain, caspase-3, vascular endothelial growth factor (VEGF), one or more endothelins (e.g., endothelin-1, endothelin-2, and endothelin-3), interleukin-8, Atrial natriuretic peptide, B-type natriuretic peptide (for myocardial ischemia and necrosis), and C-type natriuretic peptide; and/or one or more acute phase reactants such as C-reactive protein, ceruloplasmin, fibrinogen, $\alpha1$-acid glycoprotein, $\alpha1$-antitrypsin, haptoglobin, insulin-like growth factor-1, interleukin-1$\beta$, interleukin-1 receptor antagonist, interleukin-6, transforming growth factor $\beta$, tumor necrosis factor $\alpha$, E-selectin, intercellular adhesion molecule-1, matrix metalloproteinases (e.g., matrix metalloproteinase 9 (MMP-9)), monocyte chemotactic protein-1, and vascular cell adhesion molecule.

Stroke is a pathological condition with acute onset that is caused by the occlusion or rupture of a vessel supplying blood, and thus oxygen and nutrients, to the brain. The immediate area of injury is referred to as the "core," which contains brain cells that have died as a result of ischemia or physical damage. The "penumbra" is composed of brain cells that are neurologically or chemically connected to cells in the core. Cells within the penumbra are injured, but still have the ability to completely recover following removal of the insult caused during stroke. However, as ischemia or bleeding from hemorrhage continues, the core of dead cells can expand from the site of insult, resulting in a concurrent expansion of cells in the penumbra. The initial volume and rate of core expansion is related to the severity of the stroke and, in most cases, neurological outcome.

The brain contains two major types of cells, neurons and glial cells. Neurons are the most important cells in the brain, and are responsible for maintaining communication within the brain via electrical and chemical signaling. Glial cells function mainly as structural components of the brain, and they are approximately 10 times more abundant than neurons. Glial cells of the central nervous system (CNS) are astrocytes and oligodendrocytes. Astrocytes are the major interstitial cells of the brain, and they extend cellular processes that are intertwined with and surround neurons, isolating them from other neurons. Astrocytes can also form 'end feet" at the end of their processes that surround capillaries. Oligodendrocytes are cells that form myelin sheathes around axons in the CNS. Each oligodendrocyte has the ability to ensheathe up to 50 axons. Schwann cells are glial cells of the peripheral nervous system (PNS). Schwann cells form myelin sheathes around axons in the periphery, and each Schwann cell ensheathes a single axon.

Cell death during stroke occurs as a result of ischemia or physical damage to the cells of the CNS. During ischemic stroke, an infarct occurs, greatly reducing or stopping blood flow beyond the site of infarction. The zone immediately beyond the infarct soon lacks suitable blood concentrations of the nutrients essential for cell survival. Cells that lack nutrients essential for the maintenance of important functions like metabolism soon perish. Hemorrhagic stroke can induce cell death by direct trauma, elevation in intracranial pressure, and the release of damaging biochemical substances in blood. When cells die, they release their cytosolic contents into the extracellular milieu.

The barrier action of tight junctions between the capillary endothelial cells of the central nervous system is referred to as the "blood-brain barrier". This barrier is normally impermeable to proteins and other molecules, both large and small. In other tissues such as skeletal, cardiac, and smooth muscle, the junctions between endothelial cells are loose enough to allow passage of most molecules, but not proteins.

Substances that are secreted by the neurons and glial cells (intracellular brain compartment) of the central nervous system (CNS) can freely pass into the extracellular milieu (extracellular brain compartment). Likewise, substances from the extracellular brain compartment can pass into the intracellular brain compartment. The passage of substances between the intracellular and extracellular brain compartments are restricted by the normal cellular mechanisms that regulate substance entry and exit. Substances that are found in the extracellular brain compartment also are able to pass freely into the cerebrospinal fluid, and vice versa. This movement is controlled by diffusion.

The movement of substances between the vasculature and the CNS is restricted by the blood-brain barrier. This restriction can be circumvented by facilitated transport mechanisms in the endothelial cells that transport, among other substances, nutrients like glucose and amino acids across the barrier for consumption by the cells of the CNS. Furthermore, lipid-soluble substances such as molecular oxygen and carbon dioxide, as well as any lipid-soluble drugs or narcotics can freely diffuse across the blood-brain barrier.

Depending upon their size, specific markers of cerebral injury that are released from injured brain cells during stroke or other neuropathies will only be found in peripheral blood when CNS injury is coupled with or followed by an increase in the permeability of the blood-brain barrier. This is particularly true of larger molecules. Smaller molecules may appear in the peripheral blood as a result of passive diffusion, active transport, or an increase in the permeability of the blood-brain barrier. Increases in blood-brain barrier permeability can arise as a result of physical disruption in cases such as tumor invasion and extravasation or vascular rupture, or as a result of endothelial cell death due to ischemia. During stroke, the blood-brain barrier is compromised by endothelial cell death, and any cytosolic components of dead cells that are present within the local extracellular milieu can enter the bloodstream.

Therefore, specific markers of cerebral injury may also be found in the blood or in blood components such as serum and plasma, as well as the CSF of a patient experiencing stroke or TIAs. Furthermore, clearance of the obstructing object in ischemic stroke can cause injury from oxidative insult during reperfusion, and patients with ischemic stroke can sometimes experience hemorrhagic transformation as a result of reperfusion or thrombolytic therapy. Additionally, injury can be caused by vasospasm, which is a focal or diffuse narrowing of the large capacity arteries at the base of the brain following hemorrhage. The increase in blood-brain barrier permeability is related to the insult severity, and its integrity is reestablished following the resolution of insult. Specific markers of cerebral injury will only be present in peripheral blood if there has been a sufficient increase in the permeability of the blood-brain barrier that allows these large molecules to diffuse across. In this regard, most specific markers of cerebral injury can be found in cerebrospinal fluid after stroke or any other neuropathy that affects the CNS. Furthermore, many investigations of coagulation or fibrinolysis markers in stroke are performed using cerebrospinal fluid.

*The Coagulation Cascade in Stroke*

There are essentially two mechanisms that are used to halt or prevent blood loss following vessel injury. The first mechanism involves the activation of platelets to facilitate adherence to the site of vessel injury. The activated platelets then aggregate to form a platelet plug that reduces or temporarily stops blood loss. The processes of platelet aggregation, plug formation and tissue repair are all accelerated and enhanced by numerous factors secreted by activated platelets. Platelet aggregation and plug formation is mediated by the formation of a fibrinogen bridge between activated platelets. Concurrent activation of the second mechanism, the coagulation cascade, results in the generation of fibrin from fibrinogen and the formation of an insoluble fibrin clot that strengthens the platelet plug.

The coagulation cascade is an enzymatic pathway that involves numerous serine proteinases normally present in an inactive, or zymogen, form. The presence of a foreign surface in the vasculature or vascular injury results in the activation of the intrinsic and extrinsic coagulation pathways, respectively. A final common pathway is then followed, which results in the generation of fibrin by the serine proteinase thrombin and, ultimately, a crosslinked fibrin clot. In the coagulation cascade, one active enzyme is formed initially, which can activate other enzymes that active others, and this process, if left unregulated, can continue until all coagulation enzymes are activated. Fortunately, there are mechanisms in place, including fibrinolysis and the action of endogenous proteinase inhibitors that can regulate the activity of the coagulation pathway and clot formation.

Fibrinolysis is the process of proteolytic clot dissolution. In a manner analogous to coagulation, fibrinolysis is mediated by serine proteinases that are activated from their zyrnogen form. The serine proteinase plasmin is responsible for the degradation of fibrin into smaller degradation products that are liberated from the clot, resulting in clot dissolution. Fibrinolysis is activated soon after coagulation in order to regulate clot formation. Endogenous serine proteinase inhibitors also function as regulators of fibrinolysis.

The presence of a coagulation or fibrinolysis marker in cerebrospinal fluid would indicate that activation of coagulation or fibrinolysis, depending upon the marker used, coupled with increased permeability of the blood-brain barrier has occurred. In this regard, more definitive conclusions regarding the presence of coagulation or fibrinolysis markers associated with acute stroke may be obtained using cerebrospinal fluid.

Platelets are round or oval disks with an average diameter of 2-4 $\mu$m that are normally found in blood at a concentration of 200,000-300,000/$\mu$l. They play an essential role in maintaining hemostasis by maintaining vascular integrity, initially stopping bleeding by forming a platelet plug at the site of vascular injury, and by contributing to the process of fibrin formation to stabilize the platelet plug. When vascular injury occurs, platelets adhere to the site of injury and each other and are stimulated to aggregate by various agents released from adherent platelets and injured endothelial cells. This is followed by the release reaction, in which platelets secrete the contents of their intracellular granules, and formation of the platelet plug. The formation of fibrin by thrombin in the coagulation cascade allows for consolidation of the plug, followed by clot retraction and stabilization of the plug by crosslinked fibrin. Active thrombin, generated in the concurrent coagulation cascade, also has the ability to induce platelet activation and aggregation.

The coagulation cascade can be activated through either the extrinsic or intrinsic pathways. These enzymatic pathways share one final common pathway. The result of coagulation activation is the formation of a crosslinked fibrin clot. Fibrinolysis is the process of proteolytic clot dissolution that is activated soon after coagulation activation, perhaps in an effort to control the rate and amount of clot formation. Urokinase-type plasminogen activator (uPA) and tissue-type plasminogen activator (tPA) proteolytically cleave plasminogen, generating the active serine proteinase plasmin. Plasmin proteolytically digests crosslinked fibrin, resulting in clot dissolution and the production and release of fibrin degradation products.

The first step of the common pathway of the coagulation cascade involves the proteolytic cleavage of prothrombin by the factor Xa/factor Va prothrombinase complex to yield active thrombin. Thrombin is a serine proteinase that proteolytically cleaves fibrinogen to form fibrin, which is ultimately integrated into a crosslinked network during clot formation.

Methods and marker sets for differential diagnosis of stroke and other cerebral injuries are described in U.S. Patent No. 10/225,082, filed August 20, 2002, which is hereby incorporated in its entirety, including all tables figures and claims. As described therein, preferred marker panels diagnose and/or differentiate between stroke, subarachnoid hemorrhage, intracerebral hemorrhage, and/or hemorrhagic stroke; and/or can distinguish between ischemic and hemorrhagic stroke. Particularly preferred are markers that differentiate between thrombotic, embolic, lacunar, hypoperfusion, intracerebral hemorrhage, and subarachnoid hemorrhage types of strokes. Particularly preferred marker sets include BNP, IL-6, S-100β, MMP-9, TAT complex, and vWF A1-integrin; BNP, S-100β, MMP-9, and vWF-A1-integrin; vWF-A1, VEGF, and MMP-9; caspase-3, MMP-9, and GFAP; caspase-3, MMP-9, vWF-A1, and BNP; NCAM, BDNF, Caspase-3, MMP-9, vWF-A1, and VEGF; NCAM, BDNF, Caspase-3, MMP-9, vWF-A1, and S-100β; VEGF; NCAM, BDNF, Caspase-3, MMP-9, vWF-A1, and MCP-1; VEGF; NCAM, BDNF, Caspase-3, MMP-9, VEGF, and vWF A1-integrin; BDNF, MMP-9, S-100β, vWF A1-integrin, MCP-1, and GFAP; BDNF, caspase-3, MMP-9, vWF-A1, S-100β, and GFAP; NCAM, BDNF, MMP-9, vWF-A1, S-100β, and GFAP; NCAM, BDNF, caspase-3, MMP-9, S-100β, and GFAP; caspase-3, NCAM, MCP-1, S100β, MMP-9, vWF A1-integrin, and BNP; caspase-3, NCAM, MCP-1, S100β, MMP-9, vWF A1, BNP, and GFAP; CRP, NT-3, vWF, MMP-9, VEGF, and CKBB; CRP, MMP-9, VEGF, CKBB, and MCP-1; CRP, NT-3, MMP-9, VEGF, CKBB, and MCP-1; and CRP, MMP-9, VEGF, CKBB, MCP-1. Calbindin, vWF VP1, vWF A3, vWF A1-A3, TAT complex, proteolipid protein, IL-6, IL-8, myelin basic protein, S-100β, tissue factor, GFAP, vWF A1-integrin, CNP, and NCAM.

A panel consisting of the markers referenced herein may be constructed to provide relevant information related to the differential diagnosis of interest. Such a panel may be constructed using 1, 2, 3, 4, 5, 6, 7, 8, 9, 10, 11, 12, 13, 14, 15, 16, 17, 18, 19 or 20 individual markers. The analysis of a single marker or subsets of markers comprising a larger panel of markers could be carried out by one skilled in the art to optimize clinical sensitivity or specificity in various clinical settings. These include, but are not limited to ambulatory, urgent care, critical care, intensive care, monitoring unit, inpatient, outpatient, physician office, medical clinic, and health screening settings. Furthermore, one skilled in the art can use a single marker or a subset of markers comprising a larger panel of markers in combination with an adjustment of the diagnostic threshold in each of the aforementioned settings to optimize clinical sensitivity and specificity. The clinical sensitivity of an assay is defined as the percentage of those with the disease that the assay correctly predicts, and the specificity of an assay is defined as the percentage of those without the disease that the assay corrects predicts (Tietz Textbook of Clinical Chemistry, 2nd edition, Carl Burtis and Edward Ashwood eds., W.B. Saunders and Company, p. 496). The following provides a brief discussion of additional exemplary markers for use in identifying suitable marker panels by the methods described herein.

The Acute Coronary Syndrome

Myocardial ischemia is caused by an imbalance of myocardial oxygen supply and demand. Specifically, demand exceeds supply due to inadequate blood supply. The heart accounts for a small percentage of total body weight, but is responsible for 7% of body oxygen consumption. Cardiac tissue metabolism is highly aerobic and has very little reserve to compensate for inadequate blood supply. When the blood supply is reduced to levels that are inadequate for myocardial demand, the tissue rapidly becomes hypoxic and toxic cellular metabolites can not be removed. Myocardial cells rapidly use oxygen supplies remaining in the local microvasculature, and the length of time that aerobic metabolism continues is indirectly proportional to the degree of arterial occlusion. Once the oxygen supply has been exhausted, oxidative phosphorylation can not continue because oxygen is no longer available as an electron acceptor, pyruvate can not be converted to acetyl coenzyme A and enter the citric acid cycle. Myocardial metabolism switches to anaerobic metabolism using glycogen and glucose stores, and pyruvate is fermented to lactate. Lactate accumulation is the primary cause of chest pain in individuals with ACS. As ischemia continues, cardiac tissue becomes more acidic as lactate and other acidic intermediates accumulate, ATP levels decrease, and available energy sources are depleted. Cardiac tissue can recover if it is reperfused 15-20 minutes after an ischemic event. After the cellular glycogen stores have been depleted, the cell gradually displays features of necrosis, including mitochondrial swelling and loss of cell membrane integrity. Upon reperfusion, these damaged cells die, possibly as a result of the cell's inability to maintain ionic equilibrium. A loss of membrane integrity causes the cell's cytosolic contents to be released into the circulation.

Stable angina, unstable angina, and myocardial infarction all share one common feature: constricting chest pain associated with myocardial ischemia. Angina is classified as stable or unstable through a physician's interpretation of clinical symptoms, with or without diagnostic ECG changes. The classification of angina as "stable" or "unstable" does not refer to the stability of the plaque itself, but rather, the degree of exertion that is required to elicit chest pain. Most notably, the classification of chest pain as stable or unstable angina (or even mild myocardial infarction) in cases other than definitive myocardial infarction is completely subjective. The diagnosis, and in this case the distinction, is made not by angiography, which may quantify the degree of arterial occlusion, but rather by a physician's interpretation of clinical symptoms.

Stable angina is characterized by constricting chest pain that occurs upon exertion or stress, and is relieved by rest or sublingual nitroglycerin. Coronary angiography of patients with stable angina usually reveals 50-70% obstruction of at least one coronary artery. Stable angina is usually diagnosed by the evaluation of clinical symptoms and ECG changes. Patients with stable angina may have transient ST segment abnormalities, but the sensitivity and specificity of these changes associated with stable angina are low.

Unstable angina is characterized by constricting chest pain at rest that is relieved by sublingual nitroglycerin. Anginal chest pain is usually relieved by sublingual nitroglycerin, and the pain usually subsides within 30 minutes. There are three classes of unstable angina severity: class I, characterized as new onset, severe, or accelerated angina; class II, subacute angina at rest characterized by increasing severity, duration, or requirement for nitroglycerin; and class III, characterized as acute angina at rest. Unstable angina represents the clinical state between stable angina and AMI and is thought to be primarily due to the progression in the seventy and extent of atherosclerosis, coronary artery spasm, or hemorrhage into non-occluding plaques with subsequent thrombotic occlusion. Coronary angiography of patients with unstable angina usually reveals 90% or greater obstruction of at least one coronary artery, resulting in an inability of oxygen supply to meet even baseline myocardial oxygen demand. Slow growth of stable atherosclerotic plaques or rupture of unstable atherosclerotic plaques with subsequent thrombus formation can cause unstable angina. Both of these causes result in critical narrowing of the coronary artery. Unstable angina is usually associated with atherosclerotic plaque rupture, platelet activation, and thrombus formation. Unstable angina is usually diagnosed by clinical symptoms, ECG changes, and changes in cardiac markers (if any). Treatments for patients with unstable angina include nitrates, aspirin, GPIIb/IIIa inhibitors, heparin, and beta-blockers. Thrombolytic therapy has not been demonstrated to be beneficial for unstable angina patients, and calcium channel blockers may have no effect. Patients may also receive angioplasty and stents. Finally, patients with unstable angina are at risk for developing AMI.

Myocardial infarction is characterized by constricting chest pain lasting longer than 30 minutes that can be accompanied by diagnostic ECG Q waves. Most patients with AMI have coronary artery disease, and as many as 25% of AMI cases are "silent" or asymptomatic infarctions, and individuals with diabetes tend to be more susceptible to silent infarctions. Population studies suggest that 20-60% of nonfatal myocardial infarctions are silent infarctions that are not recognized by the patient. Atypical clinical presentations of AMI can include congestive heart failure, angina pectoris without a severe or prolonged attack, atypical location of pain, central nervous system manifestations resembling stroke, apprehension and nervousness, sudden mania or psychosis, syncope, weakness, acute indigestion, and peripheral embolization. AMI is usually diagnosed by clinical symptoms, ECG changes, and elevations of cardiac proteins, most notably cardiac troponin, creatine kinase-MB and myoglobin. Treatments of AMI have improved over the past decade, resulting in improved patient outcome and a 30% decrease in the death rate associated with AMI. Treatment of AMI patients is accomplished by administering agents that limit infarct size and improve outcome by removing occlusive material, increasing the oxygen supply to cardiac tissue, or decreasing the oxygen demand of cardiac tissue. Treatments can include the following: supplemental oxygen, aspirin, GPIIb/IIIa inhibitors, heparin, thrombolytics (tPA), nitrates (nitroglycerin), magnesium, calcium channel antagonists, $\beta$-adrenergic receptor blockers, angiotensin-converting enzyme inhibitors, angioplasty (PTCA), and intraluminal coronary artery stents.

The 30 minute time point from chest pain onset is thought to represent the window of reversible myocardial damage caused by ischemia. Stable angina and unstable angina are characterized angiographically as 50-70% and 90% or greater arterial occlusion, respectively, and myocardial infarction is characterized by complete or nearly complete occlusion. A common misconception is that stable angina and unstable angina refer to plaque stability, or that they, along with myocardial infarction, are separate diseases. Because stable angina often progresses to unstable angina, and unstable angina often progresses to myocardial infarction, stable angina, unstable angina, and myocardial infarction can all be characterized as coronary artery disease of varying severity. Recently, the following physiological model of coronary artery disease progression has been proposed: Inflammation → Plaque Rupture → Platelet Activation → Early Thrombosis → Early Necrosis. This model is designed to fit the theory that inflammation occurs during stable angina, and that markers of plaque rupture, platelet activation, and early thrombosis can be used to identify and monitor the progressing severity of unstable angina. The myocardial damage caused during an anginal attack is, by definition, reversible, while damage caused during a myocardial infarction is irreversible. Therefore, there are two proposed break points in this model for the discrimination of stable angina, unstable angina, and AMI. The first occurs between inflammation and plaque rupture, with the theory that plaque rupture does not occur in stable angina. The second occurs between early thrombosis and early necrosis, with the theory that myocardial damage incurred during unstable angina is reversible. It is important to realize that these events, with the exception of early myocardial necrosis, can be associated with all forms of coronary artery disease, and that progression along this diagnostic pathway does not necessarily indicate disease progression. The progression of coronary artery disease from mild unstable angina to severe unstable angina and myocardial infarction is related to plaque instability and the degree of arterial occlusion. This progression can occur slowly, as stable plaques enlarge and become more occlusive, or it can occur rapidly, as unstable plaques rupture, causing platelet activation and occlusive thrombus formation. Because myocardial infarction most frequently shares the same pathophysiology as unstable angina, it is possible that the only distinction between these two events is the reversibility of myocardial damage. By definition, unstable angina causes reversible damage, while myocardial infarction causes irreversible damage. There have been published reports that indicate the presence of myocardial necrosis in patients with unstable angina. By definition, these patients may actually be experiencing early AMI. Nevertheless, even if these patients are diagnosed with unstable angina instead of early AMI, the high degree of severity suggests that they will benefit greatly from early aggressive treatment. Myocardial ischemia is the major determinant in the pathogenesis of stable angina, unstable angina, and myocardial infarction, and they should not be thought of as individual diseases. Rather, they reflect the increasing severity of myocardial damage from ischemia.

Inflammatory mechanisms play a pivotal role in the atherosclerotic process. At the base of atherogenesis there are complex interactions between macrophages, T lymphocytes and smooth muscle cells. A growing body of experimental evidence suggests that inflammation is involved in the pathogenesis of ACS and influences its clinical evolution. In patients with ACS, coronary atherosclerotic plaques are characterized by an abundant inflammatory infiltrate. Moreover, in these patients systemic signs of inflammatory reaction can be observed: activated circulating inflammatory cells (neutrophil, monocytes and lymphocytes) and increased concentrations of pro-inflammatory cytokines, such as interleukin (1L)-1 and 6, and of acute phase reactants, in particular C-reactive protein (CRP).

Thrombus Precursor Protein

Thrombin first removes fibrinopeptide A from fibrinogen, yielding desAA fibrin monomer, which can form complexes with all other fibrinogen-derived proteins, including fibrin degradation products, fibrinogen degradation products, desAA fibrin, and fibrinogen. The desAA fibrin monomer is generically referred to as soluble fibrin, as it is the first product of fibrinogen cleavage, but it is not yet crosslinked via factor XIIIa into an insoluble fibrin clot. DesAA fibrin monomer also can undergo further proteolytic cleavage by thrombin to remove fibrinopeptide B, yielding desAABB fibrin monomer. This monomer can polymerize with other desAABB fibrin monomers to form soluble desAABB fibrin polymer, also referred to as soluble fibrin or thrombus precursor protein (TpP™).

TpP™ is the immediate precursor to insoluble fibrin, which forms a "mesh-like" structure to provide structural rigidity to the newly formed thrombus. In this regard, measurement of TpP™ in plasma is a direct measurement of active clot formation. The normal plasma concentration of TpP™ was reported to be < 6 ng/ml (Laurino, J.P. et al., Ann. Clin. Lab. Sci. 27:338-345, 1997). American Biogenetic Sciences has developed an assay for TpP™ (US Patent Nos. 5,453,359, 5,837,540 and 5,843,690). Studies have measured elevated TpP™ in patients with AMI (Laurino et al., Ann. Clin. Lab. Sci. 27:338-345, 1997; Carville et al., Clin. Chem. 42:1537-1541, 1996). The plasma concentration of TpP™ is also reported to be elevated in patients with unstable angina (Laurino et al., Ann. Clin. Lab. Sci. 27:338-345, 1997), though other workers have found TpP™ levels to be similar in controls, unstable angina, and chronic stable effort angina (Fiotta et al., Blood Coagul. Fibrinolysis 13: 247-255, 2002).

The concentration of TpP™ in plasma will theoretically be elevated during any condition that causes or is a result of coagulation activation, including disseminated intravascular coagulation, sepsis, pulmonary embolism, deep venous thrombosis, congestive heart failure, surgery, cancer, gastroenteritis, and cocaine overdose (Laurino et al., Ann. Clin. Lab. Sci. 27:338-345, 1997; Song et al., Haematologica 87: 1062-1067, 2002; La Capra et al., Blood Coagul. Fibrinolysis 11: 371-377, 2000). TpP™ is released into the bloodstream immediately following thrombin activation. TpP™ likely has a short half-life in the bloodstream because it will be rapidly converted to insoluble fibrin at the site of clot formation. Plasma TpP™ concentrations are reported to peak within 3 hours of AMI onset, returning to normal after 12 hours from onset. The plasma concentration of TpP™ can exceed 30 ng/ml in CVD (Laurino et al., Ann. Clin. Lab. Sci. 27:338-345, 1997).

MCP-1

Monocyte chemotactic protein-1 (also called monocyte chemoattractant protein-1) (MCP-1) is a 10 kDa chemotactic factor that attracts monocytes and basophils, but not neutrophils or eosiniphils. MCP-1 is normally found in equilibrium between a monomeric and homodimeric form, and it is normally produced in and secreted by monocytes and vascular endothelial cells (Yoshimura, T. et al., FEBS Lett. 244:487-493, 1989; Li, Y.S. et al., Mol. Cell. Biochem. 126:61-68, 1993). MCP-1 has been implicated in the pathogenesis of a variety of diseases that involve monocyte infiltration, including psoriasis, rheumatoid arthritis, and atherosclerosis. The normal concentration of MCP-1 in plasma is < 0.1 ng/ml. The plasma concentration of MCP-1 is elevated in patients with AMI, and may be elevated in the plasma of patients with unstable angina, but no elevations have been associated with stable angina (Soejima, H. et al., J. A. Coll. Cardiol. 34:983-988, 1999; Nishiyama, K. et al., Jpn. Circ. J 62:710-712, 1998; Matsumori, A. et al., J. Mol. Cell. Cardiol. 29:419-423, 1997). Interestingly, MCP-1 also may be involved in the recruitment of monocytes into the arterial wall during atherosclerosis.

Elevations of the serum concentration of MCP-1 are associated with various conditions associated with inflammation, including alcoholic liver disease, interstitial lung disease, sepsis, and systemic lupus erythematosus (Fisher, N.C. et al., Gut 45:416-420, 1999; Suga, M. et al., Eur. Respir. J. 14:376-382, 1999; Bossink, A.W. et al., Blood 86: 3841-3847, 1995; Kaneko, H. et al. J. Rheumatol. 26:568-573, 1999). MCP-1 is released into the bloodstream upon activation of monocytes and endothelial cells. The concentration of MCP-1 in plasma form patients with AMI has been reported to approach 1 ng/ml (100 pM), and can remain elevated for one month (Soejima, H. et al., J. Am. Coll. Cardiol. 34:983-988, 1999). The kinetics of MCP-1 release into and clearance from the bloodstream in the context of ACS are currently unknown. MCP-1 is a specific marker of the presence of a pro-inflammatory condition that involves monocyte migration.

Exemplary Markers For Use in Panels

*(i) Specific Markers of Myocardial Injury*

In addition to cardiac troponins, described in detail above, the following are exemplary specific markers of myocardial injury. This list is not meant to be limiting.

Annexin V, also called lipocortin V, endonexin II, calphobindin I, calcium binding protein 33, placental anticoagulant protein I, thromboplastin inhibitor, vascular anticoagulant-$\alpha$, and anchorin CII, is a 33 kDa calcium-binding protein that is an indirect inhibitor and regulator of tissue factor. Giambanco, I. et al., J. Histochem. Cytochem. 39:P1189-1198, 1991; Doubell, A.F. et al., Cardiovasc. Res. 27:1359-1367, 1993. The normal plasma concentration of annexin V is < 2 ng/ml (Kaneko, N, et al., Clin. Chim. Acta 251:65-80, 1996). One study has found that the plasma concentration of annexin V is elevated in individuals with AMI, but not significantly elevated in patients with old myocardial infarction, chest pain syndrome, valvular heart disease, lung disease, and kidney disease. Kaneko, N. et al., Clin. Chim. Acta 251: 65-80, 1996.

Enolase is a 78 kDa homo- or heterodimeric cytosolic protein produced from $\alpha$, $\beta$, and $\gamma$ subunits. Enolase catalyzes the interconversion of 2-phosphoglycerate and phosphoenolpyruvate in the glycolytic pathway. Enolase is present as $\alpha\alpha$, $\alpha\beta$, $\beta\beta$, $\alpha\gamma$, and $\gamma\gamma$ isoforms. The $\alpha$ subunit is found in most tissues, the $\beta$ subunit is found in cardiac and skeletal muscle, and the $\gamma$ subunit is found primarily in neuronal and neuroendocrine tissues. $\beta$-enolase is composed of $\alpha\beta$ and $\beta\beta$ enolase, and is specific for muscle. $\beta$-enolase is reported to be elevated in the serum of individuals with AMI, but not in individuals with angina (Nomura, M. et al., Br. Heart J. 58:29-33, 1987; Herraez-Dominguez, M.V. et al., Clin. Chim. Acta 64:307-315, 1975). The plasma concentration of $\beta$-enolase is also elevated during heart surgery, muscular dystrophy, and skeletal muscle injury (Usui, A. et al., Cardiovasc. Res. 23:737-740, 1989; Kato, K. et al., Clin. Chim. Acta 131:75-85, 1983; Matsuda, H. et al., Forensic Sci. Int. 99:197-208, 1999).

Creatine kinase (CK) is an 85 kDa cytosolic enzyme that catalyzes the reversible formation ADP and phosphocreatine from ATP and creatine. CK is a homo- or heterodimer composed of M and B chains. CK is composed of 2 subunits, each with a molecular weight of 43 kDa. Three isoenzymes result from various pairings of two different subunits: B (for brain) and M (for muscle). CK-MM predominates in skeletal muscle (approximately 99 percent of total CK) and heart muscle (approximately 55 percent of total CK); CK-BB predominates in brain tissue (over 90 percent of total CK); and CK-MB is most prevalent in heart muscle (up to about 45 percent of total CK). After myocardial infarction, CK-MB levels become elevated within 3 to 8 hours, peak within 9 to 30 hours, and return to normal after 48 to 72 hours.Thygesen, K. et al., Eur. J. Clin. Invest. 16:1-4, 1986; Koukkunen, H. et al., Ann. Med. 30:488-496, 1998; Bertinchant, J.P. et al., Clin. Biochem. 29:587-594, 1996; Benamer, H. et al., Am. J Cardiol. 82:845-850, 1998; Norregaard-Hansen, K. et al., Eur. Heart J. 13:188-193, 1992. CK-MB may be useful in determining the severity of unstable angina because the extent of myocardial ischemia is directly proportional to unstable angina severity.

Glycogen phosphorylase (GP) is a 188 kDa intracellular allosteric enzyme that catalyzes the removal of glucose (liberated as glucose-1-phosphate) from the nonreducing ends of glycogen in the presence of inorganic phosphate during glycogenolysis. GP is present as a homodimer, which associates with another homodimer to form a tetrameric enzymatically active phosphorylase A. There are three isoforms of GP that can be immunologically distinguished. The BB isoform is found in brain and cardiac tissue, the MM isoform is found in skeletal muscle and cardiac tissue, and the LL isoform is predominantly found in liver (Mair, J. et al., Br. Heart J. 72:125-127, 1994). The plasma GP-BB concentration is significantly elevated in patients with AMI and unstable angina with transient ST-T elevations, but not stable angina (Mair, J. et al., Br. Heart J. 72:125-127, 1994; Mair, J., Clin. Chim. Acta 272:79-86, 1998; Rabitzsch, G. et al., Clin. Chem. 41:966-978, 1995; Rabitzsch, G. et al., Lancet 341:1032-1033, 1993). GP-BB also can be used to detect perioperative AMI and myocardial ischemia in patients undergoing coronary artery bypass surgery (Rabitzsch, G. et al., Biomed. Biochim. Acta 46:S584-S588, 1987; Mair, P. et al., Eur.J Clin. Chem. Clin. Biochem. 32:543-547, 1994). Because it is also found in the brain, the plasma GP-BB concentration also may be elevated during ischemic cerebral injury.

Heart-type fatty acid binding protein (H-FABP) is a cytosolic 15 kDa lipid-binding protein involved in lipid metabolism. Heart-type FABP antigen is found not only in heart tissue, but also in kidney, skeletal muscle, aorta, adrenals, placenta, and brain (Veerkamp, J.H. and Maatman, R.G., Prog. Lipid Res. 34:17-52, 1995; Yoshimoto, K. et al., Heart Vessels 10:304-309, 1995). The plasma H-F ABP concentration is elevated in patients with AMI and unstable angina (Ishii, J. et al., Clin. Chem. 43:1372-178, 1997; Tsuji, R. et al., Int. J. Cardiol. 41:209-217, 1993). Myocardial tissue as a source of H-FABP can be confirmed by determining the ratio of myoglobin/FABP (grams/grams). Van Nieuwenhoven, F.A. et al., Circulation 92:2848-2854, 1995. The plasma H-FABP concentration can be significantly elevated 1-2 hours after the onset of chest pain, earlier than CK-MB and myoglobin (Tsuji, R. et al., Int. J. Cardiol. 41:209-217, 1993; Van Nieuwenhoven, F.A. et al., Circulation 92:2848-2854, 1995; Tanaka, T. et al., Clin. Biochem. 24:195-201, 1991).

Phosphoglyceric acid mutase (PGAM) is a 57 kDa homo- or heterodimeric intracellular glycolytic enzyme composed of 29 kDa M or B subunits that catalyzes the interconversion of 3-phosphoglycerate to 2-phosphoglycerate in the presence of magnesium. Cardiac tissue contains isozymes MM, MB, and BB, while skeletal muscle contains primarily PGAM-MM, and most other tissues contain PGAM-BB (Durany, N. and Carreras, J., Comp. Biochem. Physiol. B. Biochem. Mol. Biol. 114:217-223, 1996).

S-100 is a 21 kDa homo- or heterodimeric cytosolic $Ca^{2+}$-binding protein produced from $\alpha$ and $\beta$ subunits. It is thought to participate in the activation of cellular processes along the $Ca^{2+}$-dependent signal transduction pathway (Bonfrer, J.M. et al., Br. J. Cancer 77:2210-2214, 1998). S-100ao ($\alpha\alpha$ isoform) is found in striated muscles, heart and kidney, S-100a ($\alpha\beta$ isoform) is found in glial cells, but not in Schwann cells, and S-100$\beta$ ($\beta\beta$ isoform) is found in high concentrations in glial cells and Schwann cells, where it is a major cytosolic component (Kato, K. and Kimura, S., Biochim.

Biophys. Acta 842:146-150, 1985; Hasegawa, S. et al., Eur. Urol. 24:393-396, 1993). The serum concentration of S-100ao was reported to be elevated in patients with AMI, but not in patients with angina pectoris with suspected AMI (Usui, A. et al., Clin. Chem. 36:639-641, 1990). The serum concentration of S-100ao is significantly elevated on admission in patients with AMI, increases to peak levels 8 hours after admission, decreases and returns to baseline one week later (Usui, A. et al., Clin. Chem. 36:639-641, 1990). Furthermore, S-100ao appears to be significantly elevated earlier after AMI onset than CK-MB (Usui, A. et al., Clin. Chem. 36:639-641, 1990).

*(ii) Exemplary Markers Related To Blood Pressure Regulation*

In addition to ANP, BNP, and markers related thereto, discussed in detail above, the following represent exemplary markers that are known in the art to be related to blood pressure regulation. This list is not meant to be limiting.

C-type natriuretic peptide (CNP) is a 22-amino acid peptide that is the primary active natriuretic peptide in the human brain; CNP is also considered to be an endothelium-derived relaxant factor, which acts in the same way as nitric oxide (NO) (Davidson et al., Circulation 93:1155-9, 1996). CNP is structurally related to Atrial natriuretic peptide (ANP) and B-type natriuretic peptide (BNP); however, while ANP and BNP are synthesized predominantly in the myocardium, CNP is synthesized in the vascular endothelium as a precursor (pro-CNP) (Prickett et al., Biochem. Biophys. Res. Commun. 286:513-7, 2001).

Urotensin II is a peptide having the sequence Ala-Gly-Thr-Ala-Asp-Cys-Phe-Trp-Lys-Tyr-Cys-Val, with a disulfide bridge between Cys6 and Cys 11. Human urotensin 2 (UTN) is synthesized in a prepro form. Processed urotensin 2 has potent vasoactive and cardiostimulatory effects, acting on the G protein-linked receptor GPR14.

Vasopressin (arginine vasopressin, AVP; antidiuretic hormone, ADH) is a peptide hormone released from the posterior pituitary. Its primary function in the body is to regulate extracellular fluid volume by affecting renal handling of water. There are several mechanisms regulating release of AVP. Hypovolemia, as occurs during hemorrhage, results in a decrease in atrial pressure. Specialized stretch receptors within the atrial walls and large veins (cardiopulmonary baroreceptors) entering the atria decrease their firing rate when there is a fall in atrial pressure. Afferent from these receptors synapse within the hypothalamus; atrial receptor firing normally inhibits the release of AVP by the posterior pituitary. With hypovolemia or decreased central venous pressure, the decreased firing of atrial stretch receptors leads to an increase in AVP release. Hypothalamic osmoreceptors sense extracellular osmolarity and stimulate AVP release when osmolarity rises, as occurs with dehydration. Finally, angiotensin II receptors located in a region of the hypothalamus regulate AVP release - an increase in angiotensin II simulates AVP release.

Calcitonin gene related peptide (CGRP) is a polypeptide of 37 amino acids that is a product of the calcitonin gene derived by alternative splicing of the precursor mRNA. The calcitonin gene (CALC-I) primary RNA transcript is processed into different mRNA segments by inclusion or exclusion of different exons as part of the primary transcript. Calcitonin-encoding mRNA is the main product of CALC-I transcription in C-cells of the thyroid, whereas CGRP-I mRNA (CGRP = calcitonin-gene-related peptide) is produced in nervous tissue of the central and peripheral nervous systems. In the third mRNA sequence, the calcitonin sequence is lost and alternatively the sequence of CGRP is encoded in the mRNA. CGRP is a markedly vasoactive peptide with vasodilatative properties. CGRP has no effect on calcium and phosphate metabolism and is synthesised predominantly in nerve cells related to smooth muscle cells of the blood vessels. ProCGRP, the precursor of CGRP, and PCT have partly identical N-terminal amino acid sequences.

Procalcitonin is a 116 amino acid (1.4.5 kDa) protein encoded by the Calc-1 gene located on chromosome 11p15.4. The Calc-1 gene produces two transcripts that are the result of alternative splicing events. Pre-procalcitonin contains a 25 amino acid signal peptide which is processed by C-cells in the thyroid to a 57 amino acid N-terminal fragment, a 32 amino acid calcitonin fragment, and a 21 amino acid katacalcin fragment. Procalcitonin is secreted intact as a glycosylated product by other body cells. Whicher et al., Ann. Clin. Biochem. 38: 483-93 (2001). Plasma procalcitonin has been identified as a marker of sepsis and its severity. Yukioka et al., Ann. Acad. Med. Singapore 30: 528-31 (2001); Pettila et al., Intensive Care Med. 28: 1220-25 (2002).

Angiotensin II is an octapeptide hormone formed by renin action upon a circulating substrate, angiotensinogen, that undergoes proteolytic cleavage to from the decapeptide angiotensin I. Vascular endothelium, particularly in the lungs, has an enzyme, angiotensin converting enzyme (ACE), that cleaves off two amino acids to form the octapeptide, angiotensin II (AII).

Adrenomedullin (AM) is a 52-amino acid peptide which is produced in many tissues, including adrenal medulla, lung, kidney and heart (Yoshitomi et al., Clin. Sci. (Colch) 94:135-9, 1998). Intravenous administration of AM causes a long-lasting hypotensive effect, accompanied with an increase in the cardiac output in experimental animals. AM is synthesized as a precursor molecule (pro-AM). The N-terminal peptide processed from the AM precursor has also been reported to act as a hypotensive peptide (Kuwasako et al., Ann. Clin. Biochem. 36:622-8, 1999).

The endothelins are three related peptides (endothelin-1, endothelin-2, and endothelin-3) encoded by separate genes that are produced by vascular endothelium, each of which exhibit potent vasoconstricting activity. Endothelin-1 (ET-1) is a 21 amino acid residue peptide, synthesized as a 212 residue precursor (preproET-1), which contains a 17 residue signal sequence that is removed to provide a peptide known as big ET-1. This molecule is further processed by hydrolysis between trp21 and va.122 by endothelin converting enzyme. Both big ET-1 and ET-1 exhibit biological activity;

however the mature ET-1 form exhibits greater vasoconstricting activity (Brooks and Ergul, J. Mol. Endocrinol. 21:307-15, 1998). Similarly, endothelin-2 and endothelin-3 are also 21 amino acid residues in length, and are produced by hydrolysis of big endothelin-2 and big endothelin-3, respectively (Yap et al., Br. J. Pharmacol. 129:170-6, 2000; Lee et al., Blood 94:1440-50, 1999).

*(iii)Exemplary Markers Related to Coagulation and Hemostasis*

Elevations in the serum concentration of markers related to coagulation and hemostasis may be associated with clot presence, or any condition that causes or is a result of fibrinolysis activation, including atherosclerosis, disseminated intravascular coagulation, acute myocardial infarction, surgery, trauma, unstable angina, stroke, pulmonary embolsim, venous thrombosis, and thrombotic thrombocytopenic purpura. In addition to D-dimer and TpP, described in detail above, the following are exemplary markers related to coagulation and hemostasis. This list is not meant to be limiting.

Plasmin is a 78 kDa serine proteinase that proteolytically digests crosslinked fibrin, resulting in clot dissolution. The 70 kDa serine proteinase inhibitor $\alpha$2-antiplasmin ($\alpha$2AP) regulates plasmin activity by forming a covalent 1:1 stoichiometric complex with plasmin. The resulting ~150 kDa plasmin-$\alpha$2AP complex (PAP), also called plasmin inhibitory complex (PIC) is formed immediately after $\alpha$2AP comes in contact with plasmin that is activated during fibrinolysis.

$\beta$-thromboglobulin ($\beta$TG) is a 36 kDa platelet $\alpha$ granule component that is released upon platelet activation. Plasma levels of $\beta$-TG appear to be elevated in patients with unstable angina and acute myocardial infarction, but not stable angina (De Caterina, R. et al., Eur. Heart J. 9:913-922, 1988; Bazzan, M. et al., Cardiologia 34, 217-220, 1989). Plasma $\beta$-TG elevations also seem to be correlated with episodes of ischemia in patients with unstable angina (Sobel, M. et al., Circulation 63:300-306, 1981). Plasma concentrations of $\beta$TG associated with ACS can approach 70 ng/ml (2 nM), but this value may be influenced by platelet activation during the sampling procedure.

Platelet factor 4 (PF4) is a 40 kDa platelet $\alpha$ granule component that is released upon platelet activation. PF4 is a marker of platelet activation and has the ability to bind and neutralize heparin. The plasma concentration of PF4 appears to be elevated in patients with acute myocardial infarction and unstable angina, but not stable angina ( Gallino, A. et al., Am. Heart J. 112:285-290, 1986; Sakata, K. et al., Jpn. Circ. J. 60:277-284, 1996; Bazzan, M. et al., Cardiologia 34:217-220, 1989). Plasma PF4 elevations also seem to be correlated with episodes of ischemia in patients with unstable angina (Sobel, M. et al., Circulation 63:300-306, 1981).

Fibrinopeptide A (FPA) is a 16 amino acid, 1.5 kDa peptide that is liberated from amino terminus of fibrinogen by the action of thrombin. The plasma FPA concentration is elevated in patients with acute myocardial infarction, unstable angina, and variant angina, but not stable angina (Gensini, G.F. et al., Thromb. Res. 50:517-525, 1988; Gallino, A. et al., Am. Heart J. 112:285-290, 1986; Sakata, K. et al., Jpn. Circ. J. 60:277-284, 1996; Theroux, P. et al., Circulation 75: 156-162, 1987; Merlini, P.A. et al., Circulation 90:61-68, 1994; Manten, A. et al., Cardiovasc. Res. 40:389-395, 1998). Furthermore, plasma FPA may indicate the severity of angina (Gensini, G.F. et al., Thromb. Res. 50:517-525, 1988).

Platelet-derived growth factor (PDGF) is a 28 kDa secreted homo- or heterodimeric protein composed of the homologous subunits A and/or B (Mahadevan, D. et al., J. Biol. Chem. 270:27595-27600, 1995). PDGF is released by aggregating platelets and monocytes near sites of vascular injur, and has been implicated in the pathogenesis of atherosclerosis. Plasma PDGF concentrations are higher in individuals with acute myocardial infarction and unstable angina than in healthy controls or individuals with stable angina (Ogawa, H. et al., Am. J. Cardiol. 69:453-456, 1992; Wallace, J.M. et al., Ann. Clin. Biochem. 35:236-241, 1998; Ogawa, H. et al., Coron. Artery Dis. 4:437-442, 1993).

Prothrombin fragment 1+2 is a 32 kDa polypeptide that is liberated from the amino terminus of thrombin during thrombin activation. The plasma concentration of F1+2 is reportedly elevated in patients with acute myocardial infarction and unstable angina, but not stable angina (Merlini, P.A. et al., Circulation 90:61-68, 1994). Other reports have indicated that there is no significant change in the plasma F1+2 concentration in cardiovascular disease (Biasucci, L.M. et al., Circulation 93:2121-2127, 1996; Manten, A. et al., Cardiovasc. Res. 40:389-395, 1998).

P-selectin, also called granule membrane protein-140, GIVIP-140, PADGEM, and CD-62P, is a ~140 kDa adhesion molecule expressed in platelets and endothelial cells. P-selectin is stored in the alpha granules of platelets and in the Weibel-Palade bodies of endothelial cells. Membrane-bound and soluble forms of P-selectin have been identified. Soluble P-selectin may play an important role in regulating inflammation and thrombosis by blocking interactions between leukocytes and activated platelets and endothelial cells (Gamble, J.R. et al., Science 249:414-417, 1990). The plasma soluble P-selectin concentration was significantly elevated in patients with acute myocardial infarction and unstable angina, but not stable angina, even following an exercise stress test (Ikeda, H. et al., Circulation 92:1693-1696, 1995; Tomoda, H. and Aoki, N., Angiology 49:807-813, 1998; Hollander, J.E. et al., J. Am. Coll. Cardiol. 34:95-105, 1999; Kaikita, K. et al., Circulation 92:1726-1730, 1995; Ikeda, H. et al., Coron. Artery Dis. 5:515-518, 1994). The sensitivity and specificity of membrane-bound P-selectin versus soluble P-selectin for acute myocardial infarction is 71% versus 76% and 32% versus 45% (Hollander, J.E. et al., J. Am. Coll. Cardiol. 34:95-105, 1999). The sensitivity and specificity of membrane-bound P-selectin versus soluble P-selectin for unstable angina + acute myocardial infarction is 71% versus 79% and 30% versus 35% (Hollander, J.E. et al., J. Am. Coll. Cardiol. 34:95-105, 1999).

Thrombin is a 37 kDa serine proteinase that proteolytically cleaves fibrinogen to form fibrin, which is ultimately integrated into a crosslinked network during clot formation. Antithrombin III (ATIII) is a 65 kDa serine proteinase inhibitor that is a physiological regulator of thrombin, factor XIa, factor XIIa, and factor IXa proteolytic activity. The normal plasma concentration of the approximately 100 kDa thrombin-ATIII complex (TAT) is < 5 ng/ml (50 pM). TAT concentration is elevated in patients with acute myocardial infarction and unstable angina, especially during spontaneous ischemic episodes (Biasucci, L.M. et al., Am. J. Cardiol. 77:85-87, 1996; Kienast, J. et al., Thromb. Haemost. 70:550-553, 1993). Furthermore, TAT may be elevated in the plasma of individuals with stable angina (Manten, A. et al., Cardiovasc. Res. 40:389-395, 1998). Other published reports have found no significant differences in the concentration of TAT in the plasma of patients with ACS (Manten, A. et al., Cardiovasc. Res. 40:389-395, 1998; Hoffmeister, H.M. et al., Atherosclerosis 144:151-157, 1999).

von Willebrand factor (vWF) is a plasma protein produced by platelets, megakaryocytes, and endothelial cells composed of 220 kDa monomers that associate to form a series of high molecular weight multimers. These multimers normally range in molecular weight from 600-20,000 kDa. The A1 domain of vWF binds to the platelet glycoprotein 1b-IX-V complex and non-fibrillar collagen type VI, and the A3 domain binds fibrillar collagen types I and III (Emsley, J. et al., J. Biol. Chem. 273:10396-10401, 1998). Other domains present in the vWF molecule include the integrin binding domain, which mediates platelet-platelet interactions, the the protease cleavage domain, which appears to be relevant to the pathogenesis of type 1 lA von Willebrand disease. Measurement of the total amount of vWF would allow one who is skilled in the art to identify changes in total vWF concentration. This measurement could be performed through the measurement of various forms of the vWF molecule. Measurement of the A 1 domain would allow the measurement of active vWF in the circulation, indicating that a pro-coagulant state exists because the A1 domain is accessible for platelet binding. In this regard, an assay that specifically measures vWF molecules with both the exposed A 1 domain and either the integrin binding domain or the A3 domain would also allow for the identification of active vWF that would be available for mediating platelet-platelet interactions or mediate crosslinking of platelets to vascular subendothelium, respectively.

Tissue factor (TF) is a 45 kDa cell surface protein expressed in brain, kidney, and heart, and in a transcriptionally regulated manner on perivascular cells and monocytes. Tissue factor can be detected in the bloodstream in a soluble form, bound to factor VIIa, or in a complex with factor VIIa, and tissue factor pathway inhibitor that can also include factor Xa, TF also is expressed on the surface of macrophages, which are commonly found in atherosclerotic plaques. TF is elevated in patients with unstable angina and acute myocardial infarction, but not in patients with stable angina (Falciani, M. et al., Thromb. Haemost. 79:495-499, 1998; Suefuji, H. et al., Am. Heart J. 134:253-259, 1997; Misumi, K. et al., Am. J. Cardiol. 81:22-26, 1998). Furthermore, TF expression on macrophages and TF activity in atherosclerotic plaques is more common in unstable angina than stable angina (Soejima, H. et al., Circulation 99:2908-2913, 1999; Kaikita, K. et al., Arterioscler. Thromb. Vasc. Biol. 17:2232-2237, 1997; Ardissino, D. et al., Lancet 349:769-771, 1997).

*(iv) Exemplary Markers Related to the Acute Phase Response*

Acute phase proteins are a group of proteins, such as C-reactive protein and mannose-binding protein, produced by cells in the liver and that promote inflammation, activate the complement cascade, and stimulate chemotaxis of phagocytes. In addition to MCP-1, described in detail above, the following are exemplary markers related to the acute phase response. This list is not meant to be limiting.

Human neutrophil elastase (HNE) is a 30 kDa serine proteinase that is normally contained within the azurophilic granules of neutrophils. HNE is released upon neutrophil activation, and its activity is regulated by circulating $\alpha_1$-proteinase inhibitor. The plasma HNE concentration is usually measured by detecting HNE-$\alpha_1$-PI complexes. The normal concentration of these complexes is 50 ng/ml, which indicates a normal concentration of approximately 25 ng/ml (0.8 nM) for HNE. HNE release also can be measured through the specific detection of fibrinopeptide B$\beta_{30-43}$, a specific HNE-derived fibrinopeptide, in plasma. Plasma HNE is elevated in patients with coronary stenosis, and its elevation is greater in patients with complex plaques than those with simple plaques (Kosar, F. et al., Angiology 49:193-201, 1998; Amaro, A. et al., Eur. Heart J. 16:615-622, 1995). Plasma HNE is not significantly elevated in patients with stable angina, but is elevated inpatients with unstable angina and acute myocardial infarction, as determined by measuring fibrinopeptide B$\beta_{30-43}$, with concentrations in unstable angina being 2.5-fold higher than those associated with acute myocardial infarction (Dinerman, J.L. et al., J. Am. Coll. Cardiol. 15: 1559-1563, 1990; Mehta, J. et al., Circulation 79:549-556, 1989).

Inducible nitric oxide synthase (iNOS) is a 130 kDa cytosolic protein in epithelial cells macrophages whose expression is regulated by cytokines, including interferon-$\gamma$, interleukin-1$\beta$, interleukin-6, and tumor necrosis factor $\alpha$, and lipopolysaccharide. iNOS catalyzes the synthesis of nitric oxide (NO) from L-arginine, and its induction results in a sustained high-output production of NO, which has antimicrobial activity and is a mediator of a variety of physiological and inflammatory events. NO production by iNOS is approximately 100 fold more than the amount produced by constitutively-expressed NOS (Depre, C. et al., Cardiovasc. Res. 41:465-472, 1999). iNOS expression during myocardial ischemia may not be elevated, suggesting that iNOS may be useful in the differentiation of angina from acute myocardial infarction (Hammerman, S.I. et al., Am. J. Physiol. 277:H1579-H1592, 1999; Kaye, D.M. et al., Life Sci 62:883-887, 1998).

Lysophosphatidic acid (LPA) is a lysophospholipid intermediate formed in the synthesis of phosphoglycerides and triacylglycerols. In the context of unstable angina, LPA is most likely released as a direct result of plaque rupture..

Malondialdehyde-modified low-density lipoprotein (MDA-modified LDL) is formed during the oxidation of the apoB-100 moiety of LDL as a result of phospholipase activity, prostaglandin synthesis, or platelet activation. Plasma concentrations of oxidized LDL are elevated in stable angina, unstable angina, and acute myocardial infarction, indicating that it may be a marker of atherosclerosis (Holvoet, P., Acta Cardiol. 53:253-260, 1998; Holvoet, P. et al., Circulation 98:1487-1494, 1998). Plasma MDA-modified LDL is not elevated in stable angina, but is significantly elevated in unstable angina and acute myocardial infarction (Holvoet, P., Acta Cardiol. 53:253-260, 1998; Holvoet, P. et al., Circulation 98: 1487-1494, 1998; Holvoet, P. et al., JAMA 281:1718-1721, 1999). Plasma concentrations ofMDA-modified LDL can approach 20 $\mu$g/ml (~50 $\mu$M) in patients with acute myocardial infarction, and 15 $\mu$g/ml (~40 $\mu$M) in patients with unstable angina (Holvoet, P. et al., Circulation 98:1487-1494, 1998).

Matrix metalloproteinase-1 (MMP-1), also called collagenase-1, is a 41/44 kDa zinc- and calcium-binding proteinase that cleaves primarily type I collagen, but can also cleave collagen types II, III, VII and X. The active 41/44 kDa enzyme can undergo autolysis to the still active 22/27 kDa form. MMP-1 can be found in the bloodstream either in a free form or in complex with TIMP-1, its natural inhibitor. MMP-1 is found in the shoulder region of atherosclerotic plaques, which is the region most prone to rupture, and may be involved in atherosclerotic plaque destabilization (Johnson, J.L. et al., Arterioscler. Thromb. Vasc. Biol. 18:1707-1715, 1998). Furthermore, MMP-1 has been implicated in the pathogenesis of myocardial reperfusion injury (Shibata, M. et al., Angiology 50:573-582, 1999).

Lipopolysaccharide binding protein (LBP) is a ~ 60 kDa acute phase protein produced by the liver. LBP binds to lipopolysaccharide and is involved in LPS handling in humans. LBP has been reported to mediate transfer of LPS to the LPS receptor (CD14) on mononuclear cells, and into HDL. LBP has also been reported to protect mice from septic shock caused by LPS.

Matrix metalloproteinase-2 (MMP-2), also called gelatinase A, is a 66 kDa zinc- and calcium-binding proteinase that is synthesized as an inactive 72 kDa precursor. Mature MMP-3 cleaves type I gelatin and collagen of types IV, V, VII, and X. MMP-2 is usually found in plasma in complex with TIMP-2, its physiological regulator (Murawaki, Y. et al., J. Hepatol. 30:1090-1098, 1999). MMP-2 expression is elevated in vascular smooth muscle cells within atherosclerotic lesions, and it may be released into the bloodstream in cases of plaque instability (Kai, H. et al., J. Am. Coll. Cardiol. 32:368-372, 1998). Serum MMP-2 concentrations were elevated in patients with stable angina, unstable angina, and acute myocardial infarction, with elevations being significantly greater in unstable angina and acute myocardial infarction than in stable angina (Kai, H. et al., J. Am. Coll. Cardiol. 32:368-372, 1998). MMP-2 was elevated on admission in the serum of individuals with unstable angina and acute myocardial infarction, with maximum levels approaching 1.5 $\mu$g/ml (25 nM) (Kai, H. et al., J. Am. Coll. Cardiol. 32:368-372, 1998).

Matrix metalloproteinase-3 (MMP-3), also called stromelysin-1, is a 45 kDa zinc- and calcium-binding proteinase that is synthesized as an inactive 60 kDa precursor. The serum MMP-3 concentration in males is approximately 2 times higher than in females (Manicourt, D.H. et al., Arthritis Rheum. 37:1774-1783, 1994). MMP-3 is found in the shoulder region of atherosclerotic plaques, which is the region most prone to rupture, and may be involved in atherosclerotic plaque destabilization (Johnson, J.L. et al., Arterioscler. Thromb. Vasc. Biol. 18:1707-1715, 1998

Matrix metalloproteinase-9 (MMP-9) also called gelatinase B, is an 84 kDa zinc- and calcium-binding proteinase that is synthesized as an inactive 92 kDa precursor. MMP-9 exists as a monomer, a homodimer, and a heterodimer with a 25 kDa $\alpha_2$-microglobulin-related protein (Triebel, S. et al., FEBS Lett. 314:386-388, 1992). Plasma MMP-9 concentrations are significantly elevated in patients with unstable angina and acute myocardial infarction, but not stable angina (Kai, H. et al., J. Am. Coll. Cardiol. 32:368-372, 1998).

The balance between matrix metalloproteinases and their inhibitors is a critical factor that affects tumor invasion and metastasis. The TIMP family represents a class of small (21-28 kDa) related proteins that inhibit the metalloproteinases. Tissue inhibitor of metalloproteinase 1 (TIMP1) is reportedly involved in the regulation of bone modeling and remodeling in normal developing human bone, involved in the invasive phenotype of acute myelogenous leukemia, demonstrating polymorphic X-chrouiosome inactivation. TIMP1 is known to act on MMP-1, MMP-2, MMP-3, MMP-7, MMP-8, MMP-9, MMP-10, MMP-11, N1MP-12, MMP-13 and MMP-16. Tissue inhibitor of metalloproteinase 2 (TIMP2) complexes with metalloproteinases (such as collagenases) and irreversibly inactivates them. TIMP 2 is known to act on MMP-1, MMP-2, MMP-3, MMP-7, MMP-8, MMP-9, MMP-10, MMP-13, MMP-14, MMP-15, MMP-16 and MMP-19. Two alternatively spliced forms may be associated with SYN4, and involved in the invasive phenotype of acute myelogenous leukemia. Unlike the inducible expression of some other TIMP gene family members, the expression of this gene is largely constitutive. Tissue inhibitor of metalloproteinase 3 (TIMP3) antagonizes matrix metalloproteinase activity and can suppress tumor growth, angiogenesis, invasion, and metastasis. Loss of TIMP-3 has been related to the acquisition of tumorigenesis.

*(v) Exemplary Markers Related to Inflammation*

Acute phase proteins are part of a larger group of proteins that are related to local or systemic inflammation. The following exemplary list of additional markers related to inflammation is not meant to be limiting.

Interleukins (ILs) are part of a larger class of polypeptides known as cytokines. These are messenger molecules that transmit signals between various cells of the immune system. They are mostly secreted by macrophages and lymphocytes and their production is induced in response to injury or infection. Their actions influence other cells of the immune system as well as other tissues and organs including the liver and brain. There are at least 18 ILs described. IL-1β, IL-2, IL-4. IL-6, IL-8 and IL-10 are preferred for use as markers in the present invention. The following table shows selected functions of representative intcrleukins.

Table 1: Selected Functions of Representative Interleukins*

| Functions | IL-1 | IL-2 | IL-4 | IL-6 | IL-8 | IL-10 |
|---|---|---|---|---|---|---|
| Enhance immune responses | + | + | + | + | - | + |
| Suppress immune responses | - | - | - | - | - | + |
| Enhance inflammation | + | + | + | + | + | - |
| Suppress inflammation | - | - | - | - | - | + |
| Promote cell growth | + | + | - | - | - | - |
| Chemotactic (chemokines) | - | - | - | - | + | - |
| Pyrogenic | + | - | - | - | - | - |

Interleukin-1β (IL-1β) is a 17 kDa secreted proinflammatory cytokine that is involved in the acute phase response and is a pathogenic mediator of many diseases. IL-1β is normally produced by macrophages and epithelial cells. IL-1β is also released from cells undergoing apoptosis. Elevations of the plasma IL-1β concentration are associated with activation of the acute phase response in proinflammatory conditions such as trauma and infection.

Interleukin-1 receptor antagonist (IL-1ra) is a 17 kDa member of the IL-1 family predominantly expressed in hepatocytes, epithelial cells, monocytes, macrophages, and neutrophils. IL-1ra has both intracellular and extracellular forms produced through alternative splicing. IL-1ra is thought to participate in the regulation of physiological IL-1 activity. The plasma concentration of IL-lra is elevated in patients with acute myocardial infarction and unstable angina that proceeded to acute myocardial infarction, death, or refractory angina (Biasucci, L.M. et al., Circulation 99:2079-2084, 1999; Latini, R. et al., J. Cardiovasc. Pharmacol. 23:1-6, 1994). Furthermore, IL-1ra was significantly elevated in severe acute myocardial infarction as compared to uncomplicated acute myocardial infarction (Latini, R. *et al., J. Cardiovasc. Pharmacol.* 23:1-6, 1994).

Interleulcin-6 (IL-6) is a 20 kDa secreted protein that is a hematopoietin family proinflammatory cytokine. Its major function is to mediate the acute phase production of hepatic proteins, and its synthesis is induced by the cytokine IL-1. IL-6 is normally produced by macrophages and T lymphocytes. The plasma concentration of IL-6 is elevated in patients with acute myocardial infarction and unstable angina, to a greater degree in acute myocardial infarction (Biasucci, L.M. et al., Circulation 94:874-877, 1996; Manten, A. et al., Cardiovasc. Res. 40:389-395, 1998; Biasucci, L.M. et al., Circulation 99:2079-2084, 1999). IL-6 is not significantly elevated in the plasma of patients with stable angina (Biasucci, L.M. et al., Circulation 94:874-877, 1996; Manten, A. et al., Cardiovasc. Res. 40:389-395, 1998). The plasma concentration of IL-6 is elevated within 8-12 hours of acute myocardial infarction onset, and can approach 100 pg/ml. The plasma concentration of IL-6 in patients with unstable angina was elevated at peak levels 72 hours after onset, possibly due to the severity of insult (Biasucci, L.M. et al., Circulation 94:874-877, 1996).

Interleukin-8 (IL-8) is a 6.5 kDa chemokine produced by monocytes, endothelial cells, alveolar macrophages and fibroblasts. IL-8 induces chemotaxis and activation of neutrophils and T cells.

Tumor necrosis factor α (TNFα) is a 17 kDa secreted proinflammatory cytokine that is involved in the acute phase response and is a pathogenic mediator of many diseases. TNF-alpha is a protein of 185 amino acids glycosylated at positions 73 and 172. It is synthesized as a precursor protein of 212 amino acids. Monocytes express at least five different molecular forms of TNF-alpha with molecular masses of 21.5-28 kDa. They mainly differ by post-translational alterations such as glycosylation and phosphorylation. The normal serum concentration of TNFα is < 40 pg/ml (2 pM). The plasma concentration of TNFα is elevated in patients with acute myocardial infarction, and is marginally elevated in patients with unstable angina (Li, D. et al., Am. Heart J. 137:1145-1152, 1999; Squadrito, F. et al., Inflamm. Res. 45: 14-19, 1996; Latini, R. et al., J Cardiovasc. Pharmacol. 23:1-6, 1994; Carlstedt, F. et al., J. Intern. Med. 242:361-365, 1997). The concentration of TNFα in the plasma of acute myocardial infarction patients exceeded 300 pg/ml (15 pM) (Squadrito, F. et al., Inflamm. Res. 45:14-19, 1996).

Soluble intercellular adhesion molecule (sICAM-1), also called CD54, is a 85-110 kDa cell surface-bound immunoglobulin-like integrin ligand that facilitates binding of leukocytes to antigen-presenting cells and endothelial cells during leukocyte recruitment and migration. The plasma concentration of sICAM-1 is significantly elevated in patients with acute myocardial infarction and unstable angina, but not stable angina (Pellegatta, F. et al., J Cardiovasc. Pharmacol. 30:455-460, 1997; Miwa, K. et al., Cardiovasc. Res. 36:37-44, 1997; Ghaisas, N.K. et al., Am. J. Cardiol. 80:617-619, 1997; Ogawa, H. et al., Am. J. Cardiol. 83:38-42, 1999). Furthermore, ICAM-1 is expressed in atherosclerotic lesions and in areas predisposed to lesion formation, so it may be released into the bloodstream upon plaque rupture (Iiyama, K. et al., Circ. Res. 85:199-207, 1999; Tenaglia, A.N. et al., Am. J. Cardiol. 79:742-747, 1997). Additional ICAM molecules are well known in the art, including ICAM-2 (also called CD102) and ICAM-3 (also called CD50), which may also be present in the blood.

Vascular cell adhesion molecule (VCAM), also called CD106, is a 100-110 kDa cell surface-bound immunoglobulin-like integrin ligand that facilitates binding of B lymphocytes and developing T lymphocytes to antigen-presenting cells during lymphocyte recruitment. The plasma concentration of sVCAM-1 is marginally elevated in patients with acute myocardial infarction, unstable angina, and stable angina (Mulvihill, N. et al., Am. J. Cardiol. 83:1265-7, A9, 1999; Ghaisas, N.K. et al., Am. J. Cardiol. 80:617--619, 1997). However, sVCAM-1 is expressed in atherosclerotic lesions and its plasma concentration may correlate with the extent of atherosclerosis (Iiyama, K. et al., Circ. Res. 85:199-207, 1999; Peter, K. et al., Arterioscler. Thromb. Vasc. Biol. 17:505-512, 1997).

Macrophage migration inhibitory factor (M1F) is a lymphokine involved in cell-mediated immunity, immunoregulation, and inflammation. Like TNF$\alpha$ and IL-1$\beta$, MIF plays a central role in the host response to endotoxemia. Coinjection of recombinant MIF and LPS exacerbates LPS lethality, whereas neutralizing anti-MIF antibodies fully protect mice from endotoxic shock.

Hemoglobin (Hb) is an oxygen-carrying iron-containing globular protein found in erythrocytes. It is a heterodimer of two globin subunits. $\alpha_2\gamma_2$ is referred to as fetal Hb, $\alpha_2\beta_2$ is called adult HbA, and $\alpha_2\delta_2$ is called adult HbA$_2$. 90-95% of hemoglobin is HbA, and the $\alpha_2$ globin chain is found in all Hb types, even sickle cell hemoglobin. Hb is responsible for carrying oxygen to cells throughout the body. Hb$\alpha_2$ is not normally detected in serum.

Oxysterols (oxidized derivatives of cholesterol) and oxidized lipoproteins have been identified in atherosclerotic lesions, and are suggested to play a role in the pathogenesis of coronary heart disease. *See, e.g.,* Staprans et al., Arterioscler. Thromb. Vasc. Biol. 20: 708-14, 2000. Recently, an aldol condensation product believed to be formed by ozonolysis of cholesterol in atherosclerotic plaques was reported to be detectable in plasma from subjects with advanced atherosclerotic disease. It was suggested that this molecule may be a marker of arterial inflammation in atherosclerosis. Wentworth et al., Science 302: 1053-6, 2003. This publication is hereby incorporated by reference in its entirety.

Human lipocalin-type prostaglandin D synthase (hPDGS), also called $\beta$-trace, is a 30 kDa glycoprotein that catalyzes the formation of prostaglandin D2 from prostaglandin H. Elevations of hPDGS have been identified in blood from patients with unstable angina and cerebral infarction (Patent No. EP0999447A1). Furthermore, hPDGS appears to be a useful marker of ischemic episodes (Patent No. EP0999447A1).

Mast cell tryptase, also known as alpha tryptase, is a 275 amino acid (30.7 kDa) protein that is the major neutral protease present in mast cells. Mast cell tryptase is a specific marker for mast cell activation, and is a marker of allergic airway inflammation in asthma and in allergic reactions to a diverse set of allergens. *See, e.g.,* Taira et al., J. Asthma 39: 315-22 (2002); Schwartz et al., N. Engl. J. Med. 316: 1622-26 (1987). Elevated serum tryptase levels (> 1 ng/mL) between 1 and 6 hours after an event provides a specific indication of mast cell degranulation.

Eosinophil cationic protein (ECP) is a heterogeneous protein with molecular weight variants from 16-24 kDa and a pI of pH 10.8. Assessment of serum ECP may be assumed to reflect pulmonary inflammation in bronchial asthma. Koller et al., Arch. Dis. Childhood 73: 413-7 (1995); *see also,* Sorkness et al., Clin. Exp. Allergy 32: 1355-59 (2002); Badr-elDin et al., East Mediterr. Health J. 5: 664-75 (1999).

Interleukin 10 ("IL-10") is a 160 amino acid (18.5 kDa predicted mass) cytokine that is a member of the four $\alpha$-helix bundle family of cytokines. In solution, IL-10 forms a homodimer having an apparent molecular weight of 39 kDa. The human IL-10 gene is located on chromosome 1. Viera et al., Proc. Natl. Acad Sci. USA 88: 1172-76 (1991); Kim et al., J. Immunol. 148: 3618-23 (1992). Overproduction of IL-10 has been identified as a marker in sepsis, and is predictive of severity and mortality. Gogos et al., J. Infect. Dis. 181: 176-80 (2000).

*(vi) Exemplary Markers of Pulmonary Injury*

KL-6 (also referred to as MUC1) is a high molecular weight (> 300 kDa) mucinous glycoprotein expressed on pneumonocytes. Serum levels of KL-6 are reportedly elevated in interstitial lung diseases, which are characterized by exertional dyspnea. KL-6 has been shown to be a marker of various interstitial lung diseases, including pulmonary fibrosis, interstitial pneumonia, sarcoidosis, and interstitial pneumonitis. *See, e.g.,* Kobayashi and Kitamura, Cbest 108: 311-15 (1995); Kohno, J. Med. Invest. 46: 151-58 (1999); Bandoh et al., Ann. Rheum. Dis. 59: 257-62 (2000); and Yamane et al., J. Rheumatol. 27: 930-4 (2000).

Surfactant proteins are a family of apoproteins, which are associated in a complex with phospholipids. There are four main surfactant proteins, known as SP-A, B, C, and D. Various of the surfactant proteins have been associated with pulmonary disease. *See, e.g.,* Doyle et al., Am. J. Respir. Crit. Care Med. 156: 1217-29, 1997; Bersten et al., Am. J. Respir. Crit. Care Med.164: 648-52, 2001; Suwabe, Ribnsho Byori 50: 1061-66, 2002; Cheng et al., Crit. Care Med. 31: 311-13, 2003; Hastings, J. Clin. Monit. Comput. 16: 385-92, 2000.

Neutrophil elastase, a proteolytic enzyme, has long been measured as a marker of pulmonary injury, both in the systemic circulation and in bronchoalveolar lavage fluid. *See, e.g.*, Moraes et al., Crit. Care Med. 31(4 Suppl): S189-94, 2003

The products associated with the breakdown of type IV collagen (a main constituent of basement membrane), such as the 7S protein fragment of collagen, have been used to mark lung injury. Increased 7S protein levels have been shown to be associated with high matrix metalloproteinase (MMP; proteolytic enzyme) and neutrophil concentrations in the bronchoalveolar lavage fluid of patients after they have undergone cardiopulmonary bypass. *See*, *e.g.,* Owen et al., Am. J. Respir. Cell Mol. Biol. 29: 283-94, 2003.

*(vii) Exemplary Specific Markers of Neural Tissue Injury*

In the case where a vascular disease affects tissues other than myocardium (e.g., in stroke), specific markers of tissue damage other than markers of myocardial tissue damage may be particularly useful. Considering stroke as an example, the following list of exemplary specific markers of neural tissue injury is provided. This list is not meant to be limiting.

Adenylate kinase (AK) is a ubiquitous 22 kDa cytosolic enzyme that catalyzes the interconversion of ATP and AMP to ADP. Four isoforms of adenylate kinase have been identified in mammalian tissues (Yoneda, T. et al., Brain Res Mol Brain Res 62:187-195, 1998). The AK isoform is found in brain, skeletal muscle, heart, and aorta. Serum AK1 appears to have the greatest specificity of the AK isoforms as a marker of neural tissue injury. AK may be best suited as a cerebrospinal fluid marker of cerebral ischemia, where its dominant source would be neural tissue.

Neurotrophins are a family of growth factors expressed in the mammalian nervous system. Some examples include nerve growth factor (NGF), brain-derived neurotrophic factor (BDNF), neurotrophin-3 (NT-3) and neurotrophin-4/5 (NT-4/5). Neurotrophins exert their effects primarily as target-derived paracrine or autocrine neurotrophic factors. The role of the neurotrophins in survival, differentiation and maintenance of neurons is well known. They exhibit partially overlapping but distinct patterns of expression and cellular targets. In addition to the effects in the central nervous system, neurotrophins also affect peripheral afferent and efferent neurons.

BDNF is a potent neurotrophic factor which supports the growth and survivability of nerve and/or glial cells. BDNF is expressed as a 32 kDa precursor "pro-BDNF" molecule that is cleaved to a mature BDNF form. Mowla et al., J. Biol. Chem. 276: 12660-6 (2001). The most abundant active form of human BDNF is a 27 kDa homodimer, formed by two identical 119 amino acid subunits, which is held together by strong hydrophobic interactions; however, pro-BDNF is also released extracellularly and is biologically active.

NT-3 is also a 27 kDa homodimer consisting of two 119-amino acid subunits. The addition of NT-3 to primary cortical cell cultures has been shown to exacerbate neuronal death caused by oxygen-glucose deprivation, possible via oxygen free radical mechanisms (Bates et al., Neurobiol. Dis. 9:24-37, 2002). NT-3 is expressed as an inactive pro-NT-3 molecule, which is cleaved to the mature biologically active form.

Calbindin-D is a 28 kDa cytosolic vitamin D-dependent $Ca^{2+}$-binding protein that may serve a cellular protective function by stabilizing intracellular calcium levels. Calbindin-D is found in the central nervous system, mainly in glial cells, and in cells of the distal renal tubule (Hasegawa, S. et al., J. Urol. 149:1414-1418, 1993). The normal serum concentration of calbindin-D is <20 pg/ml (0.7 pM). Serum calbindin-D concentration is reportedly elevated following cardiac arrest, and this elevation is thought to be a result of CNS damage due to cerebral ischemia (Usui, A. et al., J Neurol. Sci. 123:134-139, 1994). Elevations of serum calbindin-D are elevated and plateau soon after reperfusion following ischemia. Maximum serum calbindin-D concentrations can be as much as 700 pg/ml (25 pM).

Creatine kinase (CK) is a cytosolic enzyme that catalyzes the reversible formation of ADP and phosphocreatine from A'Tf' and creatine. The brain-specific CK isoform (CK-BB) is an 85 kDa cytosolic protein that accounts for approximately 95% of the total brain CK activity. It is also present in significant quantities in cardiac tissue, intestine, prostate, rectum, stomach, smooth muscle, thyroid uterus, urinary bladder, and veins (Johnsson, P. J., Cardiothorac. Vasc. Anesth. 10: 120-126, 1996). Elevations of CK-BB in serum can be attributed to neural tissue injury due to ischemia, coupled with increased permeability of the blood brain barrier. In severe stroke, serum concentrations CK-13B are elevated and peak soon after the onset of stroke (within 24 hours), gradually returning to normal after 3-7 days (4).

Glial fibrillary acidic protein (GFAP) is a 55 kDa cytosolic protein that is a major structural component of astroglial filaments and is the major intermediate filament protein in astrocytes. CFAP is specific to astrocytes, which are interstitial cells located in the CNS and can be found near the blood-brain barrier. Serum GFAP is elevated following ischemic stroke (Niebroj-Dobosz, I., et al., Folia Neuropathol. 32:129-137, 1994). Serum concentrations GFAP appear to be elevated soon after the onset of stroke, continuously increase and persist for an amount of time (weeks) that may correlate with the severity of damage.

Lactate dehydrogenase (LDH) is a ubiquitous 135 kDa cytosolic enzyme. It is a tetramer of A and B chains that catalyzes the reduction of pyruvate by NADH to lactate. Five isoforms of LDH have been identified in mammalian tissues, and the tissue-specific isoforms are made of different combinations of A and B chains. Elevations in serum LDH activity are reported following both ischemic and hemorrhagic stroke, but further studies are needed in serum to confirm this observation and to determine a correlation with the severity of injury and neurological outcome (Aggarwal, S.P. et al., J. Indian Med. Assoc. 93:331-332, 1995; Maiuri, F. et al., Neurol. Res. 11:6-8, 1989).

Myelin basic protein (MBP) is actually a 14-21 kDa family of cytosolic proteins generated by alternative splicing of a single MBP gene that is likely involved in myelin compaction around axons during the myelination process. MBP is specific to oligodendrocytes in the CNS and in Schwann cells of the peripheral nervous system (PNS). Serum MBP is elevated after all types of severe stroke, specifically thrombotic stroke, embolic stroke, intracerebral hemorrhage, and subarachnoid hemorrhage, while elevations in MI3P concentration are not reported in the serum of individuals with strokes of minor to moderate severity, which would include lacunar infarcts or transient ischemic attacks (Palfreyman, J.W. et al., Clin. Chim. Acta 92:403-409, 1979). The serum concentration of MBP has been reported to correlate with the extent of damage (infarct volume), and it may also correlate with neurological outcome.

Neural cell adhesion molecule (NCAM), also called CD56, is a 170 kDa cell surface-bound immunoglobulin-like integrin ligand that is involved in the maintenance of neuronal and glial cell interactions in the nervous system, where it is expressed on the surface of astrocytes, oligodendrocytes, Schwann cells, neurons, and axons. NCAM is also localized to developing skeletal muscle myotubes, and its expression is upregulated in skeletal muscle during development, denervation and renervation.

Proteolipid protein (PLP) is a 30 kDa integral membrane protein that is a major structural component of CNS myelin. PLP is specific to oligodendrocytes in the CNS and accounts for approximately 50% of the total CNS myelin protein in the central sheath, although extremely low levels of PLP have been found (<1%) in peripheral nervous system (PNS) myelin. Serum PLP is elevated after cerebral infarction, but not after transient ischemic attack (Trotter, J.L. et al., Ann. Neurol. 14:554-558, 1983). Elevations of PLP in serum can be attributed to neural tissue injury due to physical damage or ischemia caused by infarction or cerebral hemorrhage, coupled with increased permeability of the blood brain barrier.

S-100$\beta$ is elevated in serum after 4 hours from stroke onset, with concentrations reaching a maximum 2-3 days after onset. After the serum concentration reaches its maximum, which can approach 20 ng/ml (1.9 mM), it gradually decreases to normal over approximately one week. Because the severity of damage has a direct effect on the release of S-100$\beta$, it will affect the release kinetics by influencing the length of time that S-100$\beta$ is elevated in the serum. S-100$\beta$ will be present in the serum for a longer period of time as the severity of injury increases. Furthermore, elevated serum concentrations of S-100$\beta$ can indicate complications related to neural tissue injury after AMI and cardiac surgery.

Thrombomodulin (TM) is a 70 kDa single chain integral membrane glycoprotein found on the surface of vascular endothelial cells. Current reports describing serum TM concentration alterations following ischemic stroke are mixed, reporting no changes or significant increases (Seki, Y. et al., Blood Coagul. Fibrinolysis 8:391-396, 1997). Serum elevations of TM concentration reflect endothelial cell injury and would not indicate coagulation or fibrinolysis activation.

The gamma isoform of protein kinase C (PKCg) is specific for CNS tissue and is not normally found in the circulation. PKCg is activated during cerebral ischemia and is present in the ischemic penumbra at levels 2-24-fold higher than in contralateral tissue, but is not elevated in infarcted tissue (Krupinski, J. *et al., Acta Neurobiol. Exp. (Warz)* **58**:13-21, 1998). Additional isoforms ofPKC, beta I and beta II were found in increased levels in the infarcted core of brain tissue from patients with cerebral ischemia ( Krupinski, J. et al., Acta Neurobiol. Exp. (Warz) 58:13-21, 1998). Furthermore, the alpha and delta isoforms of PKC (PKCa and PKCd, respectively) have been implicated in the development of vasospasm following subarachnoid hemorrhage using a canine model of hemorrhage. Therefore, it may be of benefit to measure various isoforms of PKC, either individually or in various combinations thereof, for the identification of cerebral damage, the presence of the ischemic penumbra, as well as the development and progression of cerebral vasospasm following subarachnoid hemorrhage. Ratios of PKC isoforms such as PKCg and either PKCbI, PKCbII, or both also may be of benefit in identifying a progressing stroke, where the ischemic penumbra is converted to irreversibly damaged infarcted tissue.

*(viii) Non-Specific Markers for Cellular Injury*

Myoglobin is a small (17.8 kDa) heme protein transports oxygen within muscle cells, and constitutes about 2 percent of muscle protein in both skeletal and cardiac muscle. Because of its low molecular weight, myoglobin is rapidly released into the circulation and is the first marker to exhibit rising levels after an AMI: elevated levels appear in the circulation after 0.5 to 2 hours. However, elevated levels may also be related to various skeletal muscle traumas and renal failure, and are therefore not specific for cardiac muscle injury.

Human vascular endothelial growth factor (VEGF) is a dimeric protein, the reported activities of which include stimulation of endothelial cell growth, angiogenesis, and capillary permeability. VEGF is secreted by a variety of vascularized tissues. In an oxygen-deficient environment, vascular endothelial cells may be damaged and may not ultimately survive. However, such endothelial damage stimulates VEGF production by vascular smooth muscle cells. Vascular endothelial cells may exhibit increased survival in the presence of VEGF, an effect that is believed to be mediated by expression of Bcl-2. VEGF can exist as a variety of splice variants known as VEGF(189), VEGF(165), VEGF(164), VEGFB(155), VEGF(148), VEGF(145), and VEGF(121).

Insulin-like growth factor-1 (IGF-1) is a ubiquitous 7.5 kDa secreted protein that mediates the anabolic and somatogenic effects of growth hormone during development (1,2). In the circulation, IGF-1 is normally bound to an IGF-binding protein that regulates IGF activity. Serum IGF-1 concentrations are reported to be significantly decreased in individuals with ischemic stroke, and the magnitude of reduction appears to correlate with the severity of injury (Schwab, S. et al., Stroke 28:1744-1748, 1997). Serum IGF-1 may be a sensitive indicator of neural tissue injury. However, the ubiquitous expression pattern of 1GF-1 indicates that all tissues can potentially affect serum concentrations of IGF-1.

Adhesion molecules are involved in the inflammatory response can also be considered as acute phase reactants, as their expression levels are altered as a result of insult. Examples of such adhesion molecules include E-selectin, intercellular adhesion molecule-1, vascular cell adhesion molecule, and the like.

E-selectin, also called ELAM-1 and CD62E, is a 140 kDa cell surface C-type lectin expressed on endothelial cells in response to IL-1 and TNFα that mediates the "rolling" interaction of neutrophils with endothelial cells during neutrophil recruitment. Some investigations report increases in serum E-selectin concentration following ischemic stroke, while others find it unchanged (Bitsch, A. et al., Stroke 29:2129-2135, 1998; Kim, J.S., J. Neurol. Sci. 137:69-78, 1996; Shyu, K.G. et al., J. Neurol. 244:90-93, 1997). E-selectin concentrations are elevated in the CSF of individuals with subarachnoid hemorrhage and may predict vasospasm (Polin, R.S. et al., J. Neurosurg. 89:559-567, 1998). Serum E-selectin concentrations are elevated in individuals with atherosclerosis, various forms of cancer, preeclampsia, diabetes, cystic fibrosis, AMI, and other nonspecific inflammatory states (Hwang, S.J. et al., Circulation 96:4219-4225, 1997; Banks, R.E. et al., Br. J. Cancer 68:122-124, 1993; Austgulen, R. et al., Eur. J. Obstet. Gynecol. Reprod. Biol. 71:53-58, 1997; Steiner, M. et al., Thromb. Haemost. 72:979-984, 1994; De Rose, V. et al., Am. J. Respir. Crit. Care Med. 157: 1234-1239, 1998).

Head activator (HA) is an 11 amino acid, 1.1 kDa neuropeptide that is found in the hypothalamus and intestine. It was originally found in the freshwater coelenterate hydra, where it acts as a head-specific growth and differentiation factor.

Glycated hemoglobin HbA1c measurement provides an assessment of the degree to which blood glucose has been elevated over an extended time period, and so has been related to the extent diabetes is controlled in a patient. Glucose binds slowly to hemoglobin A, forming the Alc subtype. The reverse reaction, or decomposition, proceeds relatively slowly, so any buildup persists for roughly 4 weeks. With normal blood glucose levels, glycated hemoglobin is expected to be 4.5% to 6.7%. As blood glucose concentration rise, however, more binding occurs. Poor blood sugar control over time is suggested when the glycated hemoglobin measure exceeds 8.0%.

*(ix) Markers related to apoptosis*

Apoptosis refers to the eukaryotic "programmed cell death" pathway. The pathway is dependent upon intracellular proteases and nucleases, leading ultimately to fragmentation of genomic DNA and cell death. The following exemplary list of markers related to apoptosis is not meant to be limiting.

Caspases are a family of proteases that relay a "doomsday" signal in a step-wise manner reminiscent of signaling by kinases. Caspases are present in all cells as latent enzymes. They are recruited to receptor-associated cytosolic complexes whose formation is initiated by receptor oligomerization (e.g., TNF receptors, FAS, and TRAIL receptors) and to other cytoplasmic adaptor proteins, such as APAF-1. Recruitment of caspases to oligomerized receptors leads to activation via dimerization or dimerization accompanied by autoproteolytic cleavage. Active caspases can proteolyze additional caspases generating a caspase cascade that cleaves proteins critical for cell survival. The final outcome of this signaling pathway is a form of controlled cell death termed apoptosis.

The subgroup of caspases involved in apoptosis has been referred to as either initiators or effectors. Caspases-8, -9, and -10 (possibly, -2 and -5) can initiate the caspase activation cascade and are therefore called initiators. Based on the prototypes, caspases-8 and 9, initiators can be activated either by dimerization alone (caspase-9) or by dimerization with concomitant autoproteolysis (caspase-8). The effector caspases-3, -6, and -7 propagate the cascade and are activated by proteolytic cleavage by other caspases. Although an initiator caspase may not be responsible for starting the caspase cascade, it can become activated and involved in later steps of the cascade. Thus, in the latter scenario, the caspase would be more appropriately termed an effector.

Caspase-3, also called CPP-32, YAMA, and apopain, is an interleukin-1β converting enzyme (ICE)-like intracellular cysteine proteinase that is activated during cellular apoptosis. Caspase-3 is present as an inactive 32 kDa precursor that is proteolytically activated during apoptosis induction into a heterodimer of 20 kDa and 11 kDa subunits (Fernandes-Alnemri, T. et al., J. Biol. Chem. 269:30761-30764, 1994). Its cellular substrates include poly(ADP-ribose) polymerase (PARP) and sterol regulatory element binding proteins (SREBPs) (Liu, X. et al., J. Biol. Chem. 271: 13371-13376, 1996). The normal plasma concentration of caspase-3 is unknown. There are no published investigations into changes in the plasma concentration of caspase-3 associated with ACS. There are increasing amounts of evidence supporting the hypothesis of apoptosis induction in cardiac myocytes associated with ischemia and hypoxia (Saraste, A., Herz 24:189-195, 1999; Ohtsuka, T. et al., Coron. Artery Dis. 10:221-225, 1999; James, T.N., Coron. Artery Dis. 9: 291-307, 1998; Bialik, S. et al., J. Clin. Invest. 100:1363-1372, 1997; Long, X. et al., J. Clin. Invest. 99:2635-2643, 1997). Elevations in the plasma caspase-3 concentration may be associated with any physiological event that involves apoptosis. There is evidence that suggests apoptosis is induced in skeletal muscle during and following exercise and in cerebral ischemia (Carraro, U. and Franceschi, C., Aging (Milano) 9:19-34, 1997; MacManus, J.P. et al., J. Cereb. Blood Flow Metab. 19:502-510, 1999).

Cathepsin D (E.C.3.4.23.5.) is a soluble lysosomal aspartic proteinase. It is synthesized in the endoplasmic reticulum as a preprocathepsin D. Having a mannose-6-phosphate tag, procathepsin D is recognized by a mannose-6-phosphate receptor. Upon entering into an acidic lysosome, the single-chain procathepsin D (52 KDa) is activated to cathepsin D and subsequently to a mature two-chain cathepsin D (31 and 14 KDa, respectively). The two mannose-6-phosphate receptors involved in the lysosomal targeting of procathepsin D are expressed both intracellularly and on the outer cell membrane. The glycosylation is believed to be crucial for normal intracellular trafficking. The fundamental role of cathepsin D is to degrade intracellular and internalized proteins. Cathepsin D has been suggested to take part in antigen processing and in enzymatic generation of peptide hormones. The tissue-specific function of cathepsin D seems to be connected to the processing of prolactin. Rat mammary glands use this enzyme for the formation of biologically active fragments of prolactin. Cathepsin D is functional in a wide variety of tissues during their remodeling or regression, and in apoptosis.

Brain α spectrin (also referred to as α fodrin) is a cytoskeletal protein of about 284 kDa that interacts with calmodulin in a calcium-dependent manner. Like erythroid spectrin, brain α spectrin forms oligomers (in particular dimers and tetramers). Brain α spectrin contains two EF-hand domains and 23 spectrin repeats. The caspase 3-mediated cleavage of α spectrin during apoptotic cell death may play an important role in altering membrane stability and the formation of apoptotic bodies.

The following table provides a list of various preferred markers, associated with a classification of the marker to a group of related markers. As understood by the skilled artisan and described herein, markers may indicate different conditions when considered with additional markers in a panel; alternatively, markers may indicate different conditions when considered in the entire clinical context of the patient.

| Marker | Classification |
| --- | --- |
| Myoglobin | Tissue injury |
| E-selectin | Tissue injury |
| VEGF | Tissue injury |
| Troponin I and complexes | Myocardial injury |
| Troponin T and complexes | Myocardial injury |
| Annexin V | Myocardial injury |
| B-cnolase | Myocardial injury |
| CK-MB | Myocardial injury |
| Glycogen phosphorylase-BB | Myocardial injury |
| Heart type fatty acid binding protein | Myocardial injury |
| Phosphoglyceric acid mutase | Myocardial injury |
| S-100ao | Myocardial injury |
| ANP | Blood pressure regulation |
| CNP | Blood pressure regulation |
| urotensin II | Blood pressure regulation |
| BNP | Blood pressure regulation |
| calcitonin gene related peptide | Blood pressure regulation |
| arg-Vasopressin | Blood pressure regulation |
| Endothelin-1 | Blood pressure regulation |
| Endothelin-2 | Blood pressure regulation |
| Endothelin-31 | Blood pressure regulation |
| procalcitonin | Blood pressure regulation |
| calcyphosine | Blood pressure regulation |
| adrenomedullin | Blood pressure regulation |
| aldosterone | Blood pressure regulation |
| angiotensin 1 | Blood pressure regulation |
| angiotensin 2 | Blood pressure regulation |
| angiotensin 3 | Blood pressure regulation |
| Bradykinin | Blood pressure regulation |
| calcitonin | Blood pressure regulation |
| Endothelin-2 | Blood pressure regulation |
| Endothelin-3 | Blood pressure regulation |
| Renin | Blood pressure regulation |
| Urodilatin | Blood pressure regulation |

(continued)

| Marker | Classification |
| --- | --- |
| Plasmin | Coagulation and hemostasis |
| Thrombin | Coagulation and hemostasis |
| Antithrombin-III | Coagulation and hemostasis |
| Fibrinogen | Coagulation and hemostasis |
| von Willebrand factor | Coagulation and hemostasis |
| D-dimer | Coagulation and hemostasis |
| PAI-1 | Coagulation and hemostasis |
| PROTEIN C | Coagulation and hemostasis |
| TAFI | Coagulation and hemostasis |
| Fibrinopeptide A | Coagulation and hemostasis |
| Plasmin alpha 2 antiplasmin complex | Coagulation and hemostasis |
| Platelet factor 4 | Coagulation and hemostasis |
| Platelet-derived growth factor | Coagulation and hemostasis |
| P-selectin | Coagulation and hemostasis |
| Prothrombin fragment 1+2 | Coagulation and hemostasis |
| B-thromboglobulin | Coagulation and hemostasis |
| Thrombin antithrombin III complex | Coagulation and hemostasis |
| Thrombomodulin | Coagulation and hemostasis |
| Thrombus Precursor Protein | Coagulation and hemostasis |
| Tissue factor | Coagulation and hemostasis |
| basic calponin 1 | Vascular tissue |
| beta like 1 integrin | Vascular tissue |
| Calponin | Vascular tissue |
| CSRP2 | Vascular tissue |
| elastin | Vascular tissue |
| Fibrillin 1 | Vascular tissue |
| LTBP4 | Vascular tissue |
| smooth muscle myosin | Vascular tissue |
| transgelin | Vascular tissue |
| Carboxyterminal propeptide of type I procollagen (PICP) | Collagen synthesis |
| Collagen carboxyterminal telopeptide (ICTP) | Collagen degradation |
| Glutathione S Transferase | Inflammatory |
| HIF 1 ALPHA | Inflammatory |
| IL-10 | Inflammatory |
| IL-1-Beta | Inflammatory |
| IL-1ra | Inflammatory |
| IL-6 | Inflammatory |
| IL-8 | Inflammatory |
| Lysophosphatidic acid | Inflammatory |
| MDA-modified LDL | Inflammatory |
| Human neutrophil elastase | Inflammatory |
| C-reactive protein | Inflammatory |
| Insulin-like growth factor | Inflammatory |
| Inducible nitric oxide synthase | Inflammatory |
| Intracellular adhesion molecule | Inflammatory |
| Lactate dehydrogenase | Inflammatory |
| MCP -1 | Inflammatory |
| MDA-LDL | Inflammatory |
| MMP-1 | Inflammatory |
| MMP-2 | Inflammatory |

(continued)

| Marker | Classification |
| --- | --- |
| MMP-3 | Inflammatory |
| MMP-9 | Inflammatory |
| TIMP-1 | Inflammatory |
| TIMP-2 | Inflammatory |
| TIMP-3 | Inflammatory |
| n-acetyl aspartate | Inflammatory |
| TNF Receptor Superfamily Member 1A | Inflammatory |
| Transforming growth factor beta | Inflammatory |
| Tumor necrosis factor alpha | Inflammatory |
| Vascular cell adhesion molecule | Inflammatory |
| Vascular endothelial growth factor | Inflammatory |
| cystatin C | Inflammatory |
| substance P | Inflammatory |
| Myeloperoxidase (MPO) | Inflammatory |
| macrophage inhibitory factor | Inflammatory |
| Fibronectin | Inflammatory |
| cardiotrophin 1 | Inflammatory |
| Haptoglobin | Inflammatory |
| PAPPA | Inflammatory |
| s-CD40 ligand* | Inflammatory |
| HMG | Inflammatory |
| IL -1 | Inflammatory |
| IL -2 | Inflammatory |
| IL -4 | Inflammatory |
| IL -6 | Inflammatory |
| IL-8 | Inflammatory |
| IL -10 | Inflammatory |
| IL-11 | Inflammatory |
| IL-13 | Inflammatory |
| IL-18 | Inflammatory |
| Eosinophil cationic protein | Inflammatory |
| Mast cell tryptase | Inflammatory |
| VCAM | Inflammatory |
| sICAM-1 | Inflammatory |
| TNF$\alpha$ | Inflammatory |
| Osteoprotegerin | Inflammatory |
| Prostaglandin D-synthase | Inflammatory |
| Prostaglandin E2 | Inflammatory |
| RANK ligand | Inflammatory |
| HSP-60 | Inflammatory |
| Serum Amyloid A | Inflammatory |
| s-iL 18 receptor | Inflammatory |
| S-iL-1 receptor | Inflammatory |
| s-TNF P55 | Inflammatory |
| s-TNF P75 | Inflammatory |
| TGF-beta | Inflammatory |
| MMP-11 | Inflammatory |
| Beta NGF | Inflammatory |
| CD44 | Inflammatory |
| EGF | Inflammatory |

(continued)

| Marker | Classification |
| --- | --- |
| E-selectin | Inflammatory |
| Fibronectin | Inflammatory |
| Neutrophil elastase | Pulmonary injury |
| KL-6 | Pulmonary injury |
| LAMP 3 | Pulmonary injury |
| LAMP3 | Pulmonary injury |
| Lung Surfactant protein A | Pulmonary injury |
| Lung Surfactant protein B | Pulmonary injury |
| Lung Surfactant protein C | Pulmonary injury |
| Lung Surfactant protein D | Pulmonary injury |
| phospholipase D | Pulmonary injury |
| PLA2G5 | Pulmonary injury |
| SFTPC | Pulmonary injury |
| MAPK10 | Neural tissue injury |
| KCNK4 | Neural tissue injury |
| KCNK9 | Neural tissue injury |
| KCNQ5 | Neural tissue injury |
| 14-3-3 | Neural tissue injury |
| 4.1B | Neural tissue injury |
| APO E4-1 | Neural tissue injury |
| myelin basic protein | Neural tissue injury |
| Atrophin 1 | Neural tissue injury |
| brain Derived neurotrophic factor | Neural tissue injury |
| Brain Fatty acid binding protein | Neural tissue injury |
| brain tubulin | Neural tissue injury |
| CACNA1A | Neural tissue injury |
| Calbindin D | Neural tissue injury |
| Calbrain | Neural tissue injury |
| Carbonic anhydrase XI | Neural tissue injury |
| CBLN 1 | Neural tissue injury |
| Cerebellin 1 | Neural tissue injury |
| Chimerin 1 | Neural tissue injury |
| Chimerin 2 | Neural tissue injury |
| CHN1 | Neural tissue injury |
| CHN2 | Neural tissue injury |
| Ciliary neurotrophic factor | Neural tissue injury |
| CK-BB | Neural tissue injury |
| CRHR1 | Neural tissue injury |
| C-tau | Neural tissue injury |
| DRPLA | Neural tissue injury |
| GFAP | Neural tissue injury |
| GPM6B | Neural tissue injury |
| GPR7 | Neural tissue injury |
| GPR8 | Neural tissue injury |
| GRIN2C | Neural tissue injury |
| GRM7 | Neural tissue injury |
| HAPIP | Neural tissue injury |
| HIP2 | Neural tissue injury |
| LDH | Neural tissue injury |
| Myelin basic protein | Neural tissue injury |

(continued)

| Marker | Classification |
| --- | --- |
| NCAM | Neural tissue injury |
| NT-3 | Neural tissue injury |
| NDPKA | Neural tissue injury |
| Neural cell adhesion molecule | Neural tissue injury |
| NEUROD2 | Neural tissue injury |
| Neurofiliment L | Neural tissue injury |
| Neuroglobin | Neural tissue injury |
| neuromodulin | Neural tissue injury |
| Neuron specific enolase | Neural tissue injury |
| Neuropeptide Y | Neural tissue injury |
| Neurotensin | Neural tissue injury |
| Neurotrophin 1,2,3,4 | Neural tissue injury |
| NRG2 | Neural tissue injury |
| PACE4 | Neural tissue injury |
| phosphoglycerate mutase | Neural tissue injury |
| PKC gamma | Neural tissue injury |
| proteolipid protein | Neural tissue injury |
| PTEN | Neural tissue injury |
| PTPRZI I | Neural tissue injury |
| RGS9 | Neural tissue injury |
| RNA Binding protein Regulatory Subunit | Neural tissue injury |
| S-100β | Neural tissue injury |
| SCA7 | Neural tissue injury |
| secretagogin | Neural tissue injury |
| SLC1A3 | Neural tissue injury |
| SORL1 | Neural tissue injury |
| SREB3 | Neural tissue injury |
| STAC | Neural tissue injury |
| STX1A | Neural tissue injury |
| STXBP1 | Neural tissue injury |
| Syntaxin | Neural tissue injury |
| thrombomodulin | Neural tissue injury |
| transthyretin | Neural tissue injury |
| adenylate kinase-1 | Neural tissue injury |
| BDNF* | Neural tissue injury |
| neurokinin A | Neural tissue injury |
| s-acetyl Glutathione | apoptosis |
| cytochrome C | apoptosis |
| Caspase 3 | apoptosis |
| Cathepsin D | apoptosis |
| α-spectrin | apoptosis |

Ubiquitination of markers

Ubiquitin-mediated degradation of proteins plays an important role in the control of numerous processes, such as the way in which extracellular materials are incorporated into a cell, the movement of biochemical signals from the cell membrane, and the regulation of cellular functions such as transcriptional on-off switches. The ubiquitin system has been implicated in the immune response and development. Ubiquitin is a 76-amino acid polypeptide that is conjugated to proteins targeted for degradation. The ubiquitin-protein conjugate is recognized by a 26S proteolytic complex that splits ubiquitin from the protein, which is subsequently degraded. Levels of ubiquitinated proteins generally, or of specific ubiquitin-protein conjugates or fragments thereof, can be measured as additional markers of the invention. Moreover, circulating levels of ubiquitin itself or its fragments can be a useful marker in the methods described herein. *See, e.g.,* Hu et al., J. Cereb. Blood Flow Metab. 21: 865-75, 2001.

The skilled artisan will recognize that an assay for ubiquitin may be designed that recognizes ubiquitin itself, ubiquitin-protein conjugates, or both ubiquitin and ubiquitin-protein conjugates. For example, antibodies used in a sandwich immunoassay may be selected so that both the solid phase antibody and the labeled antibody recognize a portion of ubiquitin that is available for binding in both unconjugated ubiquitin and ubiquitin conjugates. Alternatively, an assay specific for ubiquitin conjugates of a marker of interest could use one antibody (on a solid phase or label) that recognizes ubiquitin, and a second antibody (the other of the solid phase or label) that recognizes the marker protein.

The present invention contemplates measuring ubiquitin conjugates of any marker described herein.

Use of marker panels and the "panel response value"

As discussed above, traditional methods to evaluate marker levels in the diagnosis or prognosis of disease typically comprise establishing a "threshold" for a marker of interest. The concentration of that marker in a sample is then compared to that threshold amount, and an amount greater than the pre-established threshold is indicative of one state (e.g., disease), while an amount less than the pre-established threshold is indicative of another state (e.g., normal). One skilled in the art will recognize that univariate analysis of markers can be performed and the data from the univariate analyses of multiple markers can be combined to form panels of markers to differentiate different disease conditions.

As the number of markers in a panel increase, however, applying individual thresholds to each marker can become unwieldy. While the use of individual thresholds for one or more markers in a panel is within the scope of the present invention, the following section describes exemplary methods by which a plurality of markers are evaluated, in which particular thresholds for one or more markers in the marker panel are not relied upon in correlating a marker level to a particular diagnosis and/or prognosis. Rather, the plurality of markers is considered as a unitary whole. A simple example of integrating markers to form a unitary result can be calculating the ratio of two or more markers. In the following exemplary methods, each marker concentration measured in a sample contributes to a "panel response value," which may be compared to a "threshold" panel response as if it were simply the concentration of a single marker. This is an example of a diagnostic method wherein the amount of one or more the markers is not compared to a predetermined threshold level.

Suitable methods for identifying markers useful for the diagnosis of disease states are described in detail in U.S. Provisional Patent Application No. 60/436,392, entitled METHOD AND SYSTEM FOR DISEASE DETECTION USING MARKER COMBINATIONS (attorney docket no. 071949-6801), filed December 24, 2002; U.S. Patent Application No. 10/331,127, entitled METHOD AND SYSTEM FOR DISEASE DETECTION USING MARKER COMBINATIONS (attorney docket no. 071949-6802), filed December 27, 2002; and PCT application no. _____, filed December 23, 2003 (Atty Docket No. 071949-6805), each of which is hereby incorporated by reference in its entirety, including all tables, figures, and claims. One skilled in the art will also recognize that univariate analysis of markers can be performed and the data from the univariate analyses of multiple markers can be combined to form panels of markers to differentiate different disease conditions.

In developing a panel of markers useful in diagnosis, data for a number of potential markers may be obtained from a group of subjects by testing for the presence or level of certain markers. The group of subjects is divided into two sets, and preferably the first set and the second set each have an approximately equal number of subjects. The first set includes subjects who have been confirmed as having a disease or, more generally, being in a first condition state. For example, this first set of patients may be those ACS patients who have recently had a subsequent adverse outcome. Hereinafter, subjects in this first set will be referred to as "diseased".

The second set of subjects is simply those who do not fall within the first set. Subjects in this second set may be "non-diseased;" that is, normal subjects. Alternatively, subjects in this second set may be selected to exhibit one symptom or a constellation of symptoms that mimic those symptoms exhibited by the "diseased" subjects. In the case of the ACS example described hereinafter, the "non-diseased" group may be those ACS patients who, over the same time period, did not suffer a subsequent adverse outcome.

The data obtained from subjects in these sets includes levels of a plurality of markers. Preferably, data for the same set of markers is available for each patient. This set of markers may include all candidate markers that may be suspected as being relevant to the detection of a particular disease or condition. Actual known relevance is not required. Embodiments of the methods and systems described herein may be used to determine which of the candidate markers are most relevant to the diagnosis of the disease or condition. The levels of each marker in the two sets of subjects may be distributed across a broad range, *e.g.*, as a Gaussian distribution. However, no distribution fit is required.

As noted above, a marker often is incapable of definitively identifying a patient as either diseased or non-diseased. For example, if a patient is measured as having a marker level that falls within the overlapping region, the results of the test will be useless in diagnosing the patient. An artificial cutoff may be used to distinguish between a positive and a negative test result for the detection of the disease or condition. Regardless of where the cutoff is selected, the effectiveness of the single marker as a diagnosis tool is unaffected. Changing the cutoff merely trades off between the number of false positives and the number of false negatives resulting from the use of the single marker. The effectiveness of a test having such an overlap is often expressed using a ROC (Receiver Operating Characteristic) curve. ROC curves are well known to those skilled in the art.

The horizontal axis of the ROC curve represents (1- specificity), which increases with the rate of false positives. The vertical axis of the curve represents sensitivity, which increases with the rate of true positives. Thus, for a particular cutoff selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

While the measurement of the level of a single marker may have limited usefulness, the measurement of additional markers provides additional information. But the difficulty lies in properly combining the levels of two potentially unrelated measurements. In the methods and systems according to embodiments of the present invention, data relating to levels of various markers for the sets of diseased and non-diseased patients may be used to develop a panel of markers to provide a useful panel response. The data may be provided in a database such as Microsoft Access, Oracle, other SQL databases or simply in a data file. The database or data file may contain, for example, a patient identifier such as a name or number, the levels of the various markers present, and whether the patient is diseased or non-diseased.

Next, a "window" region may be initially selected for each marker. The location of the window region may initially be selected to include any concentrations of the marker, but the selection may affect the optimization process described below. In this regard, selection near a suspected optimal location may facilitate faster convergence of the optimizer. In a preferred method, the center of the window region is initially centered about the center of the overlap region of the two sets of patients. In one embodiment, the "window" region may simply be a cutoff point or threshold, as is known in the art. In other embodiments, the window region may span a defined concentration range for the marker. In this regard, the window region may be defined by a center value and a width. In practice, the initial selection of the limits of the window region may be determined according to a pre-selected percentile of each set of subjects. For example, a point above which a pre-selected percentile of diseased patients are measured may be used as the right (upper) end of the window range.

Each marker value for each patient may then be mapped to an indicator. The indicator is assigned one value below the window region and another value above the window region. For example, if a marker generally has a lower value for non-diseased patients and a higher value for diseased patients, a zero indicator will be assigned to a low value for a particular marker, indicating a potentially low likelihood of a positive diagnosis. In other embodiments, the indicator may be calculated based on a polynomial. The coefficients of the polynomial may be determined based on the distributions of the marker values among the diseased and non-diseased subjects. In the exemplary embodiments described in detail below, marker concentrations less than the window region are assigned a value of 0, and marker concentrations greater than the window region are assigned a value of 1. Within the window region, concentrations are linearly interpolated to a value between 0 and 1. While the choice of a linear function within the window is used in the examples below, in principle any nonlinear function may also be applied to the marker concentrations within the window, as described in the next paragraphs.

In many disease states, nonspecific markers associated with that state are elevated. But above a certain threshold, higher values of the marker may not relate to a higher probability of disease state. Below a certain threshold, lower marker values may not relate to a lower probability of disease state. In this situation the indicator function may not increase linearly with the marker value. A preferred embodiment is an indicator function that is a function that has a high and monotonic rate of change between the thresholds, and a small rate of change elsewhere. Examples of this type of function are the ramp, step, or sigmoid functions. One may associate the lower threshold with the start of an overlap region (or window region), and the upper threshold with the end of the overlap region. Below the lower threshold the probability of disease is substantially 0, while above the upper threshold the probability of disease is 1. Note that in the case where the indicator function is a step function and the weighting value is 1 for each marker, then the panel response is simply the number of markers above the window. This case is identical to the example used above where one is searching for the best panel with n of m markers above their window. Allowing the indicator to vary continuously near the threshold enables the panel response to be sensitive to a marker just under the window. This information is not lost as it is in the n of m marker example or the step function example, where the indicator value is not continuous. Another common approach of summing over M*W forces the linear relation with M. But as discussed above the most appropriate indicator function may not increase linearly with the marker value. In a further preferred embodiment the ramp function is used as an elevation indicator function. The indicator values within the window regions may vary linearly from a value of zero at one end to a value of one at the other end. In other embodiments, non-linear variations of the indicator value may be used. The ramp function has the advantage of simplicity, and may be good approximation to other function in this class. With proper choices of parameters, the ramp function can be equivalent to the step function or can increase linearly with the marker value.

[0286] [00100] In some disease states, for example unstable angina, a specific marker such as the cardiac troponins (including isoforms of cardiac troponin, comprising troponin I and T and complexes of troponin I, T and C) may be elevated above the normal population, but further elevation indicates an acute condition, in this case a myocardial infarction. Unstable angina is an ischemic condition that leads to minor necrosis of cardiac tissue. During a myocardial infarction, there is major necrosis of cardiac tissue. Cardiac troponin, which is specific to cardiac necrosis, is elevated in both conditions, but the amount of elevation is related to the amount of necrosis. The best indicator function of cardiac troponin in diagnosing unstable angina may not be an elevation indicator function. In a preferred embodiment the indicator function may be a function that is peaked near the expected values of unstable angina, and decreases when the marker value is above or below the expected value. Examples of this type of function include a Gaussian, triangle, trapezoid, or square function. These functions tend to localize the marker value of interest around a specific value. Another example of use for such an indicator function is in cases where a pattern of markers values indicates a disease state. For example, a disease condition may be indicated when one or more markers are within a range of values. When desired, the use of this type of indicator may allow for recognition of patterns of marker values.

The relative importance of the various markers may be indicated by a weighting factor. The weighting factor may initially be assigned as a coefficient for each marker. As with the cutoff region, the initial selection of the weighting factor may be selected at any acceptable value, but the selection may affect the optimization process. In this regard, selection near a suspected optimal location may facilitate faster convergence of the optimizer. In a preferred method, acceptable weighting coefficients may range between zero and one, and an initial weighting coefficient for each marker may be assigned as 0.5. In a preferred embodiment, the initial weighting coefficient for each marker may be associated with the effectiveness of that marker by itself. For example, a ROC curve may be generated for the single marker, and the area under the ROC curve may be used as the initial weighting coefficient for that marker.

Next, a panel response may be calculated for each subject in each of the two sets. The panel response is a function of the indicators to which each marker level is mapped and the weighting coefficients for each marker. In a preferred embodiment, the panel response (R) for a each subject (j) is expressed as:

$$R_j = \sum w_i I_{i,j,}$$

where i is the marker index, j is the subject index, $w_i$ is the weighting coefficient for marker i, I is the indicator value to which the marker level for marker i is mapped for subject j, and $\Sigma$ is the summation over all candidate markers i.

One advantage of using an indicator value rather than the marker value is that an extraordinarily high or low marker levels do not change the probability of a diagnosis of diseased or non-diseased for that particular marker. Typically, a marker value above a certain level generally indicates a certain condition state. Marker values above that level indicate the condition state with the same certainty. Thus, an extraordinarily high marker value may not indicate an extraordinarily high probability of that condition state. The use of an indicator which is constant on one side of the cutoff region eliminates this concern.

The panel response may also be a general function of several parameters including the marker levels and other factors including, for example, race and gender of the patient. Other factors contributing to the panel response may include the slope of the value of a particular marker over time. For example, a patient may be measured when first arriving at the hospital for a particular marker. The same marker may be measured again an hour later, and the level of change may be reflected in the panel response. Further, additional markers may be derived from other markers and may contribute to the value of the panel response. For example, the ratio of values of two markers may be a factor in calculating the panel response.

Having obtained panel responses for each subject in each set of subjects, the distribution of the panel responses for each set may now be analyzed. An objective function may be defined to facilitate the selection of an effective panel. The objective function should generally be indicative of the effectiveness of the panel, as may be expressed by, for example, overlap of the panel responses of the diseased set of subjects and the panel responses of the non-diseased set of subjects. In this manner, the objective function may be optimized to maximize the effectiveness of the panel by, for example, minimizing the overlap.

In a preferred embodiment, the ROC curve representing the panel responses of the two sets of subjects may be used to define the objective function. For example, the objective function may reflect the area under the ROC curve. By maximizing the area under the curve, one may maximize the effectiveness of the panel of markers. In other embodiments, other features of the ROC curve may be used to define the objective function. For example, the point at which the slope of the ROC curve is equal to one may be a useful feature. In other embodiments, the point at which the product of sensitivity and specificity is a maximum, sometimes referred to as the "knee," may be used. In an embodiment, the sensitivity at the knee may be maximized. In further embodiments, the sensitivity at a predetermined specificity level may be used to define the objective function. Other embodiments may use the specificity at a predetermined sensitivity level may be used. In still other embodiments, combinations of two or more of these ROC-curve features may be used.

It is possible that one of the markers in the panel is specific to the disease or condition being diagnosed. When such markers are present at above or below a certain threshold, the panel response may be set to return a "positive" test result. When the threshold is not satisfied, however, the levels of the marker may nevertheless be used as possible contributors to the objective function.

An optimization algorithm may be used to maximize or minimize the objective function. Optimization algorithms are well-known to those skilled in the art and include several commonly available minimizing or maximizing functions including the Simplex method and other constrained optimization techniques. It is understood by those skilled in the art that some minimization functions are better than others at searching for global minimums, rather than local minimums. In the optimization process, the location and size of the cutoff region for each marker may be allowed to vary to provide at least two degrees of freedom per marker. Such variable parameters are referred to herein as independent variables. In a preferred embodiment, the weighting coefficient for each marker is also allowed to vary across iterations of the optimization algorithm. In various embodiments, any permutation of these parameters may be used as independent variables.

In addition to the above-described parameters, the sense of each marker may also be used as an independent variable. For example, in many cases, it may not be known whether a higher level for a certain marker is generally indicative of a diseased state or a non-diseased state. In such a case, it may be useful to allow the optimization process to search on both sides. In practice, this may be implemented in several ways. For example, in one embodiment, the sense may be a truly separate independent variable which may be flipped between positive and negative by the optimization process. Alternatively, the sense may be implemented by allowing the weighting coefficient to be negative.

Within the teachings of this document it is often assumed for simplicity that markers that are elevated in patients with the disease or "positive sense" markers. However this is not always the case, and often, particularly with poor univariate markers, it is not clear from univariate analysis whether the marker when used in conjunction with the other markers in the panel, is best utilized in a positive or negative sense. If the sense of a marker is inverted, then it is straightforward to invert the indicator function for that marker. If the sense is not known, then the search engine may include this as a degree of freedom. For example, in one embodiment, the sense may be a truly separate independent variable, which may be flipped between positive and negative by the optimization process. For optimal performance, the sense should map smoothly from improper to proper, and there should be pressure that allows the search engine to move toward the proper sense. In a preferred embodiment the sense is switched by allowing the weighting coefficient of the analyte to go negative. If the wrong sense is selected, the weighting coefficient will be driven towards zero since inclusion of the marker in the panel response negatively impacts the objective function. The search engine will be able to drive the weighting coefficient across zero to the proper sense.

The optimization algorithm may be provided with certain constraints as well. For example, the resulting ROC curve may be constrained to provide an area-under-curve of greater than a particular value. ROC curves having an area under the curve of 0.5 indicate complete randomness, while an area under the curve of 1.0 reflects perfect separation of the two sets. Thus, a minimum acceptable value, such as 0.75, may be used as a constraint, particularly if the objective function does not incorporate the area under the curve. Other constraints may include limitations on the weighting coefficients of particular markers. Additional constraints may limit the sum of all the weighting coefficients to a particular value, such as 1.0.

The iterations of the optimization algorithm generally vary the independent parameters to satisfy the constraints while minimizing or maximizing the objective function. The number of iterations may be limited in the optimization process. Further, the optimization process may be terminated when the difference in the objective function between two consecutive iterations is below a predetermined threshold, thereby indicating that the optimization algorithm has reached a region of a local minimum or a maximum.

**[00101]** In practice diagnosis of a disease state from multiple markers can be confusing. Often the individual marker values may seem to contradict one another. In panels where the individual markers are not very effective, it is extremely difficult to understand their meaning. In a preferred embodiment, a function that combines the marker values into a scalar value that increases with increasing likelihood of disease is defined. In this manner, the information from multiple markers may be presented in a useable form. This defined function is referred to herein as the panel response (PR), and is a function of the marker values ($M_{0-n}$), written as $PR = f(M_{0-n})$. The panel response may be scaled such that all values are between 0 and 1. Because the effectiveness of the test may not depend on a scaling of the panel response, scaling may not influence the result of the method. However forcing the panel response to be a given scale may remove an unneeded redundancy, as panel response functions that differ only by a scaling factor may in fact represent the same solution. The panel response may also be a general function of several parameters including the marker levels and other factors including, for example, a patient's history, age, race and gender of the patient.

**[0300]** [00102] In a preferred embodiment, the panel response (PR) for each subject is expressed as:

$$PR = \sum_{Mar\,ker\,s} I_i\left(M_i\right)\cdot W_i,$$

where i is the marker index, $W_i$ is the weighting coefficient for the marker i, $M_i$ is the marker value for marker i, I is an indicator function for marker i, and $\Sigma$ is the summation over all candidate markers. The weighting factors scale the indicator functions and may allow for more important or specific markers to have a greater impact on the final panel response. The indicator function maps the marker value into a functional form appropriate to the marker's pathology. The indicator functions can be complex and should be chosen to match the marker. This will be illustrated in the embodiments described below. The indicator function may be a different functional form for each marker. In one example, the indicator function can map the marker value into a probability of the disease state. This mapping may not be a simple function of the marker value. In this example the said indicator from each marker can be summed to determine a relative index which is related to the probability of the patient being diseased. In a preferred embodiment the sum of all the weighting coefficients is constrained to a particular value, such as 1.0. In a preferred embodiment the indicator function is constrained to values between 0 and 1. In a further preferred embodiment, both of the above constraints are satisfied, thus, the panel response is also constrained to a value between 0 and 1.

Thus, the optimization process may provide a panel of markers including weighting coefficients for each marker and cutoff regions for the mapping of marker values to indicators. In order to develop lower-cost panels that require the measurement of fewer marker levels, certain markers may be eliminated from the panel. In this regard, the effective contribution of each marker in the panel may be determined to identify the relative importance of the markers. In one embodiment, the weighting coefficients resulting from the optimization process may be used to determine the relative importance of each marker. The markers with the lowest coefficients may be eliminated.

In order to determine a suitable panel, which for practical reasons may often mean 10 or less markers, one must find a way to systematically remove markers that do not significantly contribute to the overall result. This is accomplished by calculating the contribution from each marker. A method to accomplish this is to remove an analyte from the panel, and recalculate the objective function. The change in the objective function is related to the contribution of the marker. The markers may then be arranged in order of decreasing contribution. In embodiments where a weighting coefficient is applied to each analyte, the weight for the analyte can be set to zero to remove the analyte from the panel. In embodiments where a weighting coefficient is applied to each analyte, one cannot simply use the weights as the contribution. An example of why this does not give the proper result is the case where a marker has zero impact on the test. In this case, the weight it is given by the search program can be any value, so it is possible that its weight will be the highest.

In certain cases, the lower weighting coefficients may not be indicative of a low importance. Similarly, a higher weighting coefficient may not be indicative of a high importance. For example, the optimization process may result in a high coefficient if the associated marker is irrelevant to the diagnosis. In this instance, there may not be any advantage that will drive the coefficient lower. Varying this coefficient may not affect the value of the objective function.

Panel response values themselves may also be used as markers in the methods described herein. For example, a panel may be constructed from a plurality of markers, and each marker of the panel may be described by a function and a weighting factor to be applied to that marker (as determined by the methods described above). Each individual marker level is determined for a sample to be tested, and that level is applied to the predetermined function and weighting factor for that particular marker to arrive at a sample value for that marker. The sample values for each marker are added together to arrive at the panel response for that particular sample to be tested. For a "diseased" and "non-diseased" group of patients, the resulting panel responses may be treated as if they were just levels of another disease marker.

One cound use such a method to define new "markers" based on panel responses, and even to determine a "panel response of panel responses." For example, one may divide ACS and non-ACS subjects as follows: (1) ACS + adverse outcome; (2) ACS - adverse outcome; (3) normals. One would define a first panel constructed from a plurality of markers as described above, and obtain the panel responses from this first panel for all the subjects. Then, the members of any one of these 3 groups may be compared to the panel responses of the members of any other of these groups to define a function and weighting factor that best differentiates these two groups based on the panel responses. This can be repeated as all 3 groups are compared pairwise. The "markers" used to define a second panel might include any or all of the following as a new "marker": (1) versus (2) as marker 1; (1) versus (3) as marker 2; (2) versus (3) as marker 3.

Measures of test accuracy may be obtained as described in Fischer et al., Intensive Care Med. 29: 1043-51, 2003; Zhou et al., Statistical Methods in Diagnostic Medicine, John Wiley & Sons, 2002; and Motulsky, Intuitive Biostatistics, Oxford University Press, 1995; and other publications well known to those of skill in the art, and used to determine the effectiveness of a given marker or panel of markers. These measures include sensitivity and specificity, predictive values, likelihood ratios, diagnostic odds ratios, hazard ratios, and ROC curve areas. As discussed above, suitable tests may exhibit one or more of the following results on these various measures:

A ROC curve area of greater than about 0.5, more preferably greater than about 0.7, still more preferably greater than about 0.8, even more preferably greater than about 0.85, and most preferably greater than about 0.9;

a positive or negative likelihood ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less;

an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; and/or

a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less.

Measures of diagnostic accuracy such as those discussed above are often reported together with confidence intervals or p values. These may be calculated by methods well known in the art. *See, e.g.,* Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Assay Measurement Strategies

Numerous methods and devices are well known to the skilled artisan for the detection and analysis of the markers of the instant invention. With regard to polypeptides or proteins in patient test samples, immunoassay devices and methods are often used. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, each of which is hereby incorporated by reference in its entirety, including all tables, figures and claims. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman Access, Abbott AxSym, Roche ElecSys, Dade Behring Stratus systems are among the immunoassay analyzers that are capable of performing the immunoassays taught herein.

Preferably the markers are analyzed using an immunoassay, although other methods are well known to those skilled in the art (for example, the measurement of marker RNA levels). The presence or amount of a marker is generally determined using antibodies specific for each marker and detecting specific binding. Any suitable immunoassay may be utilized, for example, enzyme-linked immunoassays (ELISA), radioimmunoassays (RIAs), competitive binding assays, and the like. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like.

The use of immobilized antibodies specific for the markers is also contemplated by the present invention. The antibodies could be immobilized onto a variety of solid supports, such as magnetic or chromatographic matrix particles, the surface of an assay place (such as microtiter wells), pieces of a solid substrate material or membrane (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

The analysis of a plurality of markers may be carried out separately or simultaneously with one test sample. For separate or sequential assay of markers, suitable apparatuses include clinical laboratory analyzers such as the ElecSys (Roche), the AxSym (Abbott), the Access (Beckman), the ADVIA® CENTAUR® (Bayer) immunoassay systems, the NICHOLS ADVANTAGE® (Nichols Institute) immunoassay system, etc. Preferred apparatuses or protein chips perform simultaneous assays of a plurality of markers on a single surface. Particularly useful physical formats comprise surfaces having a plurality of discrete, adressable locations for the detection of a plurality of different analytes. Such formats include protein microarrays, or "protein chips" (see, *e.g.*, Ng and Hag, J. Cell Mol. Med. 6: 329-340 (2002)) and certain capillary devices *(see, e.g.,* U.S. Patent No. 6,019,944). In these embodiments, each discrete surface location may comprise antibodies to immobilize one or more analyte(s) (e.g., a marker) for detection at each location. Surfaces may alternatively comprise one or more discrete particles (e.g., microparticles or nanoparticles) immobilized at discrete locations of a surface, where the microparticles comprise antibodies to immobilize one analyte (*e.g.,* a marker) for detection.

Several markers may be combined into one test for efficient processing of a multiple of samples. In addition, one skilled in the art would recognize the value of testing multiple samples (for example, at successive time points) from the same individual. Such testing of serial samples will allow the identification of changes in marker levels over time. Increases or decreases in marker levels, as well as the absence of change in marker levels, would provide useful information about the disease status that includes, but is not limited to identifying the approximate time from onset of the event, the presence and amount of salvagable tissue, the appropriateness of drug therapies, the effectiveness of various therapies as indicated by reperfusion or resolution of symptoms, differentiation of the various types of ACS, identification of the severity of the event, identification of the disease severity, and identification of the patient's outcome, including risk of future events.

A panel consisting of the markers referenced above may be constructed to provide relevant information related to differential diagnosis and/or prognosis. Such a panel may be constucted using 1, 2, 3, 4, 5, 6, 7, 8, 9, 10, 15, 20, or more or individual markers. The analysis of a single marker or subsets of markers comprising a larger panel of markers could be carried out by one skilled in the art to optimize clinical sensitivity or specificity in various clinical settings. These include, but are not limited to ambulatory, urgent care, critical care, intensive care, monitoring unit, inpatient, outpatient, physician office, medical clinic, and health screening settings. Furthermore, one skilled in the art can use a single marker or a subset of markers comprising a larger panel of markers in combination with an adjustment of the diagnostic threshold in each of the aforementioned settings to optimize clinical sensitivity and specificity. The clinical sensitivity of an assay is defined as the percentage of those with the disease that the assay correctly predicts, and the specificity of an assay is defined as the percentage of those without the disease that the assay correctly predicts (Tietz Textbook of Clinical Chemistry, 2nd edition, Carl Burtis and Edward Ashwood eds., W.B. Saunders and Company, p. 496).

The analysis of markers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings.

In another embodiment, the present invention provides a kit for the analysis of markers. Such a kit preferably comprises devises and reagents for the analysis of at least one test sample and instructions for performing the assay. Optionally the kits may contain one or more means for using information obtained from immunoassays performed for a marker panel to rule in or out certain diagnoses.

<u>Selection of Antibodies</u>

The generation and selection of antibodies may be accomplished several ways. For example, one way is to purify polypeptides of interest or to synthesize the polypeptides of interest using, *e.g.*, solid phase peptide synthesis methods well known in the art. *See, e.g.,* Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be injected, for example, into mice or rabbits, to generate polyclonal or monoclonal antibodies. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In addition, numerous publications have reported the use of phage display technology to produce and screen libraries of polypeptides for binding to a selected target. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phages are enriched by affinity screening to the target. The identity of polypeptides displayed from these phages can be determined from their respective genomes. Using these methods a polypeptide identified as having a binding affinity for a desired target can then be synthesized in bulk by conventional means. *See, e.g.*, U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

Selecting a Treatment Regimen

The appropriate treatments for various types of vascular disease may be large and diverse. However, once a diagnosis is obtained, the clinician can readily select a treatment regimen that is compatible with the diagnosis. Accordingly, the present invention provides methods of early differential diagnosis to allow for appropriate intervention in acute time windows. The skilled artisan is aware of appropriate treatments for numerous diseases discussed in relation to the methods of diagnosis described herein. *See, e.g.,* Merck Manual of Diagnosis and Therapy, 17th Ed. Merck Research Laboratories, Whitehouse Station, NJ, 1999.

The following provides a brief discussion of additional exemplary markers for use in identifying suitable marker panels by the methods described herein.

Examples

The following examples serve to illustrate the present invention. These examples are in no way intended to limit the scope of the invention.

Example 1. Blood Sampling

Blood specimens were collected by trained study personnel using EDTA as the anticoagulant and centrifuged for greater than or equal to 10 minutes. The plasma component was transferred into a sterile cryovial and frozen at -20° C or colder. Specimens from the following population of patients and normal healthy donors were collected (Table 1). Clinical histories were available for each of the patients to aid in the statistical analysis of the assay data.

Example 2. Biochemical Analyses

Markers were measured using standard immunoassay techniques. These techniques involved the use of antibodies to specifically bind the protein targets. A monoclonal antibody directed against a selected marker was biotinylated using N-hydroxysuccinimide biotin (NHS-biotin) at a ratio of about 5 NHS-biotin moieties per antibody. The antibody-biotin conjugate was then added to wells of a standard avidin 384 well microtiter plate, and antibody conjugate not bound to the plate was removed. This formed the "anti-marker" in the microtiter plate. Another monoclonal antibody directed against the same marker was conjugated to alkaline phosphatase using succinimidyl 4-[*N*-maleimidomethyl]-cyclohexane-1-carboxylate (SMCC) and *N*-succinimidyl 3-[2-pyridyldithio]propionate (SPDP) (Pierce, Rockford, IL).

Immunoassays were performed on a TECAN Genesis RSP 200/8 Workstation. Biotinylated antibodies were pipetted into microtiter plate wells previously coated with avidin and incubated for 60 min. The solution containing unbound antibody was removed, and the wells were washed with a wash buffer, consisting of 20 mM borate (pH 7.42) containing 150 mM NaCl, 0.1 % sodium azide, and 0.02% Tween-20. The plasma samples (10 $\mu$L) were pipeted into the microtiter plate wells, and incubated for 60 min. The sample was then removed and the wells were washed with a wash buffer. The antibody- alkaline phosphatase conjugate was then added to the wells and incubated for an additional 60 min, after which time, the antibody conjugate was removed and the wells were washed with a wash buffer. A substrate, (AttoPhos®, Promega, Madison, WI) was added to the wells, and the rate of formation of the fluorescent product was related to the concentration of the marker in the patient samples.

Example 3. Dyspnea Analysis

The following table compares levels of pulmonary surfactant protein D ("SP-D"), D-dimer, BNP, total cardiac troponin I ("TnI"), and the ratio of BNP:D-dimer ("Ratio") in individual patients presenting with clinical dyspnea and in normal subjects. Dyspnea patients were subdivided into patients receiving a clinical diagnosis of congestive heart failure ("CHF"), and those receiving a clinical diagnosis of pulmonary embolism ("PE"). All units are ng/ml except BNP (pg/ml) and ratios.

| Multi-Center CHF Patients | | | | | |
|---|---|---|---|---|---|
| Patient ID | SP-D | D-Dimer | BNP | TnI | Ratio |
| 16 | 35.4 | 88 | 889 | 2.1 | 10.1 |

(continued)

| Multi-Center CHF Patients | | | | | |
|---|---|---|---|---|---|
| Patient ID | SP-D | D-Dimer | BNP | TnI | Ratio |
| 012 | 8.7 | 113 | 1228 | 2.2 | 10.9 |
| 003 | 6.9 | 62 | 552 | 0.0 | 8.9 |
| 11 | 11.7 | 160 | 987 | 0.5 | 6.2 |
| 010 | 13.3 | 145 | 466 | 0.0 | 3.2 |
| 18 | 7.9 | 39 | 330 | 0.0 | 8.6 |
| 131-2 | 7.2 | 125 | 1031 | 0.0 | 8.3 |
| 125-1 | 3.7 | 49 | 314 | 0.0 | 6.4 |
| 115 | 8.1 | 203 | 185 | 0.0 | 0.9 |
| 128-1 | 7.5 | 141 | 228 | 0.0. | 1.6 |
| 143-1 | 5.1 | 169 | 402 | 0.0 | 2.4 |
| 134-1 | 1.9 | 142 | 251 | 0.0 | 1.8 |
| 138-1 | 2.4 | 40 | 521 | 0.0 | 13.0 |
| 157-1 | 4.1 | 107 | 231 | 0.0 | 2.2 |
| 176-1 | 2.6 | 70 | 234 | 0.0 | 3.4 |
| 175-1 | 4.6 | 154 | 498 | 0.0 | 3.2 |
| 22 | 6.7 | 36 | 650 | 0.0 | 18.3 |
| 21 | 3.9 | 149 | 453 | 0.0 | 3.0 |
| 23 | 11.5 | 147 | 1024 | 0.0 | 7.0 |
| 103-2 | 3.3 | 70 | 640 | 0.0 | 9.2 |
| 20 | 2.9 | 78 | 858 | 0.0 | 11.0 |
| 148-2 | 6.2 | 79 | 1614 | 0.0 | 20.4 |
| 173-2 | 2.7 | 68 | 236 | 0.0 | 3.5 |
| 166-1 | 5.5 | 53 | 681 | 0.0 | 12.9 |
| 178-1 | 4.3 | 89 | 250 | 0.0 | 2.8 |
| 183-2 | 9.3 | 109 | 1199 | 0.0 | 11.0 |
| 189-2 | 2.2 | 270 | 335 | 0.0 | 1.2 |
| 42 | 3.5 | 143 | 846 | 0.0 | 5.9 |
| 43 | 4.2 | 63 | 287 | 0.0 | 4.5 |
| 54 | 3.5 | 51 | 302 | 0.0 | 5.9 |
| 25 | 4.6 | 61 | 768 | 0.0 | 12.5 |
| 53 | 4.5 | 77 | 1813 | 0.0 | 23.5 |
| 59 | 20.8 | 77 | 288 | 0.0 | 3.7 |
| 55 | 2.4 | 53 | 237 | 0.0 | 4.5 |
| 158-1 | 2.3 | 53 | 1030 | 0.0 | 19.6 |
| Mean | 6.7 | 100.9 | 624.5 | 0.1 | 7.8 |
| Median | 4.6 | 79.3 | 498.0 | 0.0 | 6.2 |
| St. Dev. | 6.3 | 53.1 | 415.8 | 0.5 | 5.9 |

| Multi-Center Patients with PE | | | | | |
|---|---|---|---|---|---|
| Patient ID | SP-D | D-Dimer | BNP | TnI | Ratio |
| 81 | 4.9 | 145 | 314.7 | 0.0 | 2.2 |
| 110 | 4.3 | 87 | 24.3 | 0.0 | 0.3 |
| 112 | 6.9 | 105 | 15.9 | 0.0 | 0.2 |
| 119 | 10.1 | 104 | 175.7 | 0.0 | 1.7 |
| 142 | 7.0 | 106 | 6.2 | 0.0 | 0.1 |
| 196 | 8.2 | 127 | 5.0 | 0.1 | 0.0 |
| 801-2 | 5.2 | 113 | 19.7 | 0.0 | 0.2 |
| 377-2 | 1.4 | 97 | 57.2 | 0.0 | 0.6 |
| 008264 | 1.3 | 258 | 121.3 | 0.0 | 0.5 |
| 008557 | 17.5 | 126 | 51.3 | 0.0 | 0.4 |
| 010647 | 3.8 | 106 | 355.3 | 0.0 | 3.4 |
| 10640 | 0. 7 | 43 | 9.2 | 0.0 | 0.2 |
| 7329 | 3.4 | 191 | 287.3 | 0.0 | 1.5 |
| 008605 | 6.0 | 82 | 733.5 | 0.0 | 9.0 |
| Mean | 5.8 | 120.7 | 155.5 | 0.0 | 1.4 |
| Median | 5.0 | 105.7 | 54.3 | 0.0 | 0.4 |
| St. Dev. | 4.3 | 51.7 | 207.6 | 0.0 | 2.4 |

| Normal Subjects | | | | | |
|---|---|---|---|---|---|
| Patient ID | SP-D | D-Dimer | BNP | TnI | Ratio |
| 001511 | 5.1 | 90 | 20.4 | 0.0 | 0.2 |
| 001515 | 1.9 | 36 | 8.9 | 0.0 | 0.2 |
| 001520 | 1.0 | 61 | 6.5 | 0.0 | 0.1 |
| 001521 | 4.6 | 72 | 3.8 | 0.0 | 0.1 |
| 001524 | 2.3 | 69 | 11.1 | 0.0 | 0.2 |
| 001607 | 3.6 | 72 | 23.4 | 0.0 | 0.3 |
| 001610 | 1.1 | 52 | 18.3 | 0.0 | 0.4 |
| 001613 | 0.2 | 40 | 0.0 | 0.0 | 0.0 |
| 001616 | 2.6 | 28 | 0.0 | 0.0 | 0.0 |
| 001619 | 0.3 | 44 | 0.0 | 0.0 | 0.0 |
| 001622 | 1.4 | 25 | 0.0 | 0.0 | 0.0 |
| 001625 | 4.6 | 142 | 0.0 | 0.0 | 0.0 |
| 001628 | 1.6 | 40 | 0.0 | 0.0 | 0.0 |
| 001631 | 4.6 | 57 | 0.0 | 0.0 | 0.0 |
| 001634 | 7.2 | 60 | 6.6 | 0.0 | 0.1 |
| 001637 | 5.5 | 55 | 0.0 | 0.0 | 0.0 |

(continued)

| Normal Subjects | | | | | |
|---|---|---|---|---|---|
| Patient ID | SP-D | D-Dimer | BNP | TnI | Ratio |
| 001640 | 0.0 | 260 | 19.1 | 0.0 | 0.1 |
| 001643 | 2.5 | 50 | 7.7 | 0.0 | 0.2 |
| 001646 | 0.0 | 56 | 4.7 | 0.0 | 0.1 |
| 002202 | 1.0 | 59 | 27.4 | 0.0 | 0.5 |
| 002205 | 1.7 | 39 | 23.4 | 0.0 | 0.6 |
| 002208 | 1.1 | 25 | 25.9 | 0.0 | 1.0 |
| 002211 | 0.9 | 55 | 45.9 | 0.0 | 0.8 |
| 002214 | 0.0 | 97 | 23.4 | 0.0 | 0.2 |
| 002217 | 2.8 | 117 | 15.3 | 0.0 | 0.1 |
| 002220 | 0.3 | 55 | 11.3 | 0.0 | 0.2 |
| 002223 | 2.5 | 47 | 8.1 | 0.0 | 0.2 |
| 002228 | 2.2 | 44 | 24.3 | 0.0 | 0.5 |
| 002229 | 2.6 | 61 | 11.2 | 0.0 | 0.2 |
| 002232 | 0.7 | 69 | 10.5 | 0.0 | 0.2 |
| 002235 | 0.0 | 54 | 4.0 | 0.0 | 0.1 |
| 002238 | 1.5 | 53 | 9.6 | 0.0 | 0.2 |
| 002241 | 7.5 | 16 | 10.8 | 0.0 | 0.7 |
| 002244 | 8.6 | 44 | 10.7 | 0.0 | 0.2 |
| 002247 | 3.7 | 68 | 33.1 | 0.0 | 0.5 |
| Mean | 2.5 | 63.3 | 12.2 | 0.0 | 0.2 |
| Median | 1.9 | 55.0 | 10.5 | 0.0 | 0.2 |
| St. Dev. | 2.3 | 42.3 | 11.1 | 0.0 | 0.3 |

These data indicate that the median D-dimer levels in the patients diagnosed with pulmonary embolism is higher than for the CHF patients, which is itself higher than normal subjects. Pulmonary surfactant protein D levels appears to be elevated over normals to nearly the same extent in both disease groups compared to normals. Using <82 µg/ml d-dimer as the rule-out cutoff for a diagnosis of pulmonary embolism would result in one false negative diagnosis, and would correctly rule out 18 of the 35 CHF patients and 30 of the 35 normals. For this patient population, using a d-dimer/BNP ratio of>3.4 as the rule-out cutoff would again result in one false negative diagnosis, but would correctly rule out 25 of the 35 CHF patients. The low cardiac troponin I level in all disease and normal subjects correctly rules out the occurrence of myocardial infarction in the entire test population. This example demonstrates that the differential diagnosis of causes of dyspnea can be accomplished through the measurement of d-dimer, BNP and cardiac troponin. Additionally, pulmonary embolism can be ruled in when BNP, d-dimer and pulmonary surfactant protein D levels are elevated above normal levels and troponin levels are normal. Pulmonary embolism can be ruled out when d dimer levels are in the normal range. When BNP levels are above normal, one can rule in congestive heart failure. When cardiac troponin levels are above normal, either cardiac ischemia or necrosis can be ruled in.

Example 4. Identification of diastolic dysfunction

The following table compares levels of BNP, vasopressin, endothelin-2, calcitonin gene related peptide, urotensin 2, ANP, angiotensin II, the ratios of BNP : CGRP, BNP : ANP, BNP : urotensin 2, and calcitonin in heart disease patients and normal subjects. The heart disease patients are subdivided according to the New York Heart Association classification of functional capacity and objective assessment. *See,* Nomenclature and Criteria for Diagnosis of Diseases of the Heart and Great Vessels. 9th ed. Boston, Mass: Little, Brown & Co; 1994, pp. 253-256. The classification is made as follows:

| Class | Functional Capacity | Objective Assessment |
|---|---|---|
| NYHA1 | Patients with cardiac disease but without resulting limitation of physical activity. Ordinary physical activity does not cause undue fatigue, palpitation, dyspnea, or anginal pain. | No objective evidence of cardiovascular disease. |
| NYHA2 | Patients with cardiac disease resulting in slight limitation of physical activity. They are comfortable at rest. Ordinary physical activity results in fatigue, palpitation, dyspnea, or anginal pain. | Objective evidence of minimal cardiovascular disease. |
| NYHA3 | Patients with cardiac disease resulting in marked limitation of physical activity. They are comfortable at rest. Less than ordinary activity causes fatigue, palpitation, dyspnea, or anginal pain. | Objective evidence of moderately severe cardiovascular disease. |
| NYHA4 | Patients with cardiac disease resulting in inability to carry on any physical activity without discomfort. Symptoms of heart failure or the anginal syndrome may be present even at rest. If any physical activity is undertaken, discomfort is increased. | Objective evidence of severe cardiovascular disease. |

DD indicates patients having a clinical diagnosis of diastolic dysfunction, and exhibit an ejection fraction of > 50%. Low ejection fraction (EF) patients are those exhibiting an ejection fraction of < 50%, and are NYHA4 class patients considered to exhibit systolic, rather than diastolic, dysfunction. All units are ng/ml except BNP (pg/ml) and ratios, and N is the number of subjects in each group.

| | BNP | Vasopressin | Endothelin 2 | CGRP | BNP/CGRP | Calcitonin |
|---|---|---|---|---|---|---|
| Normal | 0 | 0.98 | 3.60 | 0.94 | 0 | 0.14 |
| DD NYHA 1 | 142 | 1.13 | 4.63 | 1.06 | 247 | 0.20 |
| DD NYHA2 | 152 | 0.89 | 3.70 | 0.77 | 289 | 0.19 |
| DD NYHA3 | 325 | 0.84 | 3.72 | 1.02 | 309 | 0.16 |
| DD NYHA4 | 600 | 0.99 | 4.38 | 0.66 | 853 | 0.17 |
| DD All | 262 | 0.89 | 3.91 | 0.77 | 366 | 0.19 |
| LowEF | 839 | 1.10 | 4.38 | 0.97 | 957 | 0.19 |

| | Urotensin 2 | BNP/U2 | ANP | BNP/ANP | Angiotensin 2 | N |
|---|---|---|---|---|---|---|
| Normal | 14.6 | 0 | 1.84 | 0 | 0.09 | 20 |
| DD NYHA1 | 18.9 | 11 | 2.11 | 162 | 0.09 | 2 |
| DD NYHA2 | 18.4 | 9 | 1.48 | 113 | 0.08 | 6 |
| DD NYHA3 | 18.8 | 16 | 1.95 | 149 | 0.09 | 6 |
| DD NYHA4 | 19.8 | 30 | 1.07 | 297 | 0.06 | 6 |
| DD All | 19.0 | 14 | 1.50 | 177 | 0.08 | |
| Low EF | 28.0 | 41 | 2.17 | 413 | 0.07 | 20 |

These data indicate that Urotensin-2 and ANP can distinguish diastolic dysfunction from systolic dysfunction. In both cases, the levels are higher in systolic dysfunction than in diastolic dysfunction. Moreover, with the addition of BNP, the the ability to discriminate diastolic dysfunction from systolic dysfunction is enhanced, as elevation of both BNP and ANP appears to be indicative of systolic dysfunction while elevation of BNP with ANP at or below normal levels appears to be indicative of diastolic dysfunction. Urotensin 2 shows a similar pattern. CGRP contributes to the ability to distinguish diastolic from systolic dysfunction when expressed as a ratio with BNP where the ratio is greater in cases of systolic dysfunction relative to diastolic dysfunction.

Hammer-Lercher discusses the significance, or lack thereof, of NT-proBNP levels in controls and in patients with diastolic dysfunction (Hammer-Lercher et al., Clin. Chim. Acta 310(2):193-7 (2001). In a preferred embodiment, a panel consisting of BNP and N'1'proBNP can distinguish heart failure patients with diastolic dysfunction. When both NTproBNP and BNP are elevated above the cutoff, the patient has systolic dysfunction. When NTproBNP is not elevated, but BNP is elevated above the cutoff, this would signify that the patient suffers from diastolic dysfunction.

Example 5. Marker Panels for Cardiac Differential Diagnosis

Exemplary marker panels were selected initially comprising a marker related to blood pressure regulation and a plurality of markers related to myocardial injury in order to develop a panel for diagnosing and/or distinguishing congestive heart failure, acute coronary syndromes, and myocardial infarction, and for guiding therapy in response to the results of the assay. For this purpose, BNP, cardiac troponin I (free and complexed), creatine kinase-MB, and myoglobin were selected. Threshold levels for comparison of measured marker concentrations were established in this example using the upper end of normal values for CKMB (4.3 ng/mL), myoglobin (107 ng/mL) and troponin I (0.4 ng/mL). Elevation and/or Temporal changes in these three markers, coupled with chest pain for a period of at least 20 minutes is highly indicative of myocardial infarction. In addition, BNP concentrations in excess of 80 pg/mL BNP can provide additional risk stratification in these patients, as this level of BNP is related to increased rates of death, myocardial infarction, and congestive heart failure in comprarison to patients having a BNP level below this threshold. Moreover, even in subjects experiencing no clinical symptoms of disease, a BNP level in excess of 100 pg/mL is associated with a substantially higher incidence of congestive heart failure. Thus, this multimarker strategy can provide substantially more clinically relevant information than can individual markers.

The addition of other markers to the multimarker panel can provide additional clinical information for both risk stratification and differential diagnosis. For example, D-dimer may be added to the panel as a marker of coagulation and hemostasis. As discussed above, the addition of D-dimer can permit the differentiation of pulmonary embolism and/or deep venous thrombosis from myocardial infarction and congestive heart failure, despite the fact that the subjects may present to the clinician with substantially similar symptoms. In this case, a threshold level of about about 1 $\mu$g/mL may be established. In addition, or in the alternative, to D-dimer, C-reactive protein, a relatively nonspecific indicator of inflammation, can provide additional risk stratification to the panel. While the data is not presented here, CK-MB and myoglobin can also provide for distinguishing ST-elevation and non-ST-elevation ACS.

As the number of markers in a panel increases, the determination of a single panel response and its correlation to various disease states by the methods described herein can be advantageous. An example of such a panel may include specific markers of cardiac injury (*e.g.*, cardiac troponin I and/or T (free and complexed), creatine kinase-MB, *etc.*), and non-specific markers of tissue injury (*e.g.*, myoglobin), where none of the markers are compared to a predetermined threshold. Starting with a number of potential markers, an iterative procedure was applied. In this procedure, individual threshold concentrations for the markers were not used as cutoffs *per se.* Rather, a "window" of assay values between a minimum and maximum marker concentration was determined. Measured marker concentrations above the maximum are assigned a value of 1 and measured marker concentrations below the minimum are assigned a value of 0; measured marker concentrations within the window are linearly interpolated to a value of between 0 and 1. The value obtained for a given marker concentration was then multiplied by a weighting factor. The absolute values of the weights for all of the individual markers used in a panel add up to 1. A negative weight for a marker implies that the assay values for the control group are higher than those for the diseased group. A "panel response" is calculated by summing the weighted values for each marker in the panel. The panel responses for the entire population of "disease group" and "controls" are subjected to ROC analysis, and a panel response threshold was selected to yield the desired sensitivity and specificity for the panel. After each set of iterations, the weakest contributors to the equation may be eliminated and the iterative process started again with the reduced number of markers.

The following panels represent such marker panels identified for the ability to discriminate subjects suffering from acute myocardial infarction (labeled "Disease group") from "control" subjects. Panel 1 represents the results obtained from a "first draw" at clinical presentation, while Panels 2-4 represent the results obtained using 60, 90, and 180 minute draws, respectively. Using the "gold standard" of cardiac troponin I alone, first, 60, 90, and 180 minute draws provide 25.9%, 28.7%, 55.6% and 100% specificity, respectively, at 92.5% sensitivity.

| Panel # | 1 | 2 | 3 | 4 |
|---|---|---|---|---|
| Markers in panel | cTnI, CK-MB, myoglobin | cTnI, CK-MB, myoglobin | cTnI,CK-MB, myoglobin | cTnI,CK-MB, myoglobin |
| Control n | 474 | 184 | 186 | 74 |
| Disease n | 67 | 46 | 50 | 48 |

(continued)

| Panel # | 1 | 2 | 3 | 4 |
|---|---|---|---|---|
| Ave ROC Area | 0.939 | 0.984 | 0.992 | 0.980 |
| SD(%) | 0.008 | 0.002 | 0.001 | 0.098 |
| Ave Sens @ 92.5% Spec | 81.6% | 95.0% | 97.6% | 96.9% |
| SD(%) | 2.1 | 1.1 | 0.80 | 9.80 |
| Ave Spec @ 92.5% Sens | 76.9 | 93.3 | 96.5% | 99.0% |
| SD(%) | 2.6 | 0.6 | 0.60 | 10.00 |

In contrast to the results obtained from cardiac troponin I alone, the panels described above provide improved specificity at early time points. In these time points, myoglobin and CK-MB are included at an increased weight. Not surprisingly, at the final time point (where cardiac troponin I alone achieves 100% specificity, cardiac troponin I dominates the panel response. Additional panels may include additional markers as described herein, particularly including markers related to blood pressure regulation (e.g., BNP), markers related to coagulation and hemostasis (e.g., D-dimer, TpP), markers related to apoptosis (e.g., caspase-3, cytochrome c), and/or markers related to inflammation (e.g., MMP-9, CRP, myeloperoxidase, IL-1ra, MCP-1). In addition, the change in one or more of the foregoing markers over time is preferably included as an additional marker in such panels.

Using this same methodology, similar marker panels can be defined in order to distinguish acute myocardial infarction and mimic conditions such as non-cardiac chest pain and unstable angina. The following tables compare samples obtained from subjects suffering from these mimic conditions and samples obtained within 10 hours of presentation from subjects suffering from an acute myocardial infarction.

| Panel # | 1 | 2 | 3 |
|---|---|---|---|
| Markers in panel | cTnI, CK-MB, myoglobin, BNP | cTnI, CK-MB, myoglobin, BNP | cTnI, CK-MB, myoglobin, BNP |
| "Control" subjects | Non-cardiac chest pain | Unstable angina | Non-cardiac chest pain |
| Control n | 210 | 210 | 210 |
| "Disease" subjects | Acute myocardial infarction | Acute myocardial infarction | Unstable angina |
| Disease n | 92 | 92 | 210 |
| Ave ROC Area | 0.984 | 0.944 | 0.792 |
| SD(%) | 0.002 | 0.093 | 0.014 |
| Ave Sens @ 92.5% Spec | 97.i°ro | 86.3% | 34.6% |
| SD(%) | 0.90 | 8.7 | 1.7 |
| Ave Spec @ 92.5% Sens | 96.1% | 81.7% | 49.9% |
| SD(%) | 0.60% | 8.4 | 3.2 |

The following panels represent prognostic marker panels used to analyze test samples obtained from ACS patients having an adverse event (death, acute myocardial infarction, congestive heart failure, labeled "Disease group") within 30 days to a "control" group representing ACS patients not having such an event. An odds ratio was calculated based on these results for the ability of each panel to predict such an adverse event.

| Panel # | 1 | 2 | 3 | 4 |
|---|---|---|---|---|
| Markers in panel | cTnI, CRP, BNP | cTnI, CRP, BNP, CK-MB | cTnI, CRP, BNP, Caspase-3, MMP-9 | cTnI, CRP, BNP, MMP-9, TpP |
| Control n | 1936 | 977 | 1781 | 1762 |
| Disease n | 104 | 52 | 91 | 92 |
| Ave ROC Area | 0.679 | 0.733 | 0.708 | 0.733 |
| SD(%) | 0.006 | 0.011 | 0.013 | 0.067 |
| Ave Sens @ 92.5% Spec | 29.0% | 34.5% | 30.8% | 30.5% |
| SD(%) | 1.70 | 2.40 | 2.00 | 3.80 |
| Ave Spec @ 92.5% Sens | 23.5% | 37.9% | 32.1% | 30.2% |
| SD(%) | 2.20 | 2.00 | 2.90 | 4.60 |
| Odds Ratio | 5.038 | 6.496 | 5.489 | 5.412 |

| Panel # | 5 |
|---|---|
| Markers in panel | cTnI, CRP, BNP, MMP-9, myoglobin, MCP-1, TpP |
| Control n | 1710 |
| Disease n | 90 |
| Ave ROC Area | 0.715 |
| SD(%) | 0.012 |
| Ave Sens @ 92.5% Spec | 37.7% |
| SD(%) | 2.60 |
| Ave Spec @ 92.5% Sens | 33.4% |
| SD(%) | 3.10 |
| Odds Ratio | 7.463 |

The following panels consider death within 180 days alone as the adverse event.

| Panel # | 1 | 2 | 3 | 4 |
|---|---|---|---|---|
| Markers in panel | cTnI, CRP, BNP | Myoglobin, CRP, BNP | BNP, Myoglobin | CRP, BNP |
| Control n | 768 | 768 | 776 | 768 |
| Disease n | 63 | 63 | 63 | 63 |
| Ave ROC Area | 0.801 | 0.804 | 0.803 | 0.799 |
| SD(%) | 0.011 | 0.006 | 0.004 | 0.005 |
| Ave Sens @ 92.5% Spec | 42.1% | 42.7% | 44.0% | 42.6% |
| SD(%) | 2.80% | 2.20% | 2.30% | 1.10% |
| Ave Spec @ 92.5% Sens | 51.4% | 51.5% | 51.1% | 46.3% |
| SD(%) | 3.00% | 2.30% | 3.20% | 2.80% |
| Odds Ratio | 9.0 | 9.2 | 9.7 | 9.2 |

| Panel # | 5 | 6 | 7 | 8 |
|---|---|---|---|---|
| Markers in panel | BNP alone | BNP, cTnI, CK-MB, CRP, Myoglobin | Myoglobin, cTnI, BNP, CK-MB | CK-MB, cTnI, BNP, CRP |
| Control n | 776 | 768 | 776 | 768 |
| Disease n | 63 | 63 | 63 | 63 |
| Ave ROC Area | 0.799 | 0.802 | 0.801 | 0.796 |
| SD(%) | 0.001 | 0.020 | 0.009 | 0.015 |
| Ave Sens @ 92.5% Spec | 42.9% | 42.4% | 45.0% | 41.7% |
| SD(%) | 0.10% | 3.40% | 4.00% | 3.70% |

| Panel # | 5 | 6 | 7 | 8 |
|---|---|---|---|---|
| Ave Spec @ 92.5% Sens | 38.8% | 54.3% | 54.3% | 53.4% |
| SD(%) | 0.10% | 4.60% | 3.40% | 4.40% |
| Odds Ratio | 9.3 | 9.1 | 10.1 | 8.8 |

| Panel # | 9 |
|---|---|
| Markers in panel | CRP, CK-MB, Myoglobin, cTnI |
| Control n | 768 |
| Disease n | 63 |
| Ave ROC Area | 0.701 |
| SD(%) | 0.034 |
| Ave Sens @ 92.5% Spec | 32.7% |
| SD(%) | 3.90% |
| Ave Spec @ 92.5% Sens | 34.8% |
| SD(%) | 6.80% |
| Odds Ratio | 6.0 |

And the following panels consider death within 90, 180, 365, and 740 days, respectively, as the adverse event.

| Panel # | 1 | 2 | 3 | 4 |
|---|---|---|---|---|
| Markers in panel | cTnI, CRP, BNP, myeloperoxidase, myoglobin, CK-MB | cTnI, CRP, BNP, myeloperoxidase, myoglobin, CK-MB | cTnI, CRP, BNP, myeloperoxidase, myoglobin, CK-MB | cTnI, CRP, BNP, myeloperoxidase, myoglobin, CK-MB |
| Control n | 760 | 760 | 760 | 760 |
| Disease n | 42 | 60 | 70 | 94 |
| Ave ROC Area | 0.781 | 0.784 | 0.792 | 0.767 |
| SD(%) | 0.015 | 0.080 | 0.015 | 0.077 |
| Ave Sens @ 92.5% Spec | 37.0% | 38.7% | 39.3% | 34.4% |
| SD(%) | 3.10% | 4.90% | 3.30% | 4.60% |
| Ave Spec @ 92.5% Sens | 57.9% | 54.6% | 54.0% | 50.1% |
| SD(%) | 4.30% | 6.70% | 3.00% | 5.60% |
| Odds Ratio | 7.2 | 7.8 | 8.0 | 6.5 |

The skilled artisan will understand that additional markers may be included or substituted into the foregoing panels. Additional markers may include single concentrations of markers, or may include a marker "slope" (i.e., relative changes in markers over time, ratios of two markers, etc. The skilled artisan will also understand that the same panel may provide both diagnostic and prognostic information. The markers used for diagnosis may be the same as those used for prognosis, or may differ in that one or more markers used for one of these purposes may not be used for the other purpose.

Example 6. Diagnosis of Subclinical Atherosclerosis Using MCP-1

MCP-1 has been identified as an independent risk predictor in ACS. *See, e.g.,* de Lemos et al., Circulation 107: 690-95 (2003), which is hereby incorporated by reference in its entirety. The following data demonstrates the use of MCP-1 in the diagnosis of subclinical atherosclerosis. Baseline MCP-1 levels were measured in 3499 patients not exhibiting symptoms of atherosclerosis (based on clinical presentation). A subset of 2733 patients was given electron beam computerized tomography (EBCT) scans. EBCT is an imaging procedure that uses a CT scanner to measure the amount of calcium found in the arteries of the heart. Subclinical coronary artery disease can be detected without the need of surgery or the injection of tracking fluids by measuring coronary artery calcium ("cac"). *See, e.g.,* Khaleeli et al.,

Am. Heart J. 141: 637-44, 2001.

Distribution of MCP-1 among the 3499 patients from whom it was measured:

| Quartile | N | Median |
|---|---|---|
| 1 (<= 123 pg/mL) | 875 | 100.3 [83,112] |
| 2(>123.1-167.9 pg/mL) | 877 | 146 [134, 157] |
| 3 (168-226 pg/mL) | 874 | 194 [180, 208] |
| 4 (>226.1 pg/mL) | 873 | 285 [248, 356] |

MCP-1 Levels and Cardiovascular Risk Factors

MCP-1 Quartiles

| Variable | Quartile 1 | Quartile 2 | Quartile 3 | Quartile 4 | P value |
|---|---|---|---|---|---|
| Median Age(years) | 40 [34, 48] | 43 [36, 51] | 44 [36, 52] | 47 [39, 54] | <0.001 |
| % patients with hypertension | 21.04 | 23.21 | 25.66 | 30.09 | <0.001 |
| % patients with diabetes | 18.16 | 24.13 | 26.87 | 30.85 | 0.001 |
| Median Total Cholesterol | 172 [150, 198] | 175 [154, 201] | 180 [156, 204] | 180 [155, 207] | <0.001 |
| % patients who are current smokers | 21.52 | 22.90 | 27.64 | 27.94 | <0.001 |
| % patients with family hx CAD | 22.65 | 24.41 | 25.11 | 27.83 | 0.022 |
| Median LDL | 101 [81, 124] | 102 [82, 123] | 106 [84, 128] | 107 [82, 129] | 0.0188 |

These associations are among all 3499 patients.

LDL: Each quartile contains about 875 patients; HTN: 1060 patients had hypertension; smoking: 1013 patients were current smokers; family hx: 1139 patients had a family h/o cad; DM: 402 patients had DM.

Associations (not shown) between baseline variables and MCP-1 levels were also performed among the subset of patients that had EBCT scans (n =2733).

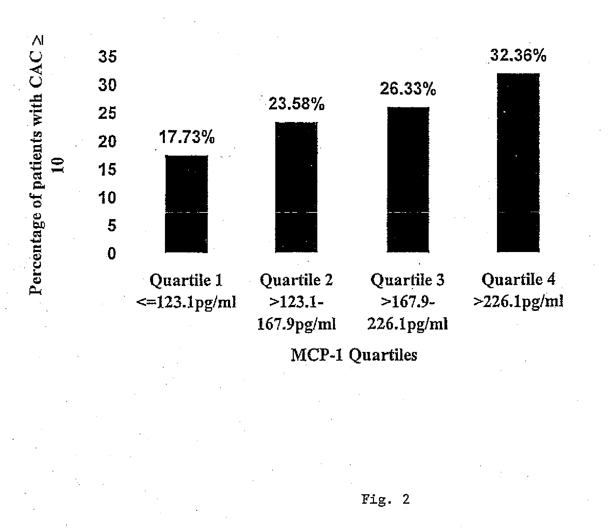

Figure 2 shows the association of MCP-1 to subclinical atherosclerosis in 2733 patients who had an EBCT scan. Of these, 581 patients had evidence of subclinical atherosclerosis defined as a coronary calcification score ≥ 10. Additional evidence suggests a significant association between the degree of cac (categorical) and MCP-1 levels (continuous).

Relative Risk for Subclinical Atherosclerosis (CAC≥10) in Multivariate Analysis (Excluding Age)

| Variable | Odds Ratio | P value |
|---|---|---|
| MCP quartile 2 | 1,339 | 0.059 |
| MCP quartile 3 | 1.445 | 0.016 |
| MCP quartile 4 | 1.716 | <0.001 |
| Sex | 2.548 | <0.001 |
| Diabetes | 2.391 | <0.001 |
| Hypertension | 3.425 | <0.001 |
| Tobacco Use | 1.781 | <0.001 |
| Total Chol | 1.521 | 0.009 |

Multivariate Model for Subclinical Atherosclerosis stratified by intermediate or highest age tertile (>= 40years; n=1831)

| Variable | Odds Ratio | P value |
|---|---|---|
| MCP quartile 2 | 1.382 | 0.051 |
| MCP quartile 3 | 1.501 | 0.013 |
| MCP quartile 4 | 1.604 | 0.003 |
| Sex | 2.561 | <0.001 |
| Diabetes | 2.164 | <0.001 |
| Hypertension | 2.424 | <0.001 |
| Tobacco Use | 1.764 | <0.001 |
| Total Chol | 1.322 | 0.099 |
| Family History of CAD | 1.409 | 0.002 |

In the case of acute myocardial infarction, panels, window values, and weighting factors are selected that, using a panel response value, preferably provide a sensitivity of at least 80% at greater than 90% specificity.

Example 7. Marker Panels for Cerebrovascular Differential Diagnosis

In the case of cerebrovascular differential diagnosis, marker panels were selected comprising a marker related to blood pressure regulation and a plurality of markers related to neural tissue injury in order to develop a panel for diagnosing and/or distinguishing stroke from patients referred to herein as "stroke mimics." Additional classes of markers tested to increase marker panel response include markers of apoptosis, markers of inflammation, and/or acute phase reactants. A final exemplary panel was identified that provided a sensitivity of at least 80% at greater than 90% specificity. Starting with a number of potential markers, an iterative procedure was applied. In this procedure, individual threshold concentrations for the markers were not used as cutoffs *per se.* Rather, a "window" of assay values between a minimum and maximum marker concentration was determined. Measured marker concentrations above the maximum are assigned a value of 1 and measured marker concentrations below the minimum are assigned a value of 0; measured marker concentrations within the window are linearly interpolated to a value of between 0 and 1. The value obtained for a given marker concentration was then multiplied by a weighting factor. The absolute values of the weights for all of the individual markers used in a panel add up to 1. A negative weight for a marker implies that the assay values for the control group are higher than those for the diseased group. Again, none of the markers are compared to a predetermined threshold. Instead, a "panel response" is calculated by summing the weighted values for each marker in the panel. The panel responses for the entire population of "disease group" and "controls" are subjected to ROC analysis, and a panel response threshold was selected to yield the desired sensitivity and specificity for the panel. After each set of iterations, the weakest contributors to the equation may be eliminated and the iterative process started again with the reduced number of markers.

| Markers in panel | NCAM, Caspase-3, IL-8, CK-I3B, CRP, S100β, BNP, MMP-9 | | |
|---|---|---|---|
| Stroke Type | All stroke | Ischemic stroke | Hemorrhagic stroke |
| "Control" n | 49 | 49 | 49 |
| "Disease" n | 48 | 41 | 7 |
| Ave ROC Area | 0.927 | 0.935 | 0.881 |
| *SD* | *0.018* | *0. 024* | *0.039* |
| Ave Sens @ 92.5% Spec | 85.6% | 86.8% | 78.7% |
| *SD* | *6. 50%* | *7.20%* | *9.60%* |
| Ave Spec @ 92.5% Sens | 83.2% | 84.4% | 39.8% |
| *SD* | *8.10%* | *8.90%* | *27.80%* |

In addition, panels were assessed for the ability to identify severity of neurologic deficit in stroke patients. In these panels, controls were subjects exhibiting an NIH stroke scale ("NIHSS") score of < 5, while the disease subjects exhibited an NIHSS score of ≥ 5. As shown in the following table, simply changing the panel parameters (*e.g.,* the width and position of the window and/or weighting) while using the same eight markers described above for stroke diagnosis, can provide important information about the severity of neurologic deficit.

|  | Panel 1 | Panel 2 |
|---|---|---|
| Markers in panel | NCAM, Caspase-3, IL-8, CK-BB, CRP, S100β, BNP, MMP-9 | |
| "Control" n | 66 | 66 |
| "Disease" n | 24 | 24 |
| Ave ROC Area | 0.928 | 0.929 |
| SD | 0.021 | 0.018 |
| Ave Sens @ 92.5% Spec | 79.6% | 80.2% |
| SD | 12.70% | 13.30% |
| Ave Spec @ 92.5% Sens | 86.9% | 87.7% |
| SD | 6.80% | 6.10% |

Interestingly, MMP-9 shows a negative correlation with neurologic deficit, indicating that, while MMP-9 is increased in stroke patients relative to mimics, MMP-9 is actually decreased in the case of stroke patients exhibiting an increased neurologic deficit, relative to subjects with less severe neurologic deficit. High MMP-9 may be indicative of increased revascularization, and therefore may be a marker of positive prognosis in stroke patients. In addition, thrombolytic treatment may be less advantageous in stroke patients with high MMP-9, as revascularization is providing additional perfusion of the lesion. Such panels may provide prognostic information in diseases and procedures that are associated with a risk of neurologic deficit. Such procedures include carotid endarterectomy, hypothermic circulatory arrest, aortic valve replacement, mitral valve replacement, coronary artery surgery, endograft repair of aortic aneurism, coronary artery bypass graft surgery, laryngeal mask insertion, and repair of congenital heart defects.

Additional panels may be provided that utilize fewer markers, with no to moderate loss of sensitivity and specificity, as shown in the following tables:

| Panel # | 3 | 4 | 5 | 6 |
|---|---|---|---|---|
| Markers in panel | CRP, BNP, MMP-9, CK-BB, Capase-3, IL-8, S-100β | CRP, BNP, MMP-9, CK-BB, Capase-3, IL-8 | CRP, BNP, MMP-9, CK-BB, Capase-3 | CRP, BNP, CK-BB, Capase-3 |
| Control n | 81 | 86 | 86 | 86 |
| Disease n | 26 | 27 | 27 | 27 |
| Ave ROC Area | 0.913 | 0.910 | 0.895 | 0.878 |
| SD(%) | 0.021 | 0.019 | 0.014 | 0.016 |
| Ave Sens @ 92.5% Spec | 74.8% | 73.4% | 65.3% | 61.0% |
| SD(%) | 12.60% | 10.50% | 7.90% | 6.00°% |
| Ave Spec @ 92.5% Sens | 83.6% | 83.7% | 80.9% | 76.1% |
| SD(%) | 6.80% | 5.50% | 5.10% | 7.40% |

Panels defined in accordance with the foregoing principles may be selected to differentiate subjects suffering from stroke (ichemic and/or hemorrhagic) from age-matched normal subjects. Such panels can be used to identify those subjects in a stroke mimic population that suffer from acute ischemia that does not rise to the level of a diagnosis of stroke. For example, ausing such panels to screen a mimic population (*e.g.*, subjects suffering from TIA, syncope, peripheral vascular disease, *etc.),* can identify a subpopulation exhibiting a panel response that could be considered a "false positive" stroke diagnosis. This subpopulation may be suffering from a significant stroke-like episode, but because of the location of the lesion, may not be exhibiting a sufficient neurologic deficit to fall within the clinical diagnosis of stroke. Such subjects may benefit from more aggressive treatment than mimic subjects who appear "normal" according to the panel response. This mimic population is referred to herein as suffering from "subclinical stroke" or "subclinical ischemia," and the methods described herein can be used for the diagnosis and/or prognosis of such subclinical conditions.

Example 8. Diagnosis of Stroke

A panel that includes any combination of the above-referenced markers may be constructed to provide relevant information regarding the diagnosis of stroke and management of patients with stroke and TIAs. In addition, a subset of markers from this larger panel may be used to optimize sensitivity and specificity for stroke and various aspects of the disease. The example presented here describes the statistical analysis of data generated from immunoassays specific for BNP, IL-6, S-100β, MMP-9, TAT complex, and the A1 and integrin domains of vWF (vWF A1-integrin) used as a 6-marker panel. The thresholds used for these assays are 55 pg/ml for BNP, 27 pg/ml for IL-6, 12 pg/ml for S-100β, 200 ng/ml for MMP-9, 63 ng/ml for TAT complex, and 1200 ng/ml for vWF A1-integrin. A statistical analysis of clinical sensitivity and specificity was performed using these thresholds in order to determine efficacy of the marker panel in identifying patients with ischemic stroke, subarachnoid hemorrhage, intracerebral hemorrhage, all hemorrhagic strokes (intracranial hemorrhage), all stroke types, and TIAs. Furthermore, the effectiveness of the marker panel was compared to a current diagnostic method, computed tomography (CT) scan, through an analysis of clinical sensitivity and specificity.

The computed tomography (CT) scan is often used in the diagnosis of stroke. Because imaging is performed on the brain, CT scan is highly specific for stroke. The sensitivity of CT scan is very high in patients with hemorrhagic stroke early after onset. In contrast, the sensitivity of CT scan in the early hours following ischemic stroke is low, with approximately one-third of patients having negative CT scans on admission. Furthermore, 50% patients may have negative CT scans within the first 24 hours after onset. The data presented here indicates that the sensitivity of CT scan at admission for 24 patients was consistent with the expectation that only one-third of patients with ischemic stroke have positive CT scans. Use of the 6-marker panel, where a patient is positively identified as having a stroke if at least two markers are elevated, yielded a sensitivity of 79%, nearly 2.5 times higher than CT scan, with high specificity (92%). The specificity of CT scan in the study population is assumed to be close to 100%. One limitation of this assumption is that CT scans were not obtained from individuals comprising the normal population. Therefore, the specificity of CT scan in this analysis is calculated by taking into consideration other diseases or conditions that may yield positive CT scans. CT scans may be positive for individuals with non-stroke conditions including intracranial tumors, arteriovenous malformations, multiple sclerosis, or encephalitis. Each of these non-stroke conditions has an estimated incidence rate of 1% of the entire U.S. population. Because positive CT scans attributed to multiple sclerosis and encephalitis can commonly be distinguished from stroke, the specificity of CT scan for the diagnosis of stroke is considered to be greater than 98%. The data presented in the following table indicates that use of a panel of markers would allow the early identification of patients experiencing ischemic stroke with high specificity and higher sensitivity than CT scan.

|         | Sensitivity | Specificity |
|---------|-------------|-------------|
| CT Scan | 33%         | >98%        |
| Markers | 92%         | 92%         |

The sensitivity and specificity of the 6-marker panel was evaluated in the context of ischemic stroke, subarachnoid hemorrhage, intracerebral hemorrhage, all hemorrhagic stroke (intracranial hemorrhage), and all stroke types combined at various times from onset. The specificity of the 6-marker panel was set to 92%, and patients were classified as having the disease if two markers were elevated. In addition, a 4-marker panel, consisting of F3NF, S-100β, MMP-9 and vWF A1-integrin was evaluated in the same context as the 6-marker panel, with specificity set to 97% using the same threshold levels. The 4-marker panel is used as a model for selecting a subset of markers from a larger panel of markers in order to improve sensitivity or specificity for the disease, as described earlier. The data presented in Tables 3-7 indicate that both panels are useful in the diagnosis of all stroke types, especially at early times form onset. Use of the 4-marker panel provides higher specificity than the 6-marker panel, with equivalent sensitivities for hemorrhagic strokes within the first 48 hours from onset. The 6-marker panel demonstrates higher sensitivity for ischemic stroke at all time points than the 4-marker panel, indicating that the 6-marker approach is useful to attain high sensitivity (*i.e.* less false negatives), and the 4-marker panel is useful to attain high specificity (*i. e.* less false positives).

Sensitivity Analysis - Ischemic Stroke

| Time from Onset of Symptoms (hr) | Number of Samples | SENSITIVITY with Specificity at 92% | SENSITIVITY with Specificity at 97% |
|---|---|---|---|
| 3 | 6 | 100 | 83.3 |

(continued)

| Time from Onset of Symptoms (hr) | Number of Samples | SENSITIVITY with Specificity at 92% | SENSITIVITY with Specificity at 97% |
|---|---|---|---|
| 6 | 19 | 100 | 94.7 |
| 12 | 36 | 91.7 | 88.9 |
| 24 | 60 | 88.3 | 86.4 |
| 48 | 96 | 88.5 | 84.4 |
| All | 175 | 89.7 | 84.0 |

Sensitivity Analysis - Subarachnoid Hemorrhage

| Time from Onset of Symptoms (hr) | Number of Samples | SENSITIVITY with Specificity at 92% | SENSITIVITY with Specificity at 97% |
|---|---|---|---|
| 3 | 3 | 100.0 | 100.0 |
| 6 | 5 | 100.0 | 100.0 |
| 12 | 6 | 100.0 | 100.0 |
| 24 | 14 | 96.3 | 92.0 |
| 48 | 32 | 95.2 | 86.8 |
| All | 283 | 91.3 | 83.0 |

Sensitivity Analysis - Intracerebral Hemorrhage

| Time from Onset of Symptoms (hr) | Number of Samples | SENSITIVITY with Specificity at 92% | SENSITIVITY with Specificity at 97% |
|---|---|---|---|
| 3 | 3 | 100.0 | 100.0 |
| 6 | 5 | 100.0 | 100.0 |
| 12 | 6 | 100.0 | 100.0 |
| 24 | 13 | 96.3 | 92.0 |
| 48 | 24 | 89.9 | 78.3 |
| All | 60 | 87.2 | 86.4 |

Sensitivity Analysis - All Hemorrhagic Stroke

| Time from Onset of Symptoms (hr) | Number of Samples | SENSITIVITY with Specificity at 92% | SENSITIVITY with Specificity at 97% |
|---|---|---|---|
| 3 | 6 | 100.0 | 100.0 |
| 6 | 10 | 100.0 | 100.0 |
| 12 | 12 | 100.0 | 100.0 |
| 24 | 27 | 96.3 | 92.0 |
| 48 | 56 | 92.9 | 84.6 |
| A11 | 343 | 90.7 | 83.6 |

Sensitivity Analysis - All Stroke

| Time from Onset of Symptoms (hr) | Number of Samples | SENSITIVITY with Specificity at 92% | SENSITIVITY with Specificity at 97% |
|---|---|---|---|
| 3 | 12 | 100.0 | 91.7 |
| 6 | 29 | 100.0 | 96.6 |
| 12 | 48 | 93.8 | 91.7 |
| 24 | 87 | 90.8 | 88.5 |
| 48 | 152 | 90.1 | 84.2 |
| All | 518 | 90.4 | 83.8 |

The 6-marker and 4-marker panels were also evaluated for their ability to identify patients with transient ischemic attacks (TIAs). By nature, TIAs are ischemic events with short duration that do not cause permanent neurological damage. TIAs may be

characterized by the localized release of markers into the bloodstream that is interrupted with the resolution of the event. Therefore, it is expected that the sensitivity of the panel of markers would decrease over time. Both the 6-marker panel, with specificity set to 92%, and the 4-marker panel, with specificity set to 97%, exhibit significant decreases in sensitivity within the first 24 hours of the event, as described in Table 8. These decreases are not observed in any of the stroke populations described in Tables 3-7. The data indicate that the collection of data from patients at successive time points may allow the differentiation of patients with TIAs from patients with other stroke types. The identification of patients with TIAs is beneficial because these patients are at increased risk for a future stroke.

Sensitivity Analysis - TIA

| Time from Onset of Symptoms (hr) | Number of Samples | SENSITIVITY with Specificity at 92% | SENSITIVITY with Specificity at 97% |
|---|---|---|---|
| 0-6 | 9 | 100.0 | 88.9 |
| 6-12 | 7 | 57.1 | 57.1 |
| 12-24 | 8 | 37.5 | 37.5 |

Example 9. Markers for cerebral vasospasm in patients presenting with subarachnoid hemorrhage.

45 consecutive patients, 38 admitted to a hospital with aneurysmal subarachnoid hemorrhage (SAH), and 7 control patients admitted for elective aneurysm clipping, were included in this study. In all patients with SAH, venous blood samples were taken by venipuncture at time of hospital admission and daily thereafter for 12 consecutive days or until the onset of vasospasm. Development of cerebral vasospasm was defined as the onset of focal neurological deficits 4-12 days after SAH or transcranial doppler (TCD) velocities > 190 cm/s. In patients undergoing elective aneurysm clipping, $3 \pm 1$ venous blood samples were taken per patient over the course of a median of 13 days after surgery. Collected blood was centrifuged (10,000g), and the resulting supernatant was immediately frozen at -70°C until analysis was completed. Measurements of vWF, VEGF, and MMP-9 were performed using immunometric enzyme immunoassays.

To determine if any changes in plasma vWF, VEGF, and MMP-9 observed in a pre-vasospasm cohort were a result of pre-clinical ischemia or specific to the development of cerebral vasospasm, these markers were also measured in the setting of embolic or thrombotic focal cerebral ischemia. A single venous blood sample was taken by venipuncture at the time of admission from a consecutive series of 59 patients admitted within 24 hours of the onset of symptomatic focal ischemia. Forty-two patients admitted with symptomatic focal ischemia subsequently demonstrated MRI evidence of cerebral infarction. Seventeen patients did not demonstrate radiological evidence of cerebral infarction, experienced symptomatic resolution, were classified as transient ischemic attack, and therefore were not included in analysis.

Three cohorts were classified as non-vasospasm (patients admitted with SAH and not developing cerebral vasospasm), pre-vasospasm (patients admitted with SAH and subsequently developing cerebral vasospasm), and focal ischemia (patients admitted with symptomatic focal ischemia subsequently defined as cerebral infarction on MRI). Mean peak plasma vWF, VEGF, and MMP-9 levels were compared between cohorts by two-way ANOVA. The alpha error was set at 0.05. When the distribution had kurtosis, significant skewing, or the variances were significantly different, the non-parametric Mann Whitney U statistic for inter-group comparison was used. Correlations between Fisher grade and plasma markers were assessed by the Spearman Rank correlation coefficient. Logistic regression analysis adjusting for patient age, gender, race, Hunt and Hess, and Fisher grade was used to calculate the odds ratio of developing vasospasm per threshold of plasma marker.

Thirty eight patients were admitted and yielded their first blood sample 1 ±1 days after SAH. Of these, 22 (57%) developed cerebral vasospasm a median seven days (range, 4-11 days) after SAH. Eighteen (47%) developed focal neurological deficits and four (10%) demonstrated TCD evidence of vasospasm only. Three patients in the SAH, non-vasospasm cohort were Fisher grade 1 and were not included in inter-cohort plasma marker comparison. Patient demographics, clinical characteristics, and Fisher grades for the non-vasospasm and pre-vasospasm cohorts are given in the following table.

Demographics, clinical presentation, and radiographical characteristics of 38 patients admitted with SAH.

|  | SAH, Non-Vasospasm (n=16) | SAH, Pre-Vasospasm (n=22) |
|---|---|---|
| Age† | 56 ± 10 years | 54 ± 13 years |
| Female | 12 (75%) | 18 (82%) |
| Admission GCS‡ | 14 (11-15) | 12 (9-14) |
| Admission HH‡ | 2 (1-3) | 3 (2-4) |
| Fisher Grade‡ | 3 (2-3) | 3 (2-4) |

† Values given as Mean ± SD, GCS, Glasgow Coma Scale
‡ Values given as Median (interquartile range) HH, Hunt and Hess Scale

In the non-vasospasm cohort, mean peak plasma vWF (p=0.974), VEGF (p=0.357), and MMP-9 (p=0.763) were unchanged versus controls (Table 10). Plasma vWF, VEGF, and MMP-9 were increased in the pre-vasospasm versus non-vasospasm cohort (Table 10). Increasing Fisher grade correlated to greater peak plasma vWF (p<0.05), VEGF (p<0.01) and MMP-9 (p<0.05).

Additionally, twenty males and 22 females (age: 59 ± 15 years) presented within 24 hours of symptomatic focal ischemia with a mean NIH stroke scale score of 6.7 ± 6.6. In the focal ischemia cohort, mean peak plasma vWF (p=0.864), VEGF (p=0.469), and MMP-9 (p=0.623) were unchanged versus controls (Table 10). Plasma vWF, VEGF, and MMP-9 were markedly increased in the pre-vasospasm versus focal ischemia cohort, as shown in the following table.

Mean peak plasma markers in the non-vasospasm, pre-vasospasm, and focal ischemia cohorts. Control group given as reference.

|  | Focal Ischemia (n=87) | p Value Versus SAH pre | SAH, no Vasospasm (n=16) | p Value Versus SAH pre | SAH, pre-Vasospasm (n=22) | Controls (n=7) |
|---|---|---|---|---|---|---|
| vWF | 4645 ± 875 | 0.010 | 4934 ± 599 | 0.025 | 5526 ± 929 | 4865 ± 868 |
| VEGF | 0.03 ± 0.04 | 0.001 | 0.06 ± 0.06 | 0.023 | 0.12 ± 0.06 | 0.04 ± 0.0 6 |
| MMP-9 | 250 ± 308 | 0.001 | 438 ± 154 | 0.006 | 705 ± 338 | 408 ± 348 |

Following SAH, elevated plasma vWF, VEGF, and MMP-9 independently increased the odds of subsequent vasospasm 17 to 25 fold with positive predictive values ranging from 75% to 92%, as shown in the following table.

Positive/negative predictive values and odds ratio for subsequent onset of vasospasm associated with various levels of plasma vWF, VEGF, and MMP-9 by logistic regression analysis.

| Plasma Marker | p Value | Odds Ratio | PPV | NPV |
|---|---|---|---|---|
| vWF (ng/ml) |  |  |  |  |
| >5800 | 0.101 | 9.2 | 88% | 57% |
| >5500 | 0.033 | 17.6 | 92% | 67% |
| >5200 | 0.144 | 4.2 | 71% | 63% |
|  |  |  |  |  |
| VEGF (ng/ml) |  |  |  |  |
| >0.12 | 0.050 | 20.7 | 75% | 58% |
| >0.08 | 0.023 | 16.8 |  |  |
|  | 60% |  | 75% |  |

(continued)

| VEGF (ng/ml) | | | | |
|---|---|---|---|---|
| >0.06 | 0.064 | 7.3 | 64% | 73% |
| | | | | |
| MMP-9 (ng/ml) | | | | |
| >700 | 0.045 | 25.4 | 91% | 64% |
| >600 | 0.105 | 5.7 | 77% | 61% |
| >500 | 0.111 | 4.9 | 68% | 65% |

Example 10. Exemplary panels for diagnosing stroke.

The following tables demonstrate the use of methods of the present invention for the diagnosis of stroke. The "analytes panel" represents the combination of markers used to analyze test samples obtained from stroke patients and from non-stroke donors (NHD indicates normal healthy donor; NSD indicates non-specific disease donor). The time (if indicated) represents the interval between onset of symptoms and sample collection. ROC curves were calculated for the sensitivity of a particular panel of markers versus 1-(specificity) for the panel at various cutoffs, and the area under the curves determined. Sensitivity of the diagnosis (Sens) was determined at 92.5% specificity (Spec); and specificity of the diagnosis was also determined at 92.5% sensitivity.

## 3-Marker Analyte Panel – Analytes: Caspase-3, MMP-9, GFAP.

| Specimens | | Stroke vs NHD+NSD | | | Stroke vs NHD | | | Stroke vs NSD | | |
|---|---|---|---|---|---|---|---|---|---|---|
| Time Interval | | All Times | | | All Times | | | All Times | | |
| Stroke (n) | | 448 | | | 448 | | | 448 | | |
| non-Stroke (n) | | 338 | | | 236 | | | 102 | | |
| | | | | | | | | | | |
| Parameter | | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | | .944 | 85.7% | 85.2% | .955 | 86.6% | 89.0% | .919 | 75.0% | 76.5% |

| Specimens | Stroke vs NHD | | | Stroke vs NSD | | | Stroke vs NHD | | | Stroke vs NSD | | |
|---|---|---|---|---|---|---|---|---|---|---|---|---|
| Time Interval | 0-6 h | | | 0-6 h | | | 6-48 h | | | 6-48 h | | |
| Stroke (n) | 16 | | | 16 | | | 89 | | | 89 | | |
| non-Stroke (n) | 236 | | | 102 | | | 236 | | | 102 | | |
| | | | | | | | | | | | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .958 | 93.8% | 95.8% | .931 | 87.5% | 92.2% | .963 | 86.5% | 90.3% | .920 | 71.9% | 76.5% |

4-Marker Panel - Analytes:  Caspase-3, MMP-9, vWF-A1 and BNP.

| Specimens | Stroke vs NHD+NSD | | | Stroke vs NHD | | | Stroke vs NSD | | |
|---|---|---|---|---|---|---|---|---|---|
| Time Interval | All Times | | | All Times | | | All Times | | |
| Stroke (n) | 482 | | | 482 | | | 482 | | |
| non-Stroke (n) | 331 | | | 234 | | | 97 | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .963 | 92.9% | 92.7% | .980 | 94.6% | 96.6% | .923 | 74.7% | 83.5% |

| Specimens | Stroke vs NHD | | | Stroke vs NSD | | | Stroke vs NHD | | | Stroke vs NSD | | |
|---|---|---|---|---|---|---|---|---|---|---|---|---|
| Time Interval | 0-6 h | | | 0-6 h | | | 6-48 h | | | 6-48 h | | |
| Stroke (n) | 18 | | | 18 | | | 101 | | | 101 | | |
| non-Stroke (n) | 234 | | | 97 | | | 234 | | | 97 | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .968 | 94.4% | 96.6% | .912 | 77.8% | 83.5% | .987 | 98.0% | 97.0% | .937 | 76.2% | 85.6% |

6-Marker Panels:  Analytes as indicated.

| | Panel 1 | Panel 2 | Panel 3 | Panel 4 |
|---|---|---|---|---|
| NCAM | ✓ | ✓ | ✓ | ✓ |
| BDNF | ✓ | ✓ | ✓ | ✓ |
| Caspase-3 | ✓ | ✓ | ✓ | ✓ |
| MMP-9 | ✓ | ✓ | ✓ | ✓ |
| vWF-A1 | ✓ | ✓ | ✓ | |
| VEGF | ✓ | | | ✓ |
| S100 | | ✓ | | |
| vWF-Integrin | | | | ✓ |
| MCP1 | | | ✓ | |
| GFAP | | | | |

| | Panel 1 | | | Panel 2 | | | Panel 3 | | | Panel 4 | | |
|---|---|---|---|---|---|---|---|---|---|---|---|---|
| | Time | | | Time | | | Time | | | Time | | |
| | all | 0-6 | 6-48 | all | 0-6 | 6-48 | all | 0-6 | 6-48 | all | 0-6 | 6-48 |
| Stroke (n) | 372 | 25 | 106 | 372 | 25 | 106 | 372 | 25 | 106 | 362 | 25 | 106 |
| non-Stroke (n) | 109 | 109 | 109 | 109 | 109 | 109 | 109 | 109 | 109 | 109 | 109 | 109 |
| ROC Area | 0.940 | 0.985 | 0.946 | 0.955 | 0.988 | 0.952 | 0.948 | 0.986 | 0.944 | 0.952 | 0.985 | 0.948 |
| Sens @ 92.5% Spec | 94.6% | 100.0% | 90.6% | 95.2% | 100.0% | 96.2% | 95.3% | 100.0% | 93.4% | 93.6% | 100.0% | 95.3% |
| Spec @ 92.5% Sens | 92.7% | 98.2% | 90.8% | 93.6% | 98.2% | 92.7% | 92.7% | 98.2% | 93.6% | 92.7% | 97.2% | 92.7% |

| | Panel 5 | Panel 6 | Panel 8 | Panel 10 |
|---|---|---|---|---|
| NCAM | | | ✓ | ✓ |
| BDNF | ✓ | ✓ | ✓ | ✓ |
| Caspase-3 | | ✓ | | ✓ |
| MMP-9 | ✓ | ✓ | ✓ | ✓ |
| vWF-A1 | | ✓ | ✓ | |
| VEGF | | | | |
| S100 | ✓ | ✓ | ✓ | ✓ |
| vWF-Integrin | ✓ | | | |
| MCP1 | ✓ | | | |
| GFAP | ✓ | ✓ | ✓ | ✓ |

| | Panel 5 | | | Panel 6 | | | Panel 8 | | | Panel 10 | | |
|---|---|---|---|---|---|---|---|---|---|---|---|---|
| | Time | | | Time | | | Time | | | Time | | |
| | all | 0-6 | 6-48 | all | 0-6 | 6-48 | all | 0-6 | 6-48 | all | 0-6 | 6-48 |
| Stroke (n) | 109 | 109 | 109 | 109 | 109 | 109 | 109 | 109 | 109 | 109 | 109 | 109 |
| non-Stroke (n) | 360 | 25 | 105 | 367 | 25 | 106 | 367 | 25 | 106 | 367 | 25 | 106 |
| ROC Area | 0.940 | 0.984 | 0.944 | 0.937 | 0.963 | 0.937 | 0.953 | 0.982 | 0.941 | 0.947 | 0.979 | 0.948 |
| Sens @ 92.5% Spec | 94.6% | 100.0% | 86.7% | 94.6% | 100.0% | 94.3% | 92.9% | 100.0% | 94.3% | 94.0% | 100.0% | 93.4% |
| Spec @ 92.5% Sens | 92.7% | 97.2% | 90.8% | 92.7% | 93.6% | 92.7% | 92.7% | 96.3% | 92.7% | 92.7% | 95.4% | 92.7% |

7-Marker Panel - Analytes: Caspase-3, NCAM, MCP-1, S100-β, MMP-9, vWF-integrin and BNP.

| Specimens | Stroke vs NHD+NSD | | Stroke vs NHD | | Stroke vs NSD | |
|---|---|---|---|---|---|---|
| Time Interval | All Times | | All Times | | All Times | |
| Stroke (n) | 419 | | 419 | | 419 | |

| non-Stroke (n) | 324 | | | 207 | | | 117 | | |
|---|---|---|---|---|---|---|---|---|---|
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .953 | 88.3% | 89.5% | .962 | 92.6% | 92.8% | .937 | 79.5% | 83.8% |

| Specimens | Stroke vs NHD | | | Stroke vs NSD | | | Stroke vs NHD | | | Stroke vs NSD | | |
|---|---|---|---|---|---|---|---|---|---|---|---|---|
| Time Interval | 0-6 h | | | 0-6 h | | | 6-48 h | | | 6-48 h | | |
| Stroke (n) | 21 | | | 21 | | | 86 | | | 86 | | |
| non-Stroke (n) | 207 | | | 117 | | | 207 | | | 117 | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .930 | 85.7% | 77.8% | .900 | 81.0% | 62.4% | .972 | 96.5% | 92.8% | .948 | 82.6% | 83.8% |

7-Marker Panel - Analytes: Caspase-3, NCAM, MCP-1, S100-β, MMP-9, vWF-integrin and BNP.

| | Analyte | | Stroke vs NHD | Stroke vs NHD+ NSD | Stroke vs NHD | Stroke vs NHD | Stroke vs NHD | Stroke vs NHD | Stroke vs NHD |
|---|---|---|---|---|---|---|---|---|---|
| | Caspase | | x | x | x | x | x | x | x |
| | NCAM | | x | x | x | x | x | x | x |
| | MCP-1 | | x | x | x | x | x | x | x |
| | S-100b | | x | x | x | x | x | x | x |
| | MMP-9 (omni)* | | | | x | | | | |
| | MMP-9 (18/16)** | | x | x | | | | | |
| | MMP-9 (18/17)*** | | | | | x | | | |
| | MMP-9 (omni+18/16) | | | | | | x | | |
| | MMP-9 (omni+18/17) | | | | | | | x | |
| | MMP-9 (18/16+18/17) | | | | | | | | x |

(continued)

|  | Analyte |  | Stroke vs NHD | Stroke vs NHD+ NSD | Stroke vs NHD | Stroke vs NHD | Stroke vs NHD | Stroke vs NHD | Stroke vs NHD |
|---|---|---|---|---|---|---|---|---|---|
|  | vWF-Integrin |  | x | x | x | x | x | x | x |
|  | BNP |  | x | x | x | x | x | x | x |
|  |  |  |  |  |  |  |  |  |  |
|  |  |  |  |  |  |  |  |  |  |
| All Times | Stroke (n) |  | 419 | 419 | 500 | 427 | 417 | 425 | 418 |
|  | non-Stroke(n) |  | 207 | 324 | 248 | 208 | 207 | 208 | 207 |
|  | ROC Area |  | 0.991 | 0.953 | 0.987 | 0.990 | 0.993 | 0.995 | 0.990 |
|  | Sens @ 92.5% Spec |  | 97.4% | 88.3% | 97.2% | 97.9% | 99.0% | 98.4% | 97.4% |
|  | Spec @ 92.5% Sens |  | 99.9% | 89.5% | 97.6% | 99.0% | 99.5% | 99.5% | 99.0% |
|  |  |  |  |  |  |  |  |  |  |
| 0-6 hours | Stroke(n) |  | 21 | 21 | 24 | 21 | 21 | 21 | 21 |
|  | non-Stroke (n) |  | 207 | 324 | 248 | 208 | 207 | 208 | 207 |
|  | ROC Area |  | 1.000 | 0.939 | 1.000 | 1.000 | 1.000 | 1.000 | 1.000 |
|  | Sens @ 92.5% Spec |  | 100.0% | 95.2% | 100.0% | 100.0% | 100.0% | 100.0% | 100.0% |
|  | Spec @ 92.5% Sens |  | 100.0% | 96.0% | 100.0% | 100.0% | 100.0% | 100.0% | 100.0% |
|  |  |  |  |  |  |  |  |  |  |
| 6-48 hours | Stroke (n) |  | 86 | 86 | 102 | 90 | 85 | 89 | 86 |
|  | non-Stroke(n) |  | 207 | 324 | 248 | 208 | 207 | 208 | 207 |
|  | ROC Area |  | 0.996 | 0.969 | 0.986 | 0.998 | 0.999 | 0.999 | 0.999 |
|  | Sens @ 92.5% Spec |  | 100.0% | 96.5% | 98.0% | 100.0% | 100.0% | 100.0% | 100.0% |
|  | Spec @ 92.5% Sens |  | 98.1% | 94.1% | 98.4% | 99.5% | 100.0% | 100.0% | 99.0% |

* - Recognizes all forms of MMP-9
* - Recognizes all forms of MMP-9 except active MMP-9
* - Recognizes all forms of MMP-9 except MMP-9/TIMP complexes

8-Marker Panel - Analytes: Caspase-3, NCAM, MCP-1, S100-β, MMP-9, vWF-A1, BNP and GFAP.

| Specimens | Stroke vs NHD+NSD | | | Stroke vs NHD | | | Stroke vs NSD | | |
|---|---|---|---|---|---|---|---|---|---|
| Time Interval | All Times | | | All Times | | | All Times | | |
| Stroke (n) | 368 | | | 380 | | | 380 | | |
| non-Stroke (n) | 298 | | | 214 | | | 93 | | |
| | | | | | | | | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .970 | 93.9% | 94.5% | .980 | 94.2% | 96.3% | .947 | 80.3% | 90.3% |

| Specimens | Stroke vs NHD | | | Stroke vs NSD | | | Stroke vs NHD | | | Stroke vs NSD | | |
|---|---|---|---|---|---|---|---|---|---|---|---|---|
| Time Interval | 0-6 h | | | 0-6 h | | | 6-48 h | | | 6-48 h | | |
| Stroke (n) | 15 | | | 15 | | | 76 | | | 76 | | |
| non-Stroke (n) | 214 | | | 93 | | | 214 | | | 93 | | |
| | | | | | | | | | | | | |
| Parameter | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens | Area | Sens @ 92.5% Spec | Spec @ 92.5% Sens |
| Value | .961 | 93.3% | 96.7% | .927 | 86.7% | 92.5% | .989 | 98.7% | 96.3% | .960 | 80.3% | 90.3% |

Additional stroke panels may be provided using 3, 4, 5, 6, 7, 8, or more markers selected from the group consisting of IL-1ra, C-reactive protein, von Willebrand factor (vWF), Tweak, creatine kinase-BB, c-Tau, D-dimer, thrombus precursor protein, vascular endothelial growth factor (VEGF), matrix metalloprotease-9 (MMP-9), neural cell adhesion molecule (NCAM), BNP, S100β, and caspasc-3. The following exemplary panels are provided for thye diagnosis of ischemic stroke, using normal healthy donor samples as a "control" group.

| Panel # | 1 | 2 | 3 |
|---|---|---|---|
| Markers in panel | CRP, NCAM, D-dimer, BNP, CK-BB, Capase-3, c-Tau, S-100β | CRP, NCAM, D-dimer, BNP, CK-BB, VEGF, MMP-9, S-100β | CRP, D-dimer, BNP, CK-BB, S-100β |
| Control n | 76 | 76 | 76 |
| Disease n | 40 | 40 | 43 |
| Ave ROC Area | 0.976 | 0.968 | 0.960 |
| SD(%) | 0.008 | 0.007 | 0.006 |
| Ave Sens @ 92.5% Spec | 94.1 | 93.6 | 93.4 |
| SD(%) | 1.40 | 1.30 | 0.90 |
| Ave Spec @ 92.5% Sens | 98.7 | 99.5 | 96.0 |
| SD(%) | 1.40 | 0.80 | 1.30 |

| Panel # | 4 | 5 | 6 |
|---|---|---|---|
| Markers in panel | CRP, IL-1ra, D-dimer, CK-BB, MMP-9, S-100β | CRP, D-dimer, Caspase-3, CK-BB, VEGF, MMP-9, S-100β | CRP, D-dimer, MMP-9, CK-BB, S-100β |
| Control n | 76 | 76 | 76 |
| Disease n | 43 | 43 | 43 |
| Ave ROC Area | 0.947 | 0.957 | 0.954 |
| SD(%) | 0.096 | 0.007 | 0.007 |
| Ave Sens @ 92.5% Spec | 92.1 | 94.5 | 93.0 |
| SD(%) | 9.50 | 1.90 | 2.30 |
| Ave Spec @ 92.5% Sens | 93.1 | 94.8 | 93.9 |
| SD(%) | 9.60 | 1.70 | 1.60 |

| Panel # | 7 | 8 | 9 |
|---|---|---|---|
| Markers in panel | CRP, BNP, D-dimer, CK-BB, MMP-9, S-100β | CRP, D-dimer, IL-1ra, CK-BB, MMP-9, S-100β | CRP, D-dimer, NCAM, BNP, S-100β |
| Control n | 76 | 76 | 76 |
| Disease n | 43 | 43 | 41 |
| Ave ROC Area | 0.960 | 0.947 | 0.960 |
| SD(%) | 0.006 | 0.096 | 0.005 |
| Ave Sens @ 92.5% Spec | 93.4 | 92.1 | 92.5 |
| SD(%) | 0.90 | 9.50 | 0.60 |
| Ave Spec @ 92.5% Sens | 96.0 | 93.1 | 96.6 |
| SD(%) | 1.30 | 9.60 | 2.40 |

| Panel # | 10 |
|---|---|
| Markers in panel | CRP, D-dimer, CK-BB, Caspase-3, MMP-9, S-100β |
| Control n | 76 |
| Disease n | 43 |
| Ave ROC Area | 0.957 |
| SD(%) | 0.007 |
| Ave Sens @ 92.5% Spec | 94.5 |
| SD(%) | 1.90 |
| Ave Spec @ 92.5% Sens | 94.8 |
| SD(%) | 1.70 |

Example 11. Exemplary panels for differentiating ischemic stroke versus hemorrhagic stroke

The following table demonstrates the use of methods of the present invention for the differentiation of different types of stroke, in this example ischemic stroke versus hemorrhagic stroke. The "analyte panel" represents the combination of markers used to analyze test samples obtained from ischemic stroke patients and from hemorrhagic stroke patients. Sensitivity of the diagnosis (Sens) was determined at 92.5% specificity (Spec); and specificity of the diagnosis was also determined at 92.5% sensitivity.

| | | | Ischemic vs. Hemorrhagic stroke | | | |
|---|---|---|---|---|---|---|
| | | | Run set 1 | Run set 2 | Run set 3 | Run set 4 |
| Analyte panel: | | CRP | x | x | x | x |
| | | NT-3 | x | | | x |
| | | vWF-total | x | | | |
| | | MMP-9 | x | x | x | x |
| | | VEGF | x | x | x | x |
| | | CKBB | x | x | x | x |
| | | MCP-1 | | x | x | x |
| | | Calbindin | | | x | |
| | | vWF-VP1 | | | x | |
| | | vWF A3 | | | x | |
| | | vWF A1-A3 | | | x | |
| | | Thrombin-antithrombin III complex | | | x | |
| | | Proteolipid protein | | | x | |
| | | IL-6 | | | x | |
| | | IL-8 | | | x | |
| | | Myelin Basic Protein | | | x | |
| | | S-100b | | | x | |
| | | Tissue factor | | | x | |
| | | GFAP | | | x | |

(continued)

| | | | Ischemic vs. Hemorrhagic stroke | | | |
|---|---|---|---|---|---|---|
| | | | Run set 1 | Run set 2 | Run set 3 | Run set 4 |
| | | vWF AI-integrin | | | x | |
| | | CNP | | | x | |
| | | NCAM | | x | | |
| | | | | | | |
| | | | | | | |
| All Times | N | Hemorrhagic stroke | 209 | 196 | 182 | 197 |
| | | Ischemic stroke | 114 | 110 | 122 | 109 |
| | | ROC Area | 0.898 | 0.867 | 0.920 | 0.882 |
| | | Sens @ 92.5% Spec | 75.1% | 62.2% | 77.9% | 64.0% |
| | | Spec @ 92.5% Sens | 77.2% | 71.8% | 85.7% | 72.5% |
| | | | | | | |

Example 12. Exemplary panels for diagnosing acute stroke

The primary endpoint in this study was the presence of clinical stroke, as defined by focal neurological signs or symptoms felt to be of vascular origin that persisted for greater than 24 hours. Blood samples from patients with stroke were stratified into two categories based on the latency from symptom onset to blood draw: less than six hours (16 samples), and 6-24 hours (38 samples). Control patients initially suspected of having a stroke but not meeting the clinical criteria served as controls. These 21 included patients with TIA (13 patients); syncope (n=1), and other (n=7). The control group was enriched with patients without vascular disease (n= 157).

Following obtaining informed consent, phlebotomy was performed and collected blood was centrifuged (10,000g), and the resulting supernatant immediately frozen at -70°C until analysis was completed as described previously (Grocott et al., 2001, McGirt et al., 2002). Measurements of biochemical markers were performed by Biosite Diagnostics (San Diego, CA) using a Genesis Robotic Sample Processor 200/8 (Tecan; Research Triangle park, NC). All assays were performed in a 10-$\mu$L reaction volume in 384-well microplates, with the amount of bound antigen detected by means of alkaline phosphatase-conjugated secondary antibodies and AttoPhos substrate (JBL Scientific, San Luis Obispo, CA).

Descriptive statistics, including frequencies and percentages for categorical data, as well as the mean and standard deviation, median, 1st and 3rd quartiles, and the minimum and maximum values for continuous variables, were calculated for all demographic and sample assay data. Demographic variables were compared by Wilcoxon test (age) or Chi-Squared test for categorical variables. Distributions of marker values were examined for outliers and non-normality. The ability to distinguish stroke by marker levels at a given sample period was tested in stages in this exploratory study in order to minimize overtesting. First, each marker was tested as the single predictor in a univariate logistic regression. Based on these results, on the clinical characteristics of the markers, and on correlation with other markers, a set of 3 markers was selected for testing in a multivariable logistic model. Non-significant markers were removed from this model and up to 2 more markers were tested additionally to arrive at a final model providing the greatest stability of estimates and predictive utility. Correlations among the included markers were checked to avoid collinearity, and influence statistics (change in Chi-Square) were examined to guard against undue influence of any one observation. Finally the validity of the model was checked by bootstrapping. Fifty test datasets of the same size as the analysis dataset were generated by random selection with replacement from the analysis dataset. Then the model was fit on each "bootstrapped" dataset, and the results inspected for consistency. In this manner separate models were developed for two time periods of marker sampling at which sufficient numbers of stroke samples were available, 0-6 hours and 6-24 hours. Multiple samples from the same patient were not used in the same analysis, preserving independence in each analysis. Where multiple samples were available from the same patient within the same time period, only the sample closest to the start of the time period was used in the analysis. To investigate the association of time after onset of symptoms with the level of serum markers, a dataset was prepared including all samples from 0-24 hours after onset for all patients with stroke. The time association was initially inspected for each marker using a Spearman rank correlation; correlations with p<0.10 were then tested with a repeated-measures multivariable regression procedure to account for non-independence of some samples.

The patient demographics from the acute (0-6 hours from symptom onset to blood collection), and subacute (6-24 hours from symptom onset to blood collection were comparable. Male patients were less likely to be diagnosed with clinical stroke in both data sets, whereas prior history of myocardial infarct and African American race were associated with increased incidence of stroke. Patient demographics for the data set in which blood was collected acutely (within six hours of symptom onset), and subacutely (between six and twenty four hours after symptom onset. There was no significant difference in age between patients with clinical stroke and patients without stroke in either data set (age expressed as mean $\pm$ standard deviation). There was an increased proportion of male patients in both subacute and acute patients without stroke. An increased proportion of stroke patients in both data sets were African American, and had a prior incidence of myocardial infarction.

| | (0-6 hours) | | | (6-24) hours | | |
|---|---|---|---|---|---|---|
| | Stroke (n=16) | No Stroke (n=165) | p | Stroke (n=38) | No Stroke (n=176) | p |
| Age | 62 $\pm$ 15 | 63.3 $\pm$ 8 | NS | 63 $\pm$ 5 | 62$\pm$9 | NS |
| Male Gender (%) | 37.5 | 67.7 | 0.026 | 42.1 | 68 | 0.005 |
| History of MI (%) | 30.8 | 1.2 | <0.001 | 37.1 | 2.3 | <0.001 |
| Race (%) | | | <0.001 | | | |
| White | 37.5 | 91.9 | | 44.7 | 89.5 | <0.001 |
| African-American | 62.5 | 3.8 | | 52.6 | 6.4 | |
| Other | 0 | 4.4 | | 2.6 | 4.1 | |

Twenty six biochemical markers involved in pathogenesis of stroke and neuronal injury were prospectively defined and divided into one of six categories: markers of glial activation, non-specific mediators of inflammation; markers of thrombosis or impaired hemostasis, markers of cellular injury; markers of peroxidized lipid/myelin breakdown; markers of apoptosis/ miscellaneous. The univariate logistic analysis demonstrated four markers that were highly correlated with stroke (p<0.001) at both time periods. These included one marker of glial activation (S100β), two markers of inflammation (vascular cell adhesion molecule, IL-6), and Won Willebrand factor (vWF). In addition, several markers were differentially upregulated as a function of time. Specifically, caspase 3, a marker of apoptosis, increased as a function of time (over a 24 hour period from symptom onset to blood draw), suggesting an increasing volume of irreversibly damaged tissue.

Two data sets were created representing serum collected from patients that presented acutely (blood drawn within six hours) and subacute stroke (blood drawn between six and twenty four hours). Markers of glial activation and inflammation were assayed in the blood of patients presenting with suspected cerebral ischemia, and univariate logistic regression performed for each marker. Given the non-normal distribution of many of the assays, data is presented as median $\pm$ interquartile range; significance represents unadjusted p value from each univariate logistic model. P>0.05 is assumed to be non-significant (NS).

| | (0-6 hours) | | | (6-24) hours | | |
|---|---|---|---|---|---|---|
| | Stroke (n= | No Stroke | p | Stroke (n=16) | No stroke (n=165) | p |
| | Median (25th, 75th percentile) | | | Median (25th, 75th percentile) | | |
| *Glial markers (unit)* | | | | | | |
| S100b (pg/ml) | 42.9 (9.0,48.7) | 0 (0,0) | <0.001 | 27.3 (9, 88) | 0 (0,0) | <0.00 1 |
| Glial fibrillary acidic protein (pg/ml) | 488.9 (0,1729) | 110.2 (0,395.1) | 0.025 | 666.9 (188,1327) | 96.8 (0,398) | 0.002 |

(continued)

| Inflammatory Mediators (unit) | | | | | | |
|---|---|---|---|---|---|---|
| (Matrix metalloproteinase 9 (MMP 9; ng/ml) | 253.0 (138,524) | 70.0 (26,109) | <0.001 | 176.8 (111,327) | 74 (27,113.7) | <0.00 1 |
| Vascular cell adhesion molecule (VCAM; $\mu$g/ml) | 2.2 (1.8,23) | 1.3 (1,1.56) | <0.001 | 2.0 (1.6,2.4) | 1.3 (1.0, 1.7) | <0.00 1 |
| Interleukin 6 (I1-6; pg/ml) | 20.4 (11.4,56) | 0.1 (0.1,9.4) | 0.039 | 33.1 (6.8,73.2) | 0.1 (0.1.11.4) | 0.008 |
| Tumor necrosis factor (TNF$\alpha$; pg/ml) | 31.2 (5.7,54.1) | 0.1 (0.1,15.5) | 0.016 | 29.8 (3.3,55) | 0.1 (0.1,17.7) | 0.039 |
| Neuronal cell adhesion molecule (NCAM, ng/ml) | 51.4 (45.6,60) | 52.0 (51.1,53) | NS | 49.3 (46,57) | 51.9 (51,52.9) | NS |
| Interleukin 1 receptor antagonist (IL-1ra, pg/ml) | 0 (0,1281) | 221.9 (0,693.7) | NS | 88.2 (0,927) | 180.8 (0,699) | NS |
| Interleukin 1$\beta$ (IL-1$\beta$; pg/ml) | 1.9 (0.2,5) | 0.1 (0.1,3.6) | NS | 0.1 (0.1,4.9) | 0.1 (0.1,4.2) | NS |
| Interleukin 8 (IL8; pg/ml) | 30.1 (10.1,39) | 2.0 (0.1,18.4) | NS | 18.2 (6.7,46) | 1.4 (0.1, 17.8) | NS |
| Monocyte chemoattractant protein- (MCP-1; pg/ml) | 203.7 (133,255) | 115.1 (79,164) | NS | 144.9 (104,222) | 114.4 (79,162) | NS |
| Vascular endothelial growth factor (VEGF; ng/ml) | 0 (0,0) | 0.1 (0,0.2) | 0.008 | 0 (0,0) | 0.1 (0,0.1) | 0.002 |

Two data sets were created representing serum collected from patients that presented acutely (blood drawn within six hours) and subacute stroke (blood drawn between six and twenty four hours). Markers of acute cerebral ischemia, including apoptosis, myelin breakdown and peroxidation, thrombosis, and cellular were assayed in the blood of patients presenting with suspected cerebral ischemia, and univariate logistic regression performed for each marker. Given the non-normal distribution of many of the assays, data is presented as median $\pm$ interquartile range; signifacance represents unadjusted p value from each univariate logistic model. P>0.05 is assumed to be non-significant (NS).

| | (0-6 hours) | | | (6-24) hours | | |
|---|---|---|---|---|---|---|
| | Stroke | No Stroke | p | Stroke (n=16) | No Stroke (n=165) | p |
| | Median (25th, 75th percentile) | | | Median (25th, 75th percentile) | | |
| *Markers of thrombosis* (unit) | | | | | | |
| Von Willebrand factor (vWFal; ng/ml)) | 7991 (6964,9059) | 5462 (4794, 6332) | <0.001 | 7720.7 (7036,8986) | 5498.8 (4815,6404) | <0.001 |
| Thrombin-antithrombin III (ng/ml) | 95 (33,151) | 15 (0.3, 38) | NS | 69.2 (39,89) | 16.5 (0.9,40.7) | NS |
| D-Dimer (ng/ml) | 2840 (2323,3452) | 3108 (2621, 4037) | NS | 2684.3 (2296,3421) | 3112.7 (2633,3955) | NS |
| *Markers of cellular injury and myelin breakdown* (unit) | | | | | | |
| Creatinine phosphokinase; brain band (CKBB; ng/ml) | 3.5 (1.3,4.4) | 0.5 (0.1,107) | 0.03 | 1.7 (0.2,3.8) | 0.5 (0.1,1.6) | 0.04 |
| Tissue factor (pg/ml) | 5766 (2828, 10596) | 9497 (5309, 19536) | NS | 4142.8 (2894,6333) | 9085.5 (4572,17264 ) | 0.013 |
| Myelin basic protein (ng/ml) | 3.1 (0.3,6.4) | 0 (0,2.8) | NS | 2.9 (0,5.5) | 0 (0,2.8) | NS |
| Proteolipid protein (RU)) | 0.1 (0.1,0.2) | 0.2 (0.1,0.6) | NS | 0.1 (0.1,0.3) | 0.2 (0.1,0.6) | NS |
| Malendialdehyde (μg/ml) | 28 (20,35) | 23 (20,26) | 0.02 | 27.7 (24.8,31.3) | 23.8 (20.1,27.2) | 0.02 |
| | | | | | | |
| *Markers of apoptosis, growth factors, miscellaneous* (unit) | | | | | | |
| Brain natriuretic peptide (BNP; pg/ml) | 53 (24,227) | 28 (21,39) | 0.019 | 120.4 (33.9,306) | 27.4 (21.1,39.2) | <0.001 |
| Caspase 3 (ng/ml) | 7.7 (4.4, 16.7) | 4.5 (3.0, 7.0) | NS | 8.1 (4.9,35.4) | 4.7 (3.0, 7.4) | 0.002 |
| | | | | | | |
| Calbindin-D (pg/ml) | 2493 (1406, 4298) | 3003 (2287, 4276) | NS | 3080.8 (1645, 3950) | 2982 (2312, 4186) | NS |

(continued)

| Markers of apoptosis, growth factors, miscellaneous (unit) | | | | | | |
|---|---|---|---|---|---|---|
| Heat shock protein 60 (HSP 60; ng/ml) | 0.1 (0,13.3) | 0 (0,0) | NS | 0 (0,15.9) | 0 (0,0) | NS |
| Cytochrome C (ng/ml) | 0 (0,0.1) | 0 (0,0) | NS | 0 (0,0.2) | 0 (0,0) | NS |
| | | | | | | |

To maximize the sensitivity and sensitivity of a diagnostic test utilizing these markers, we next created a three variable panel of stroke biomarkers using multivariable logistic regression as described above. For acute patients (time from symptom onset to blood draw less than or equal to six hours), sensitivity and specificity was optimized using the variables of MMP9, vWF, and VCAM; wherein the concentration of a marker is directly related to a predicted probability of stoke. Each of these variables contributed to the model significantly and independently (Table 21). The overall model Likelihood ratio chi-square for this logistic model was 71.4 (p < 0.0001), goodness of fit was confirmed at p=0.9317 (Hosmer & Lemeshow test), and the concordance was almost 98%(c=0.979). When the outcome probability level was set to a cutoff of 0.1, this model provided a sensitivity of 87.5% and a specificity of 91.5% for predicting stroke as clinically defined (focal neurological symptoms resulting from cerebral ischemia lasting greater than 24 hours). The bootstrapping validation showed all 50 trials with model p <0.0001 and all 50 concordance indexes >94%. MMP-9 was significant (p<0.05) in 43 samples out of 50, VCAM in 43/50, and vWFa1 in 35/50.

Confidence interval for odds ratios, in units of 1 standard deviation of predictor. A logistic regression model was created from the data set of all patients in which blood was drawn within six hours from symptom onset. The odds ratio for each of the three covariates (MMP9, vWF, and VCAM) is presented per unit of one standard deviation.

| Effect | Unit (1 sd) | Odds Ratio | Lower CL | Upper CL | p-Value |
|---|---|---|---|---|---|
| MMP9 | 137.0 | 13.202 | 3.085 | 98.035 | 0.0026 |
| VCAM | 0.5900 | 4.104 | 1.793 | 12.721 | 0.0045 |
| vWFa1 | 1462.0 | 3.581 | 1.590 | 9.450 | 0.0036 |

In similar fashion, a logistic regression model was developed for patients with subacute symptoms (6-24 hours elapsed from symptom onset to blood draw). For this time period, sensitivity and specificity was optimized using the variables of S I 00b, VCAM, and vWFal. Each of which contributed to the model significantly and independently (Table 22). The overall model Likelihood ratio chi-square for this logistic model was 95.1 (p < 0.0001), goodness of fit was confirmed at p=0.2134 (Hosmer & Lemeshow test), and the concordance was 95%(c=0.953). With the outcome probability level set to a cutoff of 0.1, this model provided a sensitivity of 97.1% and a specificity of 87.4% for discriminating stroke. The bootstrapping validation showed all 50 trials with model p <0.0001 and all 50 concordance indexes >89%. S100b was significant (p<0.05) in 47 samples out of 50, VCAM in 45/50, and vWFa1 in 49/50.

Confidence interval for odds ratios, in units of 1 standard deviation of predictor. A logistic regression model was created from the data set of all patients in which blood was drawn between six and twenty four hours from symptom onset. The odds ratio for each of the three covariates (S100β, vWF, and VCAM) is presented per unit of one standard deviation.

| Effect | Unit (1 sd) | Odds Ratio | Lower CL | Upper CL | p-Value |
|---|---|---|---|---|---|
| S100b | 65.0 | 6.371 | 2.225 | 26.246 | 0.0024 |
| VCAM | 0.660 | 2.423 | 1.417 | 4.380 | 0.0020 |
| vWFa1 | 1621.0 | 3.180 | 1.934 | 5.674 | <.0001 |

Example 13. Exemplary panels for differentiating between acute and non-acute stroke

Using the methods described in U.S. Patent Application No. 10/331,127, entitled METHOD AND SYSTEM FOR DISEASE DETECTION USING MARKER COMBINATIONS (attorney docket no. 071949-6802), filed December 27,2002, exemplary panels for differentiating between acute and non-acute stroke was identified. Starting with a large number of potential markers (e.g., 19 different markers) an iterative procedure was applied. In this procedure, individual threshold concentrations for the markers are not used as cutoffs *per se,* but are used as values to which the assay values for each patient are compared and normalized. A window factor was used to calculate the minimum and maximum values above and below the cutoff. Assay values above the maximum are set to the maximum and assay values below the minimum are set to the minimum. The absolute values of the weights for the individual markers adds up to l. A negative weight for a marker implies that the assay values for the control group are higher than those for the diseased group. A "panel response" is calculated using the cutoff, window, and weighting factors. The panel responses for the entire population of patients and controls are subjected to ROC analysis and a panel response cutoff is selected to yield the desired sensitivity and specificity for the panel. After each set of iterations, the weakest contributors to the equation are eliminated and the iterative process starts again with the that will still result in acceptable sensitivity and specificity of the panel is obtained.

The panel composition for identifying acute stroke (0-12 hours) comprised the following markers: BNP, GFAP, IL-8, β-NGF, vWF-A1, and CRP, while the panel composition for identifying non-acute stroke (12-24 hours) comprised the following markers: BNP, GFAP, IL-8, CK-BB, MCP-1, and IL-1ra. A positive result was identified as being at least 90% sensitivity at 94.4% specificity. As shown below, the markers employed can provide panels to identify acute stroke, identify non-acute stroke, and/or differentiate between acute and non-acute stroke.

0-12 hour panel results

| All Stroke | | | |
|---|---|---|---|
| Time from Onset | # of Mimic Subjects | # of Stroke Subjects | Sensitivity @ 94.4% Specifcity |
| 0-3 h | 54 | 6 | 100.0% |
| 0-6 h | 54 | 13 | 100.0% |
| 0-12 h | 54 | 24 | 95.8% |
| 12-24 h | 54 | 19 | 68.4% |
| | | | |
| Ischemic Stroke | | | |
| Time from Onset | # of Mimic Subjects | # of Stroke Subjects | Sensitivity @ 94.4% Specifcity |
| 0-3 h | 54 | 5 | 100.0% |
| 0-6 h | 54 | 11 | 100.0% |
| 0-12 h | 54 | 20 | 95.0% |
| 12-24 h | 54 | 17 | 64.7% |
| | | | |
| Hemorrhagic Stroke | | | |
| Time from Onset | # of Mimic Subjects | # of Stroke Subjects | Sensitivity @94.4% Specifcity |
| 0-3 h | 54 | 1 | 100.0% |
| 0-6 h | 54 | 2 | 100.0% |
| 0-12 h | 54 | 4 | 100.0% |
| 12-24 h | 54 | 2 | 100.0% |
| | | | |

(continued)

| 0-12 h Panel Coefficients | | | |
|---|---|---|---|
| Marker | Cutoff | Window | Weight |
| BNP | 97.13 | 0.07 | 0.15 |
| vWF-A1 | 29.35 | 0.25 | -0.07 |
| GFAP | 2.64 | 0.22 | 0.07 |
| BNGF | 0.13 | 0.88 | -0.20 |
| IL-8 | 140.32 | 0.00 | 0.21 |
| CRP | 43.68 | 0.92 | 0.30 |

### 12-24 hour panel results

| All Stroke | | | |
|---|---|---|---|
| Time from Onset | # of Mimic Subjects | # of Stroke Subjects | Sensitivity @ 94.4% Specifcity |
| 0-3 h | 20 | 6 | 83.3% |
| 0-6 h | 20 | 13 | 69.2% |
| 0-12 h | 20 | 25 | 76.0% |
| 12-24 h | 20 | 19 | 100.0% |
| | | | |
| Ischemic Stroke | | | |
| Time from Onset | # of Mimic Subjects | # of Stroke Subjects | Sensitivity @94.4% Specifcity |
| 0-3 h | 20 | 5 | 100.0% |
| 0-6 h | 20 | 11 | 72.7% |
| 0-12 h | 20 | 21 | 76.2% |
| 12-24 h | 20 | 17 | 100.0% |
| | | | |
| Hemorrhagic Stroke | | | |
| Time from Onset | # of Mimic Subjects | # of Stroke Subjects | Sensitivity @ 94.4% Specifcity |
| 0-3 h | 20 | 1 | 0.0% |
| 0-6 h | 20 | 2 | 50.0% |
| 0-12 h | 20 | 4 | 75.0% |
| 12-24 h | 20 | 2 | 100.0% |
| | | | |
| 12-24 h Panel Coefficients | | | |
| Marker | Cutoff | Window | Weight |
| MCP-1 | 67.93 | 0.69 | -004 |
| BNP | 203.00 | 0.79 | 0.21 |

(continued)

| 12-24 h Panel Coefficients | | | |
|---|---|---|---|
| Marker | Cutoff | Window | Weight |
| GFAP | 1.71 | 0.79 | 0.27 |
| IL-8 | 97.51 | 0.07 | 0.08 |
| CK-BB | 0.48 | 0.14 | -0.14 |
| IL-1ra | 367.11 | 0.68 | -0.26 |

Alternative exemplary panels for differentiating between a 0-6 time of stroke onset and post-6 hour stroke onset were also identified. The panel composition for identifying acute stroke (0-6 hours) comprised the following markers: BNP, GFAP, CRP, CK-BB, MMP-9, IL-8, and β-NGF, while the panel composition for identifying non-acute stroke (6-24 hours) comprised the following markers: BNP, GFAP, CRP, CK-BB, Caspase-3, MCP-1, and vWF-integrin. A positive result was identified as being at least 90% sensitivity at 94.4% specificity. As shown below in Tables 25 and 26, the markers employed can provide panels to identify acute stroke in the 0-6 hour window, identify stroke outside this window, and/or differentiate between time of onset windows.

0-6 hour panel results

| All Stroke | | | |
|---|---|---|---|
| Time from Onset | # of Mimic Subjects | # of Stroke Subjects | Sensitivity @ 94.4% Specifcity |
| 0-3 h | 55 | 13 | 92.3% |
| 0-6 h | 55 | 33 | 97.0% |
| 6-24 h | 55 | 76 | 65.8% |
| | | | |
| Ischemic Stroke | | | |
| Time from Onset | # of Mimic Subjects | # of Stroke Subjects | Sensitivity @94.4% Specifcity |
| 0-3 h | 55 | 11 | 90.9% |
| 0-6h | 55 | 25 | 96.0% |
| 6-24h | 55 | 51 | 64.7% |
| | | | |
| Hemorrhagic Stroke | | | |
| Time from Onset | # of Mimic Subjects | # of Stroke Subjects | Sensitivity @ 94.4% Specifcity |
| 0-3 h | 55 | 2 | 100.0% |
| 0-6h | 55 | 8 | 100.0% |
| 6-24 h | 55 | 25 | 68.0% |
| | | | |
| 0-6 h Panel Coefficients | | | |
| Marker | Cutoff | Window | Weight |
| BNP | 119,16 | 0.51 | 0.09 |
| MMP-9 | 203.57 | 0.12 | -0.08 |

(continued)

| 0-6 h Panel Coefficients | | | |
|---|---|---|---|
| Marker | Cutoff | Window | Weight |
| GFAP | 7.22 | 0.00 | 0.18 |
| BNGF | 0.05 | 0.00 | -0.14 |
| IL-8 | 32.41 | 0.00 | 0.12 |
| CK-BB | 1.69 | 0.90 | 0.16 |
| CRP | 34.86 | 0.00 | 0.24 |

6-24 hour panel results

| All Stroke | | | |
|---|---|---|---|
| Time from Onset | # of Mimic Subjects | # of Stroke Subjects | Sensitivity @ 94.4% Specifcity |
| 0-3 h | 55 | 11 | 63.6% |
| 0-6 h | 55 | 29 | 62.1% |
| 6-24 h | 55 | 66 | 93.9% |
| | | | |
| Ischemic Stroke | | | |
| Time from Onset | # of Mimic Subjects | # of Stroke Subjects | Sensitivity @ 94.4% Specifcity |
| 0-3 h | 55 | 9 | 55.6% |
| 0-6h | 55 | 22 | 77.3% |
| 6-24 h | 55 | 44 | 93.2% |
| | | | |
| Hemorrhagic Stroke | | | |
| Time from Onset | # of Mimic Subjects | # of Stroke Subjects | Sensitivity @ 94.4% Specifcity |
| 0-3 h | 55 | 2 | 100.0% |
| 0-6 h | 55 | 7 | 71.4% |
| 6-24 h | 55 | 22 | 94.5% |
| | | | |
| 6-24 h Panel Coefficients | | | |
| Marker | Cutoff | Window | Weight |
| Caspse-3 | 1.15 | 0.90 | 0.19 |
| MCP-1 | 1242.63 | 0.87 | -0.21 |
| vWF-Integrin | 5.37 | 0.90 | 0.11 |
| BNP | 738.69 | 0.97 | 0.15 |
| GFAP | 3.22 | 0.18 | 0.11 |
| CK-BB | 3.52 | 0.99 | -0.01 |

(continued)

| 6-24 h Panel Coefficients | | | |
|---|---|---|---|
| Marker | Cutoff | Window | Weight |
| CRP | 114.31 | 0.99 | 0.22 |

Example 14. Markers and marker panels for predicting cerebral vasospasm after subarrachnoid hemorrhage

Delayed ischemic neurological deficits (DIND) resulting from cerebral vasospasm is a major cause of morbidity and mortality following aneurysmal subarachnoid hemorrhage (SAH). Despite intensive efforts to reveal its pathogenesis, the biological processes underlying DIND remains unclear.

To identify exemplary markers and marker panels predictive of cerebral vasospasm, daily blood samples were drawn 48 hours after symptom onset in 52 patients presenting with aneurismal subarrachnoid hemorrhage. 23 patients (45%) developed clinical cerebral vasospasm, and only blood samples drawn prior to onset of clinical manifestations of cerebral vasospasm were considered. Univariate logistic regression was performed using peak marker levels, and the most significant variables were entered into a multiple logistic regression model.

The final logistic model included VEGF (p=0.002), NCAM (p=0.004), and caspase-3 (p=0.009), with an overall p value of <0.0001. The model had a sensitivity of 94% (negative predictive value of 95%) and a specificity of 91% (positive predictive value of 88%).

Recently, Sviri et al. (Stroke 31:118-122, 2000) identified a correlation between serum BNP levels and DIND. Sviri demonstrated a 6-fold elevation in serum BNP 7-9 days after SAH only in patients developing symptomatic cerebral vasospasm, whereas no elevation occurred in the serum BNP of patients without symptomatic vasospasm [18]. However, the temporal relationship between rising BNP and onset of DIND was not reported, raising the question as to whether serum BNP may precipitate DIND, serving as a predictive serum marker for impending DIND.

Thus, in a second study, 40 consecutive patients admitted with aneurysmal SAH were enrolled. The patient's clinical condition at admission was graded according to the Hunt and Hess classifications. The severity of SAH was classified from the initial CT appearance Diagnostic cerebral angiography was performed during the first 24 hours after admission. All patients underwent craniotomy and aneurysm clipping <48 hours after SAH. Decadron was administered pre-operatively and tapered immediately after surgery. Nimodipine, phenytoin, and gastrointestinal prophylaxis ($H_2$-blockers or proton pump inhibitors) were administered the day of admission and continued throughout the patient's stay in the intensive care unit. Serum BNP and sodium samples were taken by venipuncture at time of hospital admission and repeated every 12 hours for 12 consecutive days. All patients underwent transcranial Doppler ultrasound (TCD) evaluation between 5 times per week and at the onset of suspected DIND. The significance of differences for continuous variables was determined using Student's t-test. Non-parametric data were compared using the Mann Whitney test. Percentages were compared using the chi-squared test. Multivariate logistic regression analyses adjusting for Hunt and Hess grade and Fisher grade were used to assess the independent association between BNP and onset of DIND

16 (40%) patients developed symptomatic cerebral vasospasm after SAH. A >3-fold increase in admission serum BNP was associated with the onset of hyponatremia (p<0.05). Mean BNP levels were similar between vasospasm and non-vasospasm patients <3 days after SAH (126+/-39 vs 154+/-40, p=0.61) but were elevated in the vasospasm cohort 4-6 days after SAH (285+/-67 vs 116+/-30, p<0.01), 7-9 days after SAH (278+/-72 vs 166+/-45, p<0.01), and 9-12 days after SAH (297+/-83 vs 106+/-30, p<0.01). BNP level remained independently associated with vasospasm adjusting for Fisher and Hunt and Hess grade (OR, 1.28; 95%CI, 1.1-1.6). In patients developing vasospasm, mean serum BNP increased 5.4-fold within 24 hours after vasospasm onset, and 11.2-fold the first 3 days after vasospasm onset. Patients with increasing BNP levels from admission demonstrated no change (0 +/-3) in Glascow Coma Score (GCS) two weeks after SAH versus a 3.0 +/-2 (p<0.05) improvement in GCS in patients without increasing serum BNP.

Increasing serum BNP levels were independently associated with hyponatremia, did not significantly increase until the first 24 hours after onset of DIND, and predicted 2-week GCS. Increasing BNP may exacerbate blood flow reduction due to cerebral vasospasm and serve as a marker to determine aggressiveness of diagnostic and therapeutic management.

While the invention has been described and exemplified in sufficient detail for those skilled in this art to make and use it, various alternatives, modifications, and improvements should be apparent without departing from the spirit and scope of the invention.

Example 15. Markers and marker panels for distinguishing intracranial hemorrhage from ischemic stroke

The early management of acute ischemic stoke involves excluding the presence of intracranial hemorrhage (ICH). Blood was drawn from 113 patients who were diagnosed with either ischemic stroke or ICH. All patients presented within 48 hours from onset of symptoms. The primary clinical outcome was the presence of ICH verified by CT or the clinical diagnosis of ischemic stroke, defined as focal neurological symptoms of vascular origin persisting for greater than 24 hours with consistent radiographic findings. Univariate logistic regression was performed on each variable and the most significant ones were entered into a multiple logistic regression model. Collinearity was examined, and a final model with three variables was generated.

34 patients (30%) were diagnosed with ICH and 79 (70%) with ischemic stroke. The final logistic model included C-reactive protein (P = 0.0 1 3), vascular endothelial growth factor (P = 0.045), and BNP (P = 0.030), with an overall P value of < 0.01. Using a probability cutoff of 0.215, this model had a sensitivity of 94%, a negative predictive value of 93%, and a specificity of 40%. The same 3-variable model was significant when including only patients who presented within 24 hour of symptom onset (n = 83, P < 0.05), with a sensitivity of 94%, a negative predictive value of 96%, and a specificity of 48%. A panel of three biomarkers was able to rule out ICH with high sensitivity in patients presenting with stroke. Such a panel may prove useful as a point-of-care test to rule out ICH in patients with suspected ischemic stroke prior to therapeutic intervention.

Example 16. Markers and marker panels for predicting cerebral vasospasm after subarrachnoid hemorrhage

Delayed ischemic neurological deficits (DIND) resulting from cerebral vasospasm is a major cause of morbidity and mortality following aneurysmal subarachnoid hemorrhage (SAH). Despite intensive efforts to reveal its pathogenesis, the biological processes underlying DIND remains unclear.

To identify exemplary markers and marker panels predictive of cerebral vasospasm, daily blood samples were drawn 48 hours after symptom onset in 52 patients presenting with aneurismal subarrachnoid hemorrhage. 23 patients (45%) developed clinical cerebral vasospasm, and only blood samples drawn prior to onset of clinical manifestations of cerebral vasospasm were considered. Univariate logistic regression was performed using peak marker levels, and the most significant variables were entered into a multiple logistic regression model.

The final logistic model included VEGF (p=0.002), NCAM (p=0.004), and caspase-3 (p=0.009), with an overall p value of <0.0001. The model had a sensitivity of 94% (negative predictive value of 95%) and a specificity of 91% (positive predictive value of 88%).

Recently, Sviri et al. (Stroke 31:118-122, 2000) identified a correlation between serum BNP levels and DIND. Sviri demonstrated a 6-fold elevation in serum BNP 7-9 days after SAH only in patients developing symptomatic cerebral vasospasm, whereas no elevation occurred in the serum BNP of patients without symptomatic vasospasm [18]. However, the temporal relationship between rising BNP and onset of DIND was not reported, raising the question as to whether serum BNP may precipitate DIND, serving as a predictive serum marker for impending DIND.

Thus, in a second study, 40 consecutive patients admitted with aneurysmal SAH were enrolled. The patient's clinical condition at admission was graded according to the Hunt and Hess classifications. The severity of SAH was classified from the initial CT appearance Diagnostic cerebral angiography was performed during the first 24 hours after admission. All patients underwent craniotomy and aneurysm clipping <48 hours after SAH. Decadron was administered pre-operatively and tapered immediately after surgery. Nimodipine, phenytoin, and gastrointestinal prophylaxis ($H_2$-blockers or proton pump inhibitors) were administered the day of admission and continued throughout the patient's stay in the intensive care unit. Serum BNP and sodium samples were taken by venipuncture at time of hospital admission and repeated every 12 hours for 12 consecutive days. All patients underwent transcranial Doppler ultrasound (TCD) evaluation between 5 times per week and at the onset of suspected DIND. The significance of differences for continuous variables was determined using Student's t-test. Non-parametric data were compared using the Mann Whitney test. Percentages were compared using the chi-squared test. Multivariate logistic regression analyses adjusting for Hunt and Hess grade and Fisher grade were used to assess the independent association between BNP and onset of DIND

16 (40%) patients developed symptomatic cerebral vasospasm after SAH. A >3-fold increase in admission serum BNP was associated with the onset of hyponatremia (p<0.05). Mean BNP levels were similar between vasospasm and non-vasospasm patients <3 days after SAH (126+/-39 vs 154+/-40, p=0.61) but were elevated in the vasospasm cohort 4-6 days after SAH (285+/-67 vs 116+/-30, p<0.01), 7-9 days after SAH (278+/-72 vs 166+/-45, p<0.01), and 9-12 days after SAH (297+/-83 vs 106+/-30, p<0.01). BNP level remained independently associated with vasospasm adjusting for Fisher and Hunt and Hess grade (OR, 1.28; 95%CI, 1.1-1.6). In patients developing vasospasm, mean serum BNP increased 5.4-fold within 24 hours after vasospasm onset, and 11.2-fold the first 3 days after vasospasm onset. Patients with increasing BNP levels from admission demonstrated no change (0 +/-3) in Glascow Coma Score (GCS) two weeks after SAH versus a 3.0 +/-2 (p<0.05) improvement in GCS in patients without increasing serum BNP.

Increasing serum BNP levels were independently associated with hyponatremia, did not significantly increase until the first 24 hours after onset of DIND, and predicted 2-week GCS. Increasing BNP may exacerbate blood flow reduction due to cerebral vasospasm and serve as a marker to determine aggressiveness of diagnostic and therapeutic management.

While the invention has been described and exemplified in sufficient detail for those skilled in this art to make and use it, various alternatives, modifications, and improvements should be apparent without departing from the spirit and scope of the invention.

Example 17. Markers and marker panels for distinguishing intracranial hemorrhage from ischemic stroke

The early management of acute ischemic stoke involves excluding the presence of intracranial hemorrhage (ICH). Blood was drawn from 113 patients who were diagnosed with either ischemic stroke or ICH. A1l patients presented within 48 hours from onset of symptoms. The primary clinical outcome was the presence of ICH verified by CT or the clinical diagnosis of ischemic stroke, defined as focal neurological symptoms of vascular origin persisting for greater than 24 hours with consistent radiographic findings. Univariate logistic regression was performed on each variable and the most significant ones were entered into a multiple logistic regression model. Collinearity was examined, and a final model with three variables was generated.

34 patients (30%) were diagnosed with ICH and 79 (70%) with ischemic stroke. The final logistic model included C-reactive protein (P = 0.0 1 3), vascular endothelial growth factor (P = 0.045), and BNP (P = 0.030), with an overall P value of < 0.01. Using a probability cutoff of 0.215, this model had a sensitivity of 94%, a negative predictive value of 93%, and a specificity of 40%. The same 3-variable model was significant when including only patients who presented within 24 hour of symptom onset (n = 83, P < 0.05), with a sensitivity of 94%, a negative predictive value of 96%, and a specificity of 48%. A panel of three biomarkers was able to rule out ICH with high sensitivity in patients presenting with stroke. Such a panel may prove useful as a point-of-care test to rule out ICH in patients with suspected ischemic stroke prior to therapeutic intervention.

While the invention has been described and exemplified in sufficient detail for those skilled in this art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth below and within the claims.

In a further embodiment, the present invention relates to a method of symptom-based diagnosis of a subject, comprising

analyzing a test sample obtained from said subject for the presence or amount of a plurality of subject-derived markers, wherein said markers are selected to identify the presence or absence in said subject of a plurality of conditions within the differential diagnosis of a symptom exhibited by said subject; and

correlating the presence or amount of said markers in said test sample to the presence or absence of each of said plurality of conditions.

In one embodiment of the above method said symptom is selected from the group consisting of dyspnea, fever, chest pain, abdominal pain, disturbances in metabolic state, neurologic dysfunction, hypertension, dizziness, and headache.

In another embodiment of the above method said symptom is dyspnea, and said plurality of conditions may be selected from the group consisting of asthma, chronic obstructive pulmonary disease ("COPD"), tracheal stenosis, obstructive endobroncheal tumor, pulmonary fibrosis, pneumoconiosis, lymphangitic carcinomatosis, kyphoscoliosis, pleural effusion, amyotrophic lateral sclerosis, congestive heart failure, coronary artery disease, myocardial infarction, atrial fibrillation, cardiomyopathy, valvular dysfunction, left ventricle hypertrophy, pericarditis, arrhythmia, pulmonary embolism, metabolic acidosis, chronic bronchitis, pneumonia, anxiety, sepsis, and aneurismic dissection. In a certain embodiment, said plurality of subject-derived markers comprise at least one specific marker of neural tissue injury selected from the group consisting of adenylate kinase, brain-derived neurotrophic factor, calbindin-D, creatine kinase-BB, glial fibrillary acidic protein, lactate dehydrogenase, myelin basic protein, neural cell adhesion molecule (NCAM), neuron-specific enolase, neurotrophin-3, proteolipid protein, S-100β, thrombomodulin, and protein kinase C γ, or markers related thereto. In another certain embodiment, said plurality of conditions may comprise myocardial infarction and pulmonary embolism, myocardial infarction and congestive heart failure, pulmonary embolism and congestive heart failure, or myocardial infarction, pulmonary embolism, and congestive heart failure.

In still another embodiment of the above method, said symptom is dyspnea, and said plurality of markers comprise a plurality of markers independently selected from the group consisting of specific markers of cardiac injury, specific markers of neural tissue injury, non-specific markers of tissue injury, markers related to blood pressure regulation, markers related to pulmonary injury, markers related to inflammation, markers related to coagulation and hemostasis, and markers related to apoptosis.

In still another embodiment of the above method said symptom is dyspnea, and said plurality of markers comprise at least one subject-derived marker selected from the group consisting of free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, creatine kinase-MB, S100ao, A-type natriuretic peptide, B-type natriuretic peptide, calcitonin gene related peptide, calcitonin, urotensin 1, urodilatin, adrenomedullin, myoglobin, smooth muscle myosin heavy chain, thrombus precursor protein, D-dimer, neutrophil elastase, 7s collagen fragment, pulmonary surfactant protein(s), dipalmitoylphosphatidyl choline, KL-6, smooth muscle myosin light chain, IL-1ra, myeloperoxidase, C-reactive protein, monocyte chemoattractant peptide-1, and MMP-9, or markers related thereto.

In a further embodiment of the above method said plurality of subject-derived markers comprise at least one cardiac troponin form, B-type natriuretic peptide or a marker related to B-type natriuretic peptide, and D-dimer.

In still a further embodiment of the above method said symptom is chest pain, and said plurality of conditions are selected from the group consisting of stable angina, unstable angina, myocardial ischemia, cardiac necrosis, atrial fibrillation, myocardial infarction, musculoskeletal injury, cholecystitis, gastroesophageal reflux, pulmonary embolism, pericarditis, aortic dissection, pneumonia, and anxiety.

Said plurality of conditions can comprise aortic dissection, myocardial ischemia, myocardial necrosis, myocardial infarction, and atrial fibrillation.

In still another embodiment of the above method said symptom is chest pain, and said plurality of markers comprise a plurality of markers independently selected from the group consisting of specific markers of cardiac injury, specific markers of neural tissue injury, non-specific markers of tissue injury, markers related to blood pressure regulation, markers related to inflammation, markers related to coagulation and hemostasis, and markers related to apoptosis. In a certain embodiment, said symptom is chest pain, and said plurality of markers can comprise at least one subject-derived marker selected from the group consisting of free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, creatine kinase-MB, S100ao, A-type natriuretic peptide, B-type natriuretic peptide, NT-proBNP, calcitonin gene related peptide, calcitonin, urotensin 1, myoglobin, smooth muscle myosin heavy chain, thrombus precursor protein, D-dimer, smooth muscle myosin heavy chain, IL-1ra, myeloperoxidase, C-reactive protein, monocyte chemoattractant peptide-1, and MMP-9, or markers related thereto. In a specific embodiment, said plurality of subject-derived markers comprise at least one cardiac troponin form, B-type natriuretic peptide or a marker related to B-type natriuretic peptide, and smooth muscle myosin heavy chain. In another specific embodiment said plurality of subject-derived markers comprise at least one cardiac troponin form and smooth muscle myosin heavy chain. In still another specific embodiment said plurality of subject-derived markers comprise at least one cardiac troponin form, B-type natriuretic peptide or a marker related to B-type natriuretic peptide, atrial natriuretic peptide or a marker related to atrial natriuretic peptide, creatine kinase MB, and smooth muscle myosin heavy chain.

In another embodiment of the above method said symptom is abdominal pain, and said plurality of conditions are selected from the group consisting of aortic dissection, mesenteric embolism, pancreatitis, appendicitis, myocardial ischemia, myocardial infarction, an infectious disease, influenza, esophageal carcinoma, gastric adenocarcinoma, colorectal adenocarcinoma, pancreatic ductal adenocarcinoma, cystadenocarcinoma, and insulinoma. In a specific embodiment, said plurality of conditions comprises aortic dissection, myocardial ischemia, and myocardial infarction. In another specific embodiment said symptom is abdominal pain, and said plurality of markers comprise at least one subject-derived marker selected from the group consisting of free cardiac troponin I, free cardiac troponin T, cardiac troponin 1 in a complex comprising one or both of troponin T and troponin C, cardiac troponin T in a complex comprising one or both of troponin I and troponin C, total cardiac troponin I, total cardiac troponin T, B-type natriuretic peptide, a marker related to B-type natriuretic peptide, smooth muscle myosin heavy chain, and myoglobin. In still another specific embodiment said plurality of subject-derived markers comprise at least one cardiac troponin form, B-type natriuretic peptide or a marker related to B-type natriuretic peptide, and smooth muscle myosin heavy chain. In a further specific embodiment said plurality of subject-derived markers comprises at least one cardiac troponin form and smooth muscle myosin heavy chain.

In another embodiment of the above method said symptom is neurologic dysfunction, and said plurality of conditions are selected from the group consisting of stroke, ischemic stroke, subarachnoid hemorrhage, transient ischemic attack, intracerebral hemorrhage, hemorrhagic stroke, brain tumor, cerebral hypoxia, hypoglycemia, migraine, atrial fibrillation, myocardial infarction, cardiac ischemia, peripheral vascular disease, migraine, and seizure. In a specific embodiment said plurality of conditions comprise ischemic stroke, hemorrhagic stroke, transient ischemic attack, atrial fibrillation, myocardial ischemia, and myocardial infarction. In another specific embodiment said symptom is neurologic dysfunction, and said plurality of markers comprise and said plurality of markers comprise a plurality of markers independently selected from the group consisting of specific markers of cardiac injury, specific markers of neural tissue injury, non-specific markers of tissue injury, markers related to blood pressure regulation, markers related to inflammation, markers related to coagulation and hemostasis, and markers related to apoptosis. In still another specific embodiment said symptom is neurologic dysfunction, and said plurality of markers comprise at least one subject-derived marker selected from the group consisting of free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, c-reactive protein, creatine kinase-BB, creatine kinase-MB, IL-Ira, D-dimer, neurotrophin-3, c-Tau, vascular endothelial growth factor, thrombus precursor protein, B-type natriuretic peptide, Atrial natriuretic peptide, interleukin-6, matrix metalloproteinase-9, S-100β, thrombin-antithrombin III complex, caspase-3, cytochrome c, and a form of von Willebrand factor, or markers related thereto.

In another embodiment of the above method said test sample is selected from the group consisting of a blood sample, a serum sample, a plasma sample, a cerebrospinal fluid sample, a urine sample, a saliva sample, a sputum sample, and a pleural effusion sample.

In still a further embodiment of the above method said plurality of markers are selected from the group consisting of free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, B-type natriuretic peptide, Atrial natriuretic peptide, pulmonary surfactant protein D, D-dimer, annexin V, enolase, creatine kinase-BB, creatine kinase-MB, glycogen phosphorylase, heart-type fatty acid binding protein, phosphoglyceric acid mutase, S-100β, S-100ao, plasmin-α2-antiplasmin complex, β-thromboglobulin, platelet factor 4, fibrinopeptide A, platelet-derived growth factor, prothrombin fragment 1+2, P-selectin, thrombin-antithrombin III complex, von Willebrand factor, tissue factor, thrombus precursor protein, human neutrophil elastase, inducible nitric oxide synthase, lysophosphatidic acid, malondialdehyde-modified low density lipoprotein, matrix metalloproteinase-1, matrix metalloproteinase-2, matrix metalloproteinase-3, matrix metalloproteinase-9, TIMP1, TIMP2, TIMP3, C-reactive protein, interleukin-1β, interleukin-1 receptor antagonist, interleukin-6, tumor necrosis factor α, soluble intercellular adhesion molecule-1, vascular cell adhesion molecule, monocyte chemotactic protein-1, caspase-3, cytochrome c, human lipocalin-type prostaglandin D synthase, mast cell tryptase, eosinophil cationic protein, KL-6, procalcitonin, haptoglobin, s-CD40 ligand, S-FAS ligand, alpha 2 actin, basic calponin 1, CSRP2 elastin, LTBP4, smooth muscle myosin, smooth muscle myosin heavy chain, transgelin, aldosterone, angiotensin I, angiotensin II, angiotensin III, bradykinin, calcitonin calcitonin gene related peptide, endothelin 1, endotehlin 2, endothelin 3, renin, vasopressin, APO B48, pancreatic elastase 1, pancreatic lipase, sPLA2, trypsinogen activation peptide, alpha enolase, LAMP3, phospholipase D, PLA2G5, protein D, SFTPC, defensin HBD1, defensin HBD2, CXCL-1, CXCL-2, CXCL-3, CCL2, CCL3, CCL4, CCL8, procalcitonin, protein C, serum amyloid A, s-glutathione, s-TNF P55, s-TNF P75, TAFI, TGF beta, MMP-11, brain fatty acid binding protein, CA11, CABP1, CACNA1A, CBLN1, CHN2, cleaved Tau, CRHR1, DRPLA, EGF, GPM6B, GPR7, GPR8, GRIN2C, GRM7, HAPIP, HIF 1 alpha, HIP2 KCNK4, KCNK9, KCNQ5, MAPK10, n-acetyl aspartate, NEUROD2, NRG2, PACE4, phosphoglycerate mutase, PKC gamma, c-Tau, prostaglandin E2, PTEN, PTPRZ1, RGS9, SCA7, secretagogin, SLC1A3, SORL1, SREB3, STAC, STX1A, STXBP1, BDNF, cystatin C, neurokinin A, substance P, IL-1ra, interleukin-1, interleukin-11, interleukin-13, intcrleukin-18, interleukin-4, and interleukin-10, or markers related thereto.

In still a further embodiment of the above method said analyzing step comprises contacting said test sample with a test surface comprising a plurality of discrete addressable locations each adapted to bind for detection one of said plurality of markers. In a specific embodiment, said plurality of markers is detected in a sandwich immunoassay. In another specific embodiment, said test surface is a porous membrane. In still another embodiment, said test surface is a nonporous surface. In still a further specific embodiment, said test surface is within a capillary space. In still another specific embodiment, said test surface comprises discrete particles immobilized at said plurality of discrete addressable locations, wherein said discrete particles comprise antibodies bound thereto.

In still another aspect, the present invention relates to a test device for performing a symptom-based diagnostic method, wherein said method comprises analyzing a test sample obtained from a subject for the presence or amount of a plurality of subject-derived markers, wherein said markers are selected to identify the presence or absence in said subject of a plurality of conditions within the differential diagnosis of a symptom exhibited by said subject; and correlating the presence or amount of said markers in said test sample to the presence or absence of each of said plurality of conditions, said test device comprising a test surface comprising a plurality of discrete addressable locations, each said location comprising an antibody selected to bind for detection one of said plurality of markers immobilized at said location. In a specific embodiment, said test surface comprises discrete particles immobilized at said plurality of discrete addressable locations, wherein said discrete particles comprise said antibody. In another specific embodiment, said symptom is selected from the group consisting of dyspnea, fever, chest pain, abdominal pain, disturbances in metabolic state, neurologic dysfunction, hypertension, dizziness, and headache.

In another aspect, the present invention is directed to a method of identifying a marker panel for performing a symptom-based diagnostic method, comprising determining the ability of one or more candidate panels comprising plurality of subject-derived markers to identify the presence or absence in a subject population of a plurality of conditions within the differential diagnosis of a symptom exhibited by individual subjects in said subject population, wherein a candidate panel that correctly identifies the presence or absence of said plurality of conditions is identified as a marker panel. In a specific embodiment of this method, said symptom is selected from the group consisting of dyspnea, fever, chest pain, abdominal pain, disturbances in metabolic state, neurologic dysfunction, hypertension, dizziness, and headache.

In a further aspect, the present invention discloses a method for distinguishing between systolic heart failure and diastolic heart failure in a subject, comprising:

analyzing a test sample obtained from said subject for the presence or amount of a plurality of subject-derived markers, wherein said markers are selected to distinguish between systolic heart failure and diastolic heart failure; and correlating the presence or amount of said markers in said test sample to the presence of systolic heart failure and diastolic heart failure in said subject.

In a specific embodiment of the above method said method comprises assaying for the presence or amount of markers selected from the group consisting of B-type natriuretic peptide, a marker related to B-type natriuretic peptide, atrial natriuretic peptide, a marker related to atrial natriuretic peptide, vasopressin, endothelin-2, urodilatin, adrenomedullin, calcitonin gene related peptide, calcitonin, urotensin 2, and angiotensin 2. In another specific embodiment, said method comprises assaying for the presence or amount of markers selected from the group consisting of B-type natriuretic peptide, a marker related to B-type natriuretic peptide, atrial natriuretic peptide, a marker related to atrial natriuretic peptide, and urotensin 2.

In still another aspect of the invention, a test device for performing a diagnostic method, wherein said method comprises analyzing a test sample obtained from a subject for the presence or amount of a plurality of subject-derived markers, wherein said markers are selected to distinguish between systolic heart failure and diastolic heart failure in a subject, is disclosed.

In a further aspect, the present invention relates to a method for distinguishing between atrial fibrillation and congestive heart failure in a subject, comprising:

analyzing a test sample obtained from said subject for the presence or amount of a plurality of subject-derived markers, wherein said markers are selected to distinguish between atrial fibrillation and congestive heart failure; and correlating the presence or amount of said markers in said test sample to the presence of atrial fibrillation and congestive heart failure in said subject.

In a specific embodiment of the above method said method comprises assaying for the presence or amount of markers selected from the group consisting of atrial natriuretic peptide, a marker related to atrial natriuretic peptide, B-type natriuretic peptide, and a marker related to B-type natriuretic peptide.

In still another aspect, the present invention is directed to a test device for performing a diagnostic method, wherein said method comprises analyzing a test sample obtained from a subject for the presence or amount of a plurality of subject-derived markers, wherein said markers are selected to distinguish between atrial fibrillation and congestive heart failure in a subject.

In a further embodiment, the present invention features a method for distinguishing between atrial fibrillation and myocardial infarction in a subject, comprising:

analyzing a test sample obtained from said subject for the presence or amount of a plurality of subject-derived markers, wherein said markers are selected to distinguish between atrial fibrillation and myocardial infarction; and correlating the presence or amount of said markers in said test sample to the presence of atrial fibrillation and myocardial infarction in said subject.

In a specific embodiment, said method comprises assaying for the presence or amount of markers selected from the group consisting of atrial natriuretic peptide, a marker related to atrial natriuretic peptide, free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, and myoglobin.

In another aspect, the invention is directed to a test device for performing a diagnostic method, wherein said method comprises analyzing a test sample obtained from a subject for the presence or amount of a plurality of subject-derived markers, wherein said markers are selected to distinguish between atrial fibrillation and myocardial infarction in a subject.

In a further aspect, the present invention relates to a method for distinguishing between aortic dissection, myocardial ischemia, and myocardial infarction in a subject, comprising:

analyzing a test sample obtained from said subject for the presence or amount of a plurality of subject-derived markers, wherein said markers are selected to distinguish between aortic dissection, myocardial ischemia, and myocardial infarction; and
correlating the presence or amount of said markers in said test sample to the presence of aortic dissection, myocardial ischemia, and myocardial infarction in said subj ect.

In a specific embodiment of this method said method comprises assaying for the presence or amount of markers selected from the group consisting of B-type natriuretic peptide, a marker related to B-type natriuretic peptide, free cardiac troponin I, free cardiac troponin T, cardiac troponin I in a complex comprising one or both of troponin T and troponin C, cardiac troponin T in a complex comprising one or both of troponin I and troponin C, total cardiac troponin I, total cardiac troponin T, smooth muscle myosin heavy chain, and myoglobin.

In still another aspect, the present invention relates to a test device for performing a diagnostic method, wherein said method comprises analyzing a test sample obtained from a subject for the presence or amount of a plurality of subject-derived markers, wherein said markers are selected to distinguish between aortic dissection, myocardial ischemia, and myocardial infarction in a subject.

In a further embodiment, the present invention discloses a method of analyzing a subject sample for a plurality of subject-derived markers selected to distinguish amongst a plurality of cardiovascular disorders, comprising:

assaying said sample for the presence or amount of one or more subject-derived markers related to blood pressure regulation, and for the presence or amount of one or more subject-derived markers related to myocardial injury, and characterizing said subject's risk of having developed or of developing each of said plurality of cardiovascular disorders based upon the presence or amount of said markers, wherein the amount of one or more of said markers is not compared to a predetermined threshold amount.

In a specific embodiment, said characterization step is performed without comparing the amount of any of said markers to a predetermined threshold amount. In another specific embodiment, said subject-derived marker(s) related to blood pressure regulation are selected from the group consisting of A-type natriuretic peptide, B-type natriuretic peptide, C-type natriuretic factor, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, rennin, and urodilatin, or markers related thereto, and wherein said subject-derived marker(s) related to myocardial injury are selected from the group consisting of free cardiac troponin I, free cardiac troponin T, cardiac troponin I in a complex comprising one or both of troponin T and troponin C, cardiac troponin T in a complex comprising one or both of troponin I and troponin C, free and complexed cardiac troponin I, free and complexed cardiac troponin T, annexin V, β-enolase, creatine kinase-MB, glycogen phosphorylase-BB, heart-type fatty acid binding protein, phosphoglyceric acid mutase-MB, and S-100ao, or markers related thereto. In a certain embodiment, said method comprises assaying said sample for the presence or amount of B-type natriuretic peptide or a marker related to B-type natriuretic peptide, creatine kinase-MB, and total cardiac troponin I or total cardiac troponin T. In still another specific embodiment said method further comprises assaying said sample for the presence or amount of one or more subject-derived markers related to inflammation. In a certain embodiment, said one or more subject-derived markers related to inflammation are selected from the group consisting of acute phase reactants, cell adhesion molecules such as vascular cell adhesion molecule ("VCAM"), intercellular adhesion molecule-1 ("ICAM-1"), intercellular adhesion molecule-2 ("ICAM-2"), and intercellular adhesion molecule-3 ("ICAM-3"), myeloperoxidase (MPO), C-reactive protein, interleukins such as IL-1 β, IL-6, and IL-8, interleukin-1 receptor agonist, monocyte chemoattractant protein-1, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), and vascular endothelial growth factor ("VEGF"), or markers related thereto. In still another specific embodiment, said method further comprises assaying said sample for the presence or amount of one or more subject-derived non-specific markers of tissue injury. In a certain embodiment, said one or more subject-derived non-specific markers of tissue injury are selected from the group consisting of aspartate aminotransferase, myoglobin, actin, myosin, and lactate dehydrogenase. In still a further embodiment, said method comprises assaying said sample for the presence or amount of creatine kinase-MB, total cardiac troponin I, myoglobin, and B-type natriuretic peptide, or markers related thereto, wherein the presence or amount of D-dimer and/or myeloperoxidase are optionally added to or used to replace one of said markers in said method. In still another embodiment, said method comprises assaying said sample for the presence or amount of total cardiac troponin I, C-reactive protein, and B-type natriuretic peptide or a marker related to B-type natriuretic peptide, or markers related thereto, wherein the presence or amount of D-dimer and/or myeloperoxidase are optionally added to or used to replace one of said markers in said method. In still another embodiment, said method comprises assaying said sample for the presence or amount of creatine kinase-MB, total cardiac troponin I, myoglobin, C-reactive protein, and B-type natriuretic peptide or a marker related to B-type natriuretic peptide, or markers related thereto, wherein the presence or amount of D-dimer and/or myeloperoxidase are optionally added to or used to replace one of said markers in said method. In still a further embodiment, said method comprises assaying said sample for the presence or amount of myoglobin, C-reactive protein, and B-type natriuretic peptide or a marker related to B-type natriuretic peptide, or markers related thereto, wherein the presence or amount of D-dimer and/or myeloperoxidase are optionally added to or used to replace one of said markers in said method. In still another embodiment, said method comprises assaying said sample for the presence or amount of creatine kinase-MB, total cardiac troponin I, and myoglobin; creatine kinase-MB, total cardiac troponin I, C-reactive protein and myoglobin, or markers related thereto, wherein the presence or amount of D-dimer and/or myeloperoxidase are optionally added to or used to replace one of said markers in said method. In still another embodiment, said method comprises assaying said sample for the presence or amount of B-type natriuretic peptide or a marker related to B-type natriuretic peptide, creatine kinase-MB, total cardiac troponin I, myoglobin, and C-reactive protein, or markers related thereto, wherein the presence or amount of D-dimer and/or myeloperoxidase are optionally added to or used to replace one of said markers in said method. In another embodiment said method further comprises assaying said sample for the presence or amount of one or more subject-derived markers related to coagulation and hemostasis. In certain embodiments, said subject-derived marker(s) related to coagulation and hemostasis are selected from the group consisting of plasmin, fibrinogen, D-dimer, β-thromboglobulin, platelet factor 4, fibrinopeptide A, platelet-derived growth factor, prothrombin fragment 1+2, plasmin-α2-antiplasmin complex, thrombin-antithrombin III complex, P-selectin, thrombin, von Willebrand factor, tissue factor, and thrombus precursor protein. In still another embodiment, said plurality of cardiovascular disorders is selected from the group consisting of myocardial infarction, congestive heart failure, acute coronary syndrome, unstable angina, and pulmonary embolism.

In another aspect, the present invention relates to a test device for performing the above method, comprising a test surface comprising a plurality of discrete addressable locations corresponding to said plurality of subject-derived markers, each said location comprising an antibody immobilized at said location selected to bind for detection one of said plurality of subject-derived markers.

In another aspect, the present invention is directed to a method of analyzing a subject sample for a plurality of subject-derived markers selected to distinguish amongst a plurality of cerebrovascular disorders, comprising:

assaying said sample for the presence or amount of one or more subject-derived markers related to blood pressure regulation, and for the presence or amount of one or more subject-derived markers related to neural tissue injury, and characterizing said subject's risk of having developed or of developing each of said plurality of cerebrovascular disorders based upon the presence or amount of said markers, wherein the amount of one or more of said markers is not compared to a predetermined threshold amount.

In a specific embodiment of the above method, said characterization step is performed without comparing the amount of any of said markers to a predetermined threshold amount. In another specific embodiment, said subject-derived marker(s) related to blood pressure regulation are selected from the group consisting of A-type natriuretic peptide, B-type natriuretic peptide, C-type natriuretic factor, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, rennin, and urodilatin, or markers related thereto, and wherein said subject-derived marker(s) related to myocardial injury are selected from the group consisting of free cardiac troponin I, free cardiac troponin T, cardiac troponin I in a complex comprising one or both of troponin T and troponin C, cardiac troponin T in a complex comprising one or both of troponin I and troponin C, free and complexed cardiac troponin I, free and complexed cardiac troponin T, annexin V, β-enolase, creatine kinase-MB, glycogen phosphorylase-BB, heart-type fatty acid binding protein, phosphoglyceric acid mutase-MB, and S-100ao, or markers related thereto, and wherein said subject-derived marker(s) related to neural tissue injury are selected from the group consisting of precerebellin 1, cerebillin 1, cerebellin 3, chimerin 1, chimerin 2, calbrain, calbindin D, brain tubulin, brain fatty acid binding protein ("B-FABP"), brain derived neurotrophic factor ("BDNF"), carbonic anhydrase XI, CACNA1A calcium channel gene, nerve growth factor β, atrophin 1, apolipoprotein E4-1, protein 4.1B, 14-3-3 protein, ciliary neurotrophic factor, creatine kinase-BB, C-tau, glial fibrillary acidic protein ("GFAP"), neural cell adhesion molecule ("NCAM"), neuron specific enolase, S-100b, prostaglandin D synthase, neurokinin A, neurotensin, and secretagogin, or markers related thereto. In still another specific embodiment, said method further comprises assaying said sample for the presence or amount of one or more subject-derived markers related to inflammation. In still another embodiment, said method further comprises assaying said sample for the presence or amount of one or more subject-derived markers related to coagulation and hemostasis. In still a further embodiment, said method further comprises assaying said sample for the presence or amount of one or more subject-derived markers related to apoptosis. In still another embodiment, said characterization step is performed without comparing the amount of any of said subject-derived markers to a predetermined threshold amount. In still another specific embodiment, said plurality of cerebrovascular disorders is selected from the group consisting of ischemic stroke, hemorrhagic stroke, transient ischemic attack, and subarachnoid hemorrhage.

In another aspect, the present invention discloses a test device for performing the above method, comprising a test surface comprising a plurality of discrete addressable locations corresponding to said plurality of subject-derived markers, each said location comprising an antibody immobilized at said location selected to bind for detection one of said plurality of subject-derived markers.

In another aspect, the present invention relates to a method of analyzing a subject sample for a plurality of subject-derived markers selected to identify subjects suffering from myocardial infarction, comprising:

assaying said sample for the presence or amount of a plurality of subject-derived marker(s) related to myocardial injury selected from the group consisting of B-type natriuretic peptide, free cardiac troponin I, free cardiac troponin T, cardiac troponin I in a complex comprising one or both of troponin T and troponin C, cardiac troponin T in a complex comprising one or both of troponin I and troponin C, free and complexed cardiac troponin I, free and complexed cardiac troponin T, total cardiac troponin I, total cardiac troponin T, creatine kinase-MB, myoglobin, caspase-3, cytochrome c, glycogen phosphorylase-MB, annexin B, β-enolase, heart-type fatty acid binding protein, c-reactive protein, and S-100ao, or markers related thereto, and

characterizing said subject's risk of having suffered a myocardial infarction based upon the presence or amount of said markers, wherein the amount of each of said markers is not compared to a predetermined threshold amount.

In still another aspect, the present invention is directed to a method of diagnosing stroke in a subject, comprising:

determining the presence or amount of a plurality of subject-derived markers in a sample obtained from said subject, wherein said plurality of markers are independently selected from two or more members of the group consisting of specific markers of neural tissue injury, markers related to blood pressure regulation, markers related to coagulation and hemostasis, and markers related to inflammation, and markers related to apoptosis; and

correlating the presence or amount of said plurality of markers to the occurrence of a stroke in said subject.

In a specific embodiment of the above method, said plurality of markers are independently selected from the group consisting of adenylate kinase, brain-derived neurotrophic factor, calbindin-D, creatine kinase-BB, glial fibrillary acidic protein, lactate dehydrogenase, myelin basic protein, neural cell adhesion molecule (NCAM), c-tau, neuropeptide Y, neuron-specific enolase, neurotrophin-3, proteolipid protein, S-100β, thrombomodulin, protein kinase C γ, atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, urodilatin, acute phase reactants, cell adhesion molecules, C-reactive protein, interleukins, interleukin-1 receptor agonist, monocyte chemotactic protein-1, caspase-3, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), vascular endothelial growth factor (VEGF), caspase-3, cathepsin D, α-spectrin, plasmin, fibrinogen, D-dimer, β-thromboglobulin, platelet factor 4, fibrinopeptide A, platelet-derived growth factor, prothrombin fragment 1+2, plasmin-α2-antiplasmin complex, thrombin-antithrombin III complex, P-selectin, thrombin, von Willebrand factor, tissue factor, and thrombus precursor protein, or markers related thereto. In another specific embodiment of the above method, said plurality of subject-derived markers comprise at least one specific marker of neural tissue injury selected from the group consisting of adenylate kinase, brain-derived neurotrophic factor, c-tau, calbindin-D, creatine kinase-BB, glial fibrillary acidic protein, lactate dehydrogenase, myelin basic protein, neural cell adhesion molecule (NCAM), neuron-specific enolase, neurotrophin-3, proteolipid protein, S-100β, thrombomodulin, and protein kinase C γ, or markers related thereto. In a certain embodiment, said plurality of subject-derived markers comprises at least one of NCAM, creatine kinase-BB, c-tau, and S-100β, or markers related thereto. In another certain embodiment, said plurality of subject-derived markers comprises at least two of NCAM, creatine kinase-BB, c-tau, and S-100β, or markers related thereto. In another specific embodiment of the above method, said plurality of subject-derived markers comprise at least one marker related to apoptosis. In a certain embodiment, said plurality of subject-derived markers comprise at least one marker related to apoptosis selected from the group consisting of caspase-3, cathepsin D, and α-spectrin, or markers related thereto. In still another embodiment of the above method, said plurality of subject-derived markers comprise at least one marker related to inflammation. In a certain embodiment, said plurality of subject-derived markers comprise at least one marker related to inflammation selected from the group consisting of acute phase reactants, cell adhesion molecules, C-reactive protein, interleukins, interleukin-1 receptor agonist, monocyte chemotactic protein-1, easpase-3, lipocalin-type prostaglandin D synthase, mast cell tryptase, eosinophil cationic protein, haptoglobin, tumor necrosis factor $\alpha$, tumor necrosis factor $\beta$, Fas ligand, soluble Fas (Apo-1), TRAIL, TWEAK, fibronectin, macrophage migration inhibitory factor (MIF), and vascular endothelial growth factor (VEGF), or markers related thereto. In another certain embodiment, said plurality of subject-derived markers comprise at least one marker related to inflammation selected from the group consisting of matrix metalloprotease-9, VEGF, C-reactive protein, or a marker related thereto. In still another embodiment of the above method, said plurality of subject-derived markers comprise at least one marker related to blood pressure regulation. In certain embodiments, said plurality of subject-derived markers comprise at least one marker related to blood pressure regulation selected from the group consisting of atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, and urodilatin, or markers related thereto. In another certain embodiment, said plurality of subject-derived markers comprises BNP or a marker related thereto. In still another specific embodiment of the above method, said plurality of subject-derived markers comprise at least one specific marker of neural tissue injury, at least one marker related to inflammation, and at least one marker related to apoptosis. In certain embodiments, said plurality of subject-derived markers comprises at least one marker related to blood pressure regulation and/or at least one marker related to coagulation and hemostasis. In still a further specific embodiment of the above method, said plurality of subject-derived markers comprise at least 4 markers selected from the group consisting of IL-1ra, C-reactive protein, von Willebrand factor (vWF), creatine kinase-BB, c-Tau, D-dimer, vascular endothelial growth factor (VEGF), matrix metalloprotease-9 (MMP-9), neural cell adhesion molecule (NCAM), BNP, S100$\beta$, cytochrome c, and caspase-3. In still another embodiment of the above method, said plurality of subject-derived markers comprise BNP, creatine kinase-BB, c-tau, D-dimer, C-reactive protein, S100$\beta$, NCAM, and caspase-3, wherein from one to three of said plurality of subject-derived markers is optionally replaced by an equal number of markers selected from the group consisting of IL-1ra, MMP-9, cytochrome c, and VEGF. In still another embodiment of the above method, said plurality of subject-derived markers comprise creatine kinase-BB, D-dimer, C-reactive protein, and S100$\beta$. In still another embodiment of the above method, the assay method is an immunoassay method. In still another specific embodiment of the above method, the correlating step comprises determining the concentration of each of said plurality of subject-derived markers, and individually comparing each marker concentration to a threshold level. In still another specific embodiment of the above method, the correlating step comprises determining the concentration of each of said plurality of subject-derived markers, calculating a single index value based on the concentration of each of said plurality of subject-derived markers, and comparing the index value to a threshold level. In still another specific embodiment of the above method, the method comprises determining a temporal change in at least one of said subject-derived markers, and wherein said temporal change is used in said correlating step. In still a further embodiment of the above method, said method distinguishes stroke with a sensitivity of at least 70% at a specificity of at least 85% when compared to normal subjects. In a further specific embodiment of the above method, said method distinguishes stroke with a sensitivity of at least 80% at a specificity of at least 90% when compared to normal subjects. In a further specific embodiment of the above method, said method distinguishes stroke with a sensitivity of at least 90% at a specificity of at least 90% when compared to normal subjects. In still another specific embodiment of the above method, said method distinguishes stroke with a sensitivity of at least 70% at a specificity of at least 85% when compared to subjects exhibiting symptoms that mimic stroke symptoms. In another embodiment of the above method, said method further distinguishes stroke from one or more stroke mimic conditions selected from the group consisting of brain tumor, aneurysm, electrocution, burns, infections, cerebral hypoxia, head injury, stress, dehydration, nerve palsy, hypoglycemia, migraine, multiple sclerosis, peripheral vascular disease, peripheral neuropathy, seizure, subdural hematoma, syncope, and transient unilateral weakness. In still another embodiment of the above method, said method further distinguishes amongst types of stroke selected from the group consisting of thrombotic stroke, embolic stroke, lacunar stroke, hypoperfusion, intracebral hemorrhage, subarachnoid hemorrhage, ischemic stroke, hemorrhagic stroke, transient ischemic attack, acute stroke, and non-acute stroke. In a still further embodiment of the above method, said one or more markers are selected to selectively identify the time of onset of a stroke in said subject. In certain embodiments, one or more markers are selected to determine if the onset of said stroke was within 12 hours. In another certain embodiment, said one or more markers are selected to determine if the onset of said stroke was within 6 hours. In still another certain embodiment, said one or more markers are selected to determine if the onset of said stroke was within 3 hours. In still a further certain embodiment, said one or more markers are selected to determine if the onset of said stroke was within a window of between 12 and 48 hours. In still another embodiment of the above method, said one or more markers are selected to distinguish an acute stroke from a non-acute stroke. In still another embodiment of the above method, said plurality of subject-derived markers comprise a plurality of markers selected from the group consisting of matrix metalloproteinase-9, one or more forms of von Willebrand factor, vascular cell adhesion molecule, C-reactive protein and S-100$\beta$, or markers related thereto. In still another embodiment of the above method, said plurality of subjeci-derived markers comprise a plurality of markers selected from the group consisting of B-type natriuretic peptide, glial fibrillary acidic protein, interleukin-8, β-nerve growth factor, von Willebrand factor-A1, and C-reactive protein, or markers related thereto. In a still further embodiment of the above method, at least one marker is BNP or a related marker thereof. In another embodiment of the above method, said plurality of subject-derived markers comprise a plurality of markers selected from the group consisting of B-type natriuretic peptide, glial fibrillary acidic protein, interleukin-8, creatine kinase-BB, monocyte chemotactic protein-1, and interleukin-1 receptor antagonist, or markers related thereto. In another specific embodiment of the above method said plurality of subject-derived markers comprise a plurality of markers selected from the group consisting of B-type natriuretic peptide, glial fibrillary acidic protein, C-reactive protein, creatine kinase-BB, matrix metalloproteinase-9, interleukin-8, and β-nerve growth factor, or markers related thereto. In a still further specific embodiment of the above method, said plurality of subject-derived markers comprise a plurality of markers selected from the group consisting of B-type natriuretic peptide, glial fibrillary acidic protein, C-reactive protein, creatine kinase-BB, caspase-3, monocyte chemotactic protein-1, and von Willebrand factor-integrin, or markers related thereto.

In another aspect, the present invention discloses a device for performing the above method, comprising a plurality of discrete locations, each configured and arranged to immobilize for detection one of said plurality of subject-derived markers.

In a further aspect, the present invention is directed to a method of characterizing a risk of future cerebral vasospasm in a subject suffering from a subarrachnoid hemorrhage, comprising:

determining the presence or amount of a plurality of subject-derived markers in a sample obtained from said subject, wherein said plurality of markers are independently selected from the group consisting of specific markers of neural tissue injury, markers related to blood pressure regulation, markers related to inflammation, and markers related to apoptosis; and

correlating the presence or amount of said plurality of markers to said risk of a future cerebral vasospasm in said subject.

In a specific embodiment of the above method said plurality of markers are independently selected from the group consisting of adenylate kinase, brain-derived neurotrophic factor, calbindin-D, creatine kinase-BB, glial fibrillary acidic protein, lactate dehydrogenase, myelin basic protein, neural cell adhesion molecule (NCAM), c-tau, neuropeptide Y, neuron-specific enolase, neurotrophin-3, proteolipid protein, S-100β, thrombomodulin, protein kinase C γ, atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, urodilatin, acute phase reactants, cell adhesion molecules, C-reactive protein, interleukins, interleukin-1 receptor agonist, monocyte chemotactic protein-1, caspase-3, cytochrome c, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), vascular endothelial growth factor (VEGF), caspase-3, cathepsin D, and α-spectrin, or markers related thereto. In another specific embodiment of the above method said plurality of subject-derived markers comprise at least one specific marker of neural tissue injury. In another specific embodiment of the above method, said plurality of subject-derived markers comprise at least one marker related to apoptosis. In a certain embodiment, said plurality of subject-derived markers comprise at least one marker related to apoptosis selected from the group consisting of caspase-3, cytochrome c, cathepsin D, and α-spectrin, or markers related thereto. In another certain embodiment, said plurality of subject-derived markers comprises caspase-3 or a marker related thereto. In still another embodiment of the above method, said plurality of subject-derived markers comprise at least one marker related to inflammation. In certain embodiments, said plurality of subject-derived markers comprise at least one marker related to inflammation selected from the group consisting of acute phase reactants, cell adhesion molecules, C-reactive protein, interleukins, interleukin-1 receptor agonist, monocyte chemotactic protein-1, caspase-3, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), and vascular endothelial growth factor (VEGF), or markers related thereto. In still another embodiment of the above method, said plurality of subject-derived markers comprises at least one marker related to blood pressure regulation. In a certain embodiment, said plurality of subject-derived markers comprise at least one marker related to blood pressure regulation selected from the group consisting of atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, and urodilatin, or markers related thereto. In another certain embodiment, said plurality of subject-derived markers comprises BNP, NT-pro BNP, pro-BNP or a marker related thereto. In still another embodiment of the above method, said plurality of subject-derived markers comprise at least one specific marker of neural tissue injury, at least one marker related to inflammation, and at least one marker related to apoptosis. In still a further embodiment of the above method, said plurality of subject-derived markers comprise one or more markers selected from the group consisting of IL-1ra, C-reactive protein, von Willebrand factor (vWF), vascular endothelial growth factor (VEGF), matrix metalloprotease-9 (MMP-9), neural cell adhesion molecule (NCAM), BNP, and caspase-3. In certain embodiments, said plurality of subject-derived markers comprises VEGF, NCAM, and caspase-3. In still another embodiment of the above method, the assay method is an immunoassay method. In another embodiment of the above method, the correlating step comprises determining the concentration of each of said plurality of subject-derived markers, and individually comparing each marker concentration to a threshold level. In a further embodiment of the above method, the correlating step comprises determining the concentration of each of said plurality of subject-derived markers, calculating a single index value based on the concentration of each of said plurality of subject-derived markers, and comparing the index value to a threshold level. In another embodiment of the above method, the method comprises determining a temporal change in at least one of said subject-derived markers, and wherein said temporal change is used in said correlating step.

In another aspect, the present invention is directed to a panel comprising a plurality of markers selected to selectively identify the occurrence or nonoccurrence of a stroke in a subject exhibiting one or more symptoms associated with the diagnosis of stroke, wherein said marker(s) are selected to distinguish the occurrence of a stroke in said subject from one or more stroke mimic conditions. In a specific embodiment of the above panel, said markers are selected to identify the occurrence or nonoccurrence of a stroke with a specificity of at least 80% and a sensitivity of at least 80%. In another specific embodiment of the above panel, said markers are selected to identify the occurrence or nonoccurrence of a stroke with a specificity of at least 90% and a sensitivity of at least 90%.

In another aspect the present invention relates to a device comprising a plurality of discretely addressable locations comprising a receptor for one of a plurality of markers selected to selectively identify the occurrence or nonoccurrence of a stroke in a subject exhibiting one or more symptoms associated with the diagnosis of stroke, wherein said marker(s) are selected to distinguish the occurrence of a stroke in said subject from one or more stroke mimic conditions.

In still another aspect, the present invention discloses a method of predicting a risk of one or more future clinical outcomes for a subject suffering from a vascular disease, comprising:

determining the presence or amount of thrombus precursor protein in a sample from said subject; and
correlating the presence or amount of thrombus precursor protein to said risk of one or more clinical outcomes for the subject.

In a specific embodiment of the above method, said vascular disease is selected from the group consisting of acute coronary syndrome, atherosclerosis, ischemic stroke, intracerebral hemorrhage, subarachnoid hemorrhage, transient ischemic attack, systolic dysfunction, diastolic dysfunction, aneurysm, aortic dissections, myocardial ischemia, angina pectoris, myocardial infarction, congestive heart failure, dilated congestive cardiomyopathy, hypertrophic cardiomyopathy, restrictive cardiomyopathy, cor pulmonale, arrhythmia, valvular heart disease, endocarditis, pulmonary embolism, venous thrombosis, and peripheral vascular disease. In certain embodiments, said vascular disease is acute coronary syndrome. In further certain embodiments, said one or more future clinical outcomes are selected from the group consisting of death, nonfatal myocardial infarction, recurrent ischemia requiring rehospitalization, recurrent ischemia requiring urgent revascularization, and congestive heart failure. In still further certain embodiments, the correlating step comprises determining the concentration of thrombus precursor protein in said sample, and comparing said concentration to a threshold concentration, wherein a concentration of thrombus precursor protein less than said threshold concentration is indicative of a first risk of said one or more clinical outcomes and a concentration of thrombus precursor protein greater than said threshold concentration is indicative of a second risk of one or more clinical outcomes. In further embodiments, said threshold concentration provides a ROC curve area of at least about 0.6. In another embodiment, said threshold concentration provides an odds ratio of about 4 or greater or about 0.25 or less. In still another embodiment, said threshold concentration provides a hazard ratio of about 1.25 or greater or about 0.8 or less. In a still further certain embodiment, said threshold concentration is a median thrombus precursor protein concentration measured in samples from subjects suffering from acute coronary syndrome. In a further embodiment, said median thrombus precursor protein concentration is about 8.9 $\mu$g/mL. In another embodiment of the above method, the method further comprises determining the presence or amount of one or more other subject-derived markers in said sample, and said correlating step comprises correlating the presence or amount of thrombus precursor protein and said one or more other subject-derived markers to said risk of one or more clinical outcomes for the subject. In certain embodiments, said one or more other subject-derived markers are independently selected from the group consisting of specific markers of myocardial injury, specific markers of neural tissue injury, markers related to blood pressure regulation, markers related to coagulation and hemostasis, markers related to inflammation, and markers related to apoptosis. In further embodiments, said one or more other subject-derived markers comprise one or more specific markers of myocardial injury. In another certain embodiment, said one or more other subject-derived markers comprise one or more specific markers of neural tissue injury. In still another certain embodiment, said one or more other subject-derived markers comprise one or more markers related to blood pressure regulation. In a further certain embodiment, said one or more other subject-derived markers comprise one or more markers related to coagulation and hemostasis. In still another certain embodiment, said one or more other subject-derived markers comprise one or more markers related to apoptosis. In another embodiment, said one or more other subject-derived markers are selected from the group consisting of annexin V, B-type natriuretic peptide, β-enolase, free cardiac troponin I, complexed cardiac troponin I, free and complexed cardiac troponin I, free cardiac troponin T, complexed cardiac troponin T, free and complexed cardiac troponin T, creatine kinase-MB, glycogen phosphorylase-BB, heart-type fatty acid binding protein, phosphoglyceric acid mutase-MB, S-100ao, adenylate kinase, brain-derived neurotrophic factor, calbindin-D, creatine lcinase-BB, glial fibrillary acidic protein, lactate dehydrogenase, myelin basic protein, neural cell adhesion molecule (NCAM), c-tau, neuropeptide Y, neuron-specific enolase, neurotrophin-3, proteolipid protein, S-100β, thrombomodulin, protein kinase C γ, atrial natriuretic peptide (ANP), pro-ANP, B-type natriuretic peptide (BNP), NT-pro BNP, pro-BNP C-type natriuretic peptide, urotensin II, arginine vasopressin, aldosterone, angiotensin I, angiotensin II, angiotensin III, bradykinin, calcitonin, procalcitonin, calcitonin gene related peptide, adrenomedullin, calcyphosine, endothelin-2, endothelin-3, renin, urodilatin, acute phase reactants, cell adhesion molecules, C-reactive protein, interleukins, interleukin-1 receptor agonist, monocyte chemoattractant protein-1, caspase-3, lipocalin-type prostaglandin D synthase, mast cell tryptase, eosinophil cationic protein, haptoglobin, tumor necrosis factor α, tumor necrosis factor β, Fas ligand, soluble Fas (Apo-1), TRAIL, TWEAK, fibronectin, macrophage migration inhibitory factor (MIF), vascular endothelial growth factor (VEGF), myeloperoxidase, caspase-3, cathepsin D, α-spectrin, plasmin, fibrinogen, D-dimer, β-thromboglobulin, platelet factor 4, fibrinopeptide A, platelet-derived growth factor, prothrombin fragment 1+2, plasmin-α2-antiplasmin complex, thrombin-antithrombin III complex, P-selectin, thrombin, von Willebrand factor, and tissue factor, or markers related thereto. In another embodiment, said plurality of subject-derived markers comprises BNP or a marker related thereto. In another embodiment, said plurality of subiect-derived markers comprise free cardiac troponin I, complexed cardiac troponin I, free and complexed cardiac troponin I, free cardiac troponin T, complexed cardiac troponin T, free and complexed cardiac troponin T, or a marker related thereto. In still another embodiment, said plurality of subject-derived markers comprises C-reactive protein or a marker related thereto. In a still further embodiment, said plurality of subject-derived markers comprises caspase-3 or a marker related thereto. In still another embodiment, said plurality of subject-derived markers comprises myeloperoxidase or a marker related thereto. In another specific embodiment of the above method, the sample is from a human. In still another embodiment of the above method, the sample is selected from the group consisting of blood, serum, and plasma. In still a further specific embodiment of the above method, the assay method is an immunoassay method. In a specific embodiment of the above method, the correlating step comprises determining the concentration of thrombus precursor protein and said one or more other subject-derived markers, and individually comparing each concentration to a corresponding threshold level. In another specific embodiment of the above method, the correlating step comprises determining the concentration of thrombus precursor protein and said one or more other subject-derived markers, calculating a single index value based on each concentration, and comparing the index value to a threshold level. In another embodiment of the above method, the method comprises determining a temporal change in thrombus precursor protein concentration, and wherein said temporal change is used in said correlating step.

In another aspect, the present invention features a device for performing the above method, comprising a plurality of discrete locations, each discrete location configured and arranged to immobilize for detection thrombus precursor protein or one of said one or more other subject-derived markers.

In still another aspect, the present invention relates to a method of diagnosing atherosclerosis in a subject, comprising:

determining the presence or amount of monocyte chemoattractant protein-1 or a marker related thereto in a sample from said subject; and

correlating the presence or amount of monocyte chemoattractant protein-1 to the presence or absence of atherosclerosis in the subject.

In a specific embodiment of the above method, the correlating step comprises determining the concentration of monocyte chemoattractant protein-1 or a marker related thereto in said sample, and comparing said concentration to a threshold concentration, wherein a concentration of monocyte chemoattractant protein-1 or a marker related thereto less than said threshold concentration is indicative of a first risk of atherosclerosis and a concentration of monocyte chemoattractant protein-1 or a marker related thereto greater than said threshold concentration is indicative of a second risk of atherosclerosis. In another specific embodiment of the above method, said correlating step further comprises determining the presence or amount of one or more risk factors selected from the group consisting of the sex, age, a diagnosis of diabetes, a diagnosis of hypertension, past tobacco use, a cholesterol concentration, and a family history of atherosclerosis, for said subject, wherein the presence or absence of one or more of said risk factors and the presence or amount of monocyte chemoattractant protein-1 or a marker related thereto are correlated to the presence or absence of atherosclerosis in the subject. In another embodiment, said threshold concentration provides an odds ratio of about 1.3 or greater or about 0.77 or less. In a further embodiment, said threshold concentration is selected to provide an odds ratio of about 2 or greater or about 0.5 or less. In another embodiment, said median thrombus precursor protein concentration is greater than about 120 pg/mL. In still another embodiment, said median thrombus precursor protein concentration is greater than about 150 pg/mL. In a further embodiment, said median thrombus precursor protein concentration is greater than about 200 pg/mL. In another specific embodiment of the above method, the method further comprises determining the presence or amount of one or more other subject-derived markers in said sample, and said correlating step comprises correlating the presence or amount of monocyte chemoattractant protcin-1 or a marker related thereto and said one or more other subject-derived markers to the presence or absence of atherosclerosis in the subject. In certain embodiments, said one or more other subject-derived markers are independently selected from the group consisting of specific markers of myocardial injury, specific markers of neural tissue injury, markers related to blood pressure regulation, markers related to coagulation and hemostasis, markers related to inflammation, and markers related to apoptosis. In another specific embodiment of the above method, the assay method is an immunoassay method.

**Claims**

1. A method of diagnosis of a brain damage-related disorder or the possibility thereof in a subject suspected of suffering therefrom, which comprises detecting one or more polypeptides or variants thereof selected from the group consisting of serum amyloid A, neuromodulin, calcyphosphine, RNA binding regulatory subunit, ubiquitin fusion degradation protein 1 homolog, nucleoside diphosphate kinase A, and cathepsin D in a sample of body fluid taken from the subject.

2. Use of one or more polypeptides or variants or mutants thereof, selected from the group consisting of serum amyloid A, neuromodulin, calcyphosphine, RNA binding regulatory subunit, ubiquitin fusion degradation protein 1 homolog, nucleoside diphosphate kinase A, and cathepsin D for diagnostic, prognostic, and therapeutic applications relating to brain damage-related disorders.

3. Use for diagnostic, prognostic, and therapeutic applications relating to brain damage-related disorders of one or more materials which recognize, bind to, or have affinity for a plurality of polypeptides or variants or mutants thereof, selected from the group consisting of serum amyloid A, neuromodulin, calcyphosphine, RNA binding regulatory subunit, ubiquitin fusion degradation protein 1 homolog, nucleoside diphosphate kinase A, and cathepsin D.

4. An assay device for use in the diagnosis of brain damage-related disorders which comprises a solid substrate having one or more locations, each containing a material which recognizes, binds to, or has affinity for a polypeptide or variant or mutant thereof, selected from the group consisting of serum amyloid A, neuromodulin, calcyphosphine, RNA binding regulatory subunit, ubiquitin fusion degradation protein 1 homolog, nucleoside diphosphate kinase A, and cathepsin D.

5. A kit for use in the diagnosis of brain damage-related disorders which comprises an assay device of claim 4, and a means for detecting the amount of one or more polypeptides in a sample of body fluid taken from a subject.

Fig. 1

Fig. 2

**European Patent
Office**

## PARTIAL EUROPEAN SEARCH REPORT

which under Rule 45 of the European Patent Convention
shall be considered, for the purposes of subsequent
proceedings, as the European search report

Application Number

EP 07 10 7064

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2005/029088 A (UNIV GENEVE; HOCHSTRASSER DENIS FRANCOIS [CH]; SANCHEZ JEAN-CHARLES [C) 31 March 2005 (2005-03-31) * the whole document * ----- | 1-5 | INV. C12Q1/68 G01N33/68 |
| X | SCHWAGERL A L ET AL: "ELEVATED LEVELS OF THE ENDOSOMAL-LYSOSOMAL PROTEINASE CATHEPSIN D IN CEREBROSPINAL FLUID IN ALZHEIMER DISEASE" JOURNAL OF NEUROCHEMISTRY, NEW YORK, NY, US, vol. 64, no. 1, January 1995 (1995-01), pages 443-446, XP001199807 ISSN: 0022-3042 * the whole document * ----- -/-- | 1-5 | |

TECHNICAL FIELDS
SEARCHED      (IPC)

G01N
C12Q

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 28 September 2007 | Merlos, Ana Maria |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C07)

European Patent
Office

**PARTIAL EUROPEAN SEARCH REPORT**

Application Number

EP 07 10 7064

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | BLENNOW K ET AL: "COMBINATION OF THE DIFFERENT BIOLOGICAL MARKERS FOR INCREASING SPECIFICITY OF IN VIVO ALZHEIMER'S TESTING" JOURNAL OF NEURAL TRANSMISSION. SUPPLEMENTUM, VIENNA, AT, vol. 53, no. SUPPL, 1998, pages 223-235, XP008056363 ISSN: 0303-6995 | 1-5 | |
| Y | * page 228, paragraph 5 * | 1-5 | |
| X | RISTORI G ET AL: "Serum amyloid A protein is elevated in relapsing-remitting multiple sclerosis" JOURNAL OF NEUROIMMUNOLOGY, vol. 88, no. 1-2, 1 August 1998 (1998-08-01), pages 9-12, XP002452833 ISSN: 0165-5728 * page 10, left-hand column - page 11, left-hand column * | 1-5 | TECHNICAL FIELDS SEARCHED (IPC) |
| Y | WIJNBERGER LIA D E ET AL: "Expression in the placenta of neuronal markers for perinatal brain damage" PEDIATRIC RESEARCH, vol. 51, no. 4, April 2002 (2002-04), pages 492-496, XP002452834 ISSN: 0031-3998 * page 496, paragraph 2 * | 1-5 | |
| A | WO 00/52476 A (SKYE PHARMATECH INCORPORTED [CA]; JACKOWSKI GEORGE [CA]) 8 September 2000 (2000-09-08) * the whole document * | | |

-/--

EPO FORM 1503 03.82 (P04C10)

**European Patent Office**

**PARTIAL EUROPEAN SEARCH REPORT**

Application Number

EP 07 10 7064

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | WO 01/42793 A (UNIV GENEVE [CH]; HOCHSTRASSER DENIS FRANCOIS [CH]; SANCHEZ JEAN CHARL) 14 June 2001 (2001-06-14) * the whole document * ----- | | |
| A | WATSON MARK A ET AL: "Clinical Utility of Biochemical Analysis of Cerebrospinal Fluid" CLINICAL CHEMISTRY, vol. 41, no. 3, 1995, pages 343-360, XP002452835 ISSN: 0009-9147 * the whole document * ----- | | |

TECHNICAL FIELDS SEARCHED (IPC)

EPO FORM 1503 03.82 (P04C10)

**INCOMPLETE SEARCH
SHEET C**

Although claims 2 and 3 are directed to a diagnostic/prognostic method
and a method of treatment practised on the human/animal body (Article
52(4) EPC), the search has been carried out and based on the alleged
effects of the compound/composition.

-----

## ANNEX TO THE EUROPEAN SEARCH REPORT
## ON EUROPEAN PATENT APPLICATION NO.

EP 07 10 7064

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2007

| Patent document cited in search report | | Publication date | Patent family member(s) | | Publication date |
|---|---|---|---|---|---|
| WO 2005029088 | A | 31-03-2005 | AU | 2004274710 A1 | 31-03-2005 |
| | | | CA | 2532130 A1 | 31-03-2005 |
| | | | EP | 1664795 A2 | 07-06-2006 |
| | | | JP | 2007506086 T | 15-03-2007 |
| | | | US | 2007042425 A1 | 22-02-2007 |
| WO 0052476 | A | 08-09-2000 | AT | 283485 T | 15-12-2004 |
| | | | AU | 765923 B2 | 02-10-2003 |
| | | | AU | 2788400 A | 21-09-2000 |
| | | | BR | 0008317 A | 29-01-2002 |
| | | | CA | 2263063 A1 | 26-08-2000 |
| | | | CN | 1339108 A | 06-03-2002 |
| | | | DE | 60016178 D1 | 30-12-2004 |
| | | | DE | 60016178 T2 | 01-12-2005 |
| | | | EP | 1155325 A1 | 21-11-2001 |
| | | | ES | 2232424 T3 | 01-06-2005 |
| | | | JP | 2002538463 A | 12-11-2002 |
| | | | NZ | 513005 A | 25-07-2003 |
| | | | PT | 1155325 T | 31-03-2005 |
| | | | US | 6235489 B1 | 22-05-2001 |
| WO 0142793 | A | 14-06-2001 | AT | 360211 T | 15-05-2007 |
| | | | AU | 773595 B2 | 27-05-2004 |
| | | | AU | 1707201 A | 18-06-2001 |
| | | | CA | 2393127 A1 | 14-06-2001 |
| | | | DE | 60034480 T2 | 16-08-2007 |
| | | | DK | 1238284 T3 | 13-08-2007 |
| | | | EP | 1238284 A2 | 11-09-2002 |
| | | | JP | 2003516541 T | 13-05-2003 |
| | | | US | 2003100038 A1 | 29-05-2003 |
| | | | US | 2007134726 A1 | 14-06-2007 |

EPO FORM P0459

For more details about this annex : see Official Journal of the European Patent Office, No. 12/82

EP 1 867 734 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10419059 B
- US 6147688 A
- US 6156521 A
- US 5947124 A
- US 5795725 A
- US 10225082 B
- US 5453359 A
- US 5837540 A
- US 5843690 A
- EP 0999447 A1
- US 436392 P
- US 33112702 A
- US 6143576 A
- US 6113855 A

- US 6019944 A
- US 5985579 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Kelley's Textbook of Internal Medicine. Lippincott Williams & Wilkins, 2000, 2349-2354
- Approach to the Patient With Dyspnea. **MULROW et al.** J. Gen. Int. Med. 1993, vol. 8, 383-92
- **MORGAN ; HODGE.** Am. Fam. Physician, 1998, vol. 57, 711-16
- **HARRIS.** Aust. Fam. Physician, 2002, vol. 31, 802-06
- **GOLDHABER.** Eur. Respir. J Suppl., 2002, vol. 35, 22s-27s
- **LUNDERGAN et al.** Am. Heart J., 2002, vol. 44, 456-62
- **HANLEY et al.** Radiology, 1982, vol. 143, 29-36
- **DOWDY ; WEARDEN.** Statistics for Research. John Wiley & Sons, 1983
- **BECK.** Tutorials in Differential Diagnosis. 2002
- **ZACKON.** Pulmonary Differential Diagnosis. Elsevier, 2000
- **JAMISON.** Differential Diagnosis for Primary Practice. 1999
- **BOUCHIER et al.** French's Index of Differential Diagnosis. Oxford University Press, 1997
- **TRINDADE ; ROULEAU.** Heart Fail. Monit., 2001, vol. 2, 2-7
- Fundamental Immunology. Raven Press, 1993
- **WILSON.** J. Immunol. Methods, 1994, vol. 175, 267-273

- **YARMUSH.** J. Biochem. Biophys. Methods, 1992, vol. 25, 85-97
- **WARD et al.** Nature, 1989, vol. 341, 544-546
- **BONOW, R.O.** Circulation, 1996, vol. 93, 1946-1950
- **TATEYAMA et al.** Biochem. Biophys. Res. Commun., 1992, vol. 185, 760-7
- **HUNT et al.** Biochem. Biophys. Res. Commun., 1995, vol. 214, 1175-83
- **WILKINS, M. et al.** Lancet, 1997, vol. 349, 1307-10
- **SAGNELLA, G.A.** Clinical Science, 1998, vol. 95, 519-29
- **H.M et al.** Circulation, 1995, vol. 91, 252.0-27
- **BAYES-GENIS, A. et al.** Thromb. Haemost., 1999, vol. 81, 865-68
- **GURFINKEL, E. et al.** Br. Heart J., 1994, vol. 71, 151-55
- **KRUSKAL, J.B. et al.** N. Engl. J. Med., 1987, vol. 317, 1361-65
- **TANAKA, M. ; SUZUKI, A.** Thromb. Res., 1994, vol. 76, 289-98
- **EGERMAYER et al.** Thorax, 1998, vol. 53, 830-34
- **BENAMER, H. et al.** Am. J. Cardiol., 1998, vol. 82, 845-50
- **BERTINCHANT, J.P. et al.** Clin. Biochem., 1996, vol. 29, 587-94
- **TANASIJEVIC, M.J. et al.** Clin. Cardiol., 1999, vol. 22, 13-16

- **MUSSO, P. et al.** *J. Ital. Cardiol.,* 1996, vol. 26, 1013-23
- **HOLVOET, P. et al.** *JAMA,* 1999, vol. 281, 1718-21
- **HOLVOET, P. et al.** *Circulation,* 1998, vol. 98, 1487-94
- **FORSSMANN et al.** *Histochem Cell Biol,* 1998, vol. 110, 335-357
- **ROSSI et al.** *Journal of the American College of Cardiology,* 2000, vol. 35, 1256-62
- **RUBATU et al.** *Circulation,* 1999, vol. 100, 1722-6
- **ESTRADA et al.** *Am. J. Hypertens.,* 1994, vol. 7, 1085-9
- **GAASCH.** *JAMA,* 1994, vol. 271, 1276-80
- **SOUFER et al.** *Am. J. Cardiol.,* 1984, vol. 55, 1032-6
- **SHAMSHAM ; MITCHELL.** *Am. Fam. Physician,* 2000, vol. 61, 1319-28
- Tietz Textbook of Clinical Chemistry. W.B. Saunders and Company, 496
- **LAURINO, J.P. et al.** *Ann. Clin. Lab. Sci.,* 1997, vol. 27, 338-345
- **LAURINO et al.** *Ann. Clin. Lab. Sci.,* 1997, vol. 27, 338-345
- **CARVILLE et al.** *Clin. Chem.,* 1996, vol. 42, 1537-1541
- **FIOTTA et al.** *Blood Coagul. Fibrinolysis,* 2002, vol. 13, 247-255
- **SONG et al.** *Haematologica,* 2002, vol. 87, 1062-1067
- **LA CAPRA et al.** *Blood Coagul. Fibrinolysis,* 2000, vol. 11, 371-377
- **YOSHIMURA, T. et al.** *FEBS Lett.,* 1989, vol. 244, 487-493
- **LI, Y.S. et al.** *Mol. Cell. Biochem.,* 1993, vol. 126, 61-68
- **SOEJIMA, H. et al.** *J. A. Coll. Cardiol.,* 1999, vol. 34, 983-988
- **NISHIYAMA, K. et al.** *Jpn. Circ. J,* 1998, vol. 62, 710-712
- **MATSUMORI, A. et al.** *J. Mol. Cell. Cardiol.,* 1997, vol. 29, 419-423
- **FISHER, N.C. et al.** *Gut,* 1999, vol. 45, 416-420
- **SUGA, M. et al.** *Eur. Respir. J.,* 1999, vol. 14, 376-382
- **BOSSINK, A.W. et al.** *Blood,* 1995, vol. 86, 3841-3847
- **KANEKO, H. et al.** *J. Rheumatol.,* 1999, vol. 26, 568-573
- **SOEJIMA, H. et al.** *J. Am. Coll. Cardiol.,* 1999, vol. 34, 983-988
- **GIAMBANCO, I. et al.** *J. Histochem. Cytochem.,* 1991, vol. 39, 1189-1198
- **DOUBELL, A.F. et al.** *Cardiovasc. Res.,* 1993, vol. 27, 1359-1367
- **KANEKO, N, et al.** *Clin. Chim. Acta,* 1996, vol. 251, 65-80
- **KANEKO, N. et al.** *Clin. Chim. Acta,* 1996, vol. 251, 65-80
- **NOMURA, M. et al.** *Br. Heart J.,* 1987, vol. 58, 29-33
- **HERRAEZ-DOMINGUEZ, M.V. et al.** *Clin. Chim. Acta,* 1975, vol. 64, 307-315
- **USUI, A. et al.** *Cardiovasc. Res.,* 1989, vol. 23, 737-740
- **KATO, K. et al.** *Clin. Chim. Acta,* 1983, vol. 131, 75-85
- **MATSUDA, H. et al.** *Forensic Sci. Int.,* 1999, vol. 99, 197-208
- **THYGESEN, K. et al.** *Eur. J. Clin. Invest.,* 1986, vol. 16, 1-4
- **KOUKKUNEN, H. et al.** *Ann. Med.,* 1998, vol. 30, 488-496
- **BERTINCHANT, J.P. et al.** *Clin. Biochem.,* 1996, vol. 29, 587-594
- **BENAMER, H. et al.** *Am. J Cardiol.,* 1998, vol. 82, 845-850
- **NORREGAARD-HANSEN, K. et al.** *Eur. Heart J.,* 1992, vol. 13, 188-193
- **MAIR, J. et al.** *Br. Heart J.,* 1994, vol. 72, 125-127
- **MAIR, J.** *Clin. Chim. Acta,* 1998, vol. 272, 79-86
- **RABITZSCH, G. et al.** *Clin. Chem.,* 1995, vol. 41, 966-978
- **RABITZSCH, G. et al.** *Lancet,* 1993, vol. 341, 1032-1033
- **RABITZSCH, G. et al.** *Biomed. Biochim. Acta,* 1987, vol. 46, S584-S588
- **MAIR, P. et al.** *Eur.J Clin. Chem. Clin. Biochem.,* 1994, vol. 32, 543-547
- **VEERKAMP, J.H. ; MAATMAN, R.G.** *Prog. Lipid Res.,* 1995, vol. 34, 17-52
- **YOSHIMOTO, K. et al.** *Heart Vessels,* 1995, vol. 10, 304-309
- **ISHII, J. et al.** *Clin. Chem.,* 1997, vol. 43, 1372-178
- **TSUJI, R. et al.** *Int. J. Cardiol.,* 1993, vol. 41, 209-217
- **VAN NIEUWENHOVEN, F.A. et al.** *Circulation,* 1995, vol. 92, 2848-2854
- **TANAKA, T. et al.** *Clin. Biochem.,* 1991, vol. 24, 195-201
- **DURANY, N. ; CARRERAS, J.** *Comp. Biochem. Physiol. B. Biochem. Mol. Biol.,* 1996, vol. 114, 217-223
- **BONFRER, J.M. et al.** *Br. J. Cancer,* 1998, vol. 77, 2210-2214
- **KATO, K. ; KIMURA, S.** *Biochim. Biophys. Acta,* 1985, vol. 842, 146-150
- **HASEGAWA, S. et al.** *Eur. Urol.,* 1993, vol. 24, 393-396

- **USUI, A. et al.** *Clin. Chem.,* 1990, vol. 36, 639-641
- **DAVIDSON et al.** *Circulation,* 1996, vol. 93, 1155-9
- **PRICKETT et al.** *Biochem. Biophys. Res. Commun.,* 2001, vol. 286, 513-7
- **WHICHER et al.** *Ann. Clin. Biochem.,* 2001, vol. 38, 483-93
- **YUKIOKA et al.** *Ann. Acad. Med. Singapore,* 2001, vol. 30, 528-31
- **PETTILA et al.** *Intensive Care Med.,* 2002, vol. 28, 1220-25
- **YOSHITOMI et al.** *Clin. Sci. (Colch,* 1998, vol. 94, 135-9
- **KUWASAKO et al.** *Ann. Clin. Biochem.,* 1999, vol. 36, 622-8
- **BROOKS ; ERGUL.** *J. Mol. Endocrinol.,* 1998, vol. 21, 307-15
- **YAP et al.** *Br. J. Pharmacol.,* 2000, vol. 129, 170-6
- **LEE et al.** *Blood,* 1999, vol. 94, 1440-50
- **DE CATERINA, R. et al.** *Eur. Heart J.,* 1988, vol. 9, 913-922
- **BAZZAN, M. et al.** *Cardiologia,* 1989, vol. 34, 217-220
- **SOBEL, M. et al.** *Circulation,* 1981, vol. 63, 300-306
- **GALLINO, A. et al.** *Am. Heart J.,* 1986, vol. 112, 285-290
- **SAKATA, K. et al.** *Jpn. Circ. J.,* 1996, vol. 60, 277-284
- **GENSINI, G.F. et al.** *Thromb. Res.,* 1988, vol. 50, 517-525
- **THEROUX, P. et al.** *Circulation,* 1987, vol. 75, 156-162
- **MERLINI, P.A. et al.** *Circulation,* 1994, vol. 90, 61-68
- **MANTEN, A. et al.** *Cardiovasc. Res.,* 1998, vol. 40, 389-395
- **MAHADEVAN, D. et al.** *J. Biol. Chem.,* 1995, vol. 270, 27595-27600
- **OGAWA, H. et al.** *Am. J. Cardiol.,* 1992, vol. 69, 453-456
- **WALLACE, J.M. et al.** *Ann. Clin. Biochem.,* 1998, vol. 35, 236-241
- **OGAWA, H. et al.** *Coron. Artery Dis.,* 1993, vol. 4, 437-442
- **BIASUCCI, L.M. et al.** *Circulation,* 1996, vol. 93, 2121-2127
- **GAMBLE, J.R. et al.** *Science,* 1990, vol. 249, 414-417
- **IKEDA, H. et al.** *Circulation,* 1995, vol. 92, 1693-1696
- **TOMODA, H. ; AOKI, N.** *Angiology,* 1998, vol. 49, 807-813
- **HOLLANDER, J.E. et al.** *J. Am. Coll. Cardiol.,* 1999, vol. 34, 95-105
- **KAIKITA, K. et al.** *Circulation,* 1995, vol. 92, 1726-1730
- **IKEDA, H. et al.** *Coron. Artery Dis.,* 1994, vol. 5, 515-518
- **BIASUCCI, L.M. et al.** *Am. J. Cardiol.,* 1996, vol. 77, 85-87
- **KIENAST, J. et al.** *Thromb. Haemost.,* 1993, vol. 70, 550-553
- **HOFFMEISTER, H.M. et al.** *Atherosclerosis,* 1999, vol. 144, 151-157
- **EMSLEY, J. et al.** *J. Biol. Chem.,* 1998, vol. 273, 10396-10401
- **FALCIANI, M. et al.** *Thromb. Haemost.,* 1998, vol. 79, 495-499
- **SUEFUJI, H. et al.** *Am. Heart J.,* 1997, vol. 134, 253-259
- **MISUMI, K. et al.** *Am. J. Cardiol.,* 1998, vol. 81, 22-26
- **SOEJIMA, H. et al.** *Circulation,* 1999, vol. 99, 2908-2913
- **KAIKITA, K. et al.** *Arterioscler. Thromb. Vasc. Biol.,* 1997, vol. 17, 2232-2237
- **ARDISSINO, D. et al.** *Lancet,* 1997, vol. 349, 769-771
- **KOSAR, F. et al.** *Angiology,* 1998, vol. 49, 193-201
- **AMARO, A. et al.** *Eur. Heart J.,* 1995, vol. 16, 615-622
- **DINERMAN, J.L. et al.** *J. Am. Coll. Cardiol.,* 1990, vol. 15, 1559-1563
- **MEHTA, J. et al.** *Circulation,* 1989, vol. 79, 549-556
- **DEPRE, C. et al.** *Cardiovasc. Res.,* 1999, vol. 41, 465-472
- **HAMMERMAN, S.I. et al.** *Am. J. Physiol.,* 1999, vol. 277, H1579-H1592
- **KAYE, D.M. et al.** *Life Sci,* 1998, vol. 62, 883-887
- **HOLVOET, P.** *Acta Cardiol.,* 1998, vol. 53, 253-260
- **HOLVOET, P. et al.** *Circulation,* 1998, vol. 98, 1487-1494
- **HOLVOET, P. et al.** *JAMA,* 1999, vol. 281, 1718-1721
- **JOHNSON, J.L. et al.** *Arterioscler. Thromb. Vasc. Biol.,* 1998, vol. 18, 1707-1715
- **SHIBATA, M. et al.** *Angiology,* 1999, vol. 50, 573-582
- **MURAWAKI, Y. et al.** *J. Hepatol.,* 1999, vol. 30, 1090-1098
- **KAI, H. et al.** *J. Am. Coll. Cardiol.,* 1998, vol. 32, 368-372
- **MANICOURT, D.H. et al.** *Arthritis Rheum.,* 1994, vol. 37, 1774-1783
- **TRIEBEL, S. et al.** *FEBS Lett.,* 1992, vol. 314, 386-388
- **BIASUCCI, L.M. et al.** *Circulation,* 1999, vol. 99, 2079-2084

- **LATINI, R. et al.** *J. Cardiovasc. Pharmacol.,* 1994, vol. 23, 1-6
- **BIASUCCI, L.M. et al.** *Circulation,* 1996, vol. 94, 874-877
- **LI, D. et al.** *Am. Heart J.,* 1999, vol. 137, 1145-1152
- **SQUADRITO, F. et al.** *Inflamm. Res.,* 1996, vol. 45, 14-19
- **LATINI, R. et al.** *J Cardiovasc. Pharmacol.,* 1994, vol. 23, 1-6
- **CARLSTEDT, F. et al.** *J. Intern. Med.,* 1997, vol. 242, 361-365
- **PELLEGATTA, F. et al.** *J Cardiovasc. Pharmacol.,* 1997, vol. 30, 455-460
- **MIWA, K. et al.** *Cardiovasc. Res.,* 1997, vol. 36, 37-44
- **GHAISAS, N.K. et al.** *Am. J. Cardiol.,* 1997, vol. 80, 617-619
- **OGAWA, H. et al.** *Am. J. Cardiol.,* 1999, vol. 83, 38-42
- **IIYAMA, K. et al.** *Circ. Res.,* 1999, vol. 85, 199-207
- **TENAGLIA, A.N. et al.** *Am. J. Cardiol.,* 1997, vol. 79, 742-747
- **MULVIHILL, N. et al.** *Am. J. Cardiol.,* 1999, vol. 83 (A9), 1265-7
- **PETER, K. et al.** *Arterioscler. Thromb. Vasc. Biol.,* 1997, vol. 17, 505-512
- **STAPRANS et al.** *Arterioscler. Thromb. Vasc. Biol.,* 2000, vol. 20, 708-14
- **WENTWORTH et al.** *Science,* 2003, vol. 302, 1053-6
- **TAIRA et al.** *J. Asthma,* 2002, vol. 39, 315-22
- **SCHWARTZ et al.** *N. Engl. J. Med.,* 1987, vol. 316, 1622-26
- **KOLLER et al.** *Arch. Dis. Childhood,* 1995, vol. 73, 413-7
- **SORKNESS et al.** *Clin. Exp. Allergy,* 2002, vol. 32, 1355-59
- **BADR-ELDIN et al.** *East Mediterr. Health J.,* 1999, vol. 5, 664-75
- **VIERA et al.** *Proc. Natl. Acad Sci. USA,* 1991, vol. 88, 1172-76
- **KIM et al.** *J. Immunol.,* 1992, vol. 148, 3618-23
- **GOGOS et al.** *J. Infect. Dis.,* 2000, vol. 181, 176-80
- **KOBAYASHI ; KITAMURA.** *Cbest,* 1995, vol. 108, 311-15
- **KOHNO.** *J. Med. Invest.,* 1999, vol. 46, 151-58
- **BANDOH et al.** *Ann. Rheum. Dis.,* 2000, vol. 59, 257-62
- **YAMANE et al.** *J. Rheumatol.,* 2000, vol. 27, 930-4
- **DOYLE et al.** *Am. J. Respir. Crit. Care Med.,* 1997, vol. 156, 1217-29
- **BERSTEN et al.** *Am. J. Respir. Crit. Care Med.,* 2001, vol. 164, 648-52
- **SUWABE.** *Ribnsho Byori,* 2002, vol. 50, 1061-66
- **CHENG et al.** *Crit. Care Med.,* 2003, vol. 31, 311-13
- **HASTINGS.** *J. Clin. Monit. Comput.,* 2000, vol. 16, 385-92
- **MORAES et al.** *Crit. Care Med.,* 2003, vol. 31 (4), 189-94
- **OWEN et al.** *Am. J. Respir. Cell Mol. Biol.,* 2003, vol. 29, 283-94
- **YONEDA, T. et al.** *Brain Res Mol Brain Res,* 1998, vol. 62, 187-195
- **MOWLA et al.** *J. Biol. Chem.,* 2001, vol. 276, 12660-6
- **BATES et al.** *Neurobiol. Dis.,* 2002, vol. 9, 24-37
- **HASEGAWA, S. et al.** *J. Urol.,* 1993, vol. 149, 1414-1418
- **USUI, A. et al.** *J Neurol. Sci.,* 1994, vol. 123, 134-139
- **JOHNSSON, P. J.** *Cardiothorac. Vasc. Anesth.,* 1996, vol. 10, 120-126
- **NIEBROJ-DOBOSZ, I. et al.** *Folia Neuropathol.,* 1994, vol. 32, 129-137
- **AGGARWAL, S.P. et al.** *J. Indian Med. Assoc.,* 1995, vol. 93, 331-332
- **MAIURI, F. et al.** *Neurol. Res.,* 1989, vol. 11, 6-8
- **PALFREYMAN, J.W. et al.** *Clin. Chim. Acta,* 1979, vol. 92, 403-409
- **TROTTER, J.L. et al.** *Ann. Neurol.,* 1983, vol. 14, 554-558
- **SEKI, Y. et al.** *Blood Coagul. Fibrinolysis,* 1997, vol. 8, 391-396
- **KRUPINSKI, J. et al.** *Acta Neurobiol. Exp. (Warz),* 1998, vol. 58, 13-21
- **SCHWAB, S. et al.** *Stroke,* 1997, vol. 28, 1744-1748
- **BITSCH, A. et al.** *Stroke,* 1998, vol. 29, 2129-2135
- **KIM, J.S.** *J. Neurol. Sci.,* 1996, vol. 137, 69-78
- **SHYU, K.G. et al.** *J. Neurol.,* 1997, vol. 244, 90-93
- **POLIN, R.S. et al.** *J. Neurosurg.,* 1998, vol. 89, 559-567
- **HWANG, S.J. et al.** *Circulation,* 1997, vol. 96, 4219-4225
- **BANKS, R.E. et al.** *Br. J. Cancer,* 1993, vol. 68, 122-124
- **AUSTGULEN, R. et al.** *Eur. J. Obstet. Gynecol. Reprod. Biol.,* 1997, vol. 71, 53-58
- **STEINER, M. et al.** *Thromb. Haemost.,* 1994, vol. 72, 979-984
- **DE ROSE, V. et al.** *Am. J. Respir. Crit. Care Med.,* 1998, vol. 157, 1234-1239

- **FERNANDES-ALNEMRI, T. et al.** *J. Biol. Chem.,* 1994, vol. 269, 30761-30764
- **LIU, X. et al.** *J. Biol. Chem.,* 1996, vol. 271, 13371-13376
- **SARASTE, A.** *Herz,* 1999, vol. 24, 189-195
- **OHTSUKA, T. et al.** *Coron. Artery Dis.,* 1999, vol. 10, 221-225
- **JAMES, T.N.** *Coron. Artery Dis.,* 1998, vol. 9, 291-307
- **BIALIK, S. et al.** *J. Clin. Invest.,* 1997, vol. 100, 1363-1372
- **LONG, X. et al.** *J. Clin. Invest.,* 1997, vol. 99, 2635-2643
- **CARRARO, U. ; FRANCESCHI, C.** *Aging (Milano),* 1997, vol. 9, 19-34
- **MACMANUS, J.P. et al.** *J. Cereb. Blood Flow Metab.,* 1999, vol. 19, 502-510
- **HU et al.** *J. Cereb. Blood Flow Metab.,* 2001, vol. 21, 865-75
- **FISCHER et al.** *Intensive Care Med.,* 2003, vol. 29, 1043-51
- **ZHOU et al.** Statistical Methods in Diagnostic Medicine. John Wiley & Sons, 2002
- **MOTULSKY.** Intuitive Biostatistics. Oxford University Press, 1995
- **NG ; HAG.** *J. Cell Mol. Med.,* 2002, vol. 6, 329-340
- Guide to Protein Purification. *Meth. Enzymol.,* 1990, vol. 182
- Solid Phase Peptide Synthesis. *Meth. Enzymol.,* 1997, vol. 289
- **KISO et al.** *Chem. Pharm. Bull. (Tokyo,* 1990, vol. 38, 1192-99
- **MOSTAFAVI et al.** *Biomed. Pept. Proteins Nucleic Acids,* 1995, vol. 1, 255-60
- **FUJIWARA et al.** *Chem. Pharm. Bull. (Tokyo,* 1996, vol. 44, 1326-31
- Antibodies, A Laboratory Manual, Ed Harlow and David Lane. Cold Spring Harbor. Cold Spring Harbor Laboratory, 1988
- Antibody Engineering: A Practical Approach. Oxford University Press, 1995
- *J. Immunol.,* 1992, vol. 149, 3914-3920
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- Merck Manual of Diagnosis and Therapy. Merck Research Laboratories. Whitehouse Station, 1999
- Nomenclature and Criteria for Diagnosis of Diseases of the Heart and Great Vessels. Mass: Little, Brown & Co, 1994, 253-256
- **HAMMER-LERCHER et al.** *Clin. Chim. Acta,* 2001, vol. 310 (2), 193-7
- **LEMOS et al.** *Circulation,* 2003, vol. 107, 690-95
- **KHALEELI et al.** *Am. Heart J.,* 2001, vol. 141, 637-44
- **SVIRI et al.** *Stroke,* 2000, vol. 31, 118-122